# EUROPEAN PATENT APPLICATION

(11) **EP 4 541 188 A1**
(43) Date of publication of application: **23.04.2025**
(21) Application number: 23823967.7
(22) Date of filing: 15.06.2023
(51) Int. Cl.: A01N 43/40, A01G 7/06, A01M 1/20, A01N 25/00, A01N 43/42, A01N 43/54, A01N 43/58, A01N 43/60, A01N 43/78, A01P 3/00, C07D 213/70, C07D 213/71, C07D 237/18, C07D 239/38, C07D 401/12

(54) **PLANT DISEASE CONTROL METHOD**

(30) Priority: 17.06.2022 JP 2022098034; 11.11.2022 JP 2022180831
(71) Applicant: Sumitomo Chemical Company, Limited, Tokyo 103-6020 (JP)
(72) Inventor: ARAI, Keisuke, Takarazuka-shi, Hyogo 665-8555 (JP); NOKURA, Yoshihiko, Takarazuka-shi, Hyogo 665-8555 (JP); MAEHATA, Nao, Takarazuka-shi, Hyogo 665-8555 (JP)
(74) Representative: Vossius & Partner Patentanwälte Rechtsanwälte mbB
(86) International application number: PCT/JP2023/022202
(87) International publication number: WO 2023/243677

(57) **Abstract**

The present invention provides a method for controlling a plant disease which comprises applying a compound represented by formula (I) [wherein n is 0, 1, or 2, R¹ represents a C1-C6 alkyl group etc., R² and R³ are identical to or different from each other, and each represents a C1-C6 chain hydrocarbon group which may be optionally substituted with one or more halogen atoms, etc., R⁴ represents a C1-C6 chain hydrocarbon group, etc., q is 1, 2, or 3, and when q is 2 or 3, 2 or 3 of R⁴ are identical to or different from each other, a combination of X¹, X², X³, and X⁴ represents a combination wherein X¹ represents CR⁵, X² represents CR⁶, X³ represents CR⁷, and X⁴ represents a nitrogen atom, etc., and R⁵, R⁶, and R⁷ are identical to or different from each other, and each represents a C1-C6 chain hydrocarbon group which may be optionally substituted with one or more halogen atoms, etc.], or the N-oxide thereof, or the salt thereof to a plant or soil for cultivating the plant, which has excellent control efficacies against plant diseases.

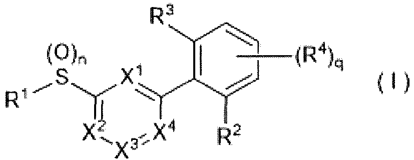

## Description

### TECHNICAL FIELD

This application claims priorities to and the benefits of Japanese Patent Application No. 2022-098034 filed on June 17, 2022 and Japanese Patent Application No. 2022-180831 filed on November 11, 2022, the entire contents of which are incorporated herein by reference.

The present invention relates to a method for controlling plant diseases.

### BACKGROUND ART

Patent documents 1 describes biaryl compounds as an agent for controlling plant diseases.

### CITATION LIST

### PATENT DOCUMENT

Patent Document 1: CN 101875639 A

### SUMMARY OF THE INVENTION

### PROBLEMS TO BE SOLVED BY INVENTION

An object of the present invention is to provide a method having an excellent efficacy for controlling plant diseases.

### MEANS TO SOLVE PROBLEMS

The present inventors have studied so as to find a method having an excellent efficacy for controlling plant diseases, and have found out that the compound represented by the following formula (I) has an excellent efficacy on controlling plant diseases.

That is, the present invention is as follows.

[1] A method for controlling a plant disease which comprises applying a compound represented by formula (I): 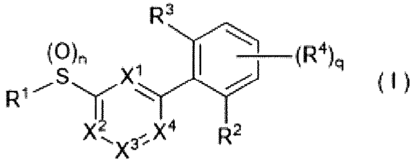 [wherein
   n is 0, 1, or 2,
   R¹ represents a C1-C6 alkyl group, a C2-C6 alkynyl group {the C1-C6 alkyl group, and the C2-C6 alkynyl group may optionally have one or more substituents selected from Group A}, a C2-C6 alkenyl group which may optionally have one or more substituents selected from Group C, a C3-C8 alicyclic hydrocarbon group, a three (3)- to eight (8)- membered non-aromatic heterocyclic group {the C3-C8 alicyclic hydrocarbon group, and the three (3)- to eight (8)- membered non-aromatic heterocyclic group may optionally have one or more substituents selected from Group B}, a C6-C10 aryl group which may optionally have one or more substituents selected from Group F, a five (5)- to ten (10)- membered aromatic heterocyclic group which may optionally have one or more substituents selected from Group D, or NR³³R³⁴,
   R² and R³ are identical to or different from each other, and each represents a C1-C6 chain hydrocarbon group which may be optionally substituted with one or more halogen atoms, a C3-C8 alicyclic hydrocarbon group which may optionally have one or more substituents selected from Group B, OR⁹, SR¹⁰, S(O)R¹¹, S(O)₂R¹², NR¹³R¹⁴, C(O)R¹⁵, a nitro group, a cyano group, a halogen atom, or a hydrogen atom,
   R⁴ represents a C1-C6 chain hydrocarbon group which may optionally have one or more substituents selected from Group A, a C3-C8 alicyclic hydrocarbon group, a three (3) - to eight (8) - membered non-aromatic heterocyclic group {the C3-C8 alicyclic hydrocarbon group, and the three (3)- to eight (8) - membered non-aromatic heterocyclic group may optionally have one or more substituents selected from Group B}, a phenyl group, a five (5) - to six (6) - membered aromatic heterocyclic group {the phenyl group, and the five (5)- to six (6)- membered aromatic heterocyclic group may optionally have one or more substituents selected from Group D}, OR¹⁶, SR¹⁷, S(O)R¹⁸, S(O)₂R¹⁹, NR²⁰R²¹, C(O)R²², C(R²³)=NOR²⁴, a cyano group, a nitro group, an amino group, a hydroxy group, or a halogen atom,
   q is 1, 2, or 3,
   when q is 2 or 3, 2 or 3 of R⁴ may be identical to or different from each other,
   a combination of X¹, X², X³, and X⁴ represents,
      a combination wherein X¹ represents CR⁵, X² represents CR⁶, X³ represents CR⁷, and X⁴ represents a nitrogen atom;
      a combination wherein X¹ represents CR⁵, X² represents CR⁶, X³ represents a nitrogen atom, and X⁴ represents CR⁸;
      a combination wherein X¹ represents CR⁵, X² represents a nitrogen atom, X³ represents CR^{7A}, and X⁴ represents CR⁸;
      a combination wherein X¹ represents a nitrogen atom, X² represents CR⁶, X³ represents CR^{7B}, and X⁴ represents CR⁸;
      a combination wherein X¹ represents a nitrogen atom, X² represents a nitrogen atom, X³ represents CR^{7C}, and X⁴ represents CH;
      a combination wherein X¹ represents a nitrogen atom, X² represents a nitrogen atom, X³ represents CH, and X⁴ represents CR⁸;
      a combination wherein X¹ represents a nitrogen atom, X² represents CR⁶, X³ represents a nitrogen atom, and X⁴ represents CR⁸;
      a combination wherein X¹ represents a nitrogen atom, X² represents CR⁶, X³ represents CR⁷, and X⁴ represents a nitrogen atom;
      a combination wherein X¹ represents CR⁵, X² represents a nitrogen atom, X³ represents a nitrogen atom, and X⁴ represents CR⁸;
      a combination wherein X¹ represents CR⁵, X² represents a nitrogen atom, X³ represents CH, and X⁴ represents a nitrogen atom;
      a combination wherein X¹ represents CH, X² represents a nitrogen atom, X³ represents CR^{7D}, and X⁴ represents a nitrogen atom; or
      a combination wherein X¹ represents CR⁵, X² represents CR⁶, X³ represents a nitrogen atom, and X⁴ represents a nitrogen atom;
   R⁵, R⁶, R⁷ and R⁸ are identical to or different from each other, and each represents a C1-C6 chain hydrocarbon group which may be optionally substituted with one or more halogen atoms, a C3-C8 alicyclic hydrocarbon group which may optionally have one or more substituents selected from Group B, OR²⁵, SR²⁶, S(O)R²⁷, S(O)₂R²⁸, NR²⁹R³⁰, C(O)R³¹, a nitro group, a cyano group, a halogen atom, or a hydrogen atom,
   R⁹, R¹⁰, R¹³, and R²⁹ are identical to or different from each other, and each represents a C1-C6 chain hydrocarbon group which may be optionally substituted with one or more halogen atoms, or a hydrogen atom,
   R^{7A} represents a C1-C6 chain hydrocarbon group which may be optionally substituted with one or more halogen atoms, a C3-C8 alicyclic hydrocarbon group which may optionally have one or more substituents selected from Group B, OR²⁵, SR²⁶, S(O)R²⁷, S(O)2R²⁸, NR²⁹R³², C(O)R³¹, a nitro group, a cyano group, a halogen atom, or a hydrogen atom,
   R^{7B} represents a C1-C6 chain hydrocarbon group which may be optionally substituted with one or more halogen atoms, a C3-C8 alicyclic hydrocarbon group which may optionally have one or more substituents selected from Group B, OR²⁵, NR²⁹R³⁰, C(O)R³¹, a nitro group, a cyano group, a halogen atom, or a hydrogen atom,
   R^{7C} represents a C1-C6 chain hydrocarbon group which may be optionally substituted with one or more halogen atoms, a C3-C8 alicyclic hydrocarbon group which may optionally have one or more substituents selected from Group B, OR²⁵, NR²⁹R³⁰, C(O)R³¹, a nitro group, a cyano group, or a halogen atom,
   R^{7D} represents a C1-C6 chain hydrocarbon group which may be optionally substituted with one or more halogen atoms, a C3-C8 alicyclic hydrocarbon group which may optionally have one or more substituents selected from Group B, OR²⁵, S(O)R²⁷, S(O)₂R²⁸, NR²⁹R³⁰, C(O)R³¹, a nitro group, a cyano group, a fluorine atom, a chlorine atom, or an iodine atom,
   R¹¹, R¹², R²⁵, R²⁶, R²⁷, and R²⁸ are identical to or different from each other, and each represents a C1-C6 chain hydrocarbon group which may be optionally substituted with one or more halogen atoms,
   R¹⁴ and R³⁰ are identical to or different from each other, and each represents a C1-C6 chain hydrocarbon group which may be optionally substituted with one or more halogen atoms, a (C1-C6 alkyl)carbonyl group, a (C1-C6 alkoxy)carbonyl group {the (C1-C6 alkyl)carbonyl group, and the (C1-C6 alkoxy)carbonyl group may be optionally substituted with one or more halogen atoms}, or a hydrogen atom,
   R¹⁵, R²² and R³¹ are identical to or different from each other, and each represents a C1-C6 chain hydrocarbon group, a C1-C6 alkoxy group, a C1-C6 alkylamino group, or a C2-C8 dialkylamino group {the C1-C6 chain hydrocarbon group, the C1-C6 alkoxy group, the C1-C6 alkylamino group, and the C2-C8 dialkylamino group may be optionally substituted with one or more halogen atoms},
   R¹⁶ represents a C1-C6 chain hydrocarbon group which may optionally have one or more substituents selected from Group A, a C1-C6 alkylsulfonyl group, a (C1-C6 alkyl) carbonyl group, a (C1-C6 alkoxy)carbonyl group, a (C1-C6 alkylamino)carbonyl group, a (C2-C8 dialkylamino)carbonyl group {the C1-C6 alkylsulfonyl group, the (C1-C6 alkyl)carbonyl group, the (C1-C6 alkoxy)carbonyl group, the (C1-C6 alkylamino) carbonyl group, and the (C2-C8 dialkylamino)carbonyl group may be optionally substituted with one or more halogen atoms}, a C3-C8 alicyclic hydrocarbon group, a three (3)- to eight (8)- membered non-aromatic heterocyclic group {the C3-C8 alicyclic hydrocarbon group, and the three (3)- to eight (8)- membered non-aromatic heterocyclic group may optionally have one or more substituents selected from Group B}, a phenyl group, a five (5)- to six (6)- membered aromatic heterocyclic group {the phenyl group, and the five (5)- to six (6)- membered aromatic heterocyclic group may optionally have one or more substituents selected from Group D}, or a hydrogen atom,
   R¹⁷, R¹⁸, R¹⁹, and R²⁴ are identical to or different from each other, and each represents a C1-C6 chain hydrocarbon group which may optionally have one or more substituents selected from Group A, a C3-C8 alicyclic hydrocarbon group, a three (3)- to eight (8)- membered non-aromatic heterocyclic group {the C3-C8 alicyclic hydrocarbon group, and the three (3)- to eight (8)- membered non-aromatic heterocyclic group may optionally have one or more substituents selected from Group B}, a phenyl group, or a five (5)- to six (6)- membered aromatic heterocyclic group {the phenyl group, and the five (5)- to six (6)- membered aromatic heterocyclic group may optionally have one or more substituents selected from Group D},
   R²⁰ represents a C1-C6 chain hydrocarbon group which may optionally have one or more substituents selected from Group A, a C3-C8 alicyclic hydrocarbon group, a three (3)- to eight (8) - membered non-aromatic heterocyclic group {the C3-C8 alicyclic hydrocarbon group, and the three (3)- to eight (8)- membered non-aromatic heterocyclic group may optionally have one or more substituents selected from Group B}, a phenyl group, a five (5)- to six (6)- membered aromatic heterocyclic group {the phenyl group, and the five (5)- to six (6)- membered aromatic heterocyclic group may optionally have one or more substituents selected from Group D}, a (C1-C6 alkyl)carbonyl group, a (C1-C6 alkoxy)carbonyl group, or a C1-C6 alkylsulfonyl group {the (C1-C6 alkyl)carbonyl group, the (C1-C6 alkoxy)carbonyl group, and the C1-C6 alkylsulfonyl group may be optionally substituted with one or more halogen atoms},
   R²¹ and R²³ are identical to or different from each other, and each represents a C1-C6 chain hydrocarbon group which may optionally have one or more substituents selected from Group A, or a hydrogen atom,
   R³² represents a C1-C6 chain hydrocarbon group which may be optionally substituted with one or more halogen atoms, a (C1-C6 alkyl)carbonyl group, or a (C1-C6 alkoxy)carbonyl group {the (C1-C6 alkyl)carbonyl group, and the (C1-C6 alkoxy) carbonyl group may be optionally substituted with one or more halogen atoms},
   R³³ and R³⁴ are identical to or different from each other, and each represents a C1-C6 chain hydrocarbon group which may optionally have one or more substituents selected from Group G, a C3-C6 alicyclic hydrocarbon group which may be optionally substituted with one or more halogen atoms, or a hydrogen atom,
   the neighboring R² and R⁴ may combine together with a carbon atom to which they are attached to form a C5-C6 alicyclic hydrocarbon, a five (5)- to six (6)- membered non-aromatic heterocyclic ring {the C5-C6 alicyclic hydrocarbon, and the five (5)- to six (6)- membered non-aromatic heterocyclic ring may optionally have one or more substituents selected from Group B}, a benzene ring, or a five (5)- to six (6)- membered aromatic heterocyclic ring {the benzene ring, and the five (5)- to six (6)- membered aromatic heterocyclic ring may optionally have one or more substituents selected from Group E},
   two of neighboring R⁴ and R⁴ may combine together with a carbon atom to which they are attached to form a C5-C6 alicyclic hydrocarbon, a five (5)- to six (6)- membered non-aromatic heterocyclic ring {the C5-C6 alicyclic hydrocarbon, and the five (5)- to six (6)- membered non-aromatic heterocyclic ring may optionally have one or more substituents selected from Group B}, a benzene ring, or a five (5) - to six (6) - membered aromatic heterocyclic ring {the benzene ring, and the five (5)- to six (6)- membered aromatic heterocyclic ring may optionally have one or more substituents selected from Group E}.

   Group A is a group consisting of a C1-C6 alkoxy group, a C1-C6 alkylsulfanyl group, a C1-C6 alkylsulfinyl group, a C1-C6 alkylsulfonyl group {the C1-C6 alkoxy group, the C1-C6 alkylsulfanyl group, the C1-C6 alkylsulfinyl group, and the C1-C6 alkylsulfonyl group may be optionally substituted with one or more halogen atoms}, a C3-C8 alicyclic hydrocarbon group, a three (3)- to eight (8)- membered non-aromatic heterocyclic group {the C3-C8 alicyclic hydrocarbon group, and the three (3)- to eight (8)- membered non-aromatic heterocyclic group may optionally have one or more substituents selected from Group B}, a phenyl group, a five (5)- to six (6)- membered aromatic heterocyclic group {the phenyl group and the five (5)- to six (6)- membered aromatic heterocyclic group may optionally have one or more substituents selected from Group D}, a halogen atom, a cyano group, a nitro group, a hydroxy group, and a sulfanyl group.
   Group B is a group consisting of an oxo group, a thioxo group, a C1-C6 chain hydrocarbon group, a C1-C6 alkoxy group, a C1-C6 alkylsulfanyl group, a C1-C6 alkylsulfinyl group, a C1-C6 alkylsulfonyl group {the C1-C6 chain hydrocarbon group, the C1-C6 alkoxy group, the C1-C6 alkylsulfanyl group, the C1-C6 alkylsulfinyl group, and the C1-C6 alkylsulfonyl group may be optionally substituted with one or more halogen atoms}, a hydroxy group, a sulfanyl group, a halogen atom, and a cyano group.
   Group C is a group consisting of a C1-C6 alkoxy group, a C1-C6 alkylsulfanyl group, a C1-C6 alkylsulfinyl group, a C1-C6 alkylsulfonyl group {the C1-C6 alkoxy group, the C1-C6 alkylsulfanyl group, the C1-C6 alkylsulfinyl group, and the C1-C6 alkylsulfonyl group may be optionally substituted with one or more halogen atoms}, a C3-C8 alicyclic hydrocarbon group, a three (3)- to eight (8)- membered non-aromatic heterocyclic group {the C3-C8 alicyclic hydrocarbon group, and the three (3) - to eight (8)- membered non-aromatic heterocyclic group may optionally have one or more substituents selected from Group B}, a phenyl group, a five (5)- to six (6)- membered aromatic heterocyclic group {the phenyl group and the five (5)- to six (6)- membered aromatic heterocyclic group may optionally have one or more substituents selected from Group D}, a chlorine atom, a bromine atom, an iodine atom, a cyano group, a nitro group, a hydroxy group, and a sulfanyl group.
   Group D is a group consisting of a C1-C6 chain hydrocarbon group, a C1-C6 alkoxy group, a C1-C6 alkylsulfanyl group, a C1-C6 alkylsulfinyl group, a C1-C6 alkylsulfonyl group, a C1-C6 alkylamino group, a C2-C8 dialkylamino group, a (C1-C6 alkyl)carbonyl group, a (C1-C6 alkoxy)carbonyl group, a (C1-C6 alkylamino)carbonyl group, a (C2-C8 dialkylamino)carbonyl group, a (C1-C6 alkyl)carbonylamino group, a (C1-C6 alkoxy)carbonylamino group {the C1-C6 chain hydrocarbon group, the C1-C6 alkoxy group, the C1-C6 alkylsulfanyl group, the C1-C6 alkylsulfinyl group, the C1-C6 alkylsulfonyl group, the C1-C6 alkylamino group, the C2-C8 dialkylamino group, the (C1-C6 alkyl)carbonyl group, the (C1-C6 alkoxy)carbonyl group, the (C1-C6 alkylamino)carbonyl group, the (C2-C8 dialkylamino)carbonyl group, the (C1-C6 alkyl)carbonylamino group, and the (C1-C6 alkoxy) carbonylamino group may be optionally substituted with one or more halogen atoms}, a C3-C8 alicyclic hydrocarbon group which may optionally have one or more substituents selected from Group B, an amino group, a nitro group, a cyano group, a hydroxy group, a sulfanyl group, and a halogen atom.
   Group E is a group consisting of a C1-C6 chain hydrocarbon group, a C1-C6 alkoxy group, a C1-C6 alkylsulfanyl group, a C1-C6 alkylsulfinyl group, a C1-C6 alkylsulfonyl group {the C1-C6 chain hydrocarbon group, the C1-C6 alkoxy group, the C1-C6 alkylsulfanyl group, the C1-C6 alkylsulfinyl group, and the C1-C6 alkylsulfonyl group may be optionally substituted with one or more halogen atoms}, a hydroxy group, a sulfanyl group, a halogen atom, and a cyano group.
   Group F is a group consisting of a C1-C6 chain hydrocarbon group, a C1-C6 alkoxy group, a C1-C6 alkylsulfanyl group, a C1-C6 alkylsulfinyl group, a C1-C6 alkylsulfonyl group, a C1-C6 alkylamino group, a C2-C8 dialkylamino group, a (C1-C6 alkyl)carbonyl group, a (C1-C6 alkoxy)carbonyl group, a (C1-C6 alkylamino)carbonyl group, a (C2-C8 dialkylamino)carbonyl group, a (C1-C6 alkyl)carbonylamino group, a (C1-C6 alkoxy)carbonylamino group {the C1-C6 chain hydrocarbon group, the C1-C6 alkoxy group, the C1-C6 alkylsulfanyl group, the C1-C6 alkylsulfinyl group, the C1-C6 alkylsulfonyl group, the C1-C6 alkylamino group, the C2-C8 dialkylamino group, the (C1-C6 alkyl)carbonyl group, the (C1-C6 alkoxy)carbonyl group, the (C1-C6 alkylamino)carbonyl group, the (C2-C8 dialkylamino)carbonyl group, the (C1-C6 alkyl)carbonylamino group, and the (C1-C6 alkoxy)carbonylamino group may be optionally substituted with one or more halogen atoms}, a C3-C8 alicyclic hydrocarbon group which may optionally have one or more substituents selected from Group B, an amino group, a cyano group, a hydroxy group, a sulfanyl group, and a halogen atom.
   Group G is a group consisting of a C3-C6 cycloalkyl group, a C1-C3 alkoxy group, a C1-C3 alkylthio group {the C3-C6 cycloalkyl group, the C1-C3 alkoxy group, and the C1-C3 alkylthio group may be optionally substituted with one or more halogen atoms}, a halogen atom, and a cyano group] (hereinafter, referred to as "Present compound X"), or a N-oxide thereof, or a salt thereof (hereinafter, the present compound X, or the N-oxide thereof, or the salt thereof is collectively referred to as "Present compound Y") to a plant or soil for cultivating the plant.
[2] The method according to [1] wherein the compound represented by formula (I), or the N-oxide thereof, or the salt thereof represents the compound wherein R¹ represents a C1-C6 alkyl group, a C2-C6 alkynyl group {the C1-C6 alkyl group, and the C2-C6 alkynyl group may optionally have one or more substituents selected from Group A}, a C2-C6 alkenyl group which may optionally have one or more substituents selected from Group C, or a C3-C8 alicyclic hydrocarbon group {the C3-C8 alicyclic hydrocarbon group may optionally have one or more substituents selected from Group B}
   (hereinafter, referred to as "Present compound N"), or the N-oxide thereof, or the salt thereof
   (hereinafter, the present compound N, or the N-oxide thereof, or the salt thereof is collectively referred to as "Present compound").
[3] The method according to [2] wherein the compound represented by formula (I), or the N-oxide thereof, or the salt thereof represents the compound wherein the combination of X¹, X², X³, and X⁴ represents a combination wherein X¹ represents CR⁵, X² represents CR⁶, X³ represents CR⁷, and X⁴ represents a nitrogen atom; a combination wherein X¹ represents CR⁵, X² represents CR⁶, X³ represents a nitrogen atom, and X⁴ represents CR⁸; a combination wherein X¹ represents CR⁵, X² represents a nitrogen atom, X³ represents CR^{7A}, and X⁴ represents CR⁸; a combination wherein X¹ represents a nitrogen atom, X² represents CR⁶, X³ represents CR^{7B}, and X⁴ represents CR⁸; a combination wherein X¹ represents a nitrogen atom, X² represents CR⁶, X³ represents a nitrogen atom, and X⁴ represents CR⁸; a combination wherein X¹ represents CR⁵, X² represents a nitrogen atom, X³ represents a nitrogen atom, and X⁴ represents CR⁸; or a combination wherein X¹ represents CR⁵, X² represents CR⁶, X³ represents a nitrogen atom, and X⁴ represents a nitrogen atom, or the N-oxide thereof, or the salt thereof.
[4] The method according to [2] or [3] wherein the compound represented by formula (I), or the N-oxide thereof, or the salt thereof represents a compound wherein R¹ represents a C2-C6 alkyl group, a C2-C6 alkynyl group {the C2-C6 alkyl group, and the C2-C6 alkynyl group may optionally have one or more substituents selected from Group A}, a methyl group which has one or more substituents selected from Group C, or a C3-C8 alicyclic hydrocarbon group {the C3-C8 alicyclic hydrocarbon group may optionally have one or more substituents selected from Group B}
   or the N-oxide thereof, or the salt thereof.
[5] A compound represented by formula (II): 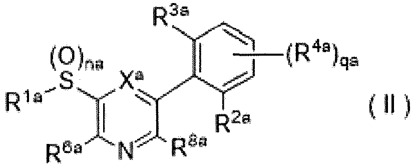 [wherein
   na is 0, 1, or 2,
   qa is 1, 2, or 3,
   when qa is 2 or 3, 2 or 3 of R^{4a} may be identical to or different from each other,
   R¹⁵ represents a C2-C6 alkyl group, a C2-C6 alkynyl group {the C2-C6 alkyl group, and the C2-C6 alkynyl group may optionally have one or more substituents selected from Group A^{a}}, a C2-C6 alkenyl group which may optionally have one or more substituents selected from Group F^{a}, a C3-C8 alicyclic hydrocarbon group which may optionally have one or more substituents selected from Group B^{a}, a methyl group which has one or more substituents selected from Group C^{a}, or a five (5)- to six (6)- membered aromatic heterocyclic group which may optionally have one or more substituents selected from Group G^{a},
   R^{8a} represents a C1-C6 chain hydrocarbon group which may be optionally substituted with one or more halogen atoms, a C3-C8 alicyclic hydrocarbon group which may optionally have one or more substituents selected from Group B^{a}, OR^{7a}, SR^{9a}, S(O)R^{10a}, S(O)₂R^{11a}, NR^{12a}R^{13a}, C(O)R^{14a}, a nitro group, a cyano group, a halogen atom, or a hydrogen atom,
   R^{2a} and R^{3a} are identical to or different from each other, and each represents a C1-C6 chain hydrocarbon group which may be optionally substituted with one or more halogen atoms, a C3-C8 alicyclic hydrocarbon group which may optionally have one or more substituents selected from Group B^{a}, OR^{15a}, SR^{16a}, S(O)R^{17a}, S(O)₂^{R18a}, NR^{19a}R^{20a}, C(O)R^{21a}, a nitro group, a cyano group, a halogen atom, or a hydrogen atom,
   a combination of X², R^{4a}, and R^{6a} represents
   a combination wherein
      X^{a} represents nitrogen atom,
      R^{4a} represents a C1-C6 chain hydrocarbon group which may optionally have one or more substituents selected from Group A^{a}, a C3-C8 alicyclic hydrocarbon group, a three (3)- to eight (8)- membered non-aromatic heterocyclic group {the C3-C8 alicyclic hydrocarbon group, and the three (3)-to eight (8)- membered non-aromatic heterocyclic group may optionally have one or more substituents selected from Group B^{a}}, a phenyl group, a five (5)- to six (6)- membered aromatic heterocyclic group {the phenyl group, and the five (5)- to six (6)- membered aromatic heterocyclic group may optionally have one or more substituents selected from Group D^{a}}, OR^{23a}, SR^{24a}, S(O)R^{25a}, S(O)₂R^{26a}, NR^{27a}R^{28a}, C(O)R^{29a}, C(R^{30a})=NOR^{31a}, a cyano group, a nitro group, an amino group, a hydroxy group, or a halogen atom,
      R^{6a} represents a C1-C6 chain hydrocarbon group which may be optionally substituted with one or more halogen atoms, a C3-C8 alicyclic hydrocarbon group which may optionally have one or more substituents selected from Group B^{a}, OR^{7a}, SR^{9a}, S(O)R^{10a}, S(O)₂R^{11a}, NR^{12a}R^{13a}, C(O)R^{14a}, a nitro group, a cyano group, a halogen atom, or a hydrogen atom; or a combination wherein
      X^{a} represents CR^{5a},
      R^{4a} represents a methyl group which may optionally have one or more substituents selected from Group F^{a}, a CH₂F group, a CHF₂ group, a C2-C6 chain hydrocarbon group which may optionally have one or more substituents selected from Group A^{a}, a C3-C8 alicyclic hydrocarbon group, a three (3)-to eight (8)- membered non-aromatic heterocyclic group {the C3-C8 alicyclic hydrocarbon group, and the three (3)- to eight (8)- membered non-aromatic heterocyclic group may optionally have one or more substituents selected from Group B^{a}}, a phenyl group, a five (5)- to six (6)- membered aromatic heterocyclic group {the phenyl group, and the five (5)- to six (6)- membered aromatic heterocyclic group may optionally have one or more substituents selected from Group D^{a}}, OR^{23a}, SR^{24a}, S(O)R^{25a}, S(O)₂R^{22a}, NR^{27a}R^{28a}, C(O)R^{29a}, C(R^{30a})=NOR^{31a}, a cyano group, a nitro group, an amino group, a hydroxy group, or a halogen atom,
      R^{6a} represents a C1-C6 chain hydrocarbon group which may be optionally substituted with one or more halogen atoms, a C3-C8 alicyclic hydrocarbon group which may optionally have one or more substituents selected from Group B^{a}, OR^{7a}, a nitro group, a fluorine atom, a bromine atom, an iodine atom, or a hydrogen atom,
   R^{5a} represents a C1-C6 chain hydrocarbon group which may be optionally substituted with one or more halogen atoms, a C3-C8 alicyclic hydrocarbon group which may optionally have one or more substituents selected from Group B^{a}, OR^{7a}, SR^{9a}, S(O)R^{10a}, S(O)₂^{11a}, NR^{12a}R^{13a}, C(O)R^{14a}, a nitro group, a cyano group, a halogen atom, or a hydrogen atom,
   R^{7a}, R^{9a} R^{10a}, R^{11a}, R^{15a}, R^{16a}, R^{17a} and R^{18a} are identical to or different from each other, and each represents a C1-C6 chain hydrocarbon group which may be optionally substituted with one or more halogen atoms,
   R^{12a} and R^{19a} are identical to or different from each other, and each represents a C1-C6 chain hydrocarbon group which may be optionally substituted with one or more halogen atoms, or a hydrogen atom,
   R^{13a} and R^{20a} are identical to or different from each other, and each represents a C1-C6 chain hydrocarbon group which may be optionally substituted with one or more halogen atoms, a (C1-C6 alkyl) carbonyl group, a (C1-C6 alkoxy)carbonyl group {the (C1-C6 alkyl)carbonyl group, and the (C1-C6 alkoxy)carbonyl group may be optionally substituted with one or more halogen atoms}, or a hydrogen atom,
   R^{14a}, R^{21a}, and R^{29a} are identical to or different from each other, and each represents a C1-C6 chain hydrocarbon group, a C1-C6 alkoxy group, a C1-C6 alkylamino group, or a C2-C8 dialkylamino group {the C1-C6 chain hydrocarbon group, the C1-C6 alkoxy group, the C1-C6 alkylamino group, and the C2-C8 dialkylamino group may be optionally substituted with one or more halogen atoms},
   R^{23a} represents a C1-C6 chain hydrocarbon group which may optionally have one or more substituents selected from Group A^{a}, a C1-C6 alkylsulfonyl group, a (C1-C6 alkyl)carbonyl group, a (C1-C6 alkoxy)carbonyl group, a (C1-C6 alkylamino) carbonyl group, a (C2-C8 dialkylamino)carbonyl group {the C1-C6 alkylsulfonyl group, the (C1-C6 alkyl)carbonyl group, the (C1-C6 alkoxy)carbonyl group, the (C1-C6 alkylamino)carbonyl group, and the (C2-C8 dialkylamino) carbonyl group may be optionally substituted with one or more halogen atoms}, a C3-C8 alicyclic hydrocarbon group, a three (3)- to eight (8)- membered non-aromatic heterocyclic group {the C3-C8 alicyclic hydrocarbon group, and the three (3)- to eight (8)- membered non-aromatic heterocyclic group may optionally have one or more substituents selected from Group B^{a}}, a phenyl group, a five (5)- to six (6)- membered aromatic heterocyclic group {the phenyl group, and the five (5)- to six (6)- membered aromatic heterocyclic group may optionally have one or more substituents selected from Group D^{a}}, or a hydrogen atom,
   R^{22a}, R^{24a}, R^{25a}, and R^{31a} are identical to or different from each other, and each represents a C1-C6 chain hydrocarbon group which may optionally have one or more substituents selected from Group A^{a}, a C3-C8 alicyclic hydrocarbon group, a three (3)- to eight (8)- membered non-aromatic heterocyclic group {the C3-C8 alicyclic hydrocarbon group, and the three (3)- to eight (8)- membered non-aromatic heterocyclic group may optionally have one or more substituents selected from Group B^{a}}, a phenyl group, or a five (5)- to six (6)- membered aromatic heterocyclic group {the phenyl group, and the five (5)- to six (6)- membered aromatic heterocyclic group may optionally have one or more substituents selected from Group D^{a}},
   R^{26a} represents a methyl group which has one or more substituents selected from Group A^{a}, a C2-C6 chain hydrocarbon group which may optionally have one or more substituents selected from Group A^{a}, a C3-C8 alicyclic hydrocarbon group, a three (3)- to eight (8)- membered non-aromatic heterocyclic group {the C3-C8 alicyclic hydrocarbon group, and the three (3)- to eight (8)- membered non-aromatic heterocyclic group may optionally have one or more substituents selected from Group B^{a}}, a phenyl group, or a five (5)- to six (6)- membered aromatic heterocyclic group {the phenyl group, and the five (5)- to six (6)- membered aromatic heterocyclic group may optionally have one or more substituents selected from Group D^{a}},
   R^{27a} represents a C1-C6 chain hydrocarbon group which may optionally have one or more substituents selected from Group A^{a}, a C3-C8 alicyclic hydrocarbon group, a three (3)-to eight (8)- membered non-aromatic heterocyclic group {the C3-C8 alicyclic hydrocarbon group, and the three (3)- to eight (8)- membered non-aromatic heterocyclic group may optionally have one or more substituents selected from Group B^{a}}, a phenyl group, a five (5)- to six (6)- membered aromatic heterocyclic group {the phenyl group, and the five (5)- to six (6)- membered aromatic heterocyclic group may optionally have one or more substituents selected from Group D^{a}}, a (C1-C6 alkyl)carbonyl group, a (C1-C6 alkoxy)carbonyl group, or a C1-C6 alkylsulfonyl group {the (C1-C6 alkyl)carbonyl group, the (C1-C6 alkoxy)carbonyl group, and the C1-C6 alkylsulfonyl group may be optionally substituted with one or more halogen atoms},
   R^{28a} and R^{30a} are identical to or different from each other, and each represents a C1-C6 chain hydrocarbon group which may optionally have one or more substituents selected from Group A^{a}, or a hydrogen atom,
   the neighboring R^{2a} and R^{4a} may combine together with a carbon atom to which they are attached to form a C5-C6 alicyclic hydrocarbon, a five (5)- to six (6)- membered non-aromatic heterocyclic ring {the C5-C6 alicyclic hydrocarbon, and the five (5)- to six (6)- membered non-aromatic heterocyclic ring may optionally have one or more substituents selected from Group B^{a}}, a benzene ring, or a five (5)- to six (6)- membered aromatic heterocyclic ring {the benzene ring, and the five (5)- to six (6)- membered aromatic heterocyclic ring may optionally have one or more substituents selected from Group E^{a}},
   two of neighboring R^{4a} and R^{4a} may combine together with a carbon atom to which they are attached to form a C5-C6 alicyclic hydrocarbon, a five (5)- to six (6)- membered non-aromatic heterocyclic ring {the C5-C6 alicyclic hydrocarbon, and the five (5)- to six (6)- membered non-aromatic heterocyclic ring may optionally have one or more substituents selected from Group B^{a}}, a benzene ring, or a five (5)- to six (6)- membered aromatic heterocyclic ring {the benzene ring, and the five (5)- to six (6)- membered aromatic heterocyclic ring may optionally have one or more substituents selected from Group E^{a}}.

   Group A^{a} is a group consisting of a C1-C6 alkoxy group, a C1-C6 alkylsulfanyl group, a C1-C6 alkylsulfinyl group, a C1-C6 alkylsulfonyl group {the C1-C6 alkoxy group, the C1-C6 alkylsulfanyl group, the C1-C6 alkylsulfinyl group, and the C1-C6 alkylsulfonyl group may be optionally substituted with one or more halogen atoms}, a C3-C8 alicyclic hydrocarbon group, a three (3)- to eight (8)- membered non-aromatic heterocyclic group {the C3-C8 alicyclic hydrocarbon group, and the three (3)- to eight (8)- membered non-aromatic heterocyclic group may optionally have one or more substituents selected from Group Ba}, a phenyl group, a five (5)- to six (6)- membered aromatic heterocyclic group {the phenyl group and the five (5)- to six (6)- membered aromatic heterocyclic group may optionally have one or more substituents selected from Group Da}, a halogen atom, a cyano group, a nitro group, a hydroxy group, and a sulfanyl group.
   Group B^{a} is a group consisting of an oxo group, a thioxo group, a C1-C6 chain hydrocarbon group, a C1-C6 alkoxy group, a C1-C6 alkylsulfanyl group, a C1-C6 alkylsulfinyl group, a C1-C6 alkylsulfonyl group {the C1-C6 chain hydrocarbon group, the C1-C6 alkoxy group, the C1-C6 alkylsulfanyl group, the C1-C6 alkylsulfinyl group, and the C1-C6 alkylsulfonyl group may be optionally substituted with one or more halogen atoms}, a hydroxy group, a sulfanyl group, a halogen atom, and a cyano group.
   Group C^{a} is a group consisting of a C1-C6 alkoxy group, a C1-C6 alkylsulfanyl group, a C1-C6 alkylsulfinyl group, a C1-C6 alkylsulfonyl group {the C1-C6 alkoxy group, the C1-C6 alkylsulfanyl group, the C1-C6 alkylsulfinyl group, and the C1-C6 alkylsulfonyl group may be optionally substituted with one or more halogen atoms}, a C3-C8 alicyclic hydrocarbon group, a three (3)- to eight (8)- membered non-aromatic heterocyclic group {the C3-C8 alicyclic hydrocarbon group, and the three (3)- to eight (8)- membered non-aromatic heterocyclic group may optionally have one or more substituents selected from Group B^{a}}, and a five (5)- to six (6)- membered aromatic heterocyclic group {the five (5)- to six (6)- membered aromatic heterocyclic group may optionally have one or more substituents selected from Group D^{a}}.
   Group D^{a} is a group consisting of a C1-C6 chain hydrocarbon group, a C1-C6 alkoxy group, a C1-C6 alkylsulfanyl group, a C1-C6 alkylsulfinyl group, a C1-C6 alkylsulfonyl group, a C1-C6 alkylamino group, a C2-C8 dialkylamino group, a (C1-C6 alkyl)carbonyl group, a (C1-C6 alkoxy)carbonyl group, a (C1-C6 alkylamino)carbonyl group, a (C2-C8 dialkylamino)carbonyl group, a (C1-C6 alkyl)carbonylamino group, a (C1-C6 alkoxy)carbonylamino group {the C1-C6 chain hydrocarbon group, the C1-C6 alkoxy group, the C1-C6 alkylsulfanyl group, the C1-C6 alkylsulfinyl group, the C1-C6 alkylsulfonyl group, the C1-C6 alkylamino group, the C2-C8 dialkylamino group, the (C1-C6 alkyl)carbonyl group, the (C1-C6 alkoxy)carbonyl group, the (C1-C6 alkylamino)carbonyl group, the (C2-C8 dialkylamino)carbonyl group, the (C1-C6 alkyl)carbonylamino group, and the (C1-C6 alkoxy)carbonylamino group may be optionally substituted with one or more halogen atoms}, a C3-C8 alicyclic hydrocarbon group which may optionally have one or more substituents selected from Group B^{a}, an amino group, a nitro group, a cyano group, a hydroxy group, a sulfanyl group, and a halogen atom.
   Group E^{a} is a group consisting of a C1-C6 chain hydrocarbon group, a C1-C6 alkoxy group, a C1-C6 alkylsulfanyl group, a C1-C6 alkylsulfinyl group, a C1-C6 alkylsulfonyl group {the C1-C6 chain hydrocarbon group, the C1-C6 alkoxy group, the C1-C6 alkylsulfanyl group, the C1-C6 alkylsulfinyl group, and the C1-C6 alkylsulfonyl group may be optionally substituted with one or more halogen atoms}, a hydroxy group, a sulfanyl group, a halogen atom, and a cyano group.
   Group F^{a} is a group consisting of a C1-C6 alkoxy group, a C1-C6 alkylsulfanyl group, a C1-C6 alkylsulfinyl group, a C1-C6 alkylsulfonyl group {the C1-C6 alkoxy group, the C1-C6 alkylsulfanyl group, the C1-C6 alkylsulfinyl group, and the C1-C6 alkylsulfonyl group may be optionally substituted with one or more halogen atoms}, a C3-C8 alicyclic hydrocarbon group, a three (3)- to eight (8)- membered non-aromatic heterocyclic group {the C3-C8 alicyclic hydrocarbon group, and the three (3)- to eight (8)- membered non-aromatic heterocyclic group may optionally have one or more substituents selected from Group B^{a}}, a phenyl group, a five (5)- to six (6)- membered aromatic heterocyclic group {the phenyl group and the five (5)- to six (6)- membered aromatic heterocyclic group may optionally have one or more substituents selected from Group D^{a}}, a chlorine atom, a bromine atom, an iodine atom, a cyano group, a nitro group, a hydroxy group, and a sulfanyl group.
   Group G^{a} is a group consisting of a C1-C6 chain hydrocarbon group, a C1-C6 alkoxy group, a C1-C6 alkylsulfanyl group, a C1-C6 alkylsulfinyl group, a C1-C6 alkylsulfonyl group, a C1-C6 alkylamino group, a C2-C8 dialkylamino group {the C1-C6 chain hydrocarbon group, the C1-C6 alkoxy group, the C1-C6 alkylsulfanyl group, the C1-C6 alkylsulfinyl group, the C1-C6 alkylsulfonyl group, the C1-C6 alkylamino group, and the C2-C8 dialkylamino group may be optionally substituted with one or more halogen atoms}, a C3-C8 alicyclic hydrocarbon group which may optionally have one or more substituents selected from Group B, an amino group, a nitro group, a cyano group, a hydroxy group, a sulfanyl group, and a halogen atom]
      (hereinafter, referred to as "Compound D of the present invention"), or a N-oxide thereof, or a salt thereof (hereinafter, the compound D of the present invention, or the N-oxide thereof, or the salt thereof are collectively referred to as "Compound R of the present invention").
[6] The compound according to [5] wherein R^{1a} represents a C2-C6 alkyl group, a C2-C6 alkynyl group {the C2-C6 alkyl group, and the C2-C6 alkynyl group may optionally have one or more substituents selected from Group A^{a}}, a C2-C6 alkenyl group which may optionally have one or more substituents selected from Group F^{a}, a C3-C8 alicyclic hydrocarbon group which may optionally have one or more substituents selected from Group B^{a}, or a methyl group which has one or more substituents selected from Group C^{a} (hereinafter, referred to as "Compound N of the present invention"), or the N-oxide thereof, or the salt thereof (hereinafter, the compound N of the present invention, or the N-oxide thereof, or the salt thereof are collectively referred to as "Compound of the present invention").
[7] The compound according to [6] wherein X^{a} represents a nitrogen atom, R^{4a} represents a C1-C6 chain hydrocarbon group which may optionally have one or more substituents selected from Group A^{a}, OR^{23a}, NR^{27a}R^{28a}, a cyano group, a nitro group, a hydroxy group, or a halogen atom,
   or the N-oxide thereof, or the salt thereof.
[8] The compound according to [6] wherein X² represents CR^{5a}, R^{4a} represents a methyl group, a C2-C6 chain hydrocarbon group which may optionally have one or more substituents selected from Group A^{a}, OR^{23a}, SR^{24a}, S(O)R^{25a}, S(O)₂R^{22a}, NR^{27a}R^{28a}, a cyano group, a nitro group, a hydroxy group, or a halogen atom,
   the N-oxide thereof, or the salt thereof.
[9] The compound according to any one of [5] to [8] wherein R^{6a} represents a hydrogen atom, or the N-oxide thereof, or the salt thereof.
[10] A compound represented by formula (III): 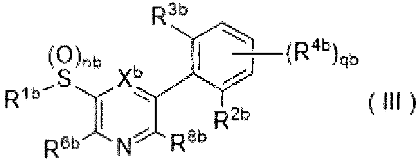 [wherein,
   nb is 0, 1, or 2,
   qb is 1, 2, or 3,
   when qb is 2 or 3, 2 or 3 of R^{4b} may be identical to or different from each other,
   R^{1b} represents a phenyl group which may optionally have one or more substituents selected from Group G^{b},
   R^{8b} represents a C1-C6 chain hydrocarbon group which may be optionally substituted with one or more halogen atoms, a C3-C8 alicyclic hydrocarbon group which may optionally have one or more substituents selected from Group B^{b}, OR^{7b}, SR^{9b}, S(O)R^{10b}, S(O)₂R^{11b}, NR^{12b}R^{13b}, C(O)R^{14b}, a nitro group, a cyano group, a halogen atom, or a hydrogen atom,
   R^{2b} and R^{3b} are identical to or different from each other, and each represents a C1-C6 chain hydrocarbon group which may be optionally substituted with one or more halogen atoms, a C3-C8 alicyclic hydrocarbon group which may optionally have one or more substituents selected from Group B^{b}, OR^{15b}, SR^{16b}, S(O)R^{17b}, S(O)₂R^{18b}, NR^{19b}R^{20b}, C(O)R^{21b}, a nitro group, a cyano group, a halogen atom, or a hydrogen atom,
   a combination of X^{b}, R^{4b} and R^{6b} represents
   a combination wherein
      X^{b} represents a nitrogen atom,
      R^{4b} represents a methyl group which may optionally have one or more substituents selected from Group F^{b}, a CH₂F group, a CHF₂ group, a C2-C3 chain hydrocarbon group which may optionally have one or more substituents selected from Group A^{b}, a C3-C8 alicyclic hydrocarbon group, a three (3)-to eight (8)- membered non-aromatic heterocyclic group {the C3-C8 alicyclic hydrocarbon group, and the three (3)- to eight (8)- membered non-aromatic heterocyclic group may optionally have one or more substituents selected from Group B^{b}}, a phenyl group, a five (5)- to six (6)- membered aromatic heterocyclic group {the phenyl group, and the five (5)- to six (6)- membered aromatic heterocyclic group may optionally have one or more substituents selected from Group D^{b}}, OR^{23b}, SR^{24b}, S(O)R^{25b}, S(O)₂R^{22b}, NR^{27b}R^{28b}, C(O)R^{29b}, C(R^{30b})=NOR^{31b}, a cyano group, a nitro group, a hydroxy group, an amino group, or a halogen atom, and
      R^{6b} represents a C1-C6 chain hydrocarbon group which may be optionally substituted with one or more halogen atoms, a C3-C8 alicyclic hydrocarbon group which may optionally have one or more substituents selected from Group B^{b}, OR^{7b}, SR^{9b}, S(O)R^{10b}, S(O)2R^{11b}, NR^{12b}R^{13b}, C(O)R^{14b}, a nitro group, a halogen atom, or a hydrogen atom; or
   a combination wherein
      X^{b} represents CR^{5b},
      R^{4b} represents a methyl group which may optionally have one or more substituents selected from Group F^{b}, a CH₂F group, a CHF₂ group, a C2-C3 chain hydrocarbon group which may optionally have one or more substituents selected from Group A^{b}, a C3-C8 alicyclic hydrocarbon group, a three (3)-to eight (8)- membered non-aromatic heterocyclic group {the C3-C8 alicyclic hydrocarbon group, and the three (3)- to eight (8)- membered non-aromatic heterocyclic group may optionally have one or more substituents selected from Group B^{b}}, a phenyl group, a five (5)- to six (6)- membered aromatic heterocyclic group {the phenyl group, and the five (5)- to six (6)- membered aromatic heterocyclic group may optionally have one or more substituents selected from Group D^{b}}, OR^{32b}, SR^{24b} , S(O)R^{25b}, S(O)₂R^{22b}, NR^{27b}R^{28b}, C(O)R^{29b}, C(R^{30b})=NOR^{31b}, a cyano group, a nitro group, a hydroxy group, or a halogen atom,
      R^{6b} represents a C1-C6 chain hydrocarbon group which may be optionally substituted with one or more halogen atoms, a C3-C8 alicyclic hydrocarbon group which may optionally have one or more substituents selected from Group B^{b}, OR^{7b}, a nitro group, a fluorine atom, a bromine atom, an iodine atom, or a hydrogen atom,
   R^{5b} represents a C1-C6 chain hydrocarbon group which may be optionally substituted with one or more halogen atoms, a C3-C8 alicyclic hydrocarbon group which may optionally have one or more substituents selected from Group B^{b}, OR^{7b}, SR^{9b}, S(O)R^{10b}, S(O)₂R^{11b}, NR^{12b}R^{13b}, C(O)R^{14b}, a nitro group, a cyano group, a halogen atom, or a hydrogen atom,
   R^{7b}, R^{9b}, R^{10b}, R^{11b}, R^{15b}, R^{16b}, R^{17b}, and R^{18b} are identical to or different from each other, and each represents a C1-C6 chain hydrocarbon group which may be optionally substituted with one or more halogen atoms,
   R^{12b} and R^{19b} are identical to or different from each other, and each represents a C1-C6 chain hydrocarbon group which may be optionally substituted with one or more halogen atoms, or a hydrogen atom,
   R^{13b} and R^{20b} are identical to or different from each other, and each represents a C1-C6 chain hydrocarbon group which may be optionally substituted with one or more halogen atoms, a (C1-C6 alkyl)carbonyl group, a (C1-C6 alkoxy)carbonyl group {the (C1-C6 alkyl)carbonyl group, and the (C1-C6 alkoxy)carbonyl group may be optionally substituted with one or more halogen atoms}, or a hydrogen atom,
   R^{14b}, R^{21b}, and R^{29b} are identical to or different from each other, and each represents a C1-C6 chain hydrocarbon group, a C1-C6 alkoxy group, a C1-C6 alkylamino group, or a C2-C8 dialkylamino group {the C1-C6 chain hydrocarbon group, the C1-C6 alkoxy group, the C1-C6 alkylamino group, and the C2-C8 dialkylamino group may be optionally substituted with one or more halogen atoms},
   R^{23b} represents a C1-C6 chain hydrocarbon group which may optionally have one or more substituents selected from Group A^{b}, a C1-C6 alkylsulfonyl group, a (C1-C6 alkyl)carbonyl group, a (C1-C6 alkoxy)carbonyl group, a (C1-C6 alkylamino) carbonyl group, a (C2-C8 dialkylamino) carbonyl group {the C1-C6 alkylsulfonyl group, the (C1-C6 alkyl)carbonyl group, the (C1-C6 alkoxy)carbonyl group, the (C1-C6 alkylamino)carbonyl group, and the (C2-C8 dialkylamino) carbonyl group may be optionally substituted with one or more halogen atoms}, a C3-C8 alicyclic hydrocarbon group, a three (3)- to eight (8)- membered non-aromatic heterocyclic group {the C3-C8 alicyclic hydrocarbon group, and the three (3)- to eight (8)- membered non-aromatic heterocyclic group may optionally have one or more substituents selected from Group B^{b}}, a phenyl group, a five (5)- to six (6)- membered aromatic heterocyclic group {the phenyl group, and the five (5)- to six (6)- membered aromatic heterocyclic group may optionally have one or more substituents selected from Group D^{b}}, or a hydrogen atom,
   R^{22b}, R^{24b}, R^{25b}, and R^{31b} are identical to or different from each other, and each represents a C1-C6 chain hydrocarbon group which may optionally have one or more substituents selected from Group A^{b}, a C3-C8 alicyclic hydrocarbon group, a three (3)- to eight (8)- membered non-aromatic heterocyclic group {the C3-C8 alicyclic hydrocarbon group, and the three (3)- to eight (8)- membered non-aromatic heterocyclic group may optionally have one or more substituents selected from Group B^{b}}, a phenyl group, or a five (5)- to six (6)- membered aromatic heterocyclic group {the phenyl group, and the five (5)- to six (6)- membered aromatic heterocyclic group may optionally have one or more substituents selected from Group D^{b}},
   R^{27b} represents a C1-C6 chain hydrocarbon group which may optionally have one or more substituents selected from Group A^{b}, a C3-C8 alicyclic hydrocarbon group, a three (3)-to eight (8)- membered non-aromatic heterocyclic group {the C3-C8 alicyclic hydrocarbon group, and the three (3)- to eight (8)- membered non-aromatic heterocyclic group may optionally have one or more substituents selected from Group B^{b}}, a phenyl group, a five (5)- to six (6)- membered aromatic heterocyclic group {the phenyl group, and the five (5)- to six (6)- membered aromatic heterocyclic group may optionally have one or more substituents selected from Group D^{b}}, a (C1-C6 alkyl)carbonyl group, a (C1-C6 alkoxy)carbonyl group, or a C1-C6 alkylsulfonyl group {the (C1-C6 alkyl)carbonyl group, the (C1-C6 alkoxy)carbonyl group, and the C1-C6 alkylsulfonyl group may be optionally substituted with one or more halogen atoms},
   R^{28b} and R^{30b} are identical to or different from each other, and each represents a C1-C6 chain hydrocarbon group which may optionally have one or more substituents selected from Group A^{b}, or a hydrogen atom,
   R^{32b} represents a methyl group which has one or more substituents selected from Group A^{b}, a C2-C6 chain hydrocarbon group which may optionally have one or more substituents selected from Group A^{b}, a C1-C6 alkylsulfonyl group, a (C1-C6 alkyl)carbonyl group, a (C1-C6 alkoxy)carbonyl group, a (C1-C6 alkylamino)carbonyl group, a (C2-C8 dialkylamino) carbonyl group {the C1-C6 alkylsulfonyl group, the (C1-C6 alkyl)carbonyl group, the (C1-C6 alkoxy) carbonyl group, the (C1-C6 alkylamino) carbonyl group, and the (C2-C8 dialkylamino) carbonyl group may be optionally substituted with one or more halogen atoms}, a C3-C8 alicyclic hydrocarbon group, a three (3)- to eight (8)- membered non-aromatic heterocyclic group {the C3-C8 alicyclic hydrocarbon group, and the three (3)- to eight (8)- membered non-aromatic heterocyclic group may optionally have one or more substituents selected from Group B^{b}}, a phenyl group, a five (5)- to six (6)- membered aromatic heterocyclic group {the phenyl group, and the five (5)- to six (6)- membered aromatic heterocyclic group may optionally have one or more substituents selected from Group D^{b}}, or a hydrogen atom,
   the neighboring R^{2b} and R^{4b} may combine together with a carbon atom to which they are attached to form a C5-C6 alicyclic hydrocarbon, a five (5)- to six (6)- membered non-aromatic heterocyclic ring {the C5-C6 alicyclic hydrocarbon, and the five (5)- to six (6)- membered non-aromatic heterocyclic ring may optionally have one or more substituents selected from Group B^{b}}, a benzene ring, or a five (5)- to six (6)- membered aromatic heterocyclic ring {the benzene ring, and the five (5)- to six (6)- membered aromatic heterocyclic ring may optionally have one or more substituents selected from Group E^{b}},
   two of neighboring R^{4b} and R^{4b} may combine together with a carbon atom to which they are attached to form a C5-C6 alicyclic hydrocarbon, a five (5)- to six (6)- membered non-aromatic heterocyclic ring {the C5-C6 alicyclic hydrocarbon, and the five (5)- to six (6)- membered non-aromatic heterocyclic ring may optionally have one or more substituents selected from Group B^{b}}, a benzene ring, or a five (5)- to six (6)- membered aromatic heterocyclic ring {the benzene ring, and the five (5)- to six (6)- membered aromatic heterocyclic ring may optionally have one or more substituents selected from Group E^{b}}.

   Group A^{b} is a group consisting of a C1-C6 alkoxy group, a C1-C6 alkylsulfanyl group, a C1-C6 alkylsulfinyl group, a C1-C6 alkylsulfonyl group {the C1-C6 alkoxy group, the C1-C6 alkylsulfanyl group, the C1-C6 alkylsulfinyl group, and the C1-C6 alkylsulfonyl group may be optionally substituted with one or more halogen atoms}, a C3-C8 alicyclic hydrocarbon group, a three (3)- to eight (8)- membered non-aromatic heterocyclic group {the C3-C8 alicyclic hydrocarbon group, and the three (3)- to eight (8)- membered non-aromatic heterocyclic group may optionally have one or more substituents selected from Group B^{b}}, a phenyl group, a five (5)- to six (6)- membered aromatic heterocyclic group {the phenyl group and the five (5)- to six (6)- membered aromatic heterocyclic group may optionally have one or more substituents selected from Group D^{b}}, a halogen atom, a cyano group, a nitro group, a hydroxy group, and a sulfanyl group.
   Group B^{b} is a group consisting of an oxo group, a thioxo group, a C1-C6 chain hydrocarbon group, a C1-C6 alkoxy group, a C1-C6 alkylsulfanyl group, a C1-C6 alkylsulfinyl group, a C1-C6 alkylsulfonyl group {the C1-C6 chain hydrocarbon group, the C1-C6 alkoxy group, the C1-C6 alkylsulfanyl group, the C1-C6 alkylsulfinyl group, and the C1-C6 alkylsulfonyl group may be optionally substituted with one or more halogen atoms}, a hydroxy group, a sulfanyl group, a halogen atom, and a cyano group.
   Group D^{b} is a group consisting of a C1-C6 chain hydrocarbon group, a C1-C6 alkoxy group, a C1-C6 alkylsulfanyl group, a C1-C6 alkylsulfinyl group, a C1-C6 alkylsulfonyl group, a C1-C6 alkylamino group, a C2-C8 dialkylamino group, a (C1-C6 alkyl)carbonyl group, a (C1-C6 alkoxy)carbonyl group, a (C1-C6 alkylamino)carbonyl group, a (C2-C8 dialkylamino)carbonyl group, a (C1-C6 alkyl)carbonylamino group, a (C1-C6 alkoxy)carbonylamino group {the C1-C6 chain hydrocarbon group, the C1-C6 alkoxy group, the C1-C6 alkylsulfanyl group, the C1-C6 alkylsulfinyl group, the C1-C6 alkylsulfonyl group, the C1-C6 alkylamino group, the C2-C8 dialkylamino group, the (C1-C6 alkyl)carbonyl group, the (C1-C6 alkoxy)carbonyl group, the (C1-C6 alkylamino)carbonyl group, the (C2-C8 dialkylamino)carbonyl group, the (C1-C6 alkyl)carbonylamino group, and the (C1-C6 alkoxy)carbonylamino group may be optionally substituted with one or more halogen atoms}, a C3-C8 alicyclic hydrocarbon group which may optionally have one or more substituents selected from Group B^{b}, an amino group, a nitro group, a cyano group, a hydroxy group, a sulfanyl group, and a halogen atom.
   Group E^{b} is a group consisting of a C1-C6 chain hydrocarbon group, a C1-C6 alkoxy group, a C1-C6 alkylsulfanyl group, a C1-C6 alkylsulfinyl group, a C1-C6 alkylsulfonyl group {the C1-C6 chain hydrocarbon group, the C1-C6 alkoxy group, the C1-C6 alkylsulfanyl group, the C1-C6 alkylsulfinyl group, and the C1-C6 alkylsulfonyl group may be optionally substituted with one or more halogen atoms}, a hydroxy group, a sulfanyl group, a halogen atom, and a cyano group.
   Group F^{b} is a group consisting of a C1-C6 alkoxy group, a C1-C6 alkylsulfanyl group, a C1-C6 alkylsulfinyl group, a C1-C6 alkylsulfonyl group {the C1-C6 alkoxy group, the C1-C6 alkylsulfanyl group, the C1-C6 alkylsulfinyl group, and the C1-C6 alkylsulfonyl group may be optionally substituted with one or more halogen atoms}, a C3-C8 alicyclic hydrocarbon group, a three (3)- to eight (8)- membered non-aromatic heterocyclic group {the C3-C8 alicyclic hydrocarbon group, and the three (3)- to eight (8)- membered non-aromatic heterocyclic group may optionally have one or more substituents selected from Group B^{b}}, a phenyl group, a five (5)- to six (6)- membered aromatic heterocyclic group {the phenyl group and the five (5)- to six (6)- membered aromatic heterocyclic group may optionally have one or more substituents selected from Group D^{b}}, a chlorine atom, a bromine atom, an iodine atom, a cyano group, a nitro group, a hydroxy group, and a sulfanyl group.
   Group G^{b} is a group consisting of a C1-C6 chain hydrocarbon group, a C1-C6 alkoxy group, a C1-C6 alkylsulfanyl group, a C1-C6 alkylsulfinyl group, a C1-C6 alkylsulfonyl group, a C1-C6 alkylamino group, a C2-C8 dialkylamino group, a (C1-C6 alkyl)carbonyl group, a (C1-C6 alkoxy)carbonyl group, a (C1-C6 alkylamino)carbonyl group, a (C2-C8 dialkylamino)carbonyl group, a (C1-C6 alkyl)carbonylamino group, a (C1-C6 alkoxy)carbonylamino group {the C1-C6 chain hydrocarbon group, the C1-C6 alkoxy group, the C1-C6 alkylsulfanyl group, the C1-C6 alkylsulfinyl group, the C1-C6 alkylsulfonyl group, the C1-C6 alkylamino group, the C2-C8 dialkylamino group, the (C1-C6 alkyl)carbonyl group, the (C1-C6 alkoxy)carbonyl group, the (C1-C6 alkylamino)carbonyl group, the (C2-C8 dialkylamino)carbonyl group, the (C1-C6 alkyl)carbonylamino group, and the (C1-C6 alkoxy)carbonylamino group may be optionally substituted with one or more halogen atoms}, a C3-C8 alicyclic hydrocarbon group which may optionally have one or more substituents selected from Group B^{b}, an amino group, a cyano group, a hydroxy group, a sulfanyl group, and a halogen atom]
      (hereinafter, referred to as "Compound E of the present invention"), or a N-oxide thereof, or a salt thereof (hereinafter, the compound E of the present invention, or the N-oxide thereof, or the salt thereof is collectively referred to as "Compound S of the present invention").
[11] A compound represented by formula (IV): 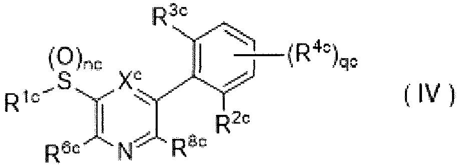 [wherein
   nc is 0, 1, or 2,
   qc is 1, 2, or 3,
   when qc is 2 or 3, 2 or 3 of R^{4c} may be identical to or different from each other,
   X^{c} is a nitrogen atom, or CR^{5c},
   R^{1c} represents a three (3)- to eight (8)- membered non-aromatic heterocyclic group which may optionally have one or more substituents selected from Group B^{c}, or NR^{33c}R^{34c},
   R^{8c} represents a C1-C6 chain hydrocarbon group which may be optionally substituted with one or more halogen atoms, a C3-C8 alicyclic hydrocarbon group which may optionally have one or more substituents selected from Group B^{c}, OR^{7c}, SR^{9c}, S(O)R^{10c}, S(O)₂R^{11c}, NR^{12c}R^{13c}, C(O)R^{14c}, a nitro group, a cyano group, a halogen atom, or a hydrogen atom,
   R^{2c} and R^{3c} are identical to or different from each other, and each represents a C1-C6 chain hydrocarbon group which may be optionally substituted with one or more halogen atoms, a C3-C8 alicyclic hydrocarbon group which may optionally have one or more substituents selected from Group B^{c}, OR^{15c}, SR^{16c}, S(O)R^{17c}, S(O)₂R^{18c}, NR^{19c}R^{20c}, C(O)R^{21c}, a nitro group, a cyano group, a halogen atom, or a hydrogen atom,
   R^{4c} represents a C1-C6 chain hydrocarbon group which may optionally have one or more substituents selected from Group A^{c}, a C3-C8 alicyclic hydrocarbon group, a three (3)- to eight (8)- membered non-aromatic heterocyclic group {the C3-C8 alicyclic hydrocarbon group, and the three (3)- to eight (8)- membered non-aromatic heterocyclic group may optionally have one or more substituents selected from the Group B^{c}}, a five (5)- to six (6)- membered aromatic heterocyclic group {the five (5)- to six (6)- membered aromatic heterocyclic group may optionally have one or more substituents selected from the Group D^{c}}, OR^{23c}, SR^{24c}, S (O)R^{25c}, S (O)₂R^{22c}, NR^{27c}R^{28c}, C(O)R^{29c}, C(R^{30c})=NOR^{31c}, a cyano group, a nitro group, an amino group, a hydroxy group, or a halogen atom,
   R^{6c} represents a C1-C6 chain hydrocarbon group which may be optionally substituted with one or more halogen atoms, a C3-C8 alicyclic hydrocarbon group which may optionally have one or more substituents selected from Group B^{c}, OR^{7c}, SR^{9c}, S(O)R^{10c}, S(O)₂R^{11c}, NR^{12c}R^{13c}, C(O)R^{14c}, a nitro group, a cyano group, a halogen atom, or a hydrogen atom,
   R^{5c} represents a C1-C6 chain hydrocarbon group which may be optionally substituted with one or more halogen atoms, a C3-C8 alicyclic hydrocarbon group which may optionally have one or more substituents selected from Group B^{c}, OR^{7c} , SR^{9c}, S(O)R^{10c}, S(O)₂R^{11c}, NR^{12c}R^{13c}, C(O)R^{14c}, a nitro group, a cyano group, a halogen atom, or a hydrogen atom,
   R7^{c}, R^{9c}, R^{10c}, R^{11c}, R^{15c}, R^{16c}, R^{17c}, and R^{18c} are identical to or different from each other, and each represents a C1-C6 chain hydrocarbon group which may be optionally substituted with one or more halogen atoms,
   R^{12c} and R^{19c} are identical to or different from each other, and each represents a C1-C6 chain hydrocarbon group which may be optionally substituted with one or more halogen atoms, or a hydrogen atom,
   R^{13c} and R^{20c} are identical to or different from each other, and each represents a C1-C6 chain hydrocarbon group which may be optionally substituted with one or more halogen atoms, a (C1-C6 alkyl)carbonyl group, a (C1-C6 alkoxy)carbonyl group {the (C1-C6 alkyl)carbonyl group, and the (C1-C6 alkoxy)carbonyl group may be optionally substituted with one or more halogen atoms}, or a hydrogen atom,
   R^{14c}, R^{21c}, and R^{29c} are identical to or different from each other, a C1-C6 chain hydrocarbon group, a C1-C6 alkoxy group, a C1-C6 alkylamino group, or a C2-C8 dialkylamino group {the C1-C6 chain hydrocarbon group, the C1-C6 alkoxy group, the C1-C6 alkylamino group, and the C2-C8 dialkylamino group may be optionally substituted with one or more halogen atoms},
   R^{23c} represents a C1-C6 chain hydrocarbon group which may optionally have one or more substituents selected from Group A^{c}, a C1-C6 alkylsulfonyl group, a (C1-C6 alkyl)carbonyl group, a (C1-C6 alkoxy)carbonyl group, a (C1-C6 alkylamino) carbonyl group, a (C2-C8 dialkylamino) carbonyl group {the C1-C6 alkylsulfonyl group, the (C1-C6 alkyl)carbonyl group, the (C1-C6 alkoxy)carbonyl group, the (C1-C6 alkylamino)carbonyl group, and the (C2-C8 dialkylamino) carbonyl group may be optionally substituted with one or more halogen atoms}, a C3-C8 alicyclic hydrocarbon group, a three (3)- to eight (8)- membered non-aromatic heterocyclic group {the C3-C8 alicyclic hydrocarbon group, and the three (3)- to eight (8)- membered non-aromatic heterocyclic group may optionally have one or more substituents selected from Group B^{c}}, a phenyl group, a five (5)- to six (6)- membered aromatic heterocyclic group {the phenyl group, and the five (5)- to six (6)- membered aromatic heterocyclic group may optionally have one or more substituents selected from Group D^{c}}, or a hydrogen atom,
   R^{22c}, R^{24c}, R^{25c}, and R^{31c} are identical to or different from each other, and each represents a C1-C6 chain hydrocarbon group which may optionally have one or more substituents selected from Group A^{c}, a C3-C8 alicyclic hydrocarbon group, a three (3)- to eight (8)- membered non-aromatic heterocyclic group {the C3-C8 alicyclic hydrocarbon group, and the three (3)- to eight (8)- membered non-aromatic heterocyclic group may optionally have one or more substituents selected from Group B^{c}}, a phenyl group, or a five (5)- to six (6)- membered aromatic heterocyclic group {the phenyl group, and the five (5)- to six (6)- membered aromatic heterocyclic group may optionally have one or more substituents selected from Group D^{c}},
   R^{27c} represents a C1-C6 chain hydrocarbon group which may optionally have one or more substituents selected from Group A^{c}, a C3-C8 alicyclic hydrocarbon group, a three (3)-to eight (8)- membered non-aromatic heterocyclic group {the C3-C8 alicyclic hydrocarbon group, and the three (3)- to eight (8)- membered non-aromatic heterocyclic group may optionally have one or more substituents selected from Group B^{c}}, a phenyl group, a five (5)- to six (6)- membered aromatic heterocyclic group {the phenyl group, and the five (5)- to six (6)- membered aromatic heterocyclic group may optionally have one or more substituents selected from Group D^{c}}, a (C1-C6 alkyl)carbonyl group, a (C1-C6 alkoxy)carbonyl group, or a C1-C6 alkylsulfonyl group {the (C1-C6 alkyl)carbonyl group, the (C1-C6 alkoxy)carbonyl group, and the C1-C6 alkylsulfonyl group may be optionally substituted with one or more halogen atoms},
   R^{28c} and R^{30c} are identical to or different from each other, and each represents a C1-C6 chain hydrocarbon group which may optionally have one or more substituents selected from Group A^{c}, or a hydrogen atom,
   R^{33c} represents a C1-C6 chain hydrocarbon group which may optionally have one or more substituents selected from Group G^{c}, a C3-C6 cycloalkyl group which may be optionally substituted with one or more halogen atoms, or a hydrogen atom,
   R^{34c} represents a C1-C6 chain hydrocarbon group which may optionally have one or more substituents selected from Group G^{c}, or a C3-C6 cycloalkyl group which may be optionally substituted with one or more halogen atoms,
   the neighboring R^{2c} and R^{4c} may combine together with a carbon atom to which they are attached to form a C5-C6 alicyclic hydrocarbon, a five (5)- to six (6)- membered non-aromatic heterocyclic ring {the C5-C6 alicyclic hydrocarbon, and the five (5)- to six (6)- membered non-aromatic heterocyclic ring may optionally have one or more substituents selected from Group B^{c}}, a benzene ring, or a five (5)- to six (6)- membered aromatic heterocyclic ring {the benzene ring, and the five (5)- to six (6)- membered aromatic heterocyclic ring may optionally have one or more substituents selected from Group E^{c}}.
   two of neighboring R^{4c} and R^{4c} may combine together with a carbon atom to which they are attached to form a C5-C6 alicyclic hydrocarbon which may optionally have one or more substituents selected from Group B^{c}, a five (5)- to six (6)-membered non-aromatic heterocyclic ring which may optionally have one or more substituents selected from Group C^{c}, a benzene ring, or a five (5)- to six (6)- membered aromatic heterocyclic ring {the benzene ring, and the five (5)- to six (6)- membered aromatic heterocyclic ring may optionally have one or more substituents selected from Group E^{c}} .

   Group A^{c} is a group consisting of a C1-C6 alkoxy group, a C1-C6 alkylsulfanyl group, a C1-C6 alkylsulfinyl group, C1-C6 alkylsulfonyl group {the C1-C6 alkoxy group, the C1-C6 alkylsulfanyl group, the C1-C6 alkylsulfinyl group, and the C1-C6 alkylsulfonyl group may be optionally substituted with one or more halogen atoms}, a C3-C8 alicyclic hydrocarbon group, a three (3)- to eight (8)- membered non-aromatic heterocyclic group {the C3-C8 alicyclic hydrocarbon group, and the three (3)- to eight (8)- membered non-aromatic heterocyclic group may optionally have one or more substituents selected from Group B^{c}}, a phenyl group, a five (5)- to six (6)- membered aromatic heterocyclic group {the phenyl group and the five (5)- to six (6)- membered aromatic heterocyclic group may optionally have one or more substituents selected from Group D^{c}}, a halogen atom, a cyano group, a nitro group, a hydroxy group, and a sulfanyl group.
   Group B^{c} is a group consisting of an oxo group, a thioxo group, a C1-C6 chain hydrocarbon group, a C1-C6 alkoxy group, a C1-C6 alkylsulfanyl group, a C1-C6 alkylsulfinyl group, a C1-C6 alkylsulfonyl group {the C1-C6 chain hydrocarbon group, the C1-C6 alkoxy group, the C1-C6 alkylsulfanyl group, the C1-C6 alkylsulfinyl group, and the C1-C6 alkylsulfonyl group may be optionally substituted with one or more halogen atoms}, a hydroxy group, a sulfanyl group, a halogen atom, and a cyano group.
   Group C^{c} is a group consisting of a thioxo group, a C1-C6 chain hydrocarbon group, a C1-C6 alkoxy group, a C1-C6 alkylsulfanyl group, a C1-C6 alkylsulfinyl group, a C1-C6 alkylsulfonyl group {the C1-C6 chain hydrocarbon group, the C1-C6 alkoxy group, the C1-C6 alkylsulfanyl group, the C1-C6 alkylsulfinyl group, and the C1-C6 alkylsulfonyl group may be optionally substituted with one or more halogen atoms}, a hydroxy group, a sulfanyl group, a halogen atom, and a cyano group.
   Group D^{c} is a group consisting of a C1-C6 chain hydrocarbon group, a C1-C6 alkoxy group, a C1-C6 alkylsulfanyl group, a C1-C6 alkylsulfinyl group, a C1-C6 alkylsulfonyl group, a C1-C6 alkylamino group, a C2-C8 dialkylamino group, a (C1-C6 alkyl)carbonyl group, a (C1-C6 alkoxy)carbonyl group, a (C1-C6 alkylamino)carbonyl group, (C2-C8 dialkylamino)carbonyl group, a (C1-C6 alkyl)carbonylamino group, a (C1-C6 alkoxy)carbonylamino group {the C1-C6 chain hydrocarbon group, the C1-C6 alkoxy group, the C1-C6 alkylsulfanyl group, the C1-C6 alkylsulfinyl group, the C1-C6 alkylsulfonyl group, the C1-C6 alkylamino group, the C2-C8 dialkylamino group, the (C1-C6 alkyl)carbonyl group, the (C1-C6 alkoxy)carbonyl group, the (C1-C6 alkylamino)carbonyl group, the (C2-C8 dialkylamino)carbonyl group, the (C1-C6 alkyl)carbonylamino group, and the (C1-C6 alkoxy)carbonylamino group may be optionally substituted with one or more halogen atoms}, a C3-C8 alicyclic hydrocarbon group which may optionally have one or more substituents selected from Group B^{c}, an amino group, a nitro group, a cyano group, a hydroxy group, a sulfanyl group, and halogen atom.
   Group E^{c} is a group consisting of a C1-C6 chain hydrocarbon group, a C1-C6 alkoxy group, a C1-C6 alkylsulfanyl group, a C1-C6 alkylsulfinyl group, a C1-C6 alkylsulfonyl group {the C1-C6 chain hydrocarbon group, the C1-C6 alkoxy group, the C1-C6 alkylsulfanyl group, the C1-C6 alkylsulfinyl group, and the C1-C6 alkylsulfonyl group may be optionally substituted with one or more halogen atoms}, a hydroxy group, a sulfanyl group, a halogen atom, and a cyano group.
   Group F^{c} is a group consisting of a C1-C6 alkoxy group, a C1-C6 alkylsulfanyl group, a C1-C6 alkylsulfinyl group, a C1-C6 alkylsulfonyl group {the C1-C6 alkoxy group, the C1-C6 alkylsulfanyl group, the C1-C6 alkylsulfinyl group, and the C1-C6 alkylsulfonyl group may be optionally substituted with one or more halogen atoms}, a C3-C8 alicyclic hydrocarbon group, a three (3)- to eight (8)- membered non-aromatic heterocyclic group {the C3-C8 alicyclic hydrocarbon group, and the three (3)- to eight (8)- membered non-aromatic heterocyclic group may optionally have one or more substituents selected from Group B^{c}}, a phenyl group, a five (5)- to six (6)- membered aromatic heterocyclic group {the phenyl group and the five (5)- to six (6)- membered aromatic heterocyclic group may optionally have one or more substituents selected from Group D^{c}}, a chlorine atom, a bromine atom, an iodine atom, a cyano group, a nitro group, a hydroxy group, and sulfanyl group.
   Group G^{c} is a group consisting of a C3-C6 cycloalkyl group, a C1-C3 alkoxy group, a C1-C3 alkylthio group {the C3-C6 cycloalkyl group, the C1-C3 alkoxy group, and the C1-C3 alkylthio group may be optionally substituted with one or more halogen atoms}, a halogen atom, and a cyano group]
      (hereinafter, referred to as "Compound F of the present invention"), or a N-oxide thereof, or a salt thereof (hereinafter, the compound F of the present invention, or the N-oxide, or the salt thereof is collectively referred to as "Compound T of the present invention").
[12] An agrochemical composition comprising the compound according to any one of [5] to [11], the N-oxide thereof, or the salt thereof, and an inert carrier.
[13] A composition comprising one or more ingredients selected from the group consisting of the following Group (a), Group (b), Group (c), and Group (d), as well as the compound according to any one of [5] to [11], or the N-oxide thereof, or the salt thereof:
   Group (a): a group consisting of insecticidal active ingredients, miticidal active ingredients, and nematicidal active ingredients;
   Group (b): fungicidal active ingredients;
   Group (c): plant growth regulatory ingredients;
   Group (d): repellent ingredients.
[14] A method for controlling a plant disease which comprises applying an effective amount of the compound according to any one of [5] to [11], or the N-oxide thereof, or the salt thereof, or an effective amount of the composition according to [13] to a plant or soil for cultivating the plant.
[15] A use of the compound according to any one of [5] to [11], or the N-oxide thereof, or the salt thereof, or the composition according to [13] for controlling a plant disease.
[16] A seed or vegetative reproductive organ carrying an effective amount of the compound according to any one of [5] to [11], or the N-oxide, or the salt thereof, or an effective amount of the composition according to [13].
[17] A compound represented by formula (V): 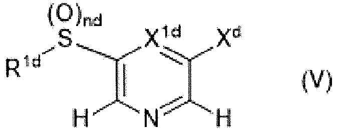 [wherein
   R^{1d} represents a C2-C4 chain hydrocarbon group, a C3-C6 alicyclic hydrocarbon group, a five (5)- to six (6)- membered aromatic heterocyclic group {the five (5)- to six (6)-membered aromatic heterocyclic group may optionally have one or more substituents selected from Group V¹},
   X^{d} represents a chlorine atom, or a bromine atom,
   the combination of X^{d} and nd represents
   a combination wherein X^{1d} represents CH, and nd is 0, 1, or 2; or
   a combination wherein X^{1d} represents a nitrogen atom, and nd is 1, or 2.
   Group V¹ is a group consisting of a C1-C4 alkyl group, a C1-C4 alkoxy group {the C1-C4 alkyl group, and the C1-C4 alkoxy group may be optionally substituted with one or more halogen atoms}, and a halogen atom]
   with the proviso that the following compounds are excluded: 3-chloro-5-(ethylthio)pyridine, 3-bromo-5-(ethylthio)pyridine, 3-chloro-5-(ethylsulfonyl)pyridine, 3-bromo-5-(ethylsulfonyl)pyridine, 3-chloro-5-(propylthio)pyridine, 3-chloro-5-[(1-methylethyl)thio]pyridine, 3-bromo-5-[(1-methylethyl)thio]pyridine, , 3-chloro-5-[(1-methylethyl)sulfonyl]pyridine, 3-bromo-5-[(1-methylethyl)sulfonyl]pyridine, 3-chloro-5-[(1,1-dimethylethyl)thio]pyridine, 3-bromo-5-[(1,1-dimethylethyl)thio]pyridine, 3-chloro-5-[(1,1-dimethylethyl)sulfonyl]pyridine, 3-bromo-5-[(1,1-dimethylethyl)sulfonyl]pyridine, 3-chloro-5-(cyclohexylsulfonyl)pyridine, 3-bromo-5-(cyclopropylthio)pyridine, 3-bromo-5-(cyclopropylsulfonyl)pyridine, 5-[(5-bromo-3-pyridinyl)thio]-2-methylpyridine, 5-[(5-bromo-3-pyridinyl)sulfonyl]-2-methylpyridine, and 3,3'-sulfonylbis(5-bromopyridine) (hereinafter, referred to as "Intermediate compound A").

### [Effect of Invention]

The present invention can control plant diseases.

### MODE FOR CARRYING OUT THE INVENTION

The substituents as used herein are explained as follows.

The expression "may optionally have" as used herein regarding the substituents has the same meaning as "may be optionally substituted with". The expression "have" or "has" as used herein regarding the substituents has the same meaning as "be substituted with".

The term "halogen atom" represents a fluorine atom, a chlorine atom, a bromine atom, or an iodine atom.

When a substituent has two or more halogen atoms or groups, the halogen atoms or groups may be identical to or different from each other.

The expression of "may have optionally one or more substituents selected from Group X" (wherein X represents any one of A, B, C, D, E, F, G, H, A^{a}, B^{a}, C^{a}, D^{a}, E^{a}, F^{a}, G^{a}, H^{a}, A^{b}, B^{b}, C^{b}, D^{b}, E^{b}, F^{b}, G^{b}, H^{b}, A^{c}, B^{c}, C^{c}, D^{c}, E^{c}, F^{c}, G^{c}, and V¹) as used herein represents that these substituents may be identical to or different from each other, when two or more substituents selected from Group X are present.

The expression "CX-CY" as used herein represents that the number of carbon atoms is from X to Y. For example, the expression "C1-C6" represents that the number of carbon atoms is from 1 to 6.

The term "chain hydrocarbon group" represents an alkyl group, an alkenyl group, or an alkynyl group.

Examples of the alkyl group include methyl group, ethyl group, propyl group, isopropyl group, 1,1-dimethylpropyl group, 1,2-dimethylpropyl group, butyl group, sec-butyl group, tert-butyl group, pentyl group, and hexyl group.

Examples of the alkenyl group include vinyl group, 1-propenyl group, 2-propenyl group, 1-methyl-1-propenyl group, 1-methyl-2-propenyl group, 1,2-dimethyl-1-propenyl group, 3-butenyl group, 4-pentenyl group, and 5-hexenyl group.

Examples of the alkynyl group include ethynyl group, 1-propynyl group, 2-propynyl group, 1-methyl-2-propynyl group, 1,1-dimethyl-2-propynyl group, 2-butynyl group, 4-pentynyl group, and 5-hexynyl group.

Examples of the alkoxy group include methoxy group, ethoxy group, propoxy group, isopropoxy group, butoxy group, tert-butoxy group, pentyloxy group, and hexyloxy group.

Examples of the "alkylsulfanyl group" include methylsulfanyl group, ethylsulfanyl group, isopropylsulfanyl group, and hexylsulfanyl group.

Examples of the "alkylsulfinyl group" include methylsulfinyl group, ethylsulfinyl group, isopropylsulfinyl group, and hexylsulfinyl group.

Examples of the "alkylsulfonyl group" include methane sulfonyl group, ethane sulfonyl group, isopropane sulfonyl group, and hexane sulfonyl group.

Examples of the "alkylcarbonyl group" include acetyl group, and propionyl group.

Examples of the "alkoxycarbonyl group" include methoxycarbonyl group, isopropoxycarbonyl group, and hexyloxycarbonyl group.

Examples of the "alicyclic hydrocarbon group" include cycloalkyl group and cycloalkenyl group.

Examples of the "cycloalkyl group" include cyclopropyl group, cyclobutyl group, cyclopentyl group, and cyclohexyl group.

Examples of the "cycloalkenyl group" include cyclopentenyl group and cyclohexenyl group.

Examples of the "aryl group" include phenyl group and naphthyl group.

Examples of the "aromatic heterocyclic group" include a five-membered aromatic heterocyclic group (such as pyrrolyl group, furanyl group, thienyl group, pyrazolyl group, imidazolyl group, triazolyl group, tetrazolyl group, oxazolyl group, isoxazolyl group, thiazolyl group, isothiazolyl group, oxadiazolyl group, and thiadiazolyl group); a six-membered aromatic heterocyclic group (such as pyridyl group, pyridazinyl group, pyrimidinyl group, pyrazinyl group, triazinyl group and tetrazinyl group).

Examples of the "non-aromatic heterocyclic group" include aziridinyl group, oxiranyl group, thiranyl group, azetidinyl group, oxetanyl group, thietanyl group, pyrrolidinyl group, tetrahydrofuranyl group, tetrahydrothienyl group, pyrazolynyl group, pyrazolidinyl group, imidazolinyl group, imidazolidinyl group, oxazolinyl group, thiazolinyl group, oxazolidinyl group, thiazolidinyl group, isoxazolinyl group, isoxazolidinyl group, isothiazolynyl group, isothiazolidinyl group, dioxolanyl group, dioxalanyl group, piperidyl group, piperaziny group, morpholinyl group, thiomorpholinyl group, pyranyl group, dihydropyranyl group, tetrahydropyranyl group, tetrahydrothiopyranyl group, azepanyl group, oxepanyl group, and thiepanyl group.

The compound D of the present invention, the compound E of the present invention, and the compound F of the present invention is collectively referred to as "Compound Q of the present invention". The compound Q of the present invention, or the N-oxide thereof, or the salt thereof is collectively referred to as "Compound Z of the present invention". Here the compound Z of the present invention means the same compound as the compound R of the present invention, the compound S of the present invention, and the compound T of the present invention.

The present compound Y, the compound Z of the present invention, or the intermediate compound A may be existed as one or more stereoisomers. Examples of the stereoisomer include enantiomer, diastereoisomer, atropisomer, and geometric isomer. Each stereoisomer, and stereoisomer mixture(s) in an arbitrary ratio thereof are included in the present invention.

The present compound X or the compound Q of the present invention, or their N-oxides may form an acid addition salts thereof such as hydrochloride salt, sulfate, nitrate, phosphate, acetate, benzoate and the like by mixing it with an acid such as hydrochloric acid, sulfuric acid, nitric acid, phosphoric acid, acetic acid, benzoic acid and the like.

Among the present compound X or the compound Q of the present invention, specific examples of the compound wherein the neighboring R² and R⁴ combine together with a carbon atom to which they are attached to form a C5-C6 alicyclic hydrocarbon, a five (5)- to six (6)- membered non-aromatic heterocyclic ring {the C5-C6 alicyclic hydrocarbon, and the five (5)- to six (6)- membered non-aromatic heterocyclic ring may optionally have one or more substituents selected from Group B}, a benzene ring, or a five (5)- to six (6)-membered aromatic heterocyclic ring {the benzene ring, and the five (5)- to six (6)- membered aromatic heterocyclic ring may optionally have one or more substituents selected from Group E} include as the present compound X, the compound represented by formula (IA-1) 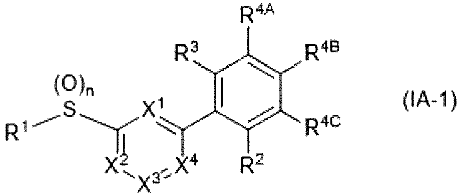 wherein
R¹, R³, X¹, X², X³, X⁴, and n are the same meanings as defined in [1],
R^{4A} and R^{4B} are identical to or different from each other, and each of these groups represents the same meanings as R⁴ defined in [1] or a hydrogen atom, and
R^{4c} and R² combine together with a carbon atom to which they are attached to form a C5-C6 alicyclic hydrocarbon, a five (5)- to six (6)- membered non-aromatic heterocyclic ring {the C5-C6 alicyclic hydrocarbon, and the five (5)- to six (6)- membered non-aromatic heterocyclic ring may optionally have one or more substituents selected from Group B}, a benzene ring, or a five (5)- to six (6)- membered aromatic heterocyclic ring {the benzene ring, and the five (5)- to six (6)- membered aromatic heterocyclic ring may optionally have one or more substituents selected from the Group E}.

Among the present compound X or the compound Q of the present invention, specific examples of the compound wherein two of neighboring R⁴ and R⁴ combine together with a carbon atom to which they are attached to form a C5-C6 alicyclic hydrocarbon, a five (5)- to six (6)- membered non-aromatic heterocyclic ring {the C5-C6 alicyclic hydrocarbon, and the five (5)- to six (6)- membered non-aromatic heterocyclic ring may optionally have one or more substituents selected from Group B}, a benzene ring, or a five (5)- to six (6)-membered aromatic heterocyclic ring {the benzene ring, and the five (5)- to six (6)- membered aromatic heterocyclic ring may optionally have one or more substituents selected from Group E} include as the present compound X,
1) the compound represented by formula (IA-1) wherein
   R¹, R², R³, X¹, X², X³, X⁴, and n have the same meanings as defined in [1],
   R^{4c} represents the same meanings as R⁴ as defined in [1] or a hydrogen atom,
   R^{4A} and R^{4B} combine together with a carbon atom to which they are attached to form a C5-C6 alicyclic hydrocarbon, a five (5)- to six (6)- membered non-aromatic heterocyclic ring {the C5-C6 alicyclic hydrocarbon, and the five (5)- to six (6)- membered non-aromatic heterocyclic ring may optionally have one or more substituents selected from Group B}, a benzene ring, or a five (5)- to six (6)- membered aromatic heterocyclic ring {the benzene ring, and the five (5)- to six (6)- membered aromatic heterocyclic ring may optionally have one or more substituents selected from Group E}; or
2) the compound represented by formula (IA-1) wherein
   R¹, R², R³, X¹, X², X³, X⁴, and n have the same meanings as defined in [1],
   R^{4A} represents the same meanings as R⁴ defined in [1] or a hydrogen atom,
   R^{4B} and R^{4C} combine together with a carbon atom to which they are attached to form a C5-C6 alicyclic hydrocarbon, a five (5)- to six (6)- membered non-aromatic heterocyclic ring {the C5-C6 alicyclic hydrocarbon, and the five (5)- to six (6)- membered non-aromatic heterocyclic ring may optionally have one or more substituents selected from Group B}, a benzene ring, or a five (5)- to six (6)- membered aromatic heterocyclic ring {the benzene ring, and the five (5)- to six (6)- membered aromatic heterocyclic ring may optionally have one or more substituents selected from Group E}.

3-Chloro-5-(ethylthio)pyridine is a compound represented by the following formula 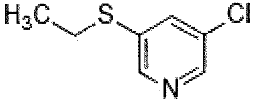 ,

3-bromo-5-(ethylthio)pyridine is a compound represented by the following formula 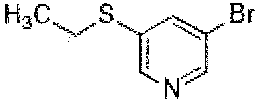 ,

3-chloro-5-(ethylsulfonyl)pyridine is a compound represented by the following formula 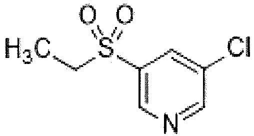 ,

3-bromo-5-(ethylsulfonyl)pyridine is a compound represented by the following formula

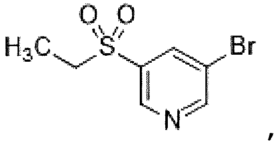

3-chloro-5-(propylthio)pyridine is a compound represented by the following formula 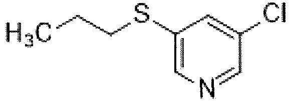 ,

3-chloro-5-[(1-methylethyl)thio]pyridine is a compound represented by the following formula 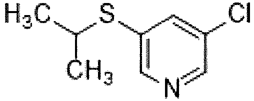 ,

3-bromo-5-[(1-methylethyl)thio]pyridine is a compound represented by the following formula 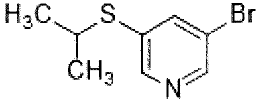 ,

3-chloro-5-[(1-methylethyl)sulfonyl]pyridine is a compound represented by the following formula 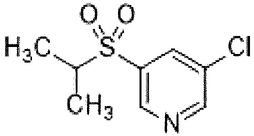 ,

3-bromo-5-[(1-methylethyl)sulfonyl]pyridine is a compound represented by the following formula 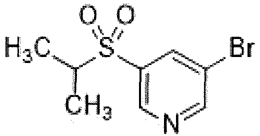 ,

3-chloro-5-[(1,1-dimethylethyl)thio]pyridine is a compound represented by the following formula 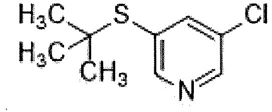 ,

3-bromo-5-[(1,1-dimethylethyl)thio]pyridine is a compound represented by the following formula 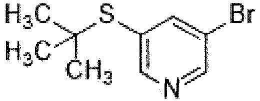 ,

3-chloro-5-[(1,1-dimethylethyl)sulfonyl]pyridine is a compound represented by the following formula

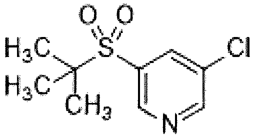

3-bromo-5-[(1,1-dimethylethyl)sulfonyl]pyridine is a compound represented by the following formula 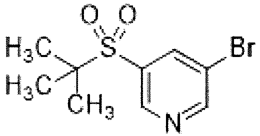 ,

3-chloro-5-(cyclohexylsulfonyl)pyridine is a compound represented by the following formula 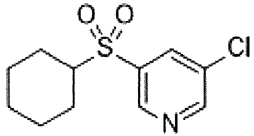 ,

3-bromo-5-(cyclopropylthio)pyridine is a compound represented by the following formula 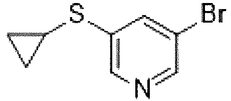 ,

3-bromo-5-(cyclopropylsulfonyl)pyridine is a compound represented by the following formula 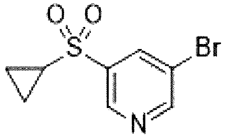 ,

5-[(5-bromo-3-pyridinyl)thio]-2-methylpyridine is a compound represented by the following formula 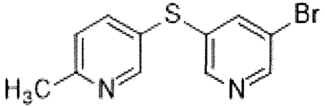 ,

5-[(5-bromo-3-pyridinyl)sulfonyl]-2-methylpyridine is a compound represented by the following formula 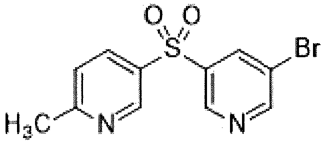 ,

3,3'-sulfonylbis(5-bromopyridine) is a compound represented by the following formula 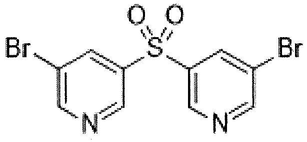 .

Embodiments of the present compound X include the following compounds.

[Embodiment X1] The present compound X wherein R¹ represents a C1-C6 alkyl group which may optionally have one or more substituents selected from Group A.
[Embodiment X2] The present compound X wherein R¹ represents a C2-C6 alkyl group which may optionally have one or more substituents selected from Group A.
[Embodiment X3] The present compound X wherein R¹ represents a C2-C6 alkyl group which may optionally have one or more substituents selected from Group A, or a methyl group which has one or more substituents selected from Group C.
[Embodiment X4] The present compound X wherein R¹ represents a C3-C8 alicyclic hydrocarbon group {the C3-C8 alicyclic hydrocarbon group may optionally have one or more substituents selected from Group B}.
[Embodiment X5] The present compound X wherein R¹ represents a C1-C6 alkyl group, or a C2-C6 alkynyl group {the C1-C6 alkyl group, and the C2-C6 alkynyl group may optionally have one or more substituents selected from Group A}.
[Embodiment X6] The present compound X wherein R¹ represents a C1-C6 alkyl group, a C2-C6 alkynyl group {the C1-C6 alkyl group, and the C2-C6 alkynyl group may optionally have one or more substituents selected from Group A}, or a C2-C6 alkenyl group which may optionally have one or more substituents selected from Group C.
[Embodiment X7] The present compound X wherein R¹ represents a C1-C6 alkyl group which may optionally have one or more substituents selected from Group A, or a C3-C8 alicyclic hydrocarbon group {the C3-C8 alicyclic hydrocarbon group may optionally have one or more substituents selected from Group B}.
[Embodiment X8] The present compound X wherein R¹ represents a C1-C6 alkyl group, a C2-C6 alkynyl group {the C1-C6 alkyl group, and the C2-C6 alkynyl group may optionally have one or more substituents selected from Group A}, or a C3-C8 alicyclic hydrocarbon group {the C3-C8 alicyclic hydrocarbon group may optionally have one or more substituents selected from Group B}.
[Embodiment X9] The present compound X wherein R¹ represents a C2-C6 alkyl group which may optionally have one or more substituents selected from Group A, a methyl group which has one or more substituents selected from Group C, or a C3-C8 alicyclic hydrocarbon group {the C3-C8 alicyclic hydrocarbon group may optionally have one or more substituents selected from Group B}.
[Embodiment X10] The present compound X wherein R² and R³ are identical to or different from each other, and each represents a C1-C6 chain hydrocarbon group which may be optionally substituted with one or more halogen atoms, or a hydrogen atom.
[Embodiment X11] The present compound X wherein R² and R³ are identical to or different from each other, and each represents a C3-C8 alicyclic hydrocarbon group which may optionally have one or more substituents selected from Group B, or a hydrogen atom.
[Embodiment X12] The present compound X wherein R² and R³ are identical to or different from each other, and each represents OR⁹, SR¹⁰, S(O)R¹¹, S(O)₂R¹², NR¹³R¹⁴, C(O)R¹⁵, a nitro group, a cyano group, or a hydrogen atom.
[Embodiment X13] The present compound X wherein R² and R³ are identical to or different from each other, and each represents a halogen atom, or a hydrogen atom.
[Embodiment X14] The present compound X wherein R² and R³ are identical to or different from each other, and each represents a C1-C6 chain hydrocarbon group which may be optionally substituted with one or more halogen atoms, a halogen atom, or a hydrogen atom.
[Embodiment X15] The present compound X wherein R² and R³ represent a hydrogen atom.
[Embodiment X16] The present compound X wherein R⁴ represents a C1-C6 chain hydrocarbon group which may optionally have one or more substituents selected from Group A.
[Embodiment X17] The present compound X wherein R⁴ represents a C3-C8 alicyclic hydrocarbon group, or a three (3)- to eight (8)- membered non-aromatic heterocyclic group {the C3-C8 alicyclic hydrocarbon group, and the three (3)-to eight (8)- membered non-aromatic heterocyclic group may optionally have one or more substituents selected from Group B}.
[Embodiment X18] The present compound X wherein R⁴ represents a phenyl group, or a five (5)- to six (6)-membered aromatic heterocyclic group {the phenyl group, and the five (5)- to six (6)- membered aromatic heterocyclic group may optionally have one or more substituents selected from Group D}.
[Embodiment X19] The present compound X wherein R⁴ represents OR¹⁶, SR¹⁷, S(O)R¹⁸, S(O)₂R¹⁹, NR²⁰R²¹, C(O)R²², C(R²³)=NOR²⁴, a cyano group, a nitro group, an amino group, or a hydroxy group.
[Embodiment X20] The present compound X wherein R⁴ represents a halogen atom.
[Embodiment X21] The present compound X wherein R⁴ represents a C1-C6 chain hydrocarbon group which may optionally have one or more substituents selected from Group A, or a halogen atom.
[Embodiment X22] The present compound X wherein R⁴ represents a C1-C6 chain hydrocarbon group which may optionally have one or more substituents selected from Group A, OR¹⁶, a cyano group, a nitro group, or a halogen atom.
[Embodiment X23] The present compound X wherein the neighboring R² and R⁴ combine together with a carbon atom to which they are attached to form a C5-C6 alicyclic hydrocarbon, or a five (5)- to six (6)- membered non-aromatic heterocyclic ring {the C5-C6 alicyclic hydrocarbon, and the five (5)- to six (6)- membered non-aromatic heterocyclic ring may optionally have one or more substituents selected from Group B}.
[Embodiment X24] The present compound X wherein the neighboring R² and R⁴ combine together with a carbon atom to which they are attached to form a benzene ring, or a five (5)- to six (6)- membered aromatic heterocyclic ring {the benzene ring, and the five (5)- to six (6)- membered aromatic heterocyclic ring may optionally have one or more substituents selected from Group E}.
[Embodiment X25] The present compound X wherein two of neighboring R⁴ and R⁴ combine together with a carbon atom to which they are attached to form a C5-C6 alicyclic hydrocarbon, or a five (5)- to six (6)- membered non-aromatic heterocyclic ring {the C5-C6 alicyclic hydrocarbon, and the five (5)- to six (6)- membered non-aromatic heterocyclic ring may optionally have one or more substituents selected from Group B}.
[Embodiment X26] The present compound X wherein two of neighboring R⁴ and R⁴ combine together with a carbon atom to which they are attached to form a benzene ring, or a five (5)- to six (6)- membered aromatic heterocyclic ring {the benzene ring, and the five (5)- to six (6)- membered aromatic heterocyclic ring may optionally have one or more substituents selected from Group E}.
[Embodiment X27] The present compound X wherein the combination of X¹, X², X³, and X⁴ represents the combination wherein X¹ represents CR⁵, X² represents CR⁶, X³ represents a nitrogen atom, and X⁴ represents CR⁸.
[Embodiment X28] The present compound X wherein the combination of X¹, X², X³, and X⁴ represents the combination wherein X¹ represents CR⁵, X² represents CR⁶, X³ represents CR⁷, and X⁴ represents a nitrogen atom; the combination wherein X¹ represents CR⁵, X² represents CR⁶, X³ represents a nitrogen atom, and X⁴ represents CR⁸; the combination wherein X¹ represents CR⁵, X² represents a nitrogen atom, X³ represents CR^{7A}, and X⁴ represents CR⁸; or the combination wherein X¹ represents a nitrogen atom, X² represents CR⁶, X³ represents CR^{7B}, and X⁴ represents CR⁸.
[Embodiment X29] The present compound X wherein the combination of X¹, X², X³, and X⁴ represents the combination wherein X¹ represents a nitrogen atom, X² represents CR⁶, X³ represents a nitrogen atom, and X⁴ represents CR⁸.
[Embodiment X30] The present compound X wherein the combination of X¹, X², X³, and X⁴ represents the combination wherein X¹ represents a nitrogen atom, X² represents a nitrogen atom, X³ represents CR^{7C}, and X⁴ represents CH; the combination wherein X¹ represents a nitrogen atom, X² represents a nitrogen atom, X³ represents CH, and X⁴ represents CR⁸; the combination wherein X¹ represents a nitrogen atom, X² represents CR⁶, X³ represents CR⁷, and X⁴ represents a nitrogen atom; the combination wherein X¹ is CR⁵, X² is a nitrogen atom, X³ represents CH, and X⁴ represents a nitrogen atom; or the combination wherein X¹ represents CH, X² represents a nitrogen atom, X³ represents CR^{7D}, and X⁴ represents a nitrogen atom.
[Embodiment X31] The present compound X wherein the combination of X¹, X², X³, and X⁴ represents the combination wherein X¹ represents CR⁵, X² represents a nitrogen atom, X³ represents a nitrogen atom, and X⁴ represents CR⁸; or the combination wherein X¹ represents CR⁵, X² represents CR⁶, X³ represents a nitrogen atom, and X⁴ represents a nitrogen atom.
[Embodiment X32] The present compound X wherein the combination of X¹, X², X³, and X⁴ represents the combination wherein X¹ represents CR⁵, X² represents CR⁶, X³ represents CR⁷, and X⁴ represents a nitrogen atom; the combination wherein X¹ represents CR⁵, X² represents CR⁶, X³ represents a nitrogen atom, and X⁴ represents CR⁸; the combination wherein X¹ represents CR⁵, X² represents a nitrogen atom, X³ represents CR^{7A}, and X⁴ represents CR⁸; the combination wherein X¹ represents a nitrogen atom, X² represents CR⁶, X³ represents CR^{7B}, and X⁴ represents CR⁸; or the combination wherein X¹ represents a nitrogen atom, X² represents CR⁶, X³ represents a nitrogen atom, and X⁴ represents CR⁸.
[Embodiment X33] The present compound X wherein the combination of X¹, X², X³, and X⁴ represents the combination wherein X¹ represents CR⁵, X² represents CR⁶, X³ represents a nitrogen atom, and X⁴ represents CR⁸; or the combination wherein X¹ represents a nitrogen atom, X² represents CR⁶, X³ represents a nitrogen atom, and X⁴ represents CR⁸.
[Embodiment X34] The present compound X wherein R⁵, R⁶, R⁷, R^{7A}, R^{7B}, and R⁸ are identical to or different from each other, and each represents a C1-C6 chain hydrocarbon group which may be optionally substituted with one or more halogen atoms, or a hydrogen atom, and R^{7C}, and R^{7D} are identical to or different from each other, and each represents a C1-C6 chain hydrocarbon group which may be optionally substituted with one or more halogen atoms.
[Embodiment X35] The present compound X wherein R⁵, R⁶, R⁷, R^{7A}, R^{7B}, and R⁸ are identical to or different from each other, and each represents a C3-C8 alicyclic hydrocarbon group which may optionally have one or more substituents selected from Group B, or a hydrogen atom, and R^{7C}, and R^{7D} are identical to or different from each other, and each represents a C3-C8 alicyclic hydrocarbon group which may optionally have one or more substituents selected from Group B.
[Embodiment X36] The present compound X wherein R⁵, R⁶, R⁷, and R⁸ are identical to or different from each other, and each represents OR²⁵, SR²⁶, S(O)R²⁷, S(O)₂R²⁸, NR²⁹R³⁰, C(O)R³¹, a nitro group, a cyano group, or a hydrogen atom.
[Embodiment X37] The present compound X wherein R⁵, R⁶, R⁷, R^{7A}, R^{7B}, and R⁸ are identical to or different from each other, and each represents a halogen atom, or a hydrogen atom, R^{7C} represents a halogen atom, R^{7D} represents a fluorine atom, a chlorine atom, or an iodine atom.
[Embodiment X38] The present compound X wherein R⁵, R⁶, R⁷, R^{7A}, R^{7B}, and R⁸ are identical to or different from each other, and each represents a C1-C6 chain hydrocarbon group which may be optionally substituted with one or more halogen atoms, a halogen atom, or a hydrogen atom, R^{7C} represents a C1-C6 chain hydrocarbon group which may be optionally substituted with one or more halogen atoms, or a halogen atom, and R^{7D} represents a C1-C6 chain hydrocarbon group which may be optionally substituted with one or more halogen atoms.
[Embodiment X39] The present compound X wherein R⁵, R⁶, R⁷, R^{7A}, R^{7B}, and R⁸ are identical to or different from each other, and each represents a C1-C6 chain hydrocarbon group which may be optionally substituted with one or more halogen atoms, OR²⁵, NR²⁹R³², C(O)R³¹, a nitro group, a cyano group, a halogen atom, or a hydrogen atom, R^{7c} represents a C1-C6 chain hydrocarbon group which may be optionally substituted with one or more halogen atoms, OR²⁵, NR²⁹R³⁰, C(O)R³¹, a nitro group, a cyano group, or a halogen atom, R^{7D} represents a C1-C6 chain hydrocarbon group which may be optionally substituted with one or more halogen atoms, OR²⁵, NR²⁹R³⁰, C(O)R³¹, a nitro group, or a cyano group.
[Embodiment X40] The present compound X wherein R⁵, R⁶, R⁷, R^{7A}, R^{7B}, and R⁸ represent a hydrogen atom.
[Embodiment X41] The present compound X wherein n is 0.
[Embodiment X42] The present compound X wherein n is 1.
[Embodiment X43] The present compound X wherein n is 2.
[Embodiment X44] The present compound X wherein q is 1.
[Embodiment X45] The present compound X wherein q is 2.
[Embodiment X46] The present compound X wherein q is 3.
[Embodiment X47] The present compound X wherein R¹ represents a C2-C6 alkyl group which may optionally have one or more substituents selected from Group A, a methyl group which has one or more substituents selected from Group C, or a C3-C8 alicyclic hydrocarbon group {the C3-C8 alicyclic hydrocarbon group may optionally have one or more substituents selected from Group B}, the combination of X¹, X², X³, and X⁴ represents the combination wherein X¹ represents CR⁵, X² represents CR⁶, X³ represents CR⁷, and X⁴ represents a nitrogen atom; the combination wherein X¹ represents CR⁵, X² represents CR⁶, X³ represents a nitrogen atom, and X⁴ represents CR⁸; the combination wherein X¹ represents CR⁵, X² represents a nitrogen atom, X³ represents CR^{7A}, and X⁴ represents CR⁸; the combination wherein X¹ represents a nitrogen atom, X² represents CR⁶, X³ represents CR^{7B}, and X⁴ represents CR⁸; the combination wherein X¹ represents a nitrogen atom, X² represents CR⁶, X³ represents a nitrogen atom, and X⁴ represents CR⁸; the combination wherein X¹ represents CR⁵, X² represents a nitrogen atom, X³ represents a nitrogen atom, and X⁴ represents CR⁸; or the combination wherein X¹ represents CR⁵, X² represents CR⁶, X³ represents a nitrogen atom, and X⁴ represents a nitrogen atom; and R⁵, R⁶, R⁷, R^{7A}, R^{7B}, and R⁸ represents a C1-C6 chain hydrocarbon group which may be optionally substituted with one or more halogen atoms, a halogen atom, or a hydrogen atom.
[Embodiment X48] The present compound X wherein R¹ represents a C1-C6 alkyl group which may optionally have one or more substituents selected from Group A, R² and R³ are identical to or different from each other, each represents a halogen atom, or a hydrogen atom, R⁴ represents a C1-C6 chain hydrocarbon group which may optionally have one or more substituents selected from Group A, OR¹⁶, SR¹⁷, NR²⁰R²¹, a cyano group, a nitro group, or a halogen atom, R⁵, R⁶, R⁷, R^{7A}, R^{7B}, R^{7C}, and R⁸ are identical to or different from each other, and each represents a C1-C6 chain hydrocarbon group which may be optionally substituted with one or more halogen atoms, OR²⁵, NR²⁹R³², C(O)R³¹, a cyano group, a halogen atom, or a hydrogen atom, and R^{7D} represents a C1-C6 chain hydrocarbon group which may be optionally substituted with one or more halogen atoms.
[Embodiment X49] A seed or vegetative reproductive organ carrying an effective amount of the present compound X, or the N-oxide, or the salt thereof.
[Embodiment X50] The present compound X wherein R¹ represents a five (5)- to ten (10)- membered aromatic heterocyclic group which may optionally have one or more substituents selected from Group D.
[Embodiment X51] The present compound X wherein R¹ represents a five (5)- to six (6)- membered aromatic heterocyclic group which may optionally have one or more substituents selected from Group D.
[Embodiment X52] The present compound X wherein R¹ represents a C6-C10 aryl group which may optionally have one or more substituents selected from Group F.
[Embodiment X53] The present compound X wherein R¹ represents a phenyl group which may optionally have one or more substituents selected from Group F.
[Embodiment X54] The present compound X wherein R¹ represents a three (3)- to eight (8)- membered non-aromatic heterocyclic group which may optionally have one or more substituents selected from Group B.
[Embodiment X55] The present compound X wherein R¹ represents a four (4)- to six (6)- membered non-aromatic heterocyclic group which may optionally have one or more substituents selected from Group B.
[Embodiment X56] The present compound X wherein R¹ represents NR³³R³⁴.
[Embodiment X57] The present compound X wherein R¹ represents NR³³R³⁴, R³³ represents a C1-C6 chain hydrocarbon group, or a C3-C6 alicyclic hydrocarbon group, and R³⁴ represents a C1-C6 chain hydrocarbon group, a C3-C6 alicyclic hydrocarbon group, or a hydrogen atom.
[Embodiment X58] The present compound X wherein R¹ represents a C1-C6 alkyl group which may optionally have one or more substituents selected from Group A, a C6-C10 aryl group which may optionally have one or more substituents selected from Group F, or a five (5)- to ten (10)- membered aromatic heterocyclic group {the five (5)- to ten (10)-membered aromatic heterocyclic group may optionally have one or more substituents selected from Group D}.
[Embodiment X59] The present compound X wherein R¹ represents a C1-C6 alkyl group which may optionally have one or more substituents selected from Group A, a phenyl group which may optionally have one or more substituents selected from Group F, or a five (5)- to six (6)- membered aromatic heterocyclic group {the five (5)- to six (6)- membered aromatic heterocyclic group may optionally have one or more substituents selected from Group D}.
[Embodiment X60] The present compound X wherein R¹ represents a C1-C6 alkyl group which may optionally have one or more substituents selected from Group A, a C6-C10 aryl group which may optionally have one or more substituents selected from Group F, a five (5)- to ten (10)- membered aromatic heterocyclic group which may optionally have one or more substituents selected from Group D, a three (3)- to eight (8)- membered non-aromatic heterocyclic group which may optionally have one or more substituents selected from Group B, or NR³³R³⁴.
[Embodiment X61] The present compound X wherein R¹ represents a C1-C6 alkyl group which may optionally have one or more substituents selected from Group A, a phenyl group which may optionally have one or more substituents selected from Group F, a five (5)- to six (6)- membered aromatic heterocyclic group which may optionally have one or more substituents selected from Group D, a four (4)- to six (6)-membered non-aromatic heterocyclic group which may optionally have one or more substituents selected from Group B, or NR³³R³⁴.
[Embodiment X62] The present compound X wherein R¹ represents a phenyl group, a pyridyl group, a pyridazinyl group, a pyrimidinyl group, a pyrazinyl group, a furanyl group, a thienyl group, an oxazolyl group, or a thiazolyl group {the phenyl group, the pyridyl group, the pyridazinyl group, the pyrimidinyl group, the pyrazinyl group, the furanyl group, the thienyl group, the oxazolyl group, and the thiazolyl group may optionally have one or more substituents selected from Group H.
   Group H is a group consisting of a C1-C6 chain hydrocarbon group, a C3-C8 alicyclic hydrocarbon group, a C1-C6 alkoxy group {the C1-C6 chain hydrocarbon group, the C3-C8 alicyclic hydrocarbon group, and the C1-C6 alkoxy group may be optionally substituted with one or more halogen atoms}, a halogen atom, a nitro group, and a cyano group.
[Embodiment X63] The present compound X wherein R¹ represents a phenyl group which may be optionally substituted with one or more halogen atoms, a five (5)- to six (6)-membered aromatic heterocyclic group which may optionally have one or more substituents selected from Group H, a five (5)- to six (6)- membered aromatic heterocyclic group, a three (3)- to eight (8)- membered non-aromatic heterocyclic group, a C1-C6 alkyl group which may optionally have one or more substituents selected from Group A, or NR³³R³⁴, R² and R³ are identical to or different from each other, and each represents a C1-C6 chain hydrocarbon group, a halogen atom, or a hydrogen atom, R⁴ represents a C1-C6 chain hydrocarbon group which may optionally have one or more substituents selected from Group A, a C3-C8 alicyclic hydrocarbon group, OR¹⁶, SR¹⁷, NR²⁰R²¹, a cyano group, a nitro group, or a halogen atom, two of neighboring R⁴ and R⁴ may combine together with a carbon atom to which they are attached to form a C5-C6 alicyclic hydrocarbon, a five (5)- to six (6)- membered non-aromatic heterocyclic ring {the C5-C6 alicyclic hydrocarbon, and the five (5)- to six (6)- membered non-aromatic heterocyclic ring may be optionally substituted with one or more halogen atoms}, or a five (5)- to six (6)- membered aromatic heterocyclic ring {the five (5)- to six (6)-membered aromatic heterocyclic ring may be optionally substituted with one or more halogen atoms}, R⁵, R⁶, R⁷, R^{7A}, R^{7B}, and R⁸ are identical to or different from each other, and each represents a C1-C6 chain hydrocarbon group which may be optionally substituted with one or more halogen atoms, OR²⁵, NR²⁹R³², C(O)R³¹, a cyano group, a halogen atom, or a hydrogen atom, R^{7C} represents a C1-C6 chain hydrocarbon group which may be optionally substituted with one or more halogen atoms, or a halogen atom, and R^{7D} represents a C1-C6 chain hydrocarbon group which may be optionally substituted with one or more halogen atoms.
[Embodiment X64] The present compound X wherein the combination of n and R¹ represents the combination wherein n is 0, 1, or 2, R¹ represents a C1-C6 alkyl group, a C2-C6 alkynyl group {the C1-C6 alkyl group, and the C2-C6 alkynyl group may optionally have one or more substituents selected from Group A}, a C2-C6 alkenyl group which may optionally have one or more substituents selected from Group C, a C6-C10 aryl group which may optionally have one or more substituents selected from Group F, a five (5)- to ten (10)-membered aromatic heterocyclic group which may optionally have one or more substituents selected from Group D, or a C3-C8 alicyclic hydrocarbon group {the C3-C8 alicyclic hydrocarbon group may optionally have one or more substituents selected from Group B}; or the combination wherein n is 2, R¹ represents a three (3)- to eight (8)-membered non-aromatic heterocyclic group which may optionally have one or more substituents selected from Group B, or NR³³R³⁴.
[Embodiment X65] The present compound X wherein the combination of X¹, X², X³, and X⁴ represents the combination wherein X¹ represents CR⁵, X² represents CR⁶, X³ represents a nitrogen atom, and X⁴ represents CR⁸; or the combination wherein X¹ represents a nitrogen atom, X² represents CR⁶, X³ represents a nitrogen atom, and X⁴ represents CR⁸; and R⁵, R⁶ and R⁸ represent a hydrogen atom.
[Embodiment X66] The present compound X wherein the combination of X¹, X², X³, and X⁴ represents the combination wherein X¹ represents CR⁵, X² represents CR⁶, X³ represents a nitrogen atom, and X⁴ represents CR⁸; and R⁵, R⁶ and R⁸ represents a hydrogen atom.
[Embodiment X67] The present compound X wherein the combination of X¹, X², X³, and X⁴ represents the combination wherein X¹ represents a nitrogen atom, X² represents CR⁶, X³ represents a nitrogen atom, and X⁴ represents CR⁸; and R⁶ and R⁸ represent a hydrogen atom.
[Embodiment X68] The present compound X wherein the combination of X¹, X², X³, and X⁴ represents the combination wherein X¹ represents CR⁵, X² represents CR⁶, X³ represents CR⁷, and X⁴ represents a nitrogen atom; the combination wherein X¹ represents CR⁵, X² represents CR⁶, X³ represents a nitrogen atom, and X⁴ represents CR⁸; the combination wherein X¹ represents CR⁵, X² represents a nitrogen atom, X³ represents CR^{7A}, and X⁴ represents CR⁸; the combination wherein X¹ represents a nitrogen atom, X² represents CR⁶, X³ represents CR^{7B}, and X⁴ represents CR⁸; the combination wherein X¹ represents a nitrogen atom, X² represents a nitrogen atom, X³ represents CH, and X⁴ represents CR⁸; the combination wherein X¹ represents a nitrogen atom, X² represents CR⁶, X³ represents a nitrogen atom, and X⁴ represents CR⁸; the combination wherein X¹ represents a nitrogen atom, X² represents CR⁶, X³ represents CR⁷, and X⁴ represents a nitrogen atom; the combination where X¹ represents CR⁵, X² represents a nitrogen atom, X³ represents a nitrogen atom, and X⁴ represents CR⁸; the combination wherein X¹ represents CR⁵, X² represents a nitrogen atom, X³ represents CH, and X⁴ represents a nitrogen atom; or the combination wherein X¹ represents CR⁵, X² represents CR⁶, X³ represents a nitrogen atom, and X⁴ represents a nitrogen atom, and R⁵, R⁶, R⁷, R^{7A}, R^{7B}, and R⁸ represent a hydrogen atom.

Embodiments of the present compound N include the following compounds.

[Embodiment 1] The present compound N wherein R¹ represents a C1-C6 alkyl group which may optionally have one or more substituents selected from Group A.
[Embodiment 2] The present compound N wherein R¹ represents a C2-C6 alkyl group which may optionally have one or more substituents selected from Group A.
[Embodiment 3] The present compound N wherein R¹ represents a C2-C6 alkyl group which may optionally have one or more substituents selected from Group A, or a methyl group which has one or more substituents selected from Group C.
[Embodiment 5] The present compound N wherein R¹ represents a C3-C8 alicyclic hydrocarbon group {the C3-C8 alicyclic hydrocarbon group may optionally have one or more substituents selected from Group B}.
[Embodiment 6] The present compound N wherein R¹ represents a C1-C6 alkyl group, or a C2-C6 alkynyl group {the C1-C6 alkyl group, and the C2-C6 alkynyl group may optionally have one or more substituents selected from Group A}.
[Embodiment 7] The present compound N wherein R¹ represents a C1-C6 alkyl group, a C2-C6 alkynyl group {the C1-C6 alkyl group, and the C2-C6 alkynyl group may optionally have one or more substituents selected from Group A}, or a C2-C6 alkenyl group which may optionally have one or more substituents selected from Group C.
[Embodiment 8] The present compound N wherein R¹ represents a C1-C6 alkyl group which may optionally have one or more substituents selected from Group A, a C3-C8 alicyclic hydrocarbon group {the C3-C8 alicyclic hydrocarbon group may optionally have one or more substituents selected from Group B}.
[Embodiment 9] The present compound N wherein R¹ represents a C1-C6 alkyl group, a C2-C6 alkynyl group {the C1-C6 alkyl group, and the C2-C6 alkynyl group may optionally have one or more substituents selected from Group A}, or a C3-C8 alicyclic hydrocarbon group {the C3-C8 alicyclic hydrocarbon group may optionally have one or more substituents selected from Group B}.
[Embodiment 10] The present compound N wherein R¹ represents a C2-C6 alkyl group which may optionally have one or more substituents selected from Group A, a methyl group which has one or more substituents selected from Group C, or a C3-C8 alicyclic hydrocarbon group {the C3-C8 alicyclic hydrocarbon group may optionally have one or more substituents selected from Group B}.
[Embodiment 11] The present compound N wherein R² and R³ are identical to or different from each other, and each represents a C1-C6 chain hydrocarbon group which may be optionally substituted with one or more halogen atoms, or a hydrogen atom.
[Embodiment 12] The present compound N wherein R² and R³ are identical to or different from each other, and each represent a C3-C8 alicyclic hydrocarbon group which may optionally have one or more substituents selected from Group B, or a hydrogen atom.
[Embodiment 13] The present compound N wherein R² and R³ are identical to or different from each other, and each represents OR⁹, SR¹⁰, S(O)R¹¹, S(O)₂R¹², NR¹³R¹⁴, C(O)R¹⁵, a nitro group, a cyano group, or a hydrogen atom.
[Embodiment 14] The present compound N wherein R² and R³ are identical to or different from each other, and each represents a halogen atom, or a hydrogen atom.
[Embodiment 15] The present compound N wherein R² and R³ are identical to or different from each other, and each represents a C1-C6 chain hydrocarbon group which may be optionally substituted with one or more halogen atoms, a halogen atom, or a hydrogen atom.
[Embodiment 16] The present compound N wherein R² and R³ represent a hydrogen atom.
[Embodiment 17] The present compound N wherein R⁴ represents a C1-C6 chain hydrocarbon group which may optionally have one or more substituents selected from Group A.
[Embodiment 18] The present compound N wherein R⁴ represents a C3-C8 alicyclic hydrocarbon group, or a three (3)- to eight (8)- membered non-aromatic heterocyclic group {the C3-C8 alicyclic hydrocarbon group, and the three (3)-to eight (8)- membered non-aromatic heterocyclic group may optionally have one or more substituents selected from Group B}.
[Embodiment 19] The present compound N wherein R⁴ represents a phenyl group, or a five (5)- to six (6)-membered aromatic heterocyclic group {the phenyl group, and the five (5)- to six (6)- membered aromatic heterocyclic group may optionally have one or more substituents selected from group D}.
[Embodiment 20] The present compound N wherein R⁴ represents OR¹⁶, SR¹⁷, S(O)R¹⁸, S(O)₂R¹⁹, NR²⁰R²¹, C(O)R²², C(R²³)=NOR²⁴, a cyano group, a nitro group, an amino group, or a hydroxy group.
[Embodiment 21] The present compound N wherein R⁴ represents a halogen atom.
[Embodiment 22] The present compound N wherein R⁴ represents a C1-C6 chain hydrocarbon group which may optionally have one or more substituents selected from Group A, or a halogen atom.
[Embodiment 23] The present compound N wherein R⁴ represents a C1-C6 chain hydrocarbon group which may optionally have one or more substituents selected from Group A, OR¹⁶, a cyano group, a nitro group, or a halogen atom.
[Embodiment 24] The present compound N wherein the neighboring R² and R⁴ combine together with a carbon atom to which they are attached to form a C5-C6 alicyclic hydrocarbon, or a five (5)- to six (6)- membered non-aromatic heterocyclic ring {the C5-C6 alicyclic hydrocarbon, and the five (5)- to six (6)- membered non-aromatic heterocyclic ring may optionally have one or more substituents selected from Group B}.
[Embodiment 25] The present compound N wherein the neighboring R² and R⁴ combine together with a carbon atom to which they are attached to form a benzene ring, or a five (5)- to six (6)- membered aromatic heterocyclic ring {the benzene ring, and the five (5)- to six (6)- membered aromatic heterocyclic ring may optionally have one or more substituents selected from Group E}.
[Embodiment 26] The present compound N wherein the neighboring R⁴ and R⁴ combine together with a carbon atom to which they are attached to form a C5-C6 alicyclic hydrocarbon, a five (5)- to six (6)- membered non-aromatic heterocyclic ring {the C5-C6 alicyclic hydrocarbon, and the five (5)- to six (6)- membered non-aromatic heterocyclic ring may optionally have one or more substituents selected from group B}.
[Embodiment 27] The present compound N wherein the neighboring R⁴ and R⁴ combine together with a carbon atom to which they are attached to form a benzene ring, or five (5)-to six (6)- membered aromatic heterocyclic ring {the benzene ring, and the five (5)- to six (6)- membered aromatic heterocyclic ring may optionally have one or more substituents selected from Group E}.
[Embodiment 28] The present compound N wherein the combination of X¹, X², X³, and X⁴ represents the combination wherein X¹ represents CR⁵, X² represents CR⁶, X³ represents a nitrogen atom, and X⁴ represents CR⁸.
[Embodiment 29] The present compound N wherein the combination of X¹, X², X³, and X⁴ represents the combination wherein X¹ represents CR⁵, X² represents CR⁶, X³ represents CR⁷, and X⁴ represents a nitrogen atom; the combination wherein X¹ represents CR⁵, X² represents CR⁶, X³ represents a nitrogen atom, and X⁴ represents CR⁸; the combination wherein X¹ represents CR⁵, X² represents a nitrogen atom, X³ represents CR^{7A}, and X⁴ represents CR⁸; or the combination wherein X¹ represents a nitrogen atom, X² represents CR⁶, X³ represents CR^{7B}, and X⁴ represents CR⁸.
[Embodiment 30] The present compound N wherein the combination of X¹, X², X³, and X⁴ represents the combination wherein X¹ represents a nitrogen atom, X² represents CR⁶, X³ represents a nitrogen atom, and X⁴ represents CR⁸.
[Embodiment 31] The present compound N wherein the combination of X¹, X², X³, and X⁴ represents the combination wherein X¹ represents a nitrogen atom, X² represents a nitrogen atom, X³ represents CR^{7c}, and X⁴ represents CH; the combination wherein X¹ represents a nitrogen atom, X² represents a nitrogen atom, X³ represents CH, and X⁴ represents CR⁸; the combination wherein X¹ represents a nitrogen atom, X² represents CR⁶, X³ represents CR⁷, and X⁴ represents a nitrogen atom; the combination wherein X¹ represents CR⁵, X² represents a nitrogen atom, X³ represents CH, and X⁴ represents a nitrogen atom; or the combination wherein X¹ represents CH, X² represents a nitrogen atom, X³ represents CR^{7D}, and X⁴ represents a nitrogen atom.
[Embodiment 32] The present compound N wherein the combination of X¹, X², X³, and X⁴ represents the combination wherein X¹ represents CR⁵, X² represents a nitrogen atom, X³ represents a nitrogen atom, and X⁴ represents CR⁸; or the combination wherein X¹ represents CR⁵, X² represents CR⁶, X³ represents a nitrogen atom, and X⁴ represents a nitrogen atom.
[Embodiment 33] The present compound N wherein the combination of X¹, X², X³, and X⁴ represents the combination wherein X¹ represents CR⁵, X² represents CR⁶, X³ represents CR⁷, and X⁴ represents a nitrogen atom; the combination wherein X¹ represents CR⁵, X² represents CR⁶, X³ represents a nitrogen atom, and X⁴ represents CR⁸; the combination wherein X¹ represents CR⁵, X² represents a nitrogen atom, X³ represents CR^{7A}, and X⁴ represents CR⁸; the combination wherein X¹ represents a nitrogen atom, X² represents CR⁶, X³ represents CR^{7B}, and X⁴ represents CR⁸; or the combination wherein X¹ represents a nitrogen atom, X² represents CR⁶, X³ represents a nitrogen atom, and X⁴ represents CR⁸.
[Embodiment 34] The present compound N wherein the combination of X¹, X², X³, and X⁴ represents the combination wherein X¹ represents CR⁵, X² represents CR⁶, X³ represents a nitrogen atom, and X⁴ represents CR⁸; or the combination wherein X¹ represents a nitrogen atom, X² represents CR⁶, X³ represents a nitrogen atom, and X⁴ represents CR⁸.
[Embodiment 35] The present compound N wherein R⁵, R⁶, R⁷, R^{7A}, R^{7B}, R^{7C}, R^{7D}, and R⁸ are identical to or different from each other, and each represents a C1-C6 chain hydrocarbon group which may be optionally substituted with one or more halogen atoms, or a hydrogen atom.
[Embodiment 36] The present compound N wherein R⁵, R⁶, R⁷, R^{7A}, R^{7B}, R^{7C}, R^{7D}, and R⁸ are identical to or different from each other, and each represents a C3-C8 alicyclic hydrocarbon group which may optionally have one or more substituents selected from Group B, or a hydrogen atom.
[Embodiment 37] The present compound N wherein R⁵, R⁶, R⁷, and R⁸ are identical to or different from each other, and each represents OR²⁵, SR²⁶, S(O)R²⁷, S(O)₂R²⁸, NR²⁹R³⁰, C(O)R³¹, a nitro group, a cyano group, or a hydrogen atom.
[Embodiment 38] The present compound N wherein R⁵, R⁶, R⁷, R^{7A}, R^{7B}, R^{7C}, and R⁸ are identical to or different from each other, and each represents a halogen atom, or a hydrogen atom, and R^{7D} represents a hydrogen atom.
[Embodiment 39] The present compound N wherein R⁵, R⁶, R⁷, R^{7A}, R^{7B}, R^{7C}, and R⁸ are identical to or different from each other, and each represents a C1-C6 chain hydrocarbon group which may be optionally substituted with one or more halogen atoms, a halogen atom, or a hydrogen atom, and R^{7D} represents a C1-C6 chain hydrocarbon group which may be optionally substituted with one or more halogen atoms, or a hydrogen atom.
[Embodiment 40] The present compound N wherein R⁵, R⁶, R⁷, R^{7A}, R^{7B}, R^{7C}, and R⁸ are identical to or different from each other, and each represents a C1-C6 chain hydrocarbon group which may be optionally substituted with one or more halogen atoms, OR²⁵, NR²⁹R³², C(O)R³¹, a nitro group, a cyano group, a halogen atom, or a hydrogen atom, and R^{7D} represents a C1-C6 chain hydrocarbon group which may be optionally substituted with one or more halogen atoms, OR²⁵, NR²⁹R³², C(O)R³¹, a nitro group, a cyano group, or a hydrogen atom.
[Embodiment 41] The present compound N wherein R⁵, R⁶, R⁷, R^{7A}, R^{7B}, R^{7C}, and R^{7D}, and R⁸ represents a hydrogen atom.
[Embodiment 42] The present compound N wherein n is 0.
[Embodiment 43] The present compound N wherein n is 1.
[Embodiment 44] The present compound N wherein n is 2.
[Embodiment 45] The present compound N wherein q is 1.
[Embodiment 46] The present compound N wherein q is 2.
[Embodiment 47] The present compound N wherein q is 3.
[Embodiment 48] The present compound N wherein R¹ represents a C2-C6 alkyl group which may optionally have one or more substituents selected from Group A, a methyl group which has one or more substituents selected from Group C, or a C3-C8 alicyclic hydrocarbon group {the C3-C8 alicyclic hydrocarbon group may optionally have one or more substituents selected from Group B}, the combination of X¹, X², X³, and X⁴ represents the combination wherein X¹ represents CR⁵, X² represents CR⁶, X³ represents CR⁷, and X⁴ represents a nitrogen atom; the combination wherein X¹ represents CR⁵, X² represents CR⁶, X³ represents a nitrogen atom, and X⁴ represents CR⁸; the combination wherein X¹ represents CR⁵, X² represents a nitrogen atom, X³ represents CR^{7A}, and X⁴ represents CR⁸; a combination wherein X¹ represents a nitrogen atom, X² represents CR⁶, X³ represents CR^{7B}, and X⁴ represents CR⁸; the combination wherein X¹ represents a nitrogen atom, X² represents CR⁶, X³ represents a nitrogen atom, and X⁴ represents CR⁸; the combination wherein X¹ represents CR⁵, X² represents a nitrogen atom, X³ represents a nitrogen atom, and X⁴ represents CR⁸; or the combination wherein X¹ represents CR⁵, X² represents CR⁶, X³ represents a nitrogen atom, and X⁴ represents a nitrogen atom; and R⁵, R⁶, R⁷, R^{7A}, R^{7B}, and R⁸ represents a C1-C6 chain hydrocarbon group which may be optionally substituted with one or more halogen atoms, a halogen atom, or a hydrogen atom.
[Embodiment 49] The present compound N wherein R¹ represents a C1-C6 alkyl group which may optionally have one or more substituents selected from Group A, R² and R³ are identical to or different from each other, and each represents a halogen atom, or a hydrogen atom, R⁴ represents a C1-C6 chain hydrocarbon group which may optionally have one or more substituents selected from group A, OR¹⁶, SR¹⁷, NR²⁰R²¹, a cyano group, a nitro group, or a halogen atom, R⁵, R⁶, R⁷, R^{7A}, R^{7B}, R^{7C}, and R⁸ are identical to or different from each other, and each represents a C1-C6 chain hydrocarbon group which may be optionally substituted with one or more halogen atoms, OR²⁵, NR²⁹R³², C(O)R³¹, a cyano group, a halogen atom, or a hydrogen atom, and R^{7D} represents a C1-C6 chain hydrocarbon group which may be optionally substituted with one or more halogen atoms.
[Embodiment 50] A seed or vegetative reproductive organ carrying an effective amount of the present compound N, or the N-oxide, or the salt thereof.

Embodiments of the compound D of the present invention include the following compounds.

[Embodiment D1] The compound D of the present invention wherein R^{1a} represents a C2-C6 alkyl group which may optionally have one or more substituents selected from Group A^{a}.
[Embodiment D2] The compound D of the present invention wherein R^{1a} represents a C2-C6 alkynyl group which may optionally have one or more substituents selected from Group A^{a}.
[Embodiment D3] The compound D of the present invention wherein R^{1a} represents a C2-C6 alkenyl group which may optionally have one or more substituents selected from Group F^{a}.
[Embodiment D4] The compound D of the present invention wherein R^{1a} represents a C3-C8 alicyclic hydrocarbon group which may optionally have one or more substituents selected from Group B^{a}.
[Embodiment D5] The compound D of the present invention wherein R^{1a} represents a methyl group which has one or more substituents selected from Group C^{a}.
[Embodiment D6] The compound D of the present invention wherein R^{1a} represents a C2-C6 alkyl group which may optionally have one or more substituents selected from Group A^{a}, a C2-C6 alkynyl group which may optionally have one or more substituents selected from Group A^{a}, or a C2-C6 alkenyl group which may optionally have one or more substituents selected from Group F^{a}.
[Embodiment D7] The compound D of the present invention wherein R^{1a} represents a C2-C6 alkyl group which may optionally have one or more substituents selected from Group A^{a}, or a methyl group which has one or more substituents selected from Group B^{a}.
[Embodiment D8] The compound D of the present invention wherein R^{1a} represents a C2-C6 alkyl group which may optionally have one or more substituents selected from Group A^{a}, or a C3-C8 alicyclic hydrocarbon group which may optionally have one or more substituents selected from Group B^{a}.
[Embodiment D9] The compound D of the present invention wherein R^{2a} and R^{3a} are identical to or different from each other, and each represents a C1-C6 chain hydrocarbon group which may be optionally substituted with one or more halogen atoms, or a hydrogen atom.
[Embodiment D10] The compound D of the present invention wherein R^{2a} and R^{3a} are identical to or different from each other, and each represents a C3-C8 alicyclic hydrocarbon group which may optionally have one or more substituents selected from Group B^{a}, or a hydrogen atom.
[Embodiment D11] The compound D of the present invention wherein R^{2a} and R^{3a} are identical to or different from each other, and each represents OR^{15a}, SR^{16a}, S(O)R^{17a}, S(O)₂R^{18a}, NR^{19a}R^{20a}, C(O)R^{21a}, a nitro group, a cyano group, or a hydrogen atom.
[Embodiment D12] The compound D of the present invention wherein R^{2a} and R^{3a} are identical to or different from each other, and each represents a halogen atom, or a hydrogen atom.
[Embodiment D13] The compound D of the present invention wherein R^{2a} and R^{3a} are identical to or different from each other, and each represents a C1-C6 chain hydrocarbon group which may be optionally substituted with one or more halogen atoms, a halogen atom, or a hydrogen atom.
[Embodiment D14] The compound D of the present invention wherein X^{a} represents a nitrogen atom, R^{4a} represents a C1-C6 chain hydrocarbon group which may optionally have one or more substituents selected from Group A^{a}, a C3-C8 alicyclic hydrocarbon group, a three (3)- to eight (8)- membered non-aromatic heterocyclic group {the C3-C8 alicyclic hydrocarbon group, and the three (3)- to eight (8)- membered non-aromatic heterocyclic group may optionally have one or more substituents selected from Group B^{a}}, a phenyl group, a five (5)- to six (6)- membered aromatic heterocyclic group {the phenyl group, and the five (5)- to six (6)- membered aromatic heterocyclic group may optionally have one or more substituents selected from Group D^{a}}, OR^{23a}, SR^{24a}, S(O)R^{25a}, S(O)₂R^{26a}, NR^{27a}R^{28a}, C(O)^{R29a}, C(R^{30a})=NOR^{31a}, a cyano group, a nitro group, an amino group, a hydroxy group, or a halogen atom, and R^{6a} represents a C1-C6 chain hydrocarbon group which may be optionally substituted with one or more halogen atoms, a C3-C8 alicyclic hydrocarbon group which may optionally have one or more substituents selected from Group B^{a}, OR^{7a}, SR^{9a}, S(O)R^{10a}, S(O)₂R^{11a}, NR^{12a}R^{13a}, C(O)R^{14a}, a nitro group, a cyano group, a halogen atom, or a hydrogen atom.
[Embodiment D15] The compound D of the present invention wherein X^{a} represents CR^{5a}, R^{4a} represents a methyl group which may optionally have one or more substituents selected from Group F^{a}, a CH₂F group, a CHF₂ group, a C2-C6 chain hydrocarbon group which may optionally have one or more substituents selected from Group A^{a}, a C3-C8 alicyclic hydrocarbon group, a three (3)- to eight (8)- membered non-aromatic heterocyclic group {the C3-C8 alicyclic hydrocarbon group, and the three (3)- to eight (8)- membered non-aromatic heterocyclic group may optionally have one or more substituents selected from Group B^{a}}, a phenyl group, a five
(5)- to six (6)- membered aromatic heterocyclic group {the phenyl group, and the five (5)- to six (6)- membered aromatic heterocyclic group may optionally have one or more substituents selected from Group D^{a}}, OR^{23a}, SR^{24a}, S(O)R^{25a}, S(O)2R^{22a}, NR^{27a}R^{28a}, C(O)R^{29a}, C(R^{30a})=NOR^{31a}, a cyano group, a nitro group, an amino group, a hydroxy group, or a halogen atom, and R^{6a} represents a C1-C6 chain hydrocarbon group which may be optionally substituted with one or more halogen atoms, a C3-C8 alicyclic hydrocarbon group which may optionally have one or more substituents selected from Group B^{a}, OR^{7a}, a nitro group, a fluorine atom, a bromine atom, an iodine atom, or a hydrogen atom.
[Embodiment D16] The compound D of the present invention wherein R^{6a} represents a C1-C6 chain hydrocarbon group which may be optionally substituted with one or more halogen atoms, or a hydrogen atom.
[Embodiment D17] The compound D of the present invention wherein R^{6a} represents a C3-C8 alicyclic hydrocarbon group which may optionally have one or more substituents selected from Group B^{a}, or a hydrogen atom.
[Embodiment D18] The compound D of the present invention wherein R^{6a} represents OR^{7a}, a nitro group, or a hydrogen atom.
[Embodiment D19] The compound D of the present invention wherein X^{a} represents CR^{5a}, and R^{6a} represents a fluorine atom, a bromine atom, an iodine atom, or a hydrogen atom.
[Embodiment D20] The compound D of the present invention wherein X^{a} represents a nitrogen atom, and R^{6a} represents a halogen atom, or a hydrogen atom.
[Embodiment D21] The compound D of the present invention wherein R^{6a} represents a hydrogen atom.
[Embodiment D22] The compound D of the present invention wherein X^{a} represents CR^{5a}, and R^{4a} represents a methyl group which may optionally have one or more substituents selected from Group F^{a}, a CH₂F group, a CHF₂ group, or a C2-C6 chain hydrocarbon group which may optionally have one or more substituents selected from Group A^{a}.
[Embodiment D23] The compound D of the present invention wherein X^{a} represents CR^{5a}, and R^{4a} represents OR^{23a}, SR^{24a}, S(O)R^{25a}, S(O)2R^{22a}, NR^{27a}R^{28a}, C(O)R^{29a}, C(R^{30a})=NOR^{31a}, a cyano group, a nitro group, an amino group, or a hydroxy group.
[Embodiment D24] The compound D of the present invention wherein X^{a} represents a nitrogen atom, and R^{4a} represents a C1-C6 chain hydrocarbon group which may optionally have one or more substituents selected from Group A^{a}.
[Embodiment D25] The compound D of the present invention wherein X^{a} represents a nitrogen atom, and R^{4a} represents OR^{23a}, SR^{24a}, S(O)R^{25a}, S(O)2R^{26a}, NR^{27a}R^{28a}, C(O)R^{29a}, C(R^{30a})=NOR^{31a}, a cyano group, a nitro group, an amino group, or a hydroxy group.
[Embodiment D26] The compound D of the present invention wherein R^{4a} represents a C3-C8 alicyclic hydrocarbon group, or a three (3)- to eight (8)- membered non-aromatic heterocyclic group {the C3-C8 alicyclic hydrocarbon group, and the three (3)- to eight (8)- membered non-aromatic heterocyclic group may optionally have one or more substituents selected from Group B^{a}}.
[Embodiment D27] The compound D of the present invention wherein R^{4a} represents a phenyl group, or a five (5)- to six (6)- membered aromatic heterocyclic group {the phenyl group, and the five (5)- to six (6)- membered aromatic heterocyclic group may optionally have one or more substituents selected from Group D^{a}}.
[Embodiment D28] The compound D of the present invention wherein R^{4a} represents a halogen atom.
[Embodiment D29] The compound D of the present invention wherein X^{a} represents CR^{5a}, and R^{4a} represents a methyl group which may optionally have one or more substituents selected from Group F^{a}, a C2-C6 chain hydrocarbon group which may optionally have one or more substituents selected from Group A^{a}, or a halogen atom.
[Embodiment D30] The compound D of the present invention wherein X^{a} represents a nitrogen atom, and R^{4a} represents a C1-C6 chain hydrocarbon group which may optionally have one or more substituents selected from Group A^{a}, or a halogen atom.
[Embodiment D31] The compound D of the present invention wherein X^{a} represents CR^{5a}, and R^{4a} represents a methyl group which may optionally have one or more substituents selected from Group F^{a}, a C2-C6 chain hydrocarbon group which may optionally have one or more substituents selected from Group A^{a}, OR^{23a}, a cyano group, a nitro group, a hydroxy group, or a halogen atom.
[Embodiment D32] The compound D of the present invention wherein X^{a} represents a nitrogen atom, and R^{4a} represents a C1-C6 chain hydrocarbon group which may optionally have one or more substituents selected from Group A^{a}, OR^{23a}, a cyano group, a nitro group, a hydroxy group, or a halogen atom.
[Embodiment D33] The compound D of the present invention wherein the neighboring R^{2a} and R^{4a} combine together with a carbon atom to which they are attached to form a C5-C6 alicyclic hydrocarbon, or a five (5)- to six (6)- membered non-aromatic heterocyclic ring {the C5-C6 alicyclic hydrocarbon, and the five (5)- to six (6)- membered non-aromatic heterocyclic ring may optionally have one or more substituents selected from Group B^{a}}.
[Embodiment D34] The compound D of the present invention wherein the neighboring R^{2a} and R^{4a} combine together with a carbon atom to which they are attached to form a benzene ring, or a five (5)- to six (6)- membered aromatic heterocyclic ring {the benzene ring, and the five (5)- to six (6)- membered aromatic heterocyclic ring may optionally have one or more substituents selected from Group E^{a}}.
[Embodiment D35] The compound D of the present invention wherein two of neighboring R^{4a} and R^{4a} combine together with a carbon atom to which they are attached to form a C5-C6 alicyclic hydrocarbon, or a five (5)- to six (6)- membered non-aromatic heterocyclic ring {the C5-C6 alicyclic hydrocarbon, and the five (5)- to six (6)- membered non-aromatic heterocyclic ring may optionally have one or more substituents selected from Group B^{a}}.
[Embodiment D36] The compound D of the present invention wherein two of neighboring R^{4a} and R^{4a} combine together with a carbon atom to which they are attached to form a benzene ring, or a five (5)- to six (6)- membered aromatic heterocyclic ring {the benzene ring, and the five (5)- to six (6)- membered aromatic heterocyclic ring may optionally have one or more substituents selected from Group E^{a}}.
[Embodiment D37] The compound D of the present invention wherein R^{5a} and R^{8a} are identical to or different from each other, and each represents a C1-C6 chain hydrocarbon group which may be optionally substituted with one or more halogen atoms, or a hydrogen atom.
[Embodiment D38] The compound D of the present invention wherein R^{5a}, and R^{8a} are identical to or different from each other, and each represents a C3-C8 alicyclic hydrocarbon group which may optionally have one or more substituents selected from Group B^{a}, or a hydrogen atom.
[Embodiment D39] The compound D of the present invention wherein R^{5a}, and R^{8a} are identical to or different from each other, and each represents OR^{7a}, SR^{9a}, S(O)R^{10a}, S(O)2R^{11a}, NR^{12a}R^{13a}, C(O)R^{14a}, a nitro group, a cyano group, or a hydrogen atom.
[Embodiment D40] The compound D of the present invention wherein R^{5a}, and R^{8a} are identical to or different from each other, and each represents a halogen atom, or a hydrogen atom.
[Embodiment D41] The compound D of the present invention wherein R^{5a}, and R^{8a} represents a hydrogen atom.
[Embodiment D42] The compound D of the present invention wherein R^{5a}, and R^{8a} are identical to or different from each other, and each represents a C1-C6 chain hydrocarbon group which may be optionally substituted with one or more halogen atoms, a halogen atom, or a hydrogen atom.
[Embodiment D43] The compound D of the present invention wherein na is 0.
[Embodiment D44] The compound D of the present invention wherein na is 1.
[Embodiment D45] The compound D of the present invention wherein na is 2.
[Embodiment D46] The compound D of the present invention wherein qa is 1.
[Embodiment D47] The compound D of the present invention wherein qa is 2.
[Embodiment D48] The compound D of the present invention wherein qa is 3.
[Embodiment D49] The compound D of the present invention wherein R^{5a}, R^{6a}, and R^{8a} represent a hydrogen atom.
[Embodiment D50] The compound D of the present invention wherein R^{2a}, and R^{3a} represent a hydrogen atom.
[Embodiment D51] The compound D of the present invention wherein R^{1a} represents a C2-C6 alkyl group which may optionally have one or more substituents selected from Group A^{a}, or a methyl group which has one or more substituents selected from Group C^{a}, R^{2a} and R^{3a} are identical to or different from each other, and each represents a C1-C6 chain hydrocarbon group which may be optionally substituted with one or more halogen atoms, a halogen atom, or a hydrogen atom, R^{4a} represents a methyl group, a C2-C6 chain hydrocarbon group which may optionally have one or more substituents selected from Group A^{a}, OR^{23a}, a cyano group, a nitro group, or a halogen atom, and R^{5a}, R^{6a}, and R^{8a} represent a hydrogen atom.
[Embodiment D52] The compound D of the present invention wherein R^{1a} represents a five (5)- to six (6)- membered aromatic heterocyclic group which may optionally have one or more substituents selected from Group G^{a}.
[Embodiment D53] The compound D of the present invention wherein R¹⁵ represents a C2-C6 alkyl group which may optionally have one or more substituents selected from Group A^{a}, or a five (5)- to six (6)- membered aromatic heterocyclic group which may optionally have one or more substituents selected from Group D^{a}.
[Embodiment D54] The compound D of the present invention wherein R^{1a} represents a five (5)- to six (6)- membered aromatic heterocyclic group which may optionally have one or more substituents selected from Group H^{a}.
   Group H^{a} is a group consisting of a C1-C6 chain hydrocarbon group, a C1-C6 alkoxy group {the C1-C6 chain hydrocarbon group, and the C1-C6 alkoxy group may be optionally substituted with one or more halogen atoms}, and a halogen atom.
[Embodiment D55] The compound D of the present invention wherein R^{1a} represents a pyridyl group, a pyridazinyl group, a pyrimidinyl group, a pyrazinyl group, a furanyl group, a thienyl group, an oxazolyl group, or a thiazolyl group {the phenyl group, the pyridyl group, the pyridazinyl group, the pyrimidinyl group, the pyrazinyl group, the furanyl group, the thienyl group, the oxazolyl group, and the thiazolyl group may optionally have one or more substituents selected from Group H^{a}}.
[Embodiment D56] The compound D of the present invention wherein R^{1a} represents a C2-C6 alkyl group which may optionally have one or more substituents selected from Group A^{a}, or a five (5)- to six (6)- membered aromatic heterocyclic group which may optionally have one or more substituents selected from Group H^{a}, a five (5)- to six (6)- membered aromatic heterocyclic group, R^{2a} and R^{3a} are identical to or different from each other, and each represents a C1-C6 chain hydrocarbon group which may be optionally substituted with one or more halogen atoms, a halogen atom, or a hydrogen atom, R^{4a} represents a methyl group which may optionally have one or more substituents selected from Group F^{a}, a CHF₂ group, a C2-C6 chain hydrocarbon group which may optionally have one or more substituents selected from Group A^{a}, a C1-C6 alkoxy group, a C1-C6 alkylsulfanyl group, a C2-C8 dialkylamino group {the C1-C6 alkoxy group, the C1-C6 alkylsulfanyl group, and the C2-C8 dialkylamino group may be optionally substituted with one or more halogen atoms}, a cyano group, a nitro group, or a halogen atom, and R^{5a}, R^{6a}, and R^{8a} represent a hydrogen atom.

Embodiments of the compound N of the present invention include the following compounds.

[Embodiment C1] The compound N of the present invention wherein R^{1a} represents a C2-C6 alkyl group which may optionally have one or more substituents selected from Group A^{a}.
[Embodiment C2] The compound N of the present invention wherein R^{1a} represents a C2-C6 alkynyl group which may optionally have one or more substituents selected from Group A^{a}.
[Embodiment C3] The compound N of the present invention wherein R^{1a} represents a C2-C6 alkenyl group which may optionally have one or more substituents selected from Group Fa.
[Embodiment C4] The compound N of the present invention wherein R^{1a} represents a C3-C8 alicyclic hydrocarbon group which may optionally have one or more substituents selected from Group B^{a}.
[Embodiment C5] The compound N of the present invention wherein R^{1a} represents a methyl group which has one or more substituents selected from Group C^{a}.
[Embodiment C6] The compound N of the present invention wherein R^{1a} represents a C2-C6 alkyl group which may optionally have one or more substituents selected from Group A^{a}, a C2-C6 alkynyl group which may optionally have one or more substituents selected from Group A^{a}, or a C2-C6 alkenyl group which may optionally have one or more substituents selected from Group F^{a}.
[Embodiment C7] The compound N of the present invention wherein R^{1a} represents a C2-C6 alkyl group which may optionally have one or more substituents selected from Group A^{a}, or a methyl group which has one or more substituents selected from Group C^{a}.
[Embodiment C8] The compound N of the present invention wherein R^{1a} represents a C2-C6 alkyl group which may optionally have one or more substituents selected from Group A^{a}, or a C3-C8 alicyclic hydrocarbon group which may optionally have one or more substituents selected from Group B^{a}.
[Embodiment C9] The compound N of the present invention wherein R^{2a} and R^{3a} are identical to or different from each other, and each represents a C1-C6 chain hydrocarbon group which may be optionally substituted with one or more halogen atoms, or a hydrogen atom.
[Embodiment C10] The compound N of the present invention wherein R^{2a} and R^{3a} are identical to or different from each other, and each represents a C3-C8 alicyclic hydrocarbon group which may optionally have one or more substituents selected from Group B^{a}, or a hydrogen atom.
[Embodiment C11] The compound N of the present invention wherein R^{2a} and R^{3a} are identical to or different from each other, and each represents OR^{15a}, SR^{16a}, S(O)R^{17a}, S(O)₂R^{18a}, NR^{19a}R^{20a}, C(O)R^{21a}, a nitro group, a cyano group, or a hydrogen atom.
[Embodiment C12] The compound N of the present invention wherein R^{2a} and R^{3a} are identical to or different from each other, and each represents a halogen atom, or a hydrogen atom.
[Embodiment C13] The compound N of the present invention wherein R^{2a} and R^{3a} are identical to or different from each other, and each represents a C1-C6 chain hydrocarbon group which may be optionally substituted with one or more halogen atoms, a halogen atom, or a hydrogen atom.
[Embodiment C14] The compound N of the present invention wherein X^{a} represents a nitrogen atom, R^{4a} represents a C1-C6 chain hydrocarbon group which may optionally have one or more substituents selected from Group A^{a}, a C3-C8 alicyclic hydrocarbon group, a three (3)- to eight (8)- membered non-aromatic heterocyclic group {the C3-C8 alicyclic hydrocarbon group, and the three (3)- to eight (8)- membered non-aromatic heterocyclic group may optionally have one or more substituents selected from Group B^{a}}, a phenyl group, a five (5)- to six (6)- membered aromatic heterocyclic group {the phenyl group, and the five (5)- to six (6)- membered aromatic heterocyclic group may optionally have one or more substituents selected from Group D^{a}}, OR^{23a}, SR^{24a}, S(O)R^{25a}, S(O)₂R^{26a}, NR^{27a}R^{28a}, C(O)R^{29a}, C(R^{30a})=NOR^{31a}, a cyano group, a nitro group, an amino group, a hydroxy group, or a halogen atom, R^{6a} represents a C1-C6 chain hydrocarbon group which may be optionally substituted with one or more halogen atoms, a C3-C8 alicyclic hydrocarbon group which may optionally have one or more substituents selected from Group B^{a}, OR^{7a}, SR^{9a}, S(O)R^{10a}, S(O)₂R^{11a}, NR^{12a}R^{13a}, C(O)R^{14a}, a nitro group, a cyano group, a halogen atom, or a hydrogen atom.
[Embodiment C15] The compound N of the present invention wherein X^{a} represents CR^{5a}, R^{4a} represents a methyl group which may optionally have one or more substituents selected from Group F^{a}, a CH₂F group, a CHF₂ group, a C2-C6 chain hydrocarbon group which may optionally have one or more substituents selected from Group A^{a}, a C3-C8 alicyclic hydrocarbon group, a three (3)- to eight (8)- membered non-aromatic heterocyclic group {the C3-C8 alicyclic hydrocarbon group, and the three (3)- to eight (8)- membered non-aromatic heterocyclic group may optionally have one or more substituents selected from Group B}, a phenyl group, a five (5)- to six (6)- membered aromatic heterocyclic group {the phenyl group, and the five (5)- to six (6)- membered aromatic heterocyclic group may optionally have one or more substituents selected from Group D^{a}}, OR^{23a}, SR^{24a}, S(O)R^{25a}, S(O)₂R^{22a}, NR^{27a}R^{28a}, C(O)R^{29a}, C(R^{30a})=NOR^{31a}, a cyano group, a nitro group, an amino group, a hydroxy group, or a halogen atom, and R^{6a} represents a C1-C6 chain hydrocarbon group which may be optionally substituted with one or more halogen atoms, a C3-C8 alicyclic hydrocarbon group which may optionally have one or more substituents selected from Group B^{a}, OR^{7a}, a nitro group, a fluorine atom, a bromine atom, an iodine atom, or a hydrogen atom.
[Embodiment C16] The compound N of the present invention wherein R^{6a} represents a C1-C6 chain hydrocarbon group which may be optionally substituted with one or more halogen atoms, or a hydrogen atom.
[Embodiment C17] The compound N of the present invention wherein R^{6a} represents a C3-C8 alicyclic hydrocarbon group which may optionally have one or more substituents selected from Group B^{a}, or a hydrogen atom.
[Embodiment C18] The compound N of the present invention wherein R^{6a} represents OR^{7a}, a nitro group, or a hydrogen atom.
[Embodiment C19] The compound N of the present invention wherein X^{a} represents CR^{5a}, and R^{6a} represents a fluorine atom, a bromine atom, an iodine atom, or a hydrogen atom.
[Embodiment C20] The compound N of the present invention wherein X^{a} represents a nitrogen atom, and R^{6a} represents a halogen atom, or a hydrogen atom.
[Embodiment C21] The compound N of the present invention wherein R^{6a} represents a hydrogen atom.
[Embodiment C22] The compound N of the present invention wherein X^{a} represents CR^{5a}, and R^{4a} represents a methyl group which may optionally have one or more substituents selected from Group F^{a}, a CH₂F group, a CHF₂ group, a C2-C6 chain hydrocarbon group which may optionally have one or more substituents selected from Group A^{a}.
[Embodiment C23] The compound N of the present invention wherein X^{a} represents CR^{5a}, R^{4a} represents OR^{23a}, SR^{24a}, S(O)R^{25a}, S(O)₂R^{22a}, NR^{27a}R^{28a}, C(O)R^{29a}, C(R^{30a})=NOR^{31a}, a cyano group, a nitro group, an amino group, or a hydroxy group.
[Embodiment C24] The compound N of the present invention wherein X² represents a nitrogen atom, R^{4a} represents a C1-C6 chain hydrocarbon group which may optionally have one or more substituents selected from Group A^{a}.
[Embodiment C25] The compound N of the present invention wherein X^{a} represents a nitrogen atom, and R^{4a} represents OR^{23a}, SR^{24a}, S(O)R^{25a}, S(O)₂R^{26a}, NR^{27a}R^{28a}, C(O)R^{29a}, C(R^{30a})=NOR^{31a}, a cyano group, a nitro group, an amino group, or a hydroxy group.
[Embodiment C26] The compound N of the present invention wherein R^{4a} represents a C3-C8 alicyclic hydrocarbon group, a three (3)- to eight (8)- membered non-aromatic heterocyclic group {the C3-C8 alicyclic hydrocarbon group, and the three (3)- to eight (8)- membered non-aromatic heterocyclic group may optionally have one or more substituents selected from Group B^{a}}.
[Embodiment C27] The compound N of the present invention wherein R^{4a} represents a phenyl group, a five (5)- to six (6)- membered aromatic heterocyclic group {the phenyl group, and the five (5)- to six (6)- membered aromatic heterocyclic group may optionally have one or more substituents selected from Group D^{a}}.
[Embodiment C28] The compound N of the present invention wherein R^{4a} represents a halogen atom.
[Embodiment C29] The compound N of the present invention wherein X^{a} represents CR^{5a}, and R^{4a} represents a methyl group which may optionally have one or more substituents selected from Group F^{a}, a C2-C6 chain hydrocarbon group which may optionally have one or more substituents selected from Group A^{a}, or a halogen atom.
[Embodiment C30] The compound N of the present invention wherein X^{a} represents a nitrogen atom, and R^{4a} represents a C1-C6 chain hydrocarbon group which may optionally have one or more substituents selected from Group A^{a}, or a halogen atom.
[Embodiment C31] The compound N of the present invention wherein X^{a} represents CR^{5a}, and R^{4a} represents a methyl group which may optionally have one or more substituents selected from Group F^{a}, a C2-C6 chain hydrocarbon group which may optionally have one or more substituents selected from Group A^{a}, OR^{23a}, a cyano group, a nitro group, a hydroxy group, or a halogen atom.
[Embodiment C32] The compound N of the present invention wherein X^{a} represents a nitrogen atom, and R^{4a} represents a C1-C6 chain hydrocarbon group which may optionally have one or more substituents selected from Group A^{a}, OR^{23a}, a cyano group, a nitro group, a hydroxy group, or a halogen atom.
[Embodiment C33] The compound N of the present invention wherein the neighboring R^{2a} and R^{4a} combine together with a carbon atom to which they are attached to form a C5-C6 alicyclic hydrocarbon, a five (5)- to six (6)- membered non-aromatic heterocyclic ring {the C5-C6 alicyclic hydrocarbon, and the five (5)- to six (6)- membered non-aromatic heterocyclic ring may optionally have one or more substituents selected from Group B^{a}}.
[Embodiment C34] The compound N of the present invention wherein the neighboring R^{2a} and R^{4a} combine together with a carbon atom to which they are attached to form a benzene ring, or a five (5)- to six (6)- membered aromatic heterocyclic ring {the benzene ring, and the five (5)- to six (6)- membered aromatic heterocyclic ring may optionally have one or more substituents selected from Group E^{a}}.
[Embodiment C35] The compound N of the present invention wherein the neighboring R^{4a} and R^{4a} combine together with a carbon atom to which they are attached to form a C5-C6 alicyclic hydrocarbon, a five (5)- to six (6)- membered non-aromatic heterocyclic ring {the C5-C6 alicyclic hydrocarbon, and the five (5)- to six (6)- membered non-aromatic heterocyclic ring may optionally have one or more substituents selected from group B^{a}}.
[Embodiment C36] The compound N of the present invention wherein the neighboring R^{4a} and R^{4a} combine together with a carbon atom to which they are attached to form a benzene ring, or five (5)- to six (6)- membered aromatic heterocyclic ring {the benzene ring, and the five (5)- to six (6)-membered aromatic heterocyclic ring may optionally have one or more substituents selected from group E^{a}}.
[Embodiment C37] The compound N of the present invention wherein R^{5a}, and R^{8a} are identical to or different from each other, and each represents a C1-C6 chain hydrocarbon group which may be optionally substituted with one or more halogen atoms, or a hydrogen atom.
[Embodiment C38] The compound N of the present invention wherein R^{5a}, and R^{8a} are identical to or different from each other, and each represents a C3-C8 alicyclic hydrocarbon group which may optionally have one or more substituents selected from Group B^{a}, or a hydrogen atom.
[Embodiment C39] The compound N of the present invention wherein R^{5a}, and R^{8a} are identical to or different from each other, and each represents OR^{7a}, SR^{9a}, S(O)R^{10a}, S(O)₂R^{11a}, NR^{12a}R^{13a}, C(O)R^{14a}, a nitro group, a cyano group, or a hydrogen atom.
[Embodiment C40] The compound N of the present invention wherein R^{5a}, and R^{8a} are identical to or different from each other, and each represents a halogen atom, or a hydrogen atom.
[Embodiment C41] The compound N of the present invention wherein R^{5a}, and R^{8a} represent a hydrogen atom.
[Embodiment C42] The compound N of the present invention wherein R^{5a}, and R^{8a} are identical to or different from each other, and each represents a C1-C6 chain hydrocarbon group which may be optionally substituted with one or more halogen atoms, a halogen atom, or a hydrogen atom.
[Embodiment C43] The compound N of the present invention wherein na is 0.
[Embodiment C44] The compound N of the present invention wherein na is 1.
[Embodiment C45] The compound N of the present invention wherein na is 2.
[Embodiment C46] The compound N of the present invention wherein qa is 1.
[Embodiment C47] The compound N of the present invention wherein qa is 2.
[Embodiment C48] The compound N of the present invention wherein qa is 3.
[Embodiment C49] The compound N of the present invention wherein R^{5a}, R^{6a}, and R^{8a} represent a hydrogen atom.
[Embodiment C50] The compound N of the present invention wherein R^{2a}, and R^{3a} represent a hydrogen atom.
[Embodiment C51] The compound N of the present invention wherein R^{1a} represents a C2-C6 alkyl group which may optionally have one or more substituents selected from Group A^{a}, or a methyl group which may optionally have one or more substituents selected from Group C^{a}, R^{2a} and R^{3a} are identical to or different from each other, and each represents a C1-C6 chain hydrocarbon group which may be optionally substituted with one or more halogen atoms, a halogen atom, or a hydrogen atom, R^{4a} represents a methyl group, a C2-C6 chain hydrocarbon group which may optionally have one or more substituents selected from Group A^{a}, OR^{23a}, a cyano group, a nitro group, or a halogen atom, and R^{5a}, R^{6a}, and R^{8a} represents a hydrogen atom.

Embodiments of the compound E of the present invention include the following compounds.

[Embodiment E1] The compound E of the present invention wherein R^{2b} and R^{3b} represent a hydrogen atom.
[Embodiment E2] The compound E of the present invention wherein R^{2b} and R^{3b} are identical to or different from each other, and each represents a halogen atom, or a hydrogen atom.
[Embodiment E3] The compound E of the present invention wherein R^{2b} and R^{3b} are identical to or different from each other, and each represents a C1-C6 chain hydrocarbon group which may be optionally substituted with one or more halogen atoms, or a hydrogen atom.
[Embodiment E4] The compound E of the present invention wherein R^{2b} and R^{3b} are identical to or different from each other, and each represents a C1-C6 chain hydrocarbon group which may be optionally substituted with one or more halogen atoms, a halogen atom, or a hydrogen atom.
[Embodiment E5] The compound E of the present invention wherein X^{b} represents a nitrogen atom, R^{4b} represents a methyl group which may optionally have one or more substituents selected from Group F^{b}, a CH₂F group, a CHF₂ group, a C2-C3 chain hydrocarbon group which may optionally have one or more substituents selected from Group A^{b}, a C3-C8 alicyclic hydrocarbon group, three (3)- to eight (8)- membered non-aromatic heterocyclic group {the C3-C8 alicyclic hydrocarbon group, and the three (3)- to eight (8)- membered non-aromatic heterocyclic group may optionally have one or more substituents selected from Group B^{b}}, a phenyl group, a five (5)- to six (6)- membered aromatic heterocyclic group {the phenyl group, and the five (5)- to six (6)- membered aromatic heterocyclic group may optionally have one or more substituents selected from Group D^{b}}, OR^{23b}, SR^{24b}, S(O)R^{25b}, S(O)₂R^{22b}, NR^{27b}R^{28b}, C(O)R^{29b}, C(R^{30b})=NOR^{31b}, a cyano group, a nitro group, a hydroxy group, an amino group, or a halogen atom, and R^{6b} represents a C1-C6 chain hydrocarbon group which may be optionally substituted with one or more halogen atoms, a C3-C8 alicyclic hydrocarbon group which may optionally have one or more substituents selected from Group B^{b}, OR^{7b}, SR^{9b}, S(O)R^{10b}, S(O)₂R^{11b}, NR^{12b}R^{13b}, C(O)R^{14b}, a nitro group, a halogen atom, or a hydrogen atom.
[Embodiment E6] The compound E of the present invention wherein X^{b} represents CR^{5b}, R^{4b} represents a methyl group which may optionally have one or more substituents selected from Group F^{b}, a CH₂F group, a CHF₂ group, a C2-C3 chain hydrocarbon group which may optionally have one or more substituents selected from Group A^{b}, a C3-C8 alicyclic hydrocarbon group, a three (3)- to eight (8)- membered non-aromatic heterocyclic group {the C3-C8 alicyclic hydrocarbon group, and the three (3)- to eight (8)- membered non-aromatic heterocyclic group may optionally have one or more substituents selected from Group B^{b}}, a phenyl group, a five (5)- to six (6)- membered aromatic heterocyclic group {the phenyl group, and the five (5)- to six (6)- membered aromatic heterocyclic group may optionally have one or more substituents selected from Group D^{b}}, OR^{32b}, SR^{24b}, S(O)R^{25b}, S(O)₂R^{22b}, NR^{27b}R^{28b}, C(O)R^{29b}, C(R^{30b})=NOR^{31b}, a cyano group, a nitro group, a hydroxy group, or a halogen atom, and R^{6b} represents C1-C6 chain hydrocarbon group which may be optionally substituted with one or more halogen atoms, a C3-C8 alicyclic hydrocarbon group which may optionally have one or more substituents selected from Group B^{b}, OR^{7b}, a nitro group, a fluorine atom, a bromine atom, an iodine atom, or a hydrogen atom.
[Embodiment E7] The compound E of the present invention wherein R^{6b} represents a C1-C6 chain hydrocarbon group which may be optionally substituted with one or more halogen atoms, or a hydrogen atom.
[Embodiment E8] The compound E of the present invention wherein X^{b} represents CR^{5b}, and R^{6b} represents a fluorine atom, a bromine atom, an iodine atom, or a hydrogen atom.
[Embodiment E9] The compound E of the present invention wherein X^{b} represents a nitrogen atom, and R^{6b} represents a halogen atom, or a hydrogen atom.
[Embodiment E10] The compound E of the present invention wherein R^{6b} represents a hydrogen atom.
[Embodiment E11] The compound E of the present invention wherein R^{4b} represents a methyl group which may optionally have one or more substituents selected from Group F^{b}, a CH₂F group, a CHF₂ group, or a C2-C3 chain hydrocarbon group which may optionally have one or more substituents selected from Group A^{b}.
[Embodiment E12] The compound E of the present invention wherein R^{4b} represents a halogen atom.
[Embodiment E13] The compound E of the present invention wherein R^{4b} represents a methyl group which may optionally have one or more substituents selected from Group F^{b}, a C2-C3 chain hydrocarbon group which may optionally have one or more substituents selected from Group A^{b}, or a halogen atom.
[Embodiment E14] The compound E of the present invention wherein the neighboring R^{2b} and R^{4b} combine together with a carbon atom to which they are attached to form a C5-C6 alicyclic hydrocarbon, or a five (5)- to six (6)- membered non-aromatic heterocyclic ring {the C5-C6 alicyclic hydrocarbon, and the five (5)- to six (6)- membered non-aromatic heterocyclic ring may optionally have one or more substituents selected from Group B^{b}}.
[Embodiment E15] The compound E of the present invention wherein the neighboring R^{2b} and R^{4b} combine together with a carbon atom to which they are attached to form a benzene ring, or five (5)- to six (6)- membered aromatic heterocyclic ring {the benzene ring, and the five (5)- to six (6)-membered aromatic heterocyclic ring may optionally have one or more substituents selected from Group E^{b}}.
[Embodiment E16] The compound E of the present invention wherein two of neighboring R^{4b} and R^{4b} combine together with a carbon atom to which they are attached to form a C5-C6 alicyclic hydrocarbon, or a five (5)- to six (6)- membered non-aromatic heterocyclic ring {the C5-C6 alicyclic hydrocarbon, and the five (5)- to six (6)- membered non-aromatic heterocyclic ring may optionally have one or more substituents selected from Group B^{b}}.
[Embodiment E17] The compound E of the present invention wherein two of neighboring R^{4b} and R^{4b} combine together with a carbon atom to which they are attached to form a benzene ring, or as five (5)- to six (6)- membered aromatic heterocyclic ring {the benzene ring, and the five (5)- to six (6)- membered aromatic heterocyclic ring may optionally have one or more substituents selected from Group E^{b}}.
[Embodiment E18] The compound E of the present invention wherein R^{5b}, and R^{8b} are identical to or different from each other, and each represents a C1-C6 chain hydrocarbon group which may be optionally substituted with one or more halogen atoms, or hydrogen atom.
[Embodiment E19] The compound E of the present invention wherein R^{5b}, and R^{8b} are identical to or different from each other, and each represents a halogen atom, or a hydrogen atom.
[Embodiment E20] The compound E of the present invention wherein R^{5b}, and R^{8b} represents a hydrogen atom.
[Embodiment E21] The compound E of the present invention wherein R^{5b}, and R^{8b} are identical to or different from each other, and each represents a C1-C6 chain hydrocarbon group which may be optionally substituted with one or more halogen atoms, a halogen atom, or a hydrogen atom.
[Embodiment E22] The compound E of the present invention wherein nb is 0.
[Embodiment E23] The compound E of the present invention wherein nb is 1.
[Embodiment E24] The compound E of the present invention wherein nb is 2.
[Embodiment E25] The compound E of the present invention wherein qb is 1.
[Embodiment E26] The compound E of the present invention wherein qb is 2.
[Embodiment E27] The compound E of the present invention wherein qb is 3.
[Embodiment E28] The compound E of the present invention wherein R^{5b}, R^{6b}, and R^{8b} represent a hydrogen atom.
[Embodiment E29] The compound E of the present invention wherein R^{1b} represents a phenyl group which may optionally have one or more substituents selected from Group H^{b}.
   Group H^{b} is a group consisting of a C1-C6 chain hydrocarbon group, a C1-C6 alkoxy group {the C1-C6 chain hydrocarbon group, and the C1-C6 alkoxy group may be optionally substituted with one or more halogen atoms}, and a halogen atom.
[Embodiment E30] The compound E of the present invention wherein R^{1b} represents a phenyl group which may be optionally substituted with one or more halogen atoms.
[Embodiment E31] The compound E of the present invention wherein R^{4b} represents a C1-C3 chain hydrocarbon group, or a halogen atom, and R^{2b}, R^{3b}, R^{5b}, R^{6b}, and R^{8b} represent a hydrogen atom.

Embodiments of the compound F of the present invention include the following compounds.

[Embodiment F1] The compound F of the present invention wherein R^{1c} represents a three (3)- to eight (8)- membered non-aromatic heterocyclic group which may optionally have one or more substituents selected from Group B^{c}, a three (3)-to eight (8)- membered non-aromatic heterocyclic group.
[Embodiment F2] The compound F of the present invention wherein R^{1c} represents a three (3)- to eight (8)- membered non-aromatic heterocyclic group which may be optionally substituted with one or more halogen atoms.
[Embodiment F3] The compound F of the present invention wherein R^{1c} represents NR^{33c}R^{34c}.
[Embodiment F4] The compound F of the present invention wherein R^{1c} represents NR^{33c}R^{34c}, R^{33c} represents a C1-C6 chain hydrocarbon group, a C3-C6 cycloalkyl group, or a hydrogen atom, and R^{34c} represents a C1-C6 chain hydrocarbon group, or a s C3-C6 cycloalkyl group.
[Embodiment F5] The compound F of the present invention wherein R^{2c} and R^{3c} represent a hydrogen atom.
[Embodiment F6] The compound F of the present invention wherein R^{2c} and R^{3c} are identical to or different from each other, and each represents a halogen atom, or a hydrogen atom.
[Embodiment F7] The compound F of the present invention wherein R^{2c} and R^{3c} are identical to or different from each other, and each represents a C1-C6 chain hydrocarbon group which may be optionally substituted with one or more halogen atoms, or a hydrogen atom.
[Embodiment F8] The compound F of the present invention wherein R^{2c} and R^{3c} are identical to or different from each other, and each represents a C1-C6 chain hydrocarbon group which may be optionally substituted with one or more halogen atoms, a halogen atom, or a hydrogen atom.
[Embodiment F9] The compound F of the present invention wherein X^{c} represents a nitrogen atom.
[Embodiment F10] The compound F of the present invention wherein X^{c} represents CR^{5c}.
[Embodiment F11] The compound F of the present invention wherein R^{6c} represents a C1-C6 chain hydrocarbon group which may be optionally substituted with one or more halogen atoms, or a hydrogen atom.
[Embodiment F12] The compound F of the present invention wherein R^{6c} represents a halogen atom, or a hydrogen atom.
[Embodiment F13] The compound F of the present invention wherein R^{6c} represents a hydrogen atom.
[Embodiment F14] The compound F of the present invention wherein R^{4c} represents a C1-C6 chain hydrocarbon group which may optionally have one or more substituents selected from Group A^{c}.
[Embodiment F15] The compound F of the present invention wherein R^{4c} represents a halogen atom.
[Embodiment F16] The compound F of the present invention wherein R^{4c} represents a C1-C6 chain hydrocarbon group which may optionally have one or more substituents selected from Group A^{c}, or a halogen atom.
[Embodiment F17] The compound F of the present invention wherein the neighboring R^{2c} and R^{4c} combine together with a carbon atom to which they are attached to form a C5-C6 alicyclic hydrocarbon, or a five (5)- to six (6)- membered non-aromatic heterocyclic ring {the C5-C6 alicyclic hydrocarbon, and the five (5)- to six (6)- membered non-aromatic heterocyclic ring may optionally have one or more substituents selected from Group B^{c}}.
[Embodiment F18] The compound F of the present invention wherein the neighboring R^{2c} and R^{4c} combine together with a carbon atom to which they are attached to form a benzene ring, or a five (5)- to six (6)- membered aromatic heterocyclic ring {the benzene ring, and the five (5)- to six (6)- membered aromatic heterocyclic ring may optionally have one or more substituents selected from Group E^{c}}.
[Embodiment F19] The compound F of the present invention wherein two of neighboring R^{4c} and R^{4c} combine together with a carbon atom to which they are attached to form a C5-C6 alicyclic hydrocarbon, or a five (5)- to six (6)- membered non-aromatic heterocyclic ring {the C5-C6 alicyclic hydrocarbon, and the five (5)- to six (6)- membered non-aromatic heterocyclic ring may optionally have one or more substituents selected from Group B^{c}}.
[Embodiment F20] The compound F of the present invention wherein two of neighboring R^{4c} and R^{4c} combine together with a carbon atom to which they are attached to form a benzene ring, or a five (5)- to six (6)- membered aromatic heterocyclic ring {the benzene ring, and the five (5)- to six (6)- membered aromatic heterocyclic ring may optionally have one or more substituents selected from Group E^{c}}.
[Embodiment F21] The compound F of the present invention wherein R^{5c}, and R^{8c} are identical to or different from each other, and each represents a C1-C6 chain hydrocarbon group which may be optionally substituted with one or more halogen atoms, or a hydrogen atom.
[Embodiment F22] The compound F of the present invention wherein R^{5c}, and R^{8c} are identical to or different from each other, and each represents a halogen atom, or a hydrogen atom.
[Embodiment F23] The compound F of the present invention wherein R^{5c}, and R^{8c} represents a hydrogen atom.
[Embodiment F24] The compound F of the present invention wherein R^{5c}, and R^{8c} are identical to or different from each other, and each represents a C1-C6 chain hydrocarbon group which may be optionally substituted with one or more halogen atoms, a halogen atom, or a hydrogen atom.
[Embodiment F25] The compound F of the present invention wherein nc is 0.
[Embodiment F26] The compound F of the present invention wherein nc is 1.
[Embodiment F27] The compound F of the present invention wherein nc is 2.
[Embodiment F28] The compound F of the present invention wherein qc is 1.
[Embodiment F29] The compound F of the present invention wherein qc is 2.
[Embodiment F30] The compound F of the present invention wherein qc is 3.
[Embodiment F31] The compound F of the present invention wherein R^{5c}, R^{6c}, and R^{8c} represent a hydrogen atom.
[Embodiment F32] The compound F of the present invention wherein X^{c} represents CR^{5c}, R^{4c} represents a C1-C3 chain hydrocarbon group, or a halogen atom, R^{2c}, R^{3c}, R^{5c}, R^{6c}, and R^{8c} represents a hydrogen atom.
[Embodiment F33] The compound F of the present invention wherein R^{5c}, R^{6c} and R^{8c} are identical to or different from each other, and each represents a methyl group, a halogen atom, or a hydrogen atom.
[Embodiment F34] The compound F of the present invention wherein R^{5c}, R^{6c} and R^{8c} represent a hydrogen atom.
[Embodiment F35] The compound F of the present invention wherein R^{1c} represents a four (4)- to six (6)- membered non-aromatic heterocyclic group, or NR^{33c}R^{34c}, R^{33c} represents a C1-C6 chain hydrocarbon group, a C3-C6 cycloalkyl group, or a hydrogen atom, R^{34c} represents a C1-C6 chain hydrocarbon group, or a C3-C6 cycloalkyl group, X^{c} represents CR^{5c}, R^{5c}, R^{6c} and R^{8c} represent a hydrogen atom, nc is 2, and R^{4c} represents a C1-C6 chain hydrocarbon group which may optionally have one or more substituents selected from Group A^{c}, or a halogen atom.

Embodiments of the intermediate compound A include the following compounds.

[Embodiment G1] The intermediate compound A wherein R^{1d} represents a propyl group, a butyl group, a cyclobutyl group, a cyclopentyl group, or a five (5)- to six (6)- membered aromatic heterocyclic group, and X^{d} represents a bromine atom.
[Embodiment G2] The intermediate compound A wherein X^{1d} represents CH or a nitrogen atom, nd is 1 or 2, and R^{1d} represents a propyl group, a butyl group, a cyclobutyl group, a cyclopentyl group, or a five (5)- to six (6)- membered aromatic heterocyclic group.
[Embodiment G3] The intermediate compound A wherein X^{1d} represents CH, nd is 1 or 2, and R^{1d} represents a propyl group, a butyl group, a cyclopentyl group, or a five (5)- to six (6)- membered aromatic heterocyclic group.
[Embodiment G4] The intermediate compound A wherein X^{1d} represents a nitrogen atom, and nd is 2.
[Embodiment G5] The intermediate compound A wherein the combination of X^{1d}, nd and R¹⁴ represents
   a combination wherein X^{1d} represents CH, nd is 1 or 2, and R^{1d} represents a propyl group, a butyl group, a cyclobutyl group, a cyclopentyl group, or a five (5) - to six (6)-membered aromatic heterocyclic group; or
   a combination wherein X^{1d} represents a nitrogen atom, nd is 1 or 2, and R^{1d} represents a C2-C4 alkyl group, a C3-C6 alicyclic hydrocarbon group, or a five (5)- to six (6)-membered aromatic heterocyclic group {the five (5)- to six (6)- membered aromatic heterocyclic group may optionally have one or more substituents selected from Group V¹}.

Next, a process for preparing the present compound Y (including the compound Z of the present invention) is explained.

### Process A

A compound represented by formula (I-b) (hereinafter, referred to as "Compound (I-b)") or a compound represented by formula (I-c) (hereinafter, referred to as "Compound (I-c)") can be prepared by reacting a compound represented by formula (I-a) (hereinafter, referred to as "Compound (I-a)") with an oxidizing agent. 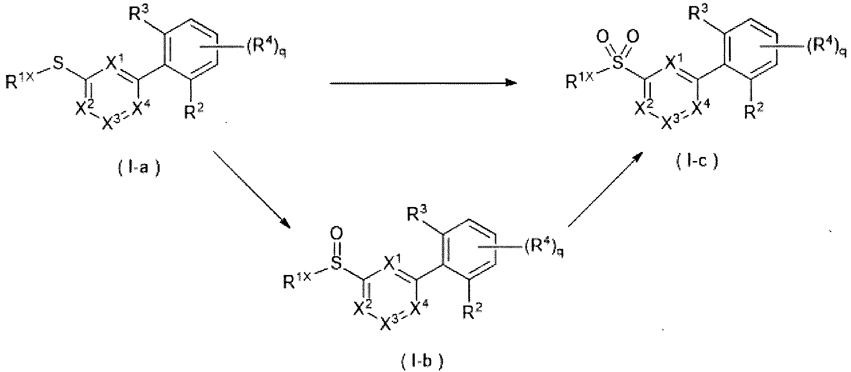 [wherein R^{1X} represents a C1-C6 alkyl group, a C2-C6 alkynyl group {the C1-C6 alkyl group, and the C2-C6 alkynyl group may optionally have one or more substituents selected from Group A}, a C2-C6 alkenyl group which may optionally have one or more substituents selected from Group C, a C3-C8 alicyclic hydrocarbon group, a three (3)- to eight (8)-membered non-aromatic heterocyclic group {the C3-C8 alicyclic hydrocarbon group, and the three (3)- to eight (8)- membered non-aromatic heterocyclic group may optionally have one or more substituents selected from Group B}, a C6-C10 aryl group which may optionally have one or more substituents selected from group F, or a five (5)- to ten (10)- membered aromatic heterocyclic group which may optionally have one or more substituents selected from Group D, and the other symbols have the same definitions as the above.]

First, a process for preparing the compound (I-a) from the compound (I-b).

The reaction is usually carried out in a solvent. Examples of the solvent to be used in the reaction include halogenated hydrocarbons such as dichloromethane, chloroform and chlorobenzene (hereinafter, collectively referred to as "halogenated hydrocarbons"); nitriles such as acetonitrile and propionitrile (hereinafter, collectively referred to as "nitriles"); alcohols such as methanol and ethanol (hereinafter, collectively referred to as "alcohols"); acetic acid; water; and mixed solvents comprising two or more of these solvents.

Examples of the oxidizing agent to be used in the reaction include sodium periodate, m-chloroperoxy benzoic acid (hereinafter, abbreviated as "mCPBA"), and hydrogen peroxide.

When a hydrogen peroxide is used as the oxidizing agent, if necessary, a base or a catalyst may be added.

Examples of the base to be used in the reaction include sodium carbonate. When the base is used in the reaction, the base is used usually within a range of 0.01 to 1 molar ratio(s), as opposed to 1 mole of the compound (I-a).

Examples of the catalysts to be used in the reaction include sodium tungstate. When the catalyst is used in the reaction, the catalyst is used within a range of 0.01 to 0.5 molar ratios as opposed to 1 mole of the compound (I-a).

In the reaction, the oxidizing agent is used usually within a range of 1 to 1.2 molar ratio(s) as opposed to 1 mole of the compound (I-a).

The reaction temperature is usually within a range of -20 to 80 °C. The reaction period of the reaction is usually within a range of 0.1 to 12 hours.

When the reaction is completed, water is added to the reaction mixtures, and the mixtures are extracted with organic solvent(s), and if necessary, the resulting organic layer is washed with an aqueous solution of a reducing agent (such as sodium sulfite, sodium thiosulfate) and an aqueous solution of a base (such as sodium hydrogen carbonate). The organic layers can be then dried and concentrated to obtain the compound (I-b).

Next, a process for preparing the compound (I-c) from the compound (I-b) is described.

The reaction is usually carried out in a solvent. Examples of the solvent to be used in the reaction include halogenated hydrocarbons, nitriles, alcohols, acetic acid, water, and mixed solvents comprising two or more of these solvents.

Examples of the oxidizing agent to be used in the reaction include mCPBA and hydrogen peroxide.

When the hydrogen peroxide is used as the oxidizing agent, if necessary, a base or a catalyst may be added.

Examples of the base to be used in the reaction include sodium carbonate. When the base is used in the reaction, the base is used usually within a range of 0.01 to 1 molar ratio(s) as opposed to 1 mole of the compound (I-b).

Examples of the catalyst to be used in the reaction include sodium tungstate. When the catalyst is used in the reaction, the catalyst is usually used within a range of 0.01 to 0.5 molar ratios as opposed to 1 mole of the compound (I-b).

In the reaction, the oxidizing agent is usually used within a range of 1 to 2 molar ratio(s) as opposed to 1 mole of the compound (I-b).

The reaction temperature is usually within a range of -20 to 120 °C. The reaction period of the reaction is usually within a range of 0.1 to 12 hours.

When the reaction is completed, water is added to the reaction mixtures, and the mixtures are extracted with organic solvent(s), and if necessary, the resulting organic layer is washed with an aqueous solution of a reducing agent (such as sodium sulfite, sodium thiosulfate) and an aqueous solution of a base (such as sodium hydrogen carbonate). The organic layers can be then dried and concentrated to obtain the compound (I-c).

Also, the compound (I-c) can be prepared by reacting the compound (I-a) with an oxidizing agent in one step reaction (that is, one-pot reaction).

The reaction can be carried out by using the oxidizing agent within a ratio of 2 to 5 molar ratios as opposed to 1 mol of the compound (I-a) according to the process for preparing the compound (I-c) from the compound (I-b).

### Process B

The compound (I-a) can be prepared by reacting a compound represented by formula (B1) (hereinafter, referred to as "Compound (B1)") with a compound represented by formula (M1) (hereinafter, referred to as "Compound (M1)") in the presence of a base. 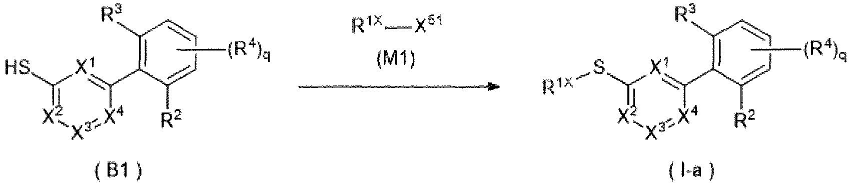 [wherein X⁵¹ represents a chlorine atom, a bromine atom, an iodine atom, a mesyloxy group, or a trifuryloxy group, and the other symbols have the same definitions as the above.]

The reaction is usually carried out in a solvent. Examples of the solvent to be used in the reaction include hydrocarbon such as hexane, toluene, xylene (hereinafter, collectively referred to as "hydrocarbons"); ethers such as methyl tert-butyl ether (hereinafter, referred to as "MTBE"), tetrahydrofuran (hereinafter, referred to as "THF"), and dimethoxyethane (hereinafter, referred to as "DME") (hereinafter, collectively referred to as "ethers"); halogenated hydrocarbons; amides such as dimethylformamide (hereinafter, referred to as "DMF") and N-methyl pyrrolidone (hereinafter, referred to as "NMP") (hereinafter, collectively referred to as "amides"); esters such as methyl acetate and ethyl acetate (hereinafter, collectively referred to as "esters"); nitriles; water; and mixed solvents comprising two or more of these solvents.

Examples of the bases include organic bases such as triethylamine and pyridine (hereinafter, collectively referred to as "organic bases"); alkali metal carbonates such as sodium carbonate and potassium carbonate (hereinafter, collectively referred to as "alkali metal carbonates"); alkali metal hydrocarbonates such as sodium hydrogen carbonate, potassium hydrocarbonate (hereinafter, collectively referred to as "alkali metal hydrocarbonates"); sodium hydride and tripotassium phosphate.

If necessary, a metal catalyst, and/or a ligand may be used in the reaction.

Examples of the metal catalyst include copper catalysts (such as copper (I) iodide, copper (I) bromide, copper (I) chloride, copper (I) oxide, copper (I) trifluoromethane sulfonate benzene complex, tetrakis(acetonitrile) copper (I) hexafluorophosphate, copper (I) 2-thiophenecarboxylate; nickel catalysts (such as bis(cyclooctadiene) nickel(0), nickel(II) chloride); palladium catalysts (such as palladium (II) acetate, tris(dibenzylideneacetone)dipalladium(0), tetrakis(triphenylphosphine) palladium (0), and [1,1'-bis(diphenylphosphino)ferrocene palladium (II) dichloride]). When the metal catalyst is used in the reaction, the metal catalyst is usually used within a range of 0.01 to 1 molar ratio(s) as opposed to 1 mole of the compound (B1).

Examples of the ligand include triphenylphosphine, Xantphos, 2,2'-bis(diphenylphosphino)-1,1'-binaphthyl, 1,1'-bis(diphenylphosphino)ferrocene, 2-dicyclohexylphosphino-2',4',6'-triisopropylbiphenyl, 2-dicyclohexylphosphino-2',6'-dimethoxybiphenlnyl, 1,2-bis (diphenylphosphino) ethane, 2,2'-bipyridine, 2-aminoethanol, 8-hydroxyqunoline, 1,10-phenanthroline, trans-1,2-cyclohexane diamine, trans-N,N'-dimethylcyclohexane-1,2-diamine, N,N'-dimethylethylenediamine, and N,N-dimethylglycine hydrochloride salt. When the ligand is used in the reaction, the ligand is usually used within a range of 0.01 to 1 molar ratio(s) as opposed to 1 mole of the compound (B1).

In the reaction, the compound (M1) is usually used within a range of 1 to 10 molar ratio(s), and the base is usually used within a range of 1 to 10 molar ratio(s), as opposed to 1 mole of the compound (B1).

The reaction temperature in the reaction is usually within a range of 0 to 150 °C. The reaction period in the reaction is usually within a range of 0.1 to 48 hours.

When the reaction is completed, water is added to the reaction mixture, and the resulting mixture was extracted with organic solvent(s), and the organic layer is worked up (for example, drying and concentration) to isolate the compound (I-a).

The compound (M1) is publicly known, or can be prepared according to a publicly known method.

### Process C

The compound represented by formula (I) can be prepared by reacting a compound represented by formula (B2) (hereinafter, referred to as "Compound (B2)") with a compound represented by formula (M2) (hereinafter, referred to as "Compound (M2)") in the presence of a palladium catalyst and a base. 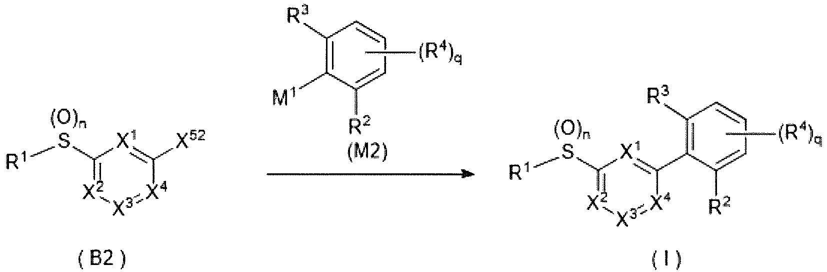 [wherein X⁵² represents a chlorine atom, a bromine atom, an iodine atom, and a trifuryloxy group, M¹ represents B(OH)₂, or 4,4,5,5-tetamethyl-1,3,2-dioxaborolan-2-yl group, and the other symbols have the same definitions as the above.]

The reaction is carried out in a solvent. Examples of the solvent to be used in the reaction include hydrocarbons, ethers, halogenated hydrocarbons, amides, esters, nitriles, dimethyl sulfoxide (hereinafter, referred to as "DMSO"), water, and mixed solvents of two or more of these solvents.

Examples of the palladium catalyst to be used in the reaction include palladium (II) acetate, and [1,1'-bis(diphenylphosphino)ferrocene] palladium (II) dichloride.

Examples of the base to be used in the reaction include organic bases, alkali metal carbonates, alkali metal hydrocarbonates, sodium fluoride, and tripotassium phosphate.

In the reaction, the compound (M2) is usually used within a range of 0.5 to 2 molar ratios, and the palladium catalyst is usually used within a range of 0.01 to 0.3 molar ratios, and the base is usually used within a range of 1 to 10 molar ratio(s), as opposed to 1 mole of the compound (B2).

The reaction temperature in the reaction is usually within a range of 0 to 150 °C. The reaction period in the reaction is usually within a range of 0.1 to 120 hours.

When the reaction is completed, water is added to the reaction mixture, and the organic layer is worked up (for example, drying and concentration) to isolate the compound represented by formula (I).

The compound (M2) is publicly known, or can be prepared according to a publicly known method.

### Process D

The compound represented by formula (I) can be prepared by reacting a compound represented by formula (B3) (hereinafter, referred to as "Compound (B3)") with a compound represented by formula (M3) (hereinafter, referred to as "Compound (M3)") in the presence of a palladium catalyst and a base. 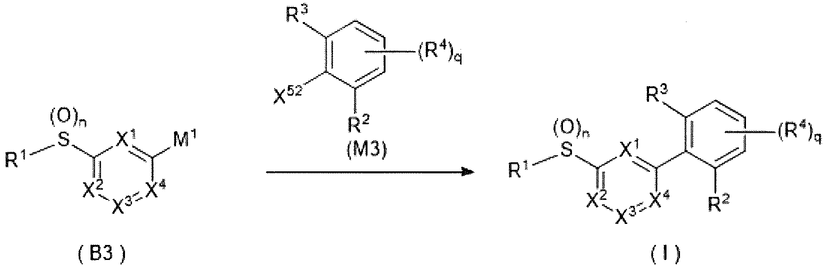 [wherein the symbols have the same definitions as the above.]

The reaction can be carried out by using the compound (B3) in place of the compound (B2) and using the compound (M3) in place of the compound (M2) according to the process C.

The compound (M3) is publicly known, or can be prepared according to a publicly known method.

### Process E

The compound (I-a) can be prepared by reacting a compound represented by formula (B4) (hereinafter, referred to as "Compound (B4)") with a compound represented by formula (M4) (hereinafter, referred to as "Compound (M4)") in the presence of a base. 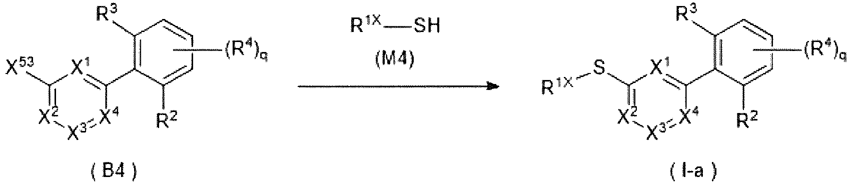 [wherein X⁵³ represents a fluorine atom, a chlorine atom, a bromine atom, an iodine atom, a mesyloxy group or a trifuryloxy group, and the other symbols have the same definitions as the above.]

The reaction is usually carried out in a solvent. Examples of the solvent to be used in the reaction include alcohols, nitriles, ethers, aromatic hydrocarbons, amides, water, and mixed solvents comprising two or more of these solvents.

Examples of the base to be used in the reaction include alkali metal hydrides, alkali metal carbonates, organic bases, sodium hydride, and tripotassium phosphate.

When X⁵³ represents a bromine atom, an iodine atom, or a trifuryloxy group, if necessary, a metal catalyst and a ligand may be added.

Examples of the metal catalyst to be used in the reaction include palladium catalysts such as palladium (II) acetate, and [1,1'-bis(diphenylphosphino)ferrocene] palladium (II) dichloride); nickel catalysts such as bis(cyclooctadiene)nickel (0) and nickel (II) chloride); and copper catalysts such as copper (I) iodide, and copper (I) chloride. When the metal catalyst is used in the reaction, the metal catalyst is usually used within a range of 0.01 to 1 molar(s) as opposed to 1 mole of the compound (B4).

Examples of the ligand to be used in the reaction include triphenylphosphine, Xantphos, 2,2'-bis(diphenylphosphino)-1,1'-binaphthyl, 1,1'-bis(diphenylphosphino)ferrocene, 2-dicyclohexylphosphino-2',4',6'-triisopropylbiphenyl, 2-dicyclohexylphosphino-2',6'-dimethoxybiphenlnyl, 1,2-bis (diphenylphosphino)ethane, 2,2'-bipyridine, 2-aminoethanol, 8-hydroxyqunoline, and 1,10-phenanthroline. When the ligand is used in the reaction, the ligand is usually used within a range of 0.01 to 1 molar ratio(s) as opposed to 1 mole of the compound (B4).

In the reaction, the compound (M4) is usually used within a range of 1 to 10 molar ratio(s), and the base is usually used within a range of 1 to 10 molar(s), as opposed to 1 mole of the compound (B4).

The reaction temperature in the reaction is usually within a range of -20 to 200 °C. The reaction period in the reaction is usually within a range of 0.1 to 72 hours.

When the reaction is completed, water is added to the reaction mixture, and the organic layer is extracted with organic solvent(s), and the organic layer is worked up (for example, drying and concentration) to obtain the compound (I-a).

The compound is publicly known, or can be prepared according to a publicly known method.

### Process F

The compound (I-c) can be prepared by reacting the compound (B4) with a compound represented by formula (M5) (hereinafter, referred to as "Compound (M5)"). 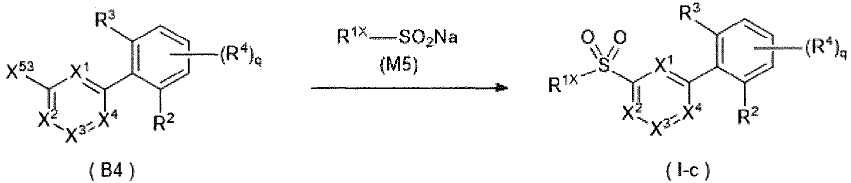 [wherein the symbols have the same definitions as the above.]

The reaction is usually carried out in a solvent. Examples of the solvents to be used in the reaction include hydrocarbons, ethers, halogenated hydrocarbons, amides, esters, nitriles, DMSO, water, and mixed solvents of two or more of these solvents.

In the reaction, the compound (M5) is usually used within a range of 1 to 3 molar ratio(s), as opposed to 1 mole of the compound (B4).

The reaction temperature in the reaction is usually within a range of 0 to 150 °C. The reaction period in the reaction is usually within a range of 0.1 to 48 hours.

When the reaction is completed, water is added to the reaction mixture, and the resulting mixture is extracted with organic solvent(s), and the organic layer is worked up (for example, drying and concentration) to isolate the compound (I-c).

The compound (M5) is publicly known, or can be prepared according to a publicly known method.

### Process G

A compound represented by formula (I-d) (hereinafter, referred to as "Compound (I-d)" can be prepared by heating the compound represented by formula (B5) (hereinafter referred to as "Compound (B5)"). 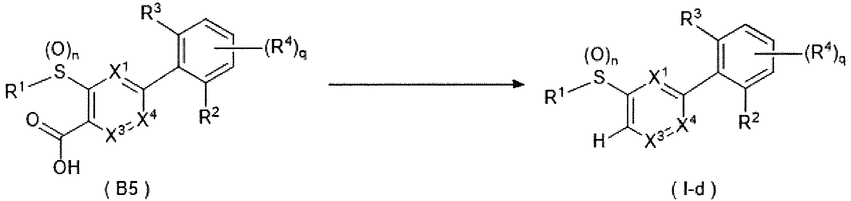 [wherein the symbols have the same definitions as the above.]

The reaction is usually carried out in a solvent. Examples of the solvents to be used in the reaction include hydrocarbons, alcohols, nitriles, ethers, esters, aromatic hydrocarbons, amides, DMSO, water, and mixed solvents of two or more of these solvents.

The reaction temperature in the reaction is usually within a range of 80 to 200 °C. The reaction period in the reaction is usually within a range of 0.1 to 24 hours.

When the reaction is completed, water is added to the reaction mixture, and the resulting mixture is extracted with organic solvent(s), and the organic layer is worked up (for example, drying and concentration) to isolate the compound (I-d).

The compound (B5) is publicly known, or can be prepared according to a publicly known method.

### Process H

A compound represented by formula (I-e) (hereinafter, referred to as "Compound (I-e)") can be prepared by reacting a compound represented by formula (B6) (hereinafter, referred to as "Compound (B6)") with a compound represented by formula (M6) (hereinafter, referred to as "Compound (M6)") in the presence of a base. 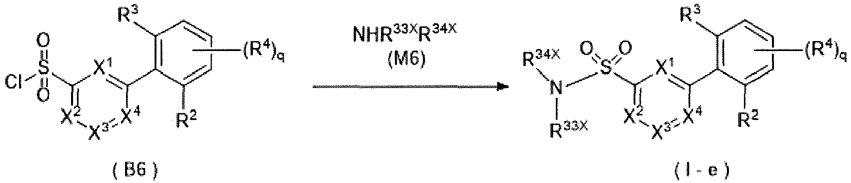 [wherein R^{33X} and R^{34X} are identical to or different from each other, and each represents a C1-C6 chain hydrocarbon group which may optionally have one or more substituents selected from Group G, a C3-C6 alicyclic hydrocarbon group which may be optionally substituted with one or more halogen atoms, or a hydrogen atom, R^{33X} and R^{34X} may combine with a nitrogen atom to which they are attached to form a five (5)- to ten (10)- membered aromatic heterocyclic ring which may optionally have one or more substituents selected from Group D, a C3-C8 alicyclic hydrocarbon, a three (3)- to eight (8)-membered non-aromatic heterocyclic ring {the C3-C8 alicyclic hydrocarbon, and a three (3)- to eight (8)- membered non-aromatic heterocyclic ring may optionally have one or more substituents selected from group B}^{,} and the other symbols have the same definitions as the above.]

The reaction is usually carried out in a solvent. Examples of the solvents to be used in the reaction include hydrocarbons, ethers, halogenated hydrocarbons, amides, esters, nitriles, water, and mixed solvents of two or more of these solvents.

Examples of the base to be used in the reaction include organic bases, alkali metal carbonates, alkali metal hydrocarbonates, sodium hydride, and tripotassium phosphate.

In the reaction, the compound (M6) is usually used within a range of 1 to 10 molar ratio(s), and the bae is usually used within a range of 1 to 10 molar ratio(s), as opposed to 1 mole of the compound (B6).

The reaction temperature in the reaction is usually within a range of 0 to 150 °C. The reaction period in the reaction is usually within a range of 0.1 to 48 hours.

When the reaction is completed, water is added to the reaction mixture, and the resulting mixture is extracted with organic solvent(s), and the organic layer is worked up (for example, drying and concentration) to isolate the compound (I-e).

The compound (M6) is publicly known, or can be prepared according to a publicly known method.

### Reference Process A

The compound (B1) can be prepared by reacting the compound (B4) with a sulfurizing agent. 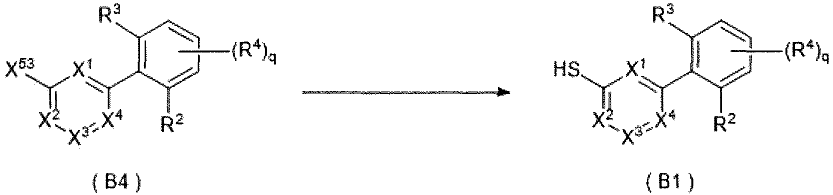 [wherein the symbols have the same definitions as the above.]

The reaction can be carried out by using the sulfurizing agent (such as hydrogen sulfide) in place of the compound (M4) according to the process E.

### Reference Process B

The compound (B4) can be prepared by reacting a compound represented by formula (C1) (hereinafter, referred to as "Compound (C1)") with the compound (M2) in the presence of a palladium catalyst and a base. 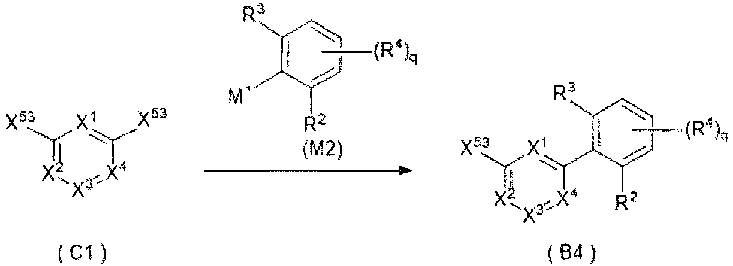 [wherein the symbols have the same definitions as the above.]

The compound (C1) is publicly known, or can be prepared according to a publicly known method.

### Reference process C

A compound represented by formula (C3) (hereinafter, referred to as "Compound (C3)") can be prepared by reacting a compound represented by formula (C2) (hereinafter, referred to as "Compound (C2)") with the compound (M1) in the presence of a base. 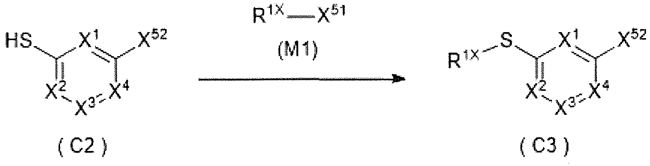 [wherein the symbols have the same definitions as the above.]

The reaction can be carried out by using the compound (C2) in place of the compound (B1) according to the process B.

The compound (C2) is publicly known, or can be prepared according to a publicly known method.

### Reference process D

A compound represented by formula (C4) (hereinafter, referred to as "Compound (C4)") or a compound represented by formula (C5) (hereinafter, referred to as "Compound (C5)") can be prepared by reacting the compound (C3) with an oxidizing agent. 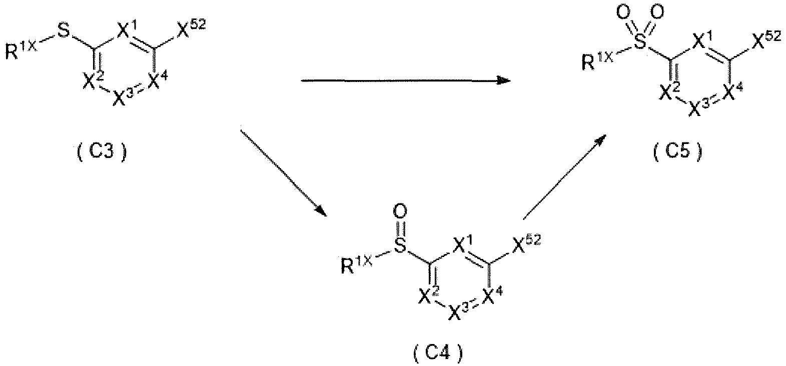 [wherein the symbols have the same definitions as the above.]

The reaction can be carried out by using the compound (C3) in place of the compound (I-a) and using the compound (C4) in place of the compound (I-b) according to the process A.

### Reference process E

A compound represented by formula (C6) (hereinafter, referred to as "Compound (C6)") can be prepared by reacting the compound (B2) with bis(pinacolato)diboron in the presence of a base and a palladium catalyst. 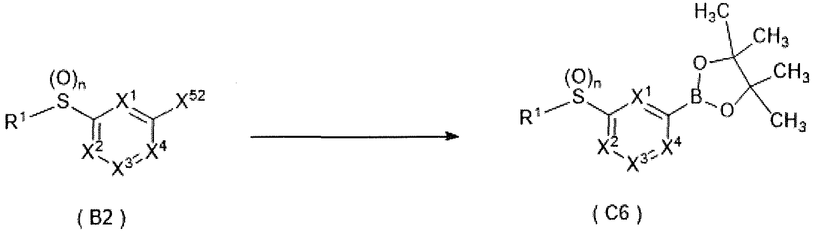 [wherein the symbols have the same definitions as the above.]

The reaction can be carried out by using bis(pinacolato)diboron in place of the compound (M2) according to the process C.

### Reference Process F

A compound represented by formula (C7) (hereinafter, referred to as "Compound (C7)") can be prepared by reacting the compound (C1) with the compound (M5). 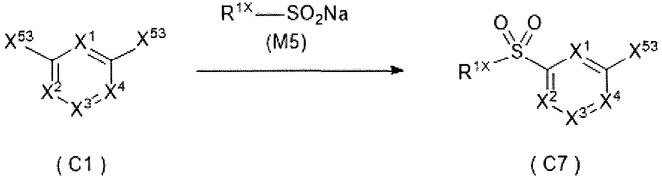 [wherein the symbols have the same definitions as the above.]

The reaction can be carried out by using the compound (C1) in place of the compound (B4) according to the process F.

### Reference Process G

A compound represented by formula (C9) (hereinafter, referred to as "Compound (C9)") can be heating a compound represented by formula (C8) (hereinafter, referred to as "Compound (C8)"). 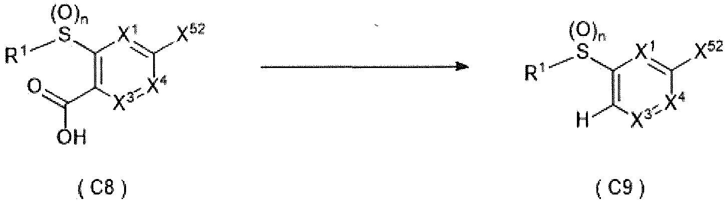 [wherein the symbols have the same definitions as the above.]

The reaction can be carried out by using the compound (C8) in place of the compound (B5) according to the process G.

The compound (C8) is publicly known, or can be prepared according to a publicly known method.

### Reference Process H

The compound (B6) can be prepared from a compound represented by formula (C10) (hereinafter, referred to as "Compound (C10)"). 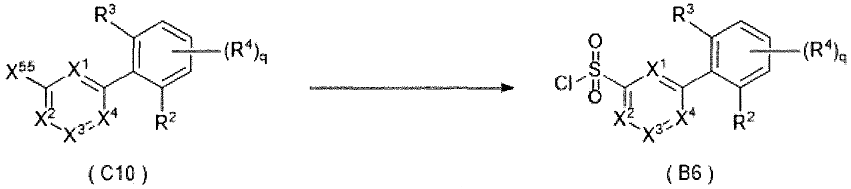 [wherein X⁵⁵ represents SH, or S(O)₂H, and the other symbols have the same definitions as the above.]

The reaction can be carried out according to the method described in WO 2016/204096 A1 or the method described in Tetrahedron Letters, 2017, 58, 2244-2247, for example.

The compound (C10) is publicly known, or can be prepared according to a publicly known method.

The compound of the present invention or the compound Z of the present invention can be mixed or combined with one or more ingredients selected from the group consisting of the following Group (a), Group (b), Group (c), and Group (d) (hereinafter, referred to as "Present ingredient").

The above-described defined mixing or combining represents that the compound of the present invention or the compound Z of the present invention and the present ingredient are used concurrently, separately, or at an interval.

When the compound of the present invention or the compound Z of the present invention and the present ingredient are concurrently used, the compound of the present invention or the compound Z of the present invention and the present ingredient may be incorporated as a separate formulation or one formulation.

One aspect of the present invention relates to a composition comprising one or more ingredients selected from the group consisting of the Group (a), the Group (b), the Group (c) and the Group (d) (that is, the present ingredient) as well as the compound of the present invention (hereinafter, the composition is referred to as "Composition A").

One aspect of the present invention relates to a composition comprising one or more ingredients selected from the group consisting of the Group (a), the Group (b), the Group (c) and the Group (d) (that is, the present ingredient) as well as the compound Z of the present invention (hereinafter, the composition is referred to as "Composition A").

The Group (a) represents acetylcholinesterase inhibitors (for example, carbamate insecticides and organophosphate insecticides), GABA-gated chloride ion channel blockers (for example, phenylpyrazole insecticides), sodium channel modulators (for example, pyrethroid insecticides), nicotinic acetylcholine receptor antagonist modulators (for example, neonicotinoid insecticides), nicotinic acetylcholine receptor allosteric modulators, glutamate-gated chloride ion channel competitive modulators (for example, macrolide insecticides), juvenile hormone mimics, multisite inhibitors, chordotonal organ TRPV channel modulators, mites growth regulators, microbial disruptors of insect midgut membranes, mitochondrial ATP synthase inhibitors, uncouplers of oxidative phosphorylation, nicotinic acetylcholine receptor channel blockers (for example, nereistoxin insecticides), inhibitors of chitin biosynthesis, moulting disruptors, ecdysone receptor agonists, octopamine receptor agonists, inhibitors of mitochondrial electron transport chain complex I, II, III, and IV, voltage-dependent sodium channel blockers, Inhibitors of acetyl CoA carboxylase, ryanodine receptor modulators (for example, diamide insecticides), chordotonal organ modulators, microbial fungicides, as well as other insecticidal active ingredients, miticidal active ingredients and nematicidal active ingredients. These agents are described in the classification based on the IRAC mode of action.

The Group (b) is a group consisting of nucleic acid synthesis inhibitors (for example, phenylamide fungicides and acylamino acid fungicides), cytostatic and cytoskeletal inhibitors (for example, MBC fungicides), respiration inhibitors (for example, QoI fungicides and QiI fungicides), amino-acid synthesis and protein synthesis inhibitors (for example, anilinopyridine fungicides), signal-transduction inhibitors, lipid synthesis and membrane synthesis inhibitors, sterol biosynthesis inhibitors (for example, DMI fungicides such as triazoles), cell wall synthesis inhibitors, melanin synthesis inhibitors, plant defense inducer, multisite fungicides, microbial fungicides, and other fungicidal active ingredients. These agents are described in the classification based on the FRAC mode of action.

The Group (c) represents a group of plant growth modulating ingredients (including mycorrhizal fungus and rhizobia).

The Group (d) represents a group of repellent active ingredients.

Examples of combinations of the present ingredient and the compound Z of the present invention (including the compound of the present invention) are recited as follows. For example, the "alanycarb + SX" indicates a combination of alanycarb and SX. The abbreviation "SX" means to any one of the compounds Z of the present invention selected from SX1 to SX210. Further, all of the present ingredients as described below are known ingredients, and can be obtained from commercially available formulations or prepared by known methods. When the present ingredient represents a microorganism, the present ingredient can be obtained from a microorganism depositary authority. The number in parentheses represents CAS RN (registered trademark).

A combination of the present ingredient in the above-mentioned Group (a) and the compound Z of the present invention:
abamectin + SX, acephate + SX, acequinocyl + SX, acetamiprid + SX, acetoprole + SX, acrinathrin + SX, acynonapyr + SX, afidopyropen + SX, afoxolaner + SX, alanycarb + SX, aldicarb + SX, allethrin + SX, alpha-cypermethrin + SX, alpha-endosulfan + SX, aluminium phosphide + SX, amitraz + SX, azadirachtin + SX, azamethiphos + SX, azinphos-ethyl + SX, azinphos-methyl + SX, azocyclotin + SX, bark of Celastrus angulatus + SX, bendiocarb + SX, benfluthrin + SX, benfuracarb + SX, bensultap + SX, benzoximate + SX, benzpyrimoXan + SX, beta-cyfluthrin + SX, beta-cypermethrin + SX, bifenazate + SX, bifenthrin + SX, bioallethrin + SX, bioresmethrin + SX, bistrifluron + SX, borax + SX, boric acid + SX, broflanilide + SX, bromopropylate + SX, buprofezin + SX, butocarboxim + SX, butoxycarboxim + SX, cadusafos + SX, calcium phosphide + SX, carbaryl + SX, carbofuran + SX, carbosulfan + SX, cartap hydrochloride + SX, cartap + SX, chinomethionat + SX, chlorantraniliprole + SX, chlordane + SX, chlorethoxyfos + SX, chlorfenapyr + SX, chlorfenvinphos + SX, chlorfluazuron + SX, chlormephos + SX, chloropicrin + SX, chlorpyrifos + SX, chlorpyrifos-methyl + SX, chromafenozide + SX, clofentezine + SX, clothianidin + SX, concanamycin A + SX, coumaphos + SX, cryolite + SX, cyanophos + SX, cyantraniliprole + SX, cyclaniliprole + SX, cyclobutrifluram + SX, cycloprothrin + SX, cycloxaprid + SX, cyenopyrafen + SX, cyetpyrafen + SX, cyflumetofen + SX, cyfluthrin + SX, cyhalodiamide + SX, cyhalothrin + SX, cyhexatin + SX, cypermethrin + SX, cyphenothrin + SX, cyproflanilide + SX, cyromazine + SX, dazomet + SX, deltamethrin + SX, demeton-S-methyl + SX, diafenthiuron + SX, diazinon + SX, dichlorvos + SX, dicloromezotiaz + SX, dicofol + SX, dicrotophos + SX, diflovidazin + SX, diflubenzuron + SX, dimefluthrin + SX, dimethoate + SX, dimethylvinphos + SX, dimpropyridaz + SX, dinotefuran + SX, disodium octaborate + SX, disulfoton + SX, DNOC (2-methyl-4,6-dinitrophenol) + SX, doramectin + SX, dried leaves of Dryopteris filix-mas + SX, emamectin-benzoate + SX, empenthrin + SX, endosulfan + SX, EPN (O-ethyl O-(4-nitrophenyl)phenylphosphonothioate) + SX, epsilon-metofluthrin + SX, epsilon-momfluorothrin + SX, esfenvalerate + SX, ethiofencarb + SX, ethion + SX, ethiprole + SX, ethoprophos + SX, etofenprox + SX, etoxazole + SX, extract of Artemisia absinthium + SX, extract of Azadirachta indica + SX, extract of Cassia nigricans + SX, extract of clitoria ternatea + SX, extract of Symphytum officinale + SX, extract of Chenopodium ambrosioides + SX, extract of Tanacetum vulgare + SX, extract of Urtica dioica + SX, extract of Viscum album + SX, famphur + SX, fenamiphos + SX, fenazaquin + SX, fenbutatin oxide + SX, fenitrothion + SX, fenmezoditiaz + SX, fenobucarb + SX, fenoxycarb + SX, fenpropathrin + SX, fenpyroximate + SX, fenthion + SX, fenvalerate + SX, fipronil + SX, flometoquin + SX, flonicamid + SX, fluacrypyrim + SX, fluazaindolizine+ SX, fluazuron + SX, flubendiamide + SX, fluchlordiniliprole + SX, flucycloxuron + SX, flucythrinate + SX, fluensulfone + SX, flufenoprox + SX, flufenoxuron + SX, flufiprole + SX, flumethrin + SX, flupentiofenox + SX, flupyradifurone + SX, flupyrimin + SX, flupyroxystrobin + SX, fluralaner + SX, fluvalinate + SX, fluxametamide + SX, formetanate + SX, fosthiazate + SX, furamethrin + SX, furathiocarb + SX, gamma-cyhalothrin + SX, GS-omega/kappa HXTX-Hv1a peptide + SX, halfenprox + SX, halofenozide + SX, heptafluthrin + SX, heptenophos + SX, hexaflumuron + SX, hexythiazox + SX, potassium salt of hop beta acid + SX, hydramethylnon + SX, hydroprene + SX, imicyafos + SX, imidacloprid + SX, imidaclothiz + SX, imiprothrin + SX, indazapyroxamet + SX, indoxacarb + SX, isocycloseram + SX, isofenphos + SX, isoprocarb + SX, isopropyl-O-(methoxyaminothiophosphoryl) salicylate + SX, isoxathion + SX, ivermectin + SX, kadethrin + SX, kappa-tefluthrin + SX, kappa-bifenthrin + SX, kinoprene + SX, lambda-cyhalothrin + SX, ledprona + SX, lenoremycin + SX, lepimectin + SX, lime sulfur + SX, lotilaner + SX, lufenuron + SX, machine oil + SX, malathion + SX, mecarbam + SX, meperfluthrin + SX, metaflumizone + SX, metam + SX, methamidophos + SX, methidathion + SX, methiocarb + SX, methomyl + SX, methoprene + SX, methoxychlor + SX, methoxyfenozide + SX, methyl bromide + SX, metofluthrin + SX, metolcarb + SX, metoxadiazone + SX, mevinphos + SX, milbemectin + SX, milbemycin oxime + SX, mivorilaner + SX, modoflaner + SX, momfluorothrin + SX, monocrotophos + SX, 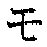 moxidectin + SX, naled + SX, nicofluprole + SX, nicotine + SX, nicotine-sulfate + SX, nitenpyram + SX, novaluron + SX, noviflumuron + SX, oil of the seeds of Chenopodium anthelminticum + SX, omethoate + SX, oxamyl + SX, oxazosulfyl + SX, oxydemeton-methyl + SX, parathion + SX, parathion-methyl + SX, permethrin + SX, phenothrin + SX, phenthoate + SX, phorate + SX, phosalone + SX, phosmet + SX, phosphamidon + SX, phosphine + SX, phoxim + SX, pirimicarb + SX, pirimiphos-methyl + SX, prallethrin + SX, profenofos + SX, profluthrin + SX, propargite + SX, propetamphos + SX, propoxur + SX, propylene glycol alginate + SX, prothiofos + SX, pyflubumide + SX, pymetrozine + SX, pyraclofos + SX, pyrethrins + SX, pyridaben + SX, pyridalyl + SX, pyridaphenthion + SX, pyrifluquinazone + SX, pyrimidifen + SX, pyriminostrobin + SX, pyriprole + SX, pyriproxyfen + SX, quinalphos + SX, resmethrin + SX, rotenone + SX, ryanodine + SX, sarolaner + SX, selamectin + SX, sigma-cypermethrin + SX, silafluofen + SX, sodium borate + SX, sodium metaborate + SX, spidoxamat + SX, spinetoram + SX, spinosad + SX, spirobudifen + SX, spirodiclofen + SX, spiromesifen + SX, spiropidion + SX, spirotetramat + SX, sulfiflumin + SX, sulfluramid + SX, sulfotep + SX, sulfoxaflor + SX, sulfur + SX, sulfuryl fluoride + SX, tartar emetic + SX, tau-fluvalinate + SX, tebufenozide + SX, tebufenpyrad + SX, tebupirimfos + SX, teflubenzuron + SX, tefluthrin + SX, temephos + SX, terbufos + SX, terpene constituents of the extract of chenopodium ambrosioides near ambrosioides + SX, tetrachlorantraniliprole + SX, tetrachlorvinphos + SX, tetradifon + SX, tetramethrin + SX, tetramethylfluthrin + SX, tetraniliprole + SX, theta-cypermethrin + SX, thiacloprid + SX, thiamethoxam + SX, thiocyclam + SX, thiodicarb + SX, thiofanox + SX, thiometon + SX, thiosultap-disodium + SX, thiosultap-monosodium + SX, tigolaner + SX, tiorantraniliprole + SX, tioxazafen + SX, tolfenpyrad + SX, tralomethrin + SX, transfluthrin + SX, triazamate + SX, triazophos + SX, trichlorfon + SX, trifluenfuronate + SX, triflumezopyrim + SX, triflumuron + SX, trimethacarb + SX, tyclopyrazoflor + SX, umifoxolaner + SX, vamidothion + SX, wood extract of Quassia amara + SX, XMC (3,5-dimethylphenyl N-methylcarbamate) + SX, xylylcarb + SX, zeta-cypermethrin + SX, zinc phosphide + SX, 4-[5-(3,5-dichlorophenyl)-5-(trifluoromethyl)-4,5-dihydro-1,2-oXazol-3-yl]-2-methyl-N-(1-oxothietan-3-yl)benzamide (1241050-20-3) + SX, 3-methoxy-N-(5-{5-(trifluoromethyl)-5-[3-(trifluoromethyl)phenyl]-4,5-dihydro-1,2-oxazol-3-yl}indan-1-yl)propanamide (1118626-57-5) + SX,, N-{4-chloro-3-[(1-cyanocyclopropyl)carbamoyl]phenyl}-1-methyl-4-(methanesulfonyl)-3-(1,1,2,2,2-pentafluoroethyl)-1H-pyrazole-3-carboXamide (1400768-21-9) + SX, N-[3-chloro-1-(pyridin-3-yl)-1H-pyrazol-4-yl]-2-(methanesulfonyl)propanamide (2396747-83-2) + SX, N-[4-chloro-2-(pyridin-3-yl)-1,3-thiazol-5-yl]-N-ethyl-3-(methanesulfonyl)propanamide + SX, 1,4-dimethyl-2-[2-(pyridin-3-yl)-2H-indazol-5-yl]-1,2,4-triazolidine-3,5-dione (2171099-09-3) + SX, 2-isopropyl-5-[(3,4,4-trifluoro-3-buten-1-yl)sulfonyl]-1,3,4-thiadiazole (2058052-95-0) + SX, N-({2-fluoro-4-[(2S,3S)-2-hydroxy-3-(3,4,5-trichlorophenyl)-3-(trifluoromethyl)pyrrolidin-1-yl]phenyl}methyl)cyclopropanecarboXamide + SX, 7-fluoro-N-[1-(methylsulfanyl)-2-methylpropan-2-yl]-2-(pyridin-3-yl)-2H-indazole-4-carboXamide + SX, 7-fluoro-N-[1-(methanesulfinyl)-2-methylpropan-2-yl]-2-(pyridin-3-yl)-2H-indazole-4-carboXamide + SX, 7-fluoro-N-[1-(methanesulfonyl)-2-methylpropan-2-yl]-2-(pyridin-3-yl)-2H-indazole-4-carboXamide + SX, N-[1-(difluoromethyl)cyclopropyl]-2-(pyridin-3-yl)-2H-indazole-4-carboXamide + SX, 2,9-dihydro-9-(methoxymethyl)-2-(pyridin-3-yl)-10H-pyrazolo[3,4-f]pyrido[2,3-b][1,4]oXazepin-10-one (2607927-97-7) + SX,
BT crop protein Cry1Ab) + SX, BT crop protein Cry1Ac + SX, BT crop protein Cry1Fa + SX, BT crop protein Cry1A.105 + SX, BT crop protein Cry2Ab + SX, BT crop protein Vip3A + SX, BT crop protein mCry3A + SX, BT crop protein Cry3Ab + SX, BT crop protein Cry3Bb + SX, BT crop protein Cry34Ab1/Cry35Ab1 + SX, Adoxophyes orana granulosis virus strain BV-0001 + SX, Anticarsia gemmatalis mNPV + SX, Autographa californica mNPV + SX, Cydia pomonella GV strain V15 + SX, Cydia pomonella GV strain V22 + SX, Cryptophlebia leucotreta GV + SX, Dendrolimus punctatus cypovirus + SX, Helicoverpa armigera NPV strain BV-0003 + SX, Helicoverpa zea NPV + SX, Lymantria dispar NPV + SX, Mamestra brassicae NPV + SX, Mamestra configurata NPV + SX, Neodiprion abietis NPV + SX, Neodiprion lecontei NPV + SX, Neodiprion sertifer NPV + SX, Nosema locustae + SX, Orgyia pseudotsugata NPV + SX, Pieris rapae GV + SX, Plodia interpunctella GV + SX, Spodoptera exigua mNPV + SX, Spodoptera littoralis mNPV + SX, Spodoptera litura NPV + SX, arthrobotrys dactyloides + SX, Bacillus firmus strain GB-126 + SX, Bacillus firmus strain I-1582 + SX, Bacillus firmus strain NCIM2637 + SX, Bacillus megaterium + SX, Bacillus sp. strain AQ175 + SX, Bacillus sp. strain AQ177 + SX, Bacillus sp. strain AQ178 + SX, Bacillus sphaericus strain 2362 serotype H5a5b + SX, Bacillus sphaericus strain ABTS1743 + SX, Bacillus thuringiensis strain AQ52 + SX, Bacillus thuringiensis strain BD#32 + SX, Bacillus thuringiensis strain CR371 + SX, Bacillus thuringiensis subsp. Aizawai strain ABTS-1857 + SX, Bacillus thuringiensis subsp. Aizawai strain AM65-52 + SX, Bacillus thuringiensis subsp. Aizawai strain GC-91 + SX, Bacillus thuringiensis subsp. Aizawai strain NB200 + SX, Bacillus thuringiensis subsp. Aizawai Serotype strain H-7 + SX, Bacillus thuringiensis subsp. Kurstaki strain ABTS351 + SX, Bacillus thuringiensis subsp. Kurstaki strain BMP123 + SX, Bacillus thuringiensis subsp. Kurstaki strain CCT1306) + SX, Bacillus thuringiensis subsp. Kurstaki strain EG2348 + SX, Bacillus thuringiensis subsp. Kurstaki strain EG7841 + SX, Bacillus thuringiensis subsp. Kurstaki strain EVB113-19 + SX, Bacillus thuringiensis subsp. Kurstaki strain F810 + SX, Bacillus thuringiensis subsp. Kurstaki strain HD-1 + SX, Bacillus thuringiensis subsp. Kurstaki strain PB54 + SX, Bacillus thuringiensis subsp. Kurstaki strain SA-11 + SX, Bacillus thuringiensis subsp. Kurstaki strain SA-12 + SX, Bacillus thuringiensis subsp. Tenebriosis strain NB176 + SX, Bacillus thuringiensis subsp. Thuringiensis strain MPPL002 + SX, Bacillus thuringiensis subsp. morrisoni + SX, Bacillus thuringiensis var. colmeri + SX, Bacillus thuringiensis var. darmstadiensis strain 24-91 + SX, Bacillus thuringiensis var. dendrolimus + SX, Bacillus thuringiensis var. galleriae + SX, Bacillus thuringiensis var. israelensis strain BMP144 + SX, Bacillus thuringiensis var. israelensis serotype strain H-14 + SX, Bacillus thuringiensis var. japonensis strain buibui + SX, Bacillus thuringiensis var. san diego strain M-7 + SX, Bacillus thuringiensis vaR7216 + SX, Bacillus thuringiensis var. aegypti + SX, Bacillus thuringiensis var. T36 + SX, Beauveria bassiana strain ANT-03 + SX, Beauveria bassiana strain ATCC74040 + SX, Beauveria bassiana strain GHA + SX, Beauveria brongniartii + SX, Burkholderia rinojensis strain A396 + SX, Chromobacterium subtsugae strain PRAA4-1T + SX, Dactyllela ellipsospora + SX, Dectylaria thaumasia + SX, Hirsutella minnesotensis + SX, Hirsutella rhossiliensis + SX, Hirsutella thompsonii + SX, Lagenidium giganteum + SX, Lecanicillium lecanii strain KV01 + SX, Lecanicillium lecanii conidia of strain DAOM198499 + SX, Lecanicillium lecanii conidia of strain DAOM216596 + SX, Lecanicillium muscarium strain Ve6 + SX, Metarhizium anisopliae strain F52 + SX, Metarhizium anisopliae var. acridum + SX, Metarhizium anisopliae var. anisopliae BIPESCO 5/F52 + SX, Metarhizium flavoviride + SX, Monacrosporium phymatopagum + SX, Paecilomyces fumosoroseus Apopka strain 97 + SX, Paecilomyces lilacinus strain 251 + SX, Paecilomyces tenuipes strain T1 + SX, Paenibacillus popilliae + SX, Pasteuria nishizawae strain Pn1 + SX, Pasteuria penetrans + SX, Pasteuria usgae + SX, Pasteuria thornei + SX, Serratia entomophila + SX, Verticillium chlamydosporium + SX, Verticillium lecani strain NCIM1312 + SX, Wolbachia pipientis + SX.

A combination of the present ingredient in the above-mentioned Group (b) and the compound Z of the present invention:
acibenzolar-S-methyl + SX, aldimorph + SX, ametoctradin + SX, aminopyrifen + SX, amisulbrom + SX, anilazine + SX, azaconazole + SX, azoxystrobin + SX, basic copper sulfate + SX, benalaxyl + SX, benalaxyl-M + SX, benodanil + SX, benomyl + SX, benthiavalicarb + SX, benthiavalicarb-isopropyl + SX, benzovindiflupyr + SX, binapacryl + SX, biphenyl + SX, bitertanol + SX, bixafen + SX, blasticidin-S + SX, Bordeaux mixture + SX, boscalid + SX, bromothalonil + SX, bromuconazole + SX, bupirimate + SX, captafol + SX, captan + SX, carbendazim + SX, carboxin + SX, carpropamid + SX, chinomethionat + SX, chitin + SX, chloroinconazide + SX, chloroneb + SX, chlorothalonil + SX, chlozolinate + SX, colletochlorin B + SX, copper(II) acetate + SX, copper(II) hydroxide + SX, copper oxychloride + SX, copper(II) sulfate + SX, coumoxystrobin + SX, cyazofamid + SX, cyflufenamid + SX, cymoxanil + SX, cyproconazole + SX, cyprodinil + SX, dichlobentiazox + SX, dichlofluanid + SX, diclocymet)+ SX, diclomezine + SX, dicloran + SX, diethofencarb + SX, difenoconazole + SX, diflumetorim + SX, dimethachlone + SX, dimethirimol + SX, dimethomorph + SX, dimoxystrobin + SX, diniconazole + SX, diniconazole-M + SX, dinocap + SX, dipotassium hydrogenphosphite + SX, dipymetitrone + SX, dithianon + SX, dodecylbenzenesulphonic acid bisethylenediamine copper(II) salt + SX, dodemorph + SX, dodine + SX, edifenphos + SX, enoxastrobin + SX, epoxiconazole + SX, etaconazole + SX, ethaboxam + SX, ethirimol + SX, etridiazole + SX, extract of Melaleuca alternifolia + SX, extract of Reynoutria sachalinensis + SX, extract of the cotyledons of lupine plantlets ("BLAD") + SX, extract of Allium sativum + SX, extract of Equisetum arvense + SX, extract of Tropaeolum majus + SX, famoxadone + SX, fenamidone + SX, fenaminstrobin + SX, fenarimol + SX, fenbuconazole + SX, fenfuram + SX, fenhexamid + SX, fenoxanil + SX, fenpiclonil + SX, fenpicoxamid + SX, fenpropidin + SX, fenpropimorph + SX, fenpyrazamine + SX, fentin acetate + SX, fentin chloride + SX, fentin hydroxide + SX, ferbam + SX, ferimzone + SX, florylpicoxamid + SX, fluazinam + SX, flubeneteram + SX, fludioxonil + SX, flufenoxadiazam + SX, flufenoxystrobin + SX, fluindapyr + SX, flumetylsulforim + SX, flumorph + SX, fluopicolide + SX, fluopyram + SX, fluopimomide + SX, fluoroimide + SX, fluoxapiprolin + SX, fluoxastrobin + SX, fluoxytioconazole + SX, fluquinconazole + SX, flusilazole + SX, flusulfamide + SX, flutianil + SX, flutolanil + SX, flutriafol + SX, fluxapyroxad + SX, folpet + SX, fosetyl + SX, fosetyl-aluminium + SX, fuberidazole + SX, furalaxyl + SX, furametpyr + SX, guazatine + SX, hexaconazole + SX, hymexazole + SX,
imazalil + SX, imibenconazole + SX, iminoctadine + SX, iminoctadine triacetate + SX, inpyrfluxam + SX, iodocarb + SX, ipconazole + SX, ipfentrifluconazole + SX, ipflufenoquin + SX, iprobenfos + SX, iprodione + SX, iprovalicarb + SX, isofetamid + SX, isoflucypram + SX, isoprothiolane + SX, isopyrazam + SX, isotianil + SX, kasugamycin + SX, kresoxim-methyl + SX, laminarin + SX, leaves and bark of Quercus + SX, mancozeb + SX, mandestrobin + SX, mandipropamid + SX, maneb + SX, mefentrifluconazole + SX, mepanipyrim + SX, mepronil + SX, meptyldinocap + SX, metalaxyl + SX, metalaxyl-M + SX, metarylpicoxamid + SX, metconazole + SX, methasulfocarb + SX, metiram + SX, metominostrobin + SX, metrafenone + SX, metyltetraprole + SX, myclobutanil + SX, naftifine + SX, nuarimol + SX, octhilinone + SX, ofurace + SX, orysastrobin + SX, oxadixyl + SX, oxathiapiprolin + SX, oxine-copper + SX, oxolinic acid + SX, oxpoconazole + SX, oxpoconazole fumarate + SX, oxycarboxin + SX, oxytetracycline + SX, pefurazoate + SX, penconazole + SX, pencycuron + SX, penflufen + SX, penthiopyrad + SX, phenamacril + SX, phosphorous acid + SX, phthalide + SX, picarbutrazox + SX, picoxystrobin + SX, piperalin + SX, polyoxins + SX, potassium hydrogencarbonate + SX, potassium dihydrogenphosphite + SX, probenazole + SX, prochloraz + SX, procymidone + SX, propamidine + SX, propamocarb + SX, propiconazole + SX, propineb + SX, proquinazid + SX, prothiocarb + SX, prothioconazole + SX, pydiflumetofen + SX, pyraclostrobin + SX, pyrametostrobin + SX, pyraoxystrobin + SX, pyrapropoyne + SX, pyraziflumid + SX, pyrazophos + SX, pyribencarb + SX, pyributicarb + SX, pyridachlometyl + SX, pyrifenox + SX, pyrimethanil + SX, pyrimorph + SX, pyriofenone + SX, pyrisoxazole + SX, pyroquilon + SX, Quillaja extract + SX, quinconazole + SX, quinofumelin + SX, quinoxyfen + SX, quintozene + SX, Saponins of Chenopodium quinoa + SX, seboctylamine + SX, sedaXane + SX, silthiofam + SX, simeconazole + SX, sodium hydrogencarbonate + SX, spiroxamine + SX, streptomycin + SX, sulfur + SX, tebuconazole + SX, tebufloquin + SX, teclofthalam + SX, tecnazene + SX, terbinafine + SX, tetraconazole + SX, thiabendazole + SX, thifluzamide + SX, thiophanate + SX, thiophanate-methyl + SX, thiram + SX, thymol + SX, tiadinil + SX, tolclofos-methyl + SX, tolfenpyrad + SX, tolprocarb + SX, tolylfluanid + SX, triadimefon + SX, triadimenol + SX, triazoxide + SX, triclopyricarb + SX, tricyclazole + SX, tridemorph + SX, trifloxystrobin + SX, triflumizole + SX, triforine + SX, triticonazole + SX, validamycin + SX, valifenalate + SX, vinclozolin + SX, yellow mustard powder + SX, zinc thiazole + SX, zineb + SX, ziram + SX, zoXamide + SX,
N'-[4-({3-[(4-chlorophenyl)methyl]-1,2,4-thiadiazol-5-yl}oxy)-2,5-dimethylphenyl]-N-ethyl-N-methylmethanimidamide (1202781-91-6) + SX, N'-{4-[(4,5-dichlorothiazol-2-yl)oxy]-2,5-dimethylphenyl}-N-ethyl-N-methylmethanimidamide (929908-57-6) + SX, N'-(2,5-dimethyl-4-phenoxyphenyl)-N-ethyl-N-methylmethanimidamide (1052688-31-9) + SX, N'-[5-chloro-4-(2-fluorophenoxy)-2-methylphenyl]-N-ethyl-N-methylmethanimidamide (2055589-28-9) + SX, N'-[2-chloro-4-(2-fluorophenoxy)-5-methylphenyl]-N-ethyl-N-methylmethanimidamide (2055756-21-1) + SX, N'-(2-chloro-4-phenoxy-5-methylphenyl)-N-ethyl-N-methylmethanimidamide (2062599-39-5) + SX, N'-[4-(1-hydroxy-1-phenyl-2,2,2-trifluoroethyl)-2-methyl-5-methoxyphenyl]-N-isopropyl-N-methylmethanimidamide (2101814-55-3) + SX, N'-[5-bromo-6-(1-methyl-2-propoxyethoxy)-2-methylpyridin-3-yl]-N-ethyl-N-methylmethanimidamide (1817828-69-5) + SX, 4-(2-bromo-4-fluorophenyl)-N-(2-chloro-6-fluorophenyl)-1,3-dimethyl-1H-pyrazol-5-amine (1362477-26-6) + SX, 2-[6-(3-fluoro-4-methoxyphenyl)-5-methylpyridin-2-yl]quinazoline (1257056-97-5) + SX, ethyl (2Z)-3-amino-2-cyano-3-phenylacrylate (39491-78-6) + SX, N-[(2-chlorothiazol-5-yl)methyl]-N-ethyl-6-methoxy-3-nitropyridin-2-amine (1446247-98-8) + SX, 5-(4-chlorobenzyl)-2-(chloromethyl)-2-methyl-1-(1H-1,2,4-triazol-1-ylmethyl)cyclopentan-1-ol (1394057-11-4) + SX, (1R,2S,5S)-5-(4-chlorobenzyl)-2-(chloromethyl)-2-methyl-1-(1H-1,2,4-triazol-1-ylmethyl)cyclopentan-1-ol (1801930-06-2) + SX, (1S,2R,5R)-5-(4-chlorobenzyl)-2-(chloromethyl)-2-methyl-1-(1H-1,2,4-triazol-1-ylmethyl)cyclopentan-1-ol (1801930-07-3) + SX, 2-(chloromethyl)-5-(4-fluorobenzyl)-2-methyl-1-(1H-1,2,4-triazol-1-ylmethyl)cyclopentan-1-ol (1394057-13-6) + SX, (1R,2S,5S)-2-(chloromethyl)-5-(4-fluorobenzyl)-2-methyl-1-(1H-1,2,4-triazol-1-ylmethyl)cyclopentan-1-ol (1801930-08-4) + SX, (1S,2R,5R)-2-(chloromethyl)-5-(4-fluorobenzyl)-2-methyl-1-(1H-1,2,4-triazol-1-ylmethyl)cyclopentan-1-ol (1801930-09-5) + SX, methyl 3-[(4-chlorophenyl)methyl]-2-hydroxy-1-methyl-2-(1H-1,2,4-triazol-1-ylmethyl)cyclopentan-1-carboxylate (1791398-02-1) + SX, 1-(2,4-difluorophenyl)-2-(1H-1,2,4-triazol-1-yl)-1-[1-(4-bromo-2,6-difluorophenoxy)cyclopropyl]ethanol (2019215-86-0) + SX, 1-(2,4-difluorophenyl)-2-(1H-1,2,4-triazol-1-yl)-1-[1-(4-chloro-2,6-difluorophenoxy)cyclopropyl]ethanol (2019215-84-8) + SX, 1-[2-(1-chlorocyclopropyl)-3-(2-fluorophenyl)-2-hydroxypropyl]-1H-imidazole-5-carbonitrile (2018316-13-5) + SX, 1-[2-(1-chlorocyclopropyl)-3-(2,3-difluorophenyl)-2-hydroxypropyl]-1H-imidazole-5-carbonitrile (2018317-25-2) + SX, 2-[6-(4-bromophenoxy)-2-(trifluoromethyl)pyridin-3-yl]-1-(1H-1,2,4-triazol-1-yl)propan-2-ol (2082661-43-4) + SX, 2-[6-(4-chlorophenoxy)-2-(trifluoromethyl)pyridin-3-yl]-1-(1H-1,2,4-triazol-1-yl)propan-2-ol (2082660-27-1) + SX, methyl ({2-methyl-5-[1-(4-methoxy-2-methylphenyl)-1H-pyrazol-3-yl]phenyl}methyl)carbamate (1605879-98-8) + SX, 2-(difluoromethyl)-N-[1,1,3-trimethyl-2,3-dihydro-1H-inden-4-yl]pyridine-3-carboXamide (1616239-21-4) + SX, 2-(difluoromethyl)-N-[3-ethyl-1,1-dimethyl-2,3-dihydro-1H-inden-4-yl]pyridine-3-carboXamide (1847460-02-9) + SX, 2-(difluoromethyl)-N-[3-propyl-1,1-dimethyl-2,3-dihydro-1H-inden-4-yl]pyridine-3-carboXamide (1847460-05-2) + SX, (2E,3Z)-5-{[1-(4-chlorophenyl)-1H-pyrazol-3-yl]oxy}-2-(methoxyimino)-N,3-dimethylpent-3-enamide (1445331-27-0) + SX, (2E,3Z)-5-{[1-(2,4-dichlorophenyl)-1H-pyrazol-3-yl]oxy}-2-(methoxyimino)-N,3-dimethylpent-3-enamide (1445331-54-3) + SX, 5-chloro-4-({2-[6-(4-chlorophenoxy)pyridin-3-yl]ethyl}amino)-6-methylpyrimidine (1605340-92-8) + SX, N-(1-benzyl-1,3-dimethylbutyl)-8-fluoroquinoline-3-carboXamide (2132414-04-9) + SX, N-(1-benzyl-3,3,3-trifluoro-1-methylpropyl)-8-fluoroquinoline-3-carboXamide (2132414-00-5) + SX, 4,4-dimethyl-2-({4-[5-(trifluoromethyl)-1,2,4-oxadiazol-3-yl]phenyl}methyl)isoXazolidin-3-one (2098918-25-1) + SX, 5,5-dimethyl-2-({4-[5-(trifluoromethyl)-1,2,4-oxadiazol-3-yl]phenyl}methyl)isoXazolidin-3-one (2098918-26-2) + SX, N-ethyl-2-methyl-N-({4-[5-(trifluoromethyl)-1,2,4-oxadiazol-3-yl]phenyl}methyl)propanamide + SX, N,2-dimethoxy-N-({4-[5-(trifluoromethyl)-1,2,4-oxadiazol-3-yl]phenyl}methyl)propanamide + SX, N-methoxy-N-({4-[5-(trifluoromethyl)-1,2,4-oxadiazol-3-yl]phenyl}methyl)cyclopropanecarboxamide + SX, N-methoxy-N'-methyl-N-({4-[5-(trifluoromethyl)-1,2,4-oxadiazol-3-yl]phenyl}methyl)urea + SX, N'-ethyl-N-methoxy-N-({4-[5-(trifluoromethyl)-1,2,4-oxadiazol-3-yl]phenyl}methyl)urea + SX, N,N'-dimethoxy-N-({4-[5-(trifluoromethyl)-1,2,4-oxadiazol-3-yl]phenyl}methyl)urea + SX, N-acetyl-2-(ethanesulfonyl)-N-[2-(methoxycarbonyl)-4-(trifluoromethoxy)phenyl]-4-(trifluoromethyl)benzamide (2043675-28-9) + SX, 3-(4-bromo-7-fluoroindol-1-yl)butan-2-yl N-[(3-hydroxy-4-methoxypyridin-2-yl)carbonyl]-L-alaninate + SX, 3-(7-bromoindol-1-yl)butan-2-yl N-[(3-hydroxy-4-methoxypyridin-2-yl)carbonyl]-L-alaninate + SX, 3-(7-bromo-4-fluoroindol-1-yl)butan-2-yl N-[(3-hydroxy-4-methoxypyridin-2-yl)carbonyl]-L-alaninate + SX, 3-(3,5-dichloropyridin-2-yl)butan-2-yl N-[(3-hydroxy-4-methoxypyridin-2-yl)carbonyl]-L-alaninate + SX, 3-(3,5-dichloropyridin-2-yl)butan-2-yl N-{[3-(acetoxymethoxy)-4-methoxypyridin-2-yl]carbonyl}-L-alaninate + SX, (1S)-1-[1-(naphthalen-1-yl)cyclopropyl]ethyl N-[(3-hydroxy-4-methoxypyridin-2-yl)carbonyl]-L-alaninate + SX, (1S)-1-[1-(naphthalen-1-yl)cyclopropyl]ethyl N-[(3-acetoxy-4-methoxypyridin-2-yl)carbonyl]-L-alaninate + SX, (1S)-1-[1-(naphthalen-1-yl)cyclopropyl]ethyl N-{[3-(acetoxymethoxy)-4-methoxypyridin-2-yl]carbonyl}-L-alaninate + SX, N-({ 4-[5-(trifluoromethyl)-1,2,4-oxadiazol-3-yl]phenyl}methyl)cyclopropanecarboxamide + SX, N-allyl-N-({4-[5-(trifluoromethyl)-1,2,4-oxadiazol-3-yl]phenyl}methyl)acetamide + SX, N-allyl-N-({4-[5-(trifluoromethyl)-1,2,4-oxadiazol-3-yl]phenyl}methyl)propanamide + SX, N-({4-[5-(trifluoromethyl)-1,2,4-oxadiazol-3-yl]phenyl}methyl)propanamide + SX, 3,3,3-trifluoro-N-({ 2-fluoro-4-[5-(trifluoromethyl)-1,2,4-oxadiazol-3-yl]phenyl}methyl)propanamide + SX, 3,3,3-trifluoro-N-({ 3-fluoro-4-[5-(trifluoromethyl)-1,2,4-oxadiazol-3-yl]phenyl}methyl)propanamide + SX, 3,3,3-trifluoro-N-({ 2,3-difluoro-4-[5-(trifluoromethyl)-1,2,4-oxadiazol-3-yl]phenyl}methyl)propanamide + SX, N-({2,3-difluoro-4-[5-(trifluoromethyl)-1,2,4-oxadiazol-3-yl]phenyl}methyl)butanamide + SX, N-methoxy-N-methyl-N'-({4-[5-(trifluoromethyl)-1,2,4-oxadiazol-3-yl]phenyl}methyl)urea + SX, N,N-diethyl-N'-({4-[5-(trifluoromethyl)-1,2,4-oxadiazol-3-yl]phenyl}methyl)urea + SX, N-methyl-N'-({4-[5-(trifluoromethyl)-1,2,4-oxadiazol-3-yl]phenyl}methyl)urea + SX, 1-({4-[5-(trifluoromethyl)-1,2,4-oxadiazol-3-yl]phenyl}methyl)pyrrolidin-2-one + SX, 1-({4-[5-(trifluoromethyl)-1,2,4-oxadiazol-3-yl]phenyl}methyl)piperidin-2-one + SX, 4-({4-[5-(trifluoromethyl)-1,2,4-oxadiazol-3-yl]phenyl}methyl)morpholin-3-one + SX, 2-({4-[5-(trifluoromethyl)-1,2,4-oxadiazol-3-yl]phenyl}methyl)isoxazolidin-3-one + SX, 3,3-dimethyl-1-({4-[5-(trifluoromethyl)-1,2,4-oxadiazol-3-yl]phenyl}methyl)piperidin-2-one + SX, 2-({4-[5-(trifluoromethyl)-1,2,4-oXadiazol-3-yl]phenyl}methyl)-1,2-oxazinan-3-one + SX, 1-({3-fluoro-4-[5-(trifluoromethyl)-1,2,4-oxadiazol-3-yl]phenyl}methyl)azepan-2-one + SX, 4,4-dimethyl-1-({4-[5-(trifluoromethyl)-1,2,4-oxadiazol-3-yl]phenyl}methyl)pyrrolidin-2-one + SX, 5-methyl-1-({4-[5-(trifluoromethyl)-1,2,4-oxadiazol-3-yl]phenyl}methyl)pyrrolidin-2-one + SX, ethyl 1-({4-[5-(trifluoromethyl)-1,2,4-oxadiazol-3-yl]phenyl}methyl)-1H-pyrazole-4-carboxylate + SX, N-methyl-1-({4-[5-(trifluoromethyl)-1,2,4-oxadiazol-3-yl]phenyl}methyl)-1H-pyrazole-4-carboxamide + SX, N-propyl-1-({4-[5-(trifluoromethyl)-1,2,4-oxadiazol-3-yl]phenyl}methyl)-1H-pyrazole-4-carboxamide + SX, N-methoxy-1-({4-[5-(trifluoromethyl)-1,2,4-oxadiazol-3-yl]phenyl}methyl)-1H-pyrazole-4-carboxamide + SX, N-methoxy-N-methyl-1-({4-[5-(trifluoromethyl)-1,2,4-oxadiazol-3-yl]phenyl}methyl)-1H-pyrazole-4-carboxamide + SX, N,N-dimethyl-1-({4-[5-(trifluoromethyl)-1,2,4-oxadiazol-3-yl]phenyl}methyl)-1H-1,2,4-triazol-3-amine + SX, N-methyl-4-[5-(trifluoromethyl)-1,2,4-oxadiazol-3-yl]benzamide + SX, methyl 2-[2-chloro-4-(4-chlorophenoxy)phenyl]-2-hydroxy-3-(1H-1,2,4-triazol-1-yl)propanoate + SX, ethyl 2-[2-chloro-4-(4-chlorophenoxy)phenyl]-2-hydroxy-3-(1H-1,2,4-triazol-1-yl)propanoate + SX, methyl 2-[2-(trifluoromethyl)-4-(4-chlorophenoxy)phenyl]-2-hydroxy-3-(1H-1,2,4-triazol-1-yl)propanoate + SX, 1-(2,3-dimethylpyridin-5-yl)-4,4-difluoro-3,3-dimethyl-3,4-dihydroisoquinoline + SX, 1-[2-(difluoromethyl)-3-methylpyridin-5-yl]-4,4-difluoro-3,3-dimethyl-3,4-dihydroisoquinoline + SX, 2,2-difluoro-N-[6-({[1-(1-methyl-1H-tetrazol-5-yl)benzimidazol-2-yl]oxy}methyl)pyridin-2-yl]-2-phenoxyacetamide + SX, 1-[2-(1-chlorocyclopropyl)-3-(3-chloro-2-fluorophenyl)-2-hydroxypropyl]-1H-imidazole-5-carbonitrile + SX, ethyl 1-[(4-{[2-(trifluoromethyl)-1,3-dioxolan-2-yl]methoxy}phenyl)methyl]-1H-pyrazole-4-carboxylate + SX, ethyl 1-[(4-{[(1Z)-2-ethoxy-3,3,3-trifluoro-1-propen-1-yl]oxy}phenyl)methyl]-1H-pyrazole-4-carboxylate + SX, 6-chloro-3-(3-cyclopropyl-2-fluorophenoxy)-N-[2-(2,4-dimethylphenyl)-2,2-difluoroethyl]-5-methylpyridazine-4-carboxamide + SX, 6-chloro-3-(3-cyclopropyl-2-fluorophenoxy)-N-[2-(3,4-dimethylphenyl)-2,2-difluoroethyl]-5-methylpyridazine-4-carboxamide + SX, 6-chloro-N-[2-(2-chloro-4-methylphenyl)-2,2-difluoroethyl]-3-(3-cyclopropyl-2-fluorophenoxy)-5-methylpyridazine-4-carboxamide + SX, 2-[cyano(2,6-difluoropyridin-4-yl)amino]-N-(2,2-dimethylcyclobutyl)-5-methylthiazole-4-carboxamide + SX, 2-[cyano(2,6-difluoropyridin-4-yl)amino]-N-(spiro[3.4]octan-1-yl)-5-methylthiazole-4-carboxamide + SX, 2-[cyano(2,6-difluoropyridin-4-yl)amino]-N-hexyl-5-methylthiazole-4-carboxamide + SX, 2-[acetyl(2,6-difluoropyridin-4-yl)amino]-N-(2,2-dimethylcyclobutyl)-5-methylthiazole-4-carboxamide + SX, 2-[(2-methoxyacetyl) (2,6-difluoropyridin-4-yl)amino]-N-(2,2-dimethylcyclobutyl)-5-methylthiazole-4-carboxamide + SX, 2-[(2-methylpropanoyl) (2,6-difluoropyridin-4-yl)amino]-N-(2,2-dimethylcyclobutyl)-5-methylthiazole-4-carboxamide + SX, 5-[5-(difluoromethyl)-1,3,4-oxadiazol-2-yl]-N-[1-(2-fluorophenyl)ethyl]pyrimidin-2-amine + SX, 5-[5-(difluoromethyl)-1,3,4-oxadiazol-2-yl]-N-[1-(2,6-difluorophenyl)ethyl]pyrimidin-2-amine + SX, 5-[5-(difluoromethyl)-1,3,4-oxadiazol-2-yl]-N-[1-(3,5-difluorophenyl)ethyl]pyrimidin-2-amine + SX, 5-[5-(difluoromethyl)-1,3,4-oxadiazol-2-yl]-N-[1-(6-chloropyridin-3-yl)ethyl]pyrimidin-2-amine + SX, 5-[5-(difluoromethyl)-1,3,4-oxdiazol-2-yl]-N-[1-(2-fluorophenyl)cyclopropyl]pyrimidin-2-amine + SX, 5-[5-(difluoromethyl)-1,3,4-oxadiazol-2-yl]-N-[1-(2,6-difluorophenyl)cyclopropyl]pyrimidin-2-amine + SX, 5-[5-(difluoromethyl)-1,3,4-oXadiazol-2-yl]-N-[1-(2-fluoro-3-methoxyphenyl)cyclopropyl]pyrimidin-2-amine + SX, 5-[5-(difluoromethyl)-1,3,4-oxadiazol-2-yl]-2-{[1-(2,6-difluorophenyl)cyclopropyl]oxy}pyrimidine + SX, 3-[3-(3-cyclopropyl-2-fluorophenoxy)-6-methylpyridazin-4-yl]-5-[(2,4-dimethylphenyl)methyl]-5,6-dihydro-4H-1,2,4-oxadiazine + SX, (5S)-3-[3-(3-cyclopropyl-2-fluorophenoxy)-6-methylpyridazin-4-yl]-5-[(2,4-dimethylphenyl)methyl]-5,6-dihydro-4H-1,2,4-oxadiazine + SX, 3-[3-(3-chloro-2-fluorophenoxy)-6-methylpyridazin-4-yl]-5-[(4-bromo-2-methylphenyl)methyl]-5,6-dihydro-4H-1,2,4-oxadiazine + SX, 3-[3-(3-chloro-2-fluorophenoxy)-6-methylpyridazin-4-yl]-5-[(2-chloro-4-methylphenyl)methyl]-5,6-dihydro-4H-1,2,4-oxadiazine + SX, (5S)-3-[3-(3-chloro-2-fluorophenoxy)-6-methylpyridazin-4-yl]-5-[(2-chloro-4-methylphenyl)methyl]-5,6-dihydro-4H-1,2,4-oxadiazine + SX, (5R)-3-[3-(3-chloro-2-fluorophenoxy)-6-methylpyridazin-4-yl]-5-[(2-chloro-4-methylphenyl)methyl]-5,6-dihydro-4H-1,2,4-oxadiazine + SX, N-((2S)-1-{3-[2-(5-fluoro-2-methoxyphenyl)-2-hydroxyethyl]-5-[(E)-1-(isopropoxyimino)ethyl]-2,6-dioxo-3,6-dihydropyrimidin-1(2H)-yl}-3-methylbutan-2-yl)-2-methylpropanamide + SX, N-((2S)-1-{3-[2-(5-fluoro-2-methoxyphenyl)-2-hydroxyethyl]-5-[(E)-1-(isopropoxyimino)ethyl]-2,6-dioxo-3,6-dihydropyrimidin-1(2H)-yl}-3-methylbutan-2-yl)-2,2-dimethylpropanamide + SX, N-((2S)-1-{3-[2-(5-fluoro-2-methoxyphenyl)-2-hydroxyethyl]-5-[(E)-1-(isopropoxyimino)ethyl]-2,6-dioxo-3,6-dihydropyrimidin-1(2H)-yl}propan-2-yl)-2-methylpropanamide + SX, N-((2S)-1-{3-[2-(2-methoxyphenyl)-2-hydroxyethyl]-5-[(E)-1-(isopropoxyimino)ethyl]-2,6-dioxo-3,6-dihydropyrimidin-1(2H)-yl}propan-2-yl)-2-methylpropanamide + SX, N-((2S)-1-{3-[2-(5-fluoro-2-methoxyphenyl)-2-(2-cyanoethoxy)ethyl]-5-[1-(isopropoxyimino)ethyl]-2,6-dioxo-3,6-dihydropyrimidin-1(2H)-yl}propan-2-yl)-2-methylpropanamide + SX,
Agrobacterium radiobactor strain K1026 + SXAgrobacterium radiobactor strain K84 + SX, Bacillus amyloliquefaciens strain PTA-4838 (Aveo(trade mark) EZ Nematicide) + SX, Bacillus amyloliquefaciens strain AT332 + SX, Bacillus amyloliquefaciens strain B3 + SX, Bacillus amyloliquefaciens strain D747 + SX, Bacillus amyloliquefaciens strain DB101 + SX, Bacillus amyloliquefaciens strain DB102 + SX, Bacillus amyloliquefaciens strain GB03 + SX, Bacillus amyloliquefaciens strain FZB24 + SX, Bacillus amyloliquefaciens strain FZB42 + SX, Bacillus amyloliquefaciens strain IN937a + SX, Bacillus amyloliquefaciens strain MBI600 + SX, Bacillus amyloliquefaciens strain QST713 + SX, Bacillus amyloliquefaciens isolate strain B246 + SX, Bacillus amyloliquefaciens strain F727 + SX, Bacillus amyloliquefaciens subsp. plantarum strain D747 + SX, Bacillus licheniformis strain HB-2 + SX, Bacillus licheniformis strain SB3086 + SX, Bacillus pumilus strain AQ717 + SX, Bacillus pumilus strain BUF-33 + SX, Bacillus pumilus strain GB34 + SX, Bacillus pumilus strain QST2808 + SX, Bacillus simplex strain CGF2856 + SX, Bacillus subtilis strain AQ153 + SX, Bacillus subtilis strain AQ743 + SX, Bacillus subtilis strain BU1814 + SX, Bacillus subtilis strain D747 + SX, Bacillus subtilis strain DB101 + SX, Bacillus subtilis strain FZB24 + SX, Bacillus subtilis strain GB03 + SX, Bacillus subtilis strain HAI0404 + SX, Bacillus subtilis strain IAB/BS03 + SX, Bacillus subtilis strain MBI600 + SX, Bacillus subtilis strain QST30002/AQ30002 + SX, Bacillus subtilis strain QST30004/AQ30004 + SX, Bacillus subtilis strain QST713 + SX, Bacillus subtilis strain QST714 + SX, Bacillus subtilis var. Amyloliquefaciens strain FZB24 + SX, Bacillus subtilis strain Y1336 + SX, Burkholderia cepacia + SX, Burkholderia cepacia type Wisconsin strain J82 + SX, Burkholderia cepacia type Wisconsin strain M54 + SX, Candida oleophila strain O + SX, Candida saitoana + SX, Chaetomium cupreum + SX, Clonostachys rosea + SX, Coniothyrium minitans strain CGMCC8325 + SX, Coniothyrium minitans strain CON/M/91-8 + SX, cryptococcus albidus + SX, Erwinia carotovora subsp. carotovora strain CGE234M403 + SX, Fusarium oxysporum strain Fo47 + SX, Gliocladium catenulatum strain J1446 + SX, Paenibacillus polymyXa strain AC-1 + SX, Paenibacillus polymyXa strain BS-0105 + SX, Pantoea agglomerans strain E325 + SX, Phlebiopsis gigantea strain VRA1992 + SX, Pseudomonas aureofaciens strain TX1 + SX, Pseudomonas chlororaphis strain 63-28 + SX, Pseudomonas chlororaphis strain AFS009 + SX, Pseudomonas chlororaphis strain MA342 + SX, Pseudomonas fluorescens strain 1629RS + SX, Pseudomonas fluorescens strain A506 + SX, Pseudomonas fluorescens strain CL145A + SX, Pseudomonas fluorescens strain G7090 + SX, Pseudomonas sp. strain CAB-02 + SX, Pseudomonas syringae strain 742RS + SX, Pseudomonas syringae strain MA-4 + SX, Pseudozyma flocculosa strain PF-A22UL + SX, Pseudomonas rhodesiae strain HAI-0804 + SX, Pythium oligandrum strain DV74 + SX, Pythium oligandrum strain M1 + SX, Streptomyces griseoviridis strain K61 + SX, Streptomyces lydicus strain WYCD108US + SX, Streptomyces lydicus strain WYEC108 + SX, Talaromyces flavus strain SAY-Y-94-01 + SX, Talaromyces flavus strain V117b + SX, Trichoderma asperellum strain ICC012 + SX, Trichoderma asperellum SKT-1 + SX, Trichoderma asperellum strain T25 + SX, Trichoderma asperellum strain T34 + SX, Trichoderma asperellum strain TV1 + SX, Trichoderma atroviride strain CNCM 1-1237 + SX, Trichoderma atroviride strain LC52 + SX, Trichoderma atroviride strain IMI 206040 + SX, Trichoderma atroviride strain SC1 + SX, Trichoderma atroviride strain SKT-1 + SX, Trichoderma atroviride strain T11 + SX, Trichoderma gamsii strain ICC080 + SX, Trichoderma harzianum strain 21 + SX, Trichoderma harzianum strain DB104 + SX, Trichoderma harzianum strain DSM 14944 + SX, Trichoderma harzianum strain ESALQ-1303 + SX, Trichoderma harzianum strain ESALQ-1306 + SX, Trichoderma harzianum strain IIHR-Th-2 + SX, Trichoderma harzianum strain ITEM908 + SX, Trichoderma harzianum strain kd + SX, Trichoderma harzianum strain MO1 + SX, Trichoderma harzianum strain SF + SX, Trichoderma harzianum strain T22 + SX, Trichoderma harzianum strain T39 + SX, Trichoderma harzianum strain T78 + SX, Trichoderma harzianum strain TH35 + SX, Trichoderma polysporum strain IMI206039 + SX, trichoderma stromaticum + SX, Trichoderma virens strain G-41 + SX, Trichoderma virens strain GL-21 + SX, Trichoderma viride + SX, Variovorax paradoxus strain CGF4526 + SX, Harpin protein + SX.

A combination of the present ingredient in the above-mentioned Group (c) and the compound Z of the present invention:
1-methylcyclopropene + SX, 1,3-diphenylurea + SX, 2, 3, 5-triiodobenzoic acid + SX, IAA ((1H-indol-3-yl)acetic acid) + SX, IBA (4-(1H-indol-3-yl)butyric acid) + SX, MCPA (2-(4-chloro-2-methylphenoxy)acetic acid) + SX, MCPB (4-(4-chloro-2-methylphenoxy)butyric acid) + SX4-CPA (4-chlorophenoxyacetic acid) + SX, 5-aminolevulinic acid hydrochloride + SX, 6-benzylaminopurine + SX, abscisic acid + SXAVG (aminoethoxy vinylglycine) + SX, anisiflupurin + SX, ancymidol + SX, butralin + SX, calcium carbonate + SX, calcium chloride + SX, calcium formate + SX, calcium peroxide + SX, calcium polysulfide + SX, calcium sulfate + SX, chlormequat-chloride + SX, chlorpropham + SX, choline chloride + SX, cloprop + SX, cyanamide + SX, cyclanilide + SX, daminozide + SX, decan-1-ol + SX, dichlorprop + SX, dikegulac + SX, dimethipin + SX, diquat + SX, ethephon + SX, ethychlozate + SX, flumetralin + SX, flurprimidol + SX, forchlorfenuron + SX, formononetin + SX, Gibberellin A + SX, Gibberellin A3 + SX, inabenfide + SX, Kinetin + SX, lipochito oligosaccharide SP104 + SX, maleic hydrazide + SX, mefluidide) + SX, mepiquat-chloride + SX, oxidized glutathione + SX, paclobutrazol + SX, pendimethalin + SX, prohexadione-calcium + SX, prohydrojasmon + SX, pyraflufen-ethyl + SX, sintofen + sodium 1-naphthaleneacetate + SX, sodium cyanate + SX, thidiazuron + SX, triapenthenol + SX, tribufos + SX, trinexapac-ethyl + SX, uniconazole-P + SX, 2-(naphthalen-1-yl)acetamide + SX, [4-oxo-4-(2-phenylethyl)amino]butyic acid + SX, methyl [5-(trifluoromethyl)benzo[b]thiophen-2-carboxylate + SX, 3-[(6-chloro-4-phenylquinazolin-2-yl)amino)propane-1-ol + SX, Claroideoglomus etunicatum + SX, Claroideoglomus claroideum + SX, Funneliformis mosseae + SX, Gigaspora margarita + SX, Gigaspora rosea + SX, Glomus aggregatum + SX, Glomus deserticola + SX, Glomus monosporum + SX, Paraglomus brasillianum + SX, Rhizophagus clarus + SX, Rhizophagus intraradices RTI-801 + SX, Rhizophagus irregularis DAOM 197198 + SX, Azorhizobium caulinodans + SX, Azospirillum amazonense + SX, Azospirillum brasilense XOH + SX, Azospirillum brasilense Ab-V5 + SX, Azospirillum brasilense Ab-V6 + SX, Azospirillum caulinodans + SX, Azospirillum halopraeferens + SX, Azospirillum irakense + SX, Azospirillum lipoferum + SX, Bradyrhizobium elkanii SEMIA 587 + SX, Bradyrhizobium elkanii SEMIA 5019 + SX, Bradyrhizobium japonicum TA-11 + SX, Bradyrhizobium japonicum USDA 110 + SX, Bradyrhizobium liaoningense + SX, Bradyrhizobium lupini + SX, Delftia acidovorans RAY209 + SX, Mesorhizobium ciceri + SX, Mesorhizobium huakii + SX, Mesorhizobium loti + SX, Rhizobium etli + SX, Rhizobium galegae + SX, Rhizobium leguminosarum bv. Phaseoli + SX, Rhizobium leguminosarum bv. Trifolii + SX, Rhizobium leguminosarum bv. Viciae + SX, Rhizobium trifolii + SX, Rhizobium tropici + SX, Sinorhizobium fredii + SX, Sinorhizobium meliloti + SX, Zucchini Yellow Mosaik Virus weak strain + SX.

A combination of the present ingredient in the above-mentioned Group (d) and the compound Z of the present invention:
anthraquinone + SX, deet + SX, and icaridin + SX.

Examples of a ratio of the compound Z of the present invention to the present ingredient include, but are not particularly limited to 1000 : 1 to 1 : 1000, 500 : 1 to 1 : 500, 100 : 1 to 1 : 100, 50 : 1, 20 : 1, 10 : 1, 9 : 1, 8 : 1, 7 : 1, 6 : 1, 5 : 1, 4 : 1, 3 : 1, 2 : 1, 1 : 1, 1 : 2, 1 : 3, 1 : 4, 1 : 5, 1 : 6, 1 : 7, 1 : 8, 1 : 9, 1 : 10, 1 : 20, and 1 : 50 in the weight ratio (the compound of the present invention : the present ingredient).

The present compound, the present compound Y, the compound of the present invention, the compound Z of the present invention, or the composition A can control plant diseases caused by phytopathogenic microorganisms (such as fungi, Oomycete, Phytomyxea, and bacteria). Examples of the fungi include Ascomycota, Basidiomycota, Blasocladiomycota, Chytridiomycota, Mucoromycota, and Olpidiomycota. Specific examples of plant diseases include the followings. The descriptions in a parenthesis indicates an academic name of phytopathogenic microorganism that causes each of the disease.

Rice diseases:
   blast (Pyricularia oryzae), brown spot (Cochliobolus miyabeanus), sheath blight (Rhizoctonia solani), bakanae disease (Gibberella fujikuroi), downy mildew (Sclerophthora macrospora), false blast and head blight (Epicoccum nigrum), seedling blight(Trichoderma viride, Rhizopus oryzae), bordered sheath spot (Waitea circinata), brown sclerotium disease (Ceratobasidium setariae), brown sheath blight (Thanatephorus cucumeris), rice false smut (Ustilaginoidea virens), and kernel smut (Tilletia horrida, Tilletia barclayana) ;
Wheat diseases:
   powdery mildew (Blumeria graminis), fusarium blight (Fusarium graminearum, Fusarium avenaceum, Fusarium culmorum, Microdochium nivale), stripe rust (Puccinia striiformis), stem rust (Puccinia graminis), leaf rust (Puccinia recondita), snow mould (Microdochium nivale, Microdochium majus), typhula snow blight (Typhula incarnata, Typhula ishikariensis), Sclerotinia snow blight (Sclerotinia borealis), Pythium snow blight (Pythium spp.), loose smut (Ustilago tritici), stinking smut (Tilletia caries, Tilletia controversa), eyespot (Pseudocercosporella herpotrichoides ), leaf blotch (Septoria tritici), glume blotch (Stagonospora nodorum), tan spot (Pyrenophora tritici-repentis), rhizoctonia seeding blight (Rhizoctonia solani), take-all disease (Gaeumannomyces graminis), and blast (Pyricularia graminis-tritici);
Barley diseases:
   powdery mildew (Blumeria graminis), fusarium head blight (Fusarium graminearum, Fusarium avenaceum, Fusarium culmorum, Microdochium nivale), stripe rust (Puccinia striiformis), stem rust (Puccinia graminis), dwarf leaf rust (Puccinia hordei), loose smut (Ustilago nuda), scald (Rhynchosporium secalis), net blotch (Pyrenophora teres), spot blotch (Cochliobolus sativus), stripe (Pyrenophora graminea), Ramularia disease (Ramularia collo-cygni), and rhizoctonia seeding blight (Rhizoctonia solani);
Corn diseases:
   rust (Puccinia sorghi), southern rust (Puccinia polysora), northern leaf blight (Setosphaeria turcica), tropical rust (Physopella zeae), southern leaf blight (Cochliobolus heterostrophus), anthracnose (Colletotrichum graminicola), gray leaf spot (Cercospora zeae-maydis), eyespot (Kabatiella zeae), phaeosphaeria leaf spot (Phaeosphaeria maydis), diplodia rot (Stenocarpella maydis, Stenocarpella macrospora), stalk rot (Fusarium graminearum, Fusarium verticilioides, Colletotrichum graminicola), smut (Ustilago maydis), and Physoderma brown spot and Physoderma stalk rot (Physoderma maydis);
Cotton diseases:
   anthracnose (Colletotrichum gossypii), grey mildew (Ramularia areola), alternaria leaf spot (Alternaria macrospora, Alternaria gossypii), and black root rot (Thielaviopsis basicola);
Coffee diseases:
   rust (Hemileia vastatrix), and leaf spot (Cercospora coffeicola);
Rape seed diseases:
   sclerotinia rot (Sclerotinia sclerotiorum), gray leaf spot (Alternaria brassicae), root rot (Phoma lingam), and light leaf spot (Pyrenopeziza brassicae);
Sugar cane diseases:
   rust (Puccinia melanocephela, Puccinia kuehnii), and smut (Ustilago scitaminea);
Sunflower diseases:
   rust (Puccinia helianthi), and downy mildew (Plasmopara halstedii);
Citrus diseases:
   melanose (Diaporthe citri), scab (Elsinoe fawcetti), green mold (Penicillium digitatum), blue mold (Penicillium italicum), Phytophthora rot (Phytophthora parasitica, Phytophthora citrophthora), and aspergillus rot (Aspergillus niger);
Apple diseases:
   blossom blight (Monilinia mali), valsa canker (Valsa ceratosperma), powdery mildew (Podosphaera leucotricha), alternaria leaf spot (Alternaria alternata apple pathotype), scab (Venturia inaequalis), bitter rot (Glomerella cingulata, Colletotrichum acutatum), blotch (Diplocarpon mali), ring rot (Botryosphaeria berengeriana), crown rot (Phytophtora cactorum), and rust (Gymnosporangium juniperi-virginianae, Gymnosporangium yamadae);
Pear diseases:
   scab (Venturia nashicola, Venturia pirina), black spot (Alternaria alternata Japanese pear pathotype), and rust (Gymnosporangium haraeanum);
Peach diseases:
   brown rot (Monilinia fructicola), scab (Cladosporium carpophilum), Phomopsis rot (Phomopsis sp.), leaf curl (Taphrina deformans), and powdery mildew (Podosphaera leucotricha);
Grapes diseases:
   anthracnose (Elsinoe ampelina), ripe rot (Glomerella cingulata, Colletotrichum acutatum), powdery mildew (Uncinula necator), rust (Phakopsora ampelopsidis), black rot (Guignardia bidwellii), downy mildew (Plasmopara viticola), brown spot (Pseudocercospora vitis), and white rot (Coniella castaneicola);
Japanese persimmon diseases:
   anthracnose (Gloeosporium kaki, Colletotrichum acutatum), leaf spot (Cercospora kaki, Mycosphaerella nawae), and powdery mildew (Phyllactinia kakicola);
Diseases of gourd family:
   anthracnose (Colletotrichum lagenarium), powdery mildew (Sphaerotheca fuliginea), gummy stem blight (Didymella bryoniae), Corynespora leaf spot (Corynespora cassiicola), fusarium wilt (Fusarium oxysporum), downy mildew (Pseudoperonospora cubensis), phytophthora rot (Phytophthora capsici), damping-off (Pythium sp.), and Phomopsis root rot (Phomopsis sp.);
Tomato diseases:
   early blight (Alternaria solani), leaf mold (Cladosporium fulvum), Cercospora leaf mold (Pseudocercospora fuligena), late blight (Phytophthora infestans), and powdery mildew (Leveillula taurica);
Eggplant diseases:
   brown spot (Phomopsis vexans), powdery mildew (Erysiphe cichoracearum), black blight (Corynespora melongenae), leaf mold (Mycovellosiella nattrassii), and brown leaf spot (Thanatephorus cucumeris);
Cruciferous vegetables diseases:
   alternaria leaf spot (Alternaria japonica), white spot (Cercosporella brassicae), clubroot (Plasmodiophora brassicae), downy mildew (Peronospora parasitica), white rust (Albugo candida), and black spot (Alternaria brassicicola);
Welsh onion disease:
   rust (Puccinia allii), alternaria leaf spot (Alternaria porri), white rot (Sclerotium cepivorum), neck rot (Botrytis squamosa), leaf blotch (Stemphylium vesicarium, Stemphylium botryosum), southern blight (Sclerotium rolfsii), and leaf blight (Botrytis squamosa);
Soybean diseases:
   purple stain (Cercospora kikuchii), sphaceloma scab (Elsinoe glycines), pod and stem blight (Diaporthe phaseolorum var. sojae), rust (Phakopsora pachyrhizi), target spot (Corynespora cassiicola), anthracnose (Colletotrichum glycines, Colletotrichum truncatum), Rhizoctonia rot (Rhizoctonia solani), septoria brown spot (Septoria glycines), Cercospora leaf spot (Cercospora sojina), stem rot (Sclerotinia sclerotiorum), powdery mildew (Microsphaera diffusa), phytophthora stem and root rot (Phytophthora sojae), downy mildew (Peronospora manshurica), sudden death syndrome (Fusarium virguliforme), red crown rot (Calonectria ilicicola), and Diaporthe/Phomopsis complex (Diaporthe longicolla, Diaporthe phaseolorum, Phomopsis longicolla);
Kidney bean diseases:
   stem rot (Sclerotinia sclerotiorum), rust (Uromyces appendiculatus), angular leaf spot (Phaeoisariopsis griseola), and anthracnose (Colletotrichum lindemuthianum), and Fusarium root-rot (Fusarium solani);
Peanut diseases:
   leaf spot (Cercospora personata), brown leaf spot (Cercospora arachidicola), southern blight (Sclerotium rolfsii), and Cylindrocladium black rot (Calonectria ilicicola);
Garden pea disease:
   powdery mildew (Erysiphe pisi), and root rot (Fusarium solani);
Potato diseases:
   early blight (Alternaria solani), late blight (Phytophthora infestans), Pink rot (Phytophthora erythroseptica), powdery scab (Spongospora subterranea f. sp. subterranea), verticillium wilt (Verticillium albo-atrum, Verticillium dahliae, Verticillium nigrescens), dry rot (Fusarium solani), and potato wart (Synchytrium endobioticum);
Strawberry disease:
   powdery mildew (Sphaerotheca humuli), and strawberry anthracnose (Glomerella cingulata, Colletotrichum acutatum);
Tea diseases:
   net blister blight (Exobasidium reticulatum), white scab (Elsinoe leucospila), gray blight (Pestalotiopsis sp.), and anthracnose (Colletotrichum theae-sinensis);
Tobacco diseases:
   brown spot (Alternaria longipes), anthracnose (Colletotrichum tabacum), blue mold (Peronospora tabacina), and black shank (Phytophthora nicotianae);
Sugar beet diseases:
   cercospora leaf spot (Cercospora beticola), leaf blight (Thanatephorus cucumeris), root rot (Thanatephorus cucumeris), and aphanomyces root rot (Aphanomyces cochlioides), and rust (Uromyces betae);
Rose diseases:
   black spot (Diplocarpon rosae), and powdery mildew (Sphaerotheca pannosa);
Chrysanthemum diseases:
   leaf blight (Septoria chrysanthemi-indici), and white rust (Puccinia horiana);
Onion diseases:
   botrytis leaf blight (Botrytis cinerea, Botrytis byssoidea, Botrytis squamosa), gray-mold neck rot (Botrytis allii), and small sclerotial neck rot (Botrytis squamosa);
Various crops diseases:
   Botrytis rot (Botrytis cinerea), sclerotinia rot (Sclerotinia sclerotiorum), seedling blight (Pythium aphanidermatum, Pythium irregulare, Pythium ultimum);
Japanese radish disease:
   alternaria leaf spot (Alternaria brassicicola);
Sweet potato disease:
   stem rot (Fusarium oxysporum f. sp. Batatas), foot rot (Diaporthe destruens), and black rot (Ceratocystis fimbriata);
Turfgrass diseases:
   Dollar spot (Sclerotinia homoeocarpa), brown patch and large patch (Rhizoctonia solani), pythium bligt (Pythium aphanidermatum), and grass anthracnose (Colletotrichum caudatum);
Banana disease:
   Sigatoka disease (Mycosphaerella fijiensis, Mycosphaerella musicola);
Lentils disease:
   ascochyta blight (Ascochyta lentis);
Chickpea disease:
   ascochyta blight (Ascochyta rabiei);
Green pepper disease:
   anthracnose (Colletotrichum scovillei), powdery mildew (Leveillula taurica), and southern blight (Sclerotium rolfsii).
Mango disease:
   anthracnose (Colletotrichum acutatum);
Sweet potato disease:
   foot rot (Diaporthe destruens);
Fruit trees diseases:
   white root rot (Rosellinia necatrix), and violet root rot (Helicobasidium mompa);
Postharvest disease of fruits (for example, apple and pear):
   Mucor rot diseases (Mucor piriformis);
Seed diseases or diseases in the early stages of the growth of various plants caused by Aspergillus spp., Penicillium spp., Fusarium spp., Gibberella spp., Tricoderma spp., Thielaviopsis spp., Rhizopus spp., Mucor spp., Corticium spp., Phoma spp., Rhizoctonia spp. or Diplodia spp.; and the like;
Viral diseases:
   Lettuce big-vein disease transmitted by Olpidium brassicae, and viral diseases of several crops transmitted by Polymixa spp. (e.g. Polymyxa betae and Polymyxa graminis);
Diseases caused by bacteria:
   bacterial seedling blight of rice (Burkholderia plantarii), bacterial palea browning of rice (Pantoea ananatis), bacterial leaf blight of rice (Xanthomonas oryzae pv. oryzae.), bacterial spot of cucumber (Pseudomonas syringae pv. lachrymans), bacterial wilt of eggplant (Ralstonia solanacearum), canker of citrus (Xanthomonas citri), bacterial soft rot of Chinese cabbage (Erwinia carotovora), scab of potato (Streptomyces scabiei), scholar of potato (Pectobacterium carotovorum, Dickeya sp.), bacterial leaf spot of peach (Pseudomona syringae, Erwinia nigrifluens, Xanthomonas campestris), and bacterial canker of Japanese apricot (Pseudomonas syringae pv. morsprunorum, Erwinia sp.), Goss's wilt of corn (Clavibacter michiganensis), Pierce's disease of grapes, olive and peach (Xylella fastidiosa), andcrown gall of Rosaceae plants such as apple, peach, cherries (Agrobacterium tumefaciens).

The term of "soybean rust fungus having an amino acid substitution of F129L on mitochondrial cytochrome b protein" represents soybean rust fungus (scientific name: *Phakopsora pachyrhizi*) which shows a resistance against QoI fungicide by having a mutation in the mitochondrial cytochrome b gene encoding mitochondrial cytochrome protein and as a result of the mutation, causing amino acid substitution of F129L.

The method for controlling plant diseases of the present invention is carried out by applying an effective amount of the present compound, the present compound Y, the compound of the present invention, the compound Z of the present invention, or the composition A to a plant or soil for cultivating a plant. Examples of the application method include foliar application, soil application, and seed application.

The present compound, the present compound Y, the compound of the present invention, the compound Z of the present invention, or the composition A is usually used by mixing it with inert carrier(s) such as solid carrier(s) and liquid carrier(s), and surfactant(s), and the like, and as needed, adding thereto auxiliary agent(s) for formulation such as binder(s), dispersant(s), and stabilizer(s) to be formulated into an aqueous suspension formulation, an oily suspension formulation, an oil solution, an emulsifiable concentrate, an emulsion formulation, a microemulsion formulation, a microcapsule formulation, a wettable powder, a granular wettable powder, a dust formulation, a granule, or the like. In addition to these formulations, the Present compound or the Composition A may be used by formulating it into a dosage form described in Manual on development and use of FAO and WHO Specifications for pesticides, FAO Plant Production and Protection Papers- 271-276, prepared by the FAO/WHO Joint Meeting on Pesticide Specifications, 2016, ISSN:0259-2517.

These formulations usually comprise 0.0001 to 99% by weight ratio of the present compound, the present compound Y, the compound of the present invention, the compound Z of the present invention, or the composition A.

Examples of the solid carrier include fine powders and granules of clays (for example, pyrophyllite clay and kaolin clay), talc, calcium carbonate, diatomaceous earth, zeolite, bentonite, acid white clay, attapulgite, white carbon, ammonium sulfate, vermiculite, perlite, pumice, silica sand, chemical fertilizers (for example, ammonium sulfate, ammonium phosphate, ammonium nitrate, urea, and ammonium chloride), and the others; as well as resins (for example, polyethylene, polypropylene, polyester, polyurethane, polyamide, and polyvinyl chloride).

Examples of the liquid carrier include water, alcohols (for example, ethanol, cyclohexanol, benzyl alcohol, propylene glycol, and polyethylene glycol), ketones (for example, acetone and cyclohexanone), aromatic hydrocarbons (for example, xylene, phenyl xylyl ethane, and methylnaphthalene), aliphatic hydrocarbons (for example, hexane and cyclohexane), esters (for example, ethyl acetate, methyl oleate, and propylene carbonate), nitriles (for example, acetonitrile), ethers (for example, ethylene glycol dimethyl ether), amides (for example, N,N-dimethylformamide and N,N-dimethyloctanamide), sulfoxides (for example, dimethyl sulfoxide), lactams (for example, N-methylpyrrolidone and N-octylpyrrolidone), fatty acids (for example, oleic acid), and vegetable oils (for example, soybean oil).

Examples of the surfactant include nonionic surfactants (for example, polyoxyethylene alkyl ethers, polyoxyethylene alkyl aryl ethers, and polyethylene glycol fatty acid esters), and anionic surfactants (for example, alkyl sulfonates, alkyl aryl sulfonates, and alkyl sulfates).

Examples of the other auxiliary agent for formulation include binders, dispersants, colorants, and stabilizers, and the specific examples thereof include polysaccharides (for example, starch, gum arabic, cellulose derivatives, and alginic acid), lignin derivatives, water-soluble synthetic polymers (for example, polyvinyl alcohol, polyvinyl pyrrolidone, and polyacrylic acids), acidic isopropyl phosphate, and dibutylhydroxytoluene.

Moreover, some adjuvants can be employed to improve or help the efficacy of the present compound, the present compound Y, the compound of the present invention, or the composition A. The specific examples thereof include Nimbus (registered trademark), Assist (registered trademark), Aureo (registered trademark), Iharol (registered trademark), Silwet L-77 (registered trademark), BreakThru (registered trademark), SundanceII (registered trademark), Induce (registered trademark), Penetrator (registered trademark), AgriDex (registered trademark), Lutensol A8 (registered trademark), NP-7 (registered trademark), Triton (registered trademark), Nufilm (registered trademark), Emulgator NP7 (registered trademark), Emulad (registered trademark), TRITON X 45 (registered trademark), AGRAL 90 (registered trademark), AGROTIN (registered trademark), ARPON (registered trademark), EnSpray N (registered trademark), and BANOLE (registered trademark).

The plant as used herein include whole plant, and a certain part of the plant. Examples of the certain part of the plant include stem and leaf, flower, ear, fruit, tree stem, branch, crown, seed, vegetative reproductive organ, and seedling.

A vegetative reproduction organ means a part of plant such as root, stem, and leaf which has a growth capability even when said part is separated from the plant body and placed into soil. Examples of the vegetative reproduction organ include tuberous root, creeping root, bulb, corm or solid bulb, tuber, rhizome, stolon, rhizophore, cane cuttings, propagule, and vine cutting. Stolon is also referred to as "runner", and propagule is also referred to as "propagulum" and categorized into broad bud and bulbil. Vine cutting means a shoot (collective term of leaf and stem) of sweet potato, glutinous yam, or the like. Bulb, corm or solid bulb, tuber, rhizome, cane cuttings, rhizophore, and tuberous root are also collectively referred to as "bulb". For example, cultivation of potato starts with planting a tuber into soil, and the tuber to be used is generally referred to as "seed potato".

The seedling as used herein include a seedling and a sapling.

Examples of a method for controlling plant diseases by applying an effective amount of the present compound, the present compound Y, the compound of the present invention, the compound Z of the present invention, or the composition A to soils include a method of applying an effective amount of the present compound, the present compound Y, the compound of the present invention, the compound Z of the present invention, or the composition A to soils before planting plants or after planting plants. Specific examples of the application method include planting hole treatment (spraying into planting holes, soil mixing after planting hole treatment), plant foot treatment (plant foot spraying, soil mixing after plant foot treatment, irrigation at plant foot, plant foot treatment at a later seeding raising stage), planting furrow treatment (planting furrow spraying, soil mixing after planting furrow treatment), planting row treatment (planting row spraying, soil mixing after planting row treatment, planting row spraying at a growing stage), planting row treatment at the time of sowing (planting row spraying at the time of sowing, soil mixing after planting row treatment at the time of sowing), broadcast treatment (overall soil surface spraying, soil mixing after broadcast treatment), side-article treatment, treatment of water surface (application to water surface, application to water surface after flooding), other soil spraying treatment (spraying of a granular formulation on leaves at a growing stage, spraying under a canopy or around a tree stem, spraying on the soil surface, mixing with surface soil, spraying into seed holes, spraying on the ground surfaces of furrows, spraying between plants), other irrigation treatment (soil irrigation, irrigation at a seedling raising stage, chemical solution injection treatment, irrigation of a plant part just above the ground, chemical solution drip irrigation, chemigation), seedling raising box treatment (spraying into a seedling raising box, irrigation of a seedling raising box, flooding into a seedling raising box with chemical solution), seedling raising tray treatment (spraying on a seedling raising tray, irrigation of a seedling raising tray, flooding into a seedling raising tray with chemical solution), seedbed treatment (spraying on a seedbed, irrigation of a seedbed, spraying on a lowland rice nursery, immersion of seedlings), seedbed soil incorporation treatment (mixing with seedbed soil, mixing with seedbed soil before sowing, spraying at sowing before covering with soils, spraying at sowing after covering with soils, mixing with covering with soils), and other treatment (mixing with culture soil, plowing under, mixing with surface soil, mixing with soil at the place where raindrops fall from a canopy, treatment at a planting position, spraying of a granule formulation on flower clusters, mixing with a paste fertilizer).

Examples of the application to seeds (or seed treatments) include an application of the present compound, the present compound Y, the compound of the present invention, the compound Z of the present invention, or the composition A to seeds or vegetative reproductive organs, and specific examples thereof include spraying treatment in which a suspension of the present compound, the present compound Y, the compound of the present invention, the compound Z of the present invention, or the composition A is sprayed onto seed surface or the vegetative reproductive organ surface in the form of mist; smearing treatment in which the Present compound, the present compound Y, the compound of the present invention, the compound Z of the present invention, or the composition A is coated a surface of seeds or the vegetative reproductive organ; a soaking treatment in which the seeds are soaked into the solution of the present compound, the present compound Y, the compound of the present invention, the compound Z of the present invention, or the composition A for a certain time; and a method for coating the seeds or the vegetative reproductive organ with a carrier containing the present compound, the present compound Y, the compound of the present invention, the compound Z of the present invention, or the composition A (film coating treatment, pellet coating treatment). Examples of the above-described vegetative reproductive organ include particularly seed potato.

As used herein, seeds or vegetative reproductive organs carrying the present compound, the present compound Y, the compound of the present invention, the compound Z of the present invention, or the composition A means seeds or vegetative reproductive organs in the state where the present compound, the present compound Y, the compound of the present invention, the compound Z of the present invention, or the composition A is adhered to a surface of the seeds or the vegetative reproductive organ. The above-described seeds or vegetative reproductive organs carrying the present compound, the present compound Y, the compound of the present invention, the compound Z of the present invention, or the composition A may be adhered by any other materials that are different from the present compound, the present compound Y, the compound of the present invention, the compound Z of the present invention, or the composition A before or after being adhered the present compound, the present compound Y, the compound of the present invention, the compound Z of the present invention, or the composition A to the seeds or vegetative reproductive organs.

When the composition A is applied to seeds or vegetative reproductive organs, the composition A may be also applied to seeds or vegetative reproductive organs as a single formulation, or the Composition A may be applied to seeds or vegetative reproductive organs as multiple different formulations by multiple times. Examples of the method in which the Composition A is applied as multiple different formulations by multiple times include, for example, a method in which the formulations comprising as an active component the Present compound only are applied, and seeds or vegetative reproductive organs are air dried, followed by applying the formulations comprising the Present ingredient: and a method in which the formulations comprising as an active component the present compound and the Present ingredients are applied, and seeds or vegetative reproductive organs are air dried, followed by applying the formulations comprising the Present ingredients other than the already-applied Present ingredients, are included.

Also, when the composition A is adhered in a form of layer(s) to a surface of seeds or vegetative reproductive organ, the layer(s) is/are composed of one layer or a multiple layers. Also, when multiple layers are formed, each of the layer may be composed of a layer comprising one or more active ingredients, or a combination of a layer comprising one or more active ingredients and a layer not comprising an active ingredient.

Seeds or vegetative reproductive organs carrying the present compound, the present compound Y, the compound of the present invention, the compound Z of the present invention, or the composition A can be obtained, for example, by applying the formulations comprising the present compound, the present compound Y, the compound of the present invention, the compound Z of the present invention, or the composition A by the above-described application method to seeds to seeds or vegetative reproductive organs.

The application dose of the present compound, the present compound Y, the compound of the present invention, the compound Z of the present invention, or the composition A may be varied depending on the climate condition, the formulation forms, the application timing, the application method, the application area, the target diseases, or the target crops, and the like, and in the case of the foliar application or soil application, a dose thereof is within a range of 1 to 500 g per 1,000 m². In the case of being applied to seeds, the dose of application dose of the present compound, the present compound Y, the compound of the present invention, the compound Z of the present invention, or the composition A is usually within a range of 0.001 to 100 g of the Present compound per 1 Kg of seeds. When the present compound, the present compound Y, the compound of the present invention, the compound Z of the present invention, or the composition A is formulated into an emulsifiable concentrate, a wettable powder or a flowable etc., they are usually applied by diluting them with water so as to make an effective concentration of the active ingredients 0.01 to 10,000 ppm, and the dust formulation or the granular formulation, etc., is usually applied as itself without diluting them.

The present compound, the present compound Y, the compound of the present invention, the compound Z of the present invention, or the composition A can be used as an agent for controlling plant diseases in the agricultural land such as field, paddy, lawn and orchard. Examples of the plants include the followings.

corn (dent corn, flint corn, flour corn, popcorn, waxy corn, sweet corn, and field corn), rice (long grain rice, short grain rice, medium grain rice, japonica rice, tropical japonica rice, indica rice, javanica rice, paddy rice, upland rice, floating rice, direct-seeded rice, transplanted rice, and glutinous rice), wheat (bread wheat (hard wheat, soft wheat, medium wheat, red wheat, and white wheat), durum wheat, spelt wheat, and club wheat, winter wheat and spring wheat of them), barley (two-rowed barley (= barley for brewery), six-rowed barley, hull-less barley, and pearl barley, winter barley and spring barley of them), rye (winter rye and spring rye), triticale (winter triticale and spring triticale), oat (winter oat and spring oat), sorghum, cotton (upland cotton and Pima cotton), soybean (ripe seed harvest soybean, green soybeans, and early harvest soybeans, indeterminate type, determinate type, and semi-determinate type of them), peanut, buckwheat, beet (beets for sugar production, beets for feed, beets for root vegetable, beets for leaf vegetable, and beets for fuel), rapeseed (winter rapeseed and spring rapeseed), canola (winter canola and spring canola), sunflower (sunflowers for oil extraction, edible sunflowers, and sunflowers for ornamental purpose), sugar cane, tobacco, tea, mulberry, solanaceous vegetables (for example, eggplant, tomato, pimento, pepper, and potato), cucurbitaceous vegetables (for example, cucumber, pumpkin, zucchini, water melon, and melon), cruciferous vegetables (for example, Japanese radish, white turnip, horseradish, kohlrabi, Chinese cabbage, cabbage, leaf mustard, broccoli, and cauliflower), asteraceous vegetables (for example, burdock, crown daisy, artichoke, and lettuce), liliaceous vegetables (for example, welsh onion, onion, garlic, and asparagus), ammiaceous vegetables (for example, carrot, parsley, celery, and parsnip), chenopodiaceous vegetables (for example, spinach and Swiss chard), lamiaceous vegetables (for example, perilla, mint, and basil), strawberry, sweet potato, glutinous yam, eddoe, pomaceous fruits (for example, apple, pear, Japanese pear, Chinese white pear, Chinese quince, and quince), stone fleshy fruits (for example, peach, plum, nectarine, Japanese apricot (Prunus mume), cherry fruit, apricot, and prune), citrus fruits (for example, Citrus unshiu, orange, lemon, lime, and grapefruit), nuts (for example, chestnuts, walnuts, hazelnuts, almond, pistachio, cashew nuts, and macadamia nuts), berry fruits (for example, blueberry, cranberry, blackberry, and raspberry), grapes, Japanese persimmon, fig, olive, Japanese plum, banana, coffee, date palm, coconuts, ornamental plants, forest plants, turfs, grasses, and the others.

The above plants are not specifically limited as long as they are generally cultivated cultivars. The above plants also include plants which may be produced by natural breeding, plants which may be generated by mutation, F1 hybrid plants, and genetically modified crops. Examples of the genetically modified crops include plants which have resistance to HPPD (4-hydroxyphenylpyruvate dioxygenase enzyme) inhibitors such as isoxaflutole, ALS (acetolactate synthase) inhibitors such as imazethapyr and thifensulfuron-methyl, EPSP (5-enolpyruvylshikimate-3-phosphate synthase) inhibitors, glutamine synthetase inhibitors, PPO (protoporphyrinogen oxidase) inhibitors, or herbicide such as bromoxynil and dicamba; plants which can synthesize a selective toxin known in Bacillus spp. such as Bacillus thuringiensis or the like; and plants which can synthesize a gene fragment or the like which is partially identical to an endogenous gene derived from a harmful insect, and induce a gene silencing (RNAi; RNA interference) in the target harmful insect to achieve a specific insecticidal activity.

### EXAMPLES

Hereinafter, the present invention is explained in more detail by using Preparation Examples, Reference Preparation Examples, Formulation Examples, and Test Examples, etc., however, the present invention should not be limited to these examples.

As used herein, "Me" represents a methyl group, "Et" represents an ethyl group, "Pr" represents a propyl group, "i-Pr" represents an isopropyl group, "c-Pr" represents a cyclopropyl group, "Bu" represents a butyl group, "i-Bu" represents an isobutyl group, "s-Bu" represents a sec-butyl group, "c-Pn" represents a cyclopentyl group, "Ph" represents a phenyl group, "Bn" represents a benzyl group. When the phenyl group has a substituent, the substituent is written with its substituted position before the symbol. For example, "4-F-Ph" represents a 4-fluorophenyl group, and "4-Me O-Ph" represents a 4-methoxyphenyl group.

In the case where a physical property value of a compound is measured by a liquid chromatography / Mass analysis (hereinafter, referred to as "LCMS"), the measured molecular ion value [M+H]⁺ or [M-H]⁻and a retention time (hereinafter, referred to as "RT") is described. The condition for liquid chromatography (hereinafter, referred to as "LC") and Mass analysis (hereinafter, referred to as "MS") are shown below.

### [LC condition]

Column: L-column2 ODS, inner diameter 4.6 mm, length 30 mm, particle size 3 µm (Chemicals Evaluation and Research Institute, Japan)
UV measurement wavelength: 254 nm
Mobile phase: A solution: 0.1 % aqueous solution of formic acid, B solution: 0.1 % acetonitrile solution of formic acid
Flow rates: 2.0 mL/min.
Pump: LC-20 AD (manufactured by Shimadzu Corporation) two machines (high pressure gradient)
Gradient condition: a solution for elution was transferred at a concentration gradient indicated in [Table LC1] .

**[Table LC1]**

| Time (min) | A solution (%) | B solution (%) |
|---|---|---|
| 0.01 | 90 | 10 |
| 2.00 | 0 | 100 |
| 4.00 | 0 | 100 |
| 4.01 | 90 | 10 |

### [MS condition]

Detector: LCMS-2020 (manufactured by Shimadzu Corporation)
Ionization Methods: DUIS

In the case where a physical property value of a compound is measured by gas chromatography (GC) / Mass analysis (MS) (hereinafter, referred to as "GCMS"), the measured molecular ion value [M]⁺ and a retention time (hereinafter, referred to as "RT") is described. The condition for GC is shown below.

### [GC Analysis condition]

Device: HP 7890A (manufactured by Agilent Technologies, Ltd.)
Column: HP-5MS (0.25 µm × 0.25 mm × 30 m) (manufactured by Agilent Technologies, Ltd.)
Inlet temperature: 250 °C
Control mode: linear velocity, Pressure: 14.7 psi, Linear velocity: 23.98 cm/sec
Carrier gas: helium
Flow volume: 1 mL/min
Injection volume: 1.0 µL (split ratio 200 : 1)
Oven temperature: 70 °C (2 min) → 15 °C/min → 300 °C (2.67 min)

### [MS analysis condition]

Detector: 5875C inert XL MSD (manufactured by Agilent Technologies, Ltd.)
Ionization method: EI
Ionization voltage: 69.9 eV, Ion source temperature: 230 °C

First, a preparation example of the Present compounds Y (including the Present compounds, the compounds of the present invention, and the compounds Z of the present invention) is shown.

### Preparation Example 1

1-Propanethiol 0.22 mL was added to a mixture of 2-chloro-4-(3-fluoro-4-methylpheny)pyrimidine 0.50 g, potassium carbonate 0.62 g, and DMF 5 mL at 0 °C, and the mixture was stirred at room temperature for 5 hours. Water was added to the resulting mixture, and the mixture was extracted with ethyl acetate. The resulting mixture was washed with saturated brine, dried over anhydrous sodium sulfate, and concentrated under reduced pressure. The resulting residue was subjected to a silica gel column chromatography (hexane : ethyl acetate = 6 : 4) to obtain the present compound 1 represented by the following formula 0.55 g. 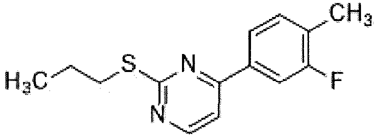 Present compound 1: ¹H-NMR (CDCl₃) δ: 8.53 (1H, d), 7.80-7.72 (2H, m), 7.33-7.27 (2H, m), 3.21 (2H, t), 2.35 (3H, d), 1.89-1.77 (2H, m), 1.09 (3H, t).

### Preparation Example 1-1

The compounds which were prepared according to the Preparation Example 1 and their physical property values are shown.

A compound represented by formula (IA-1): 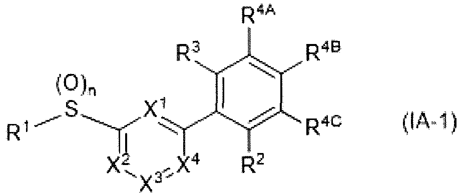 (hereinafter, referred to as "Compound (IA-1)") wherein a combination of n, X¹, X², X³, X⁴, R¹, R², R³, R^{4A}, R^{4B}, and R^{4C} represents any combination described in [Table A-1].

**[Table A-1]**

| Present compound | n | X¹ | X² | X³ | X⁴ | R¹ | R² | R³ | R^{4A} | R^{4B} | R^{4C} |
|---|---|---|---|---|---|---|---|---|---|---|---|
| 2 | 0 | N | CH | CH | CH | Et | H | H | H | Cl | H |
| 3 | 0 | CH | N | CH | CH | Pr | H | H | H | Me | F |
| 67 | 0 | CH | N | N | CH | Pr | H | H | H | Me | F |

Present compound 2: ¹H-NMR (CDCl₃) δ: 8.01-7.95 (2H, m), 7.53 (1H, t), 7.46-7.36 (3H, m), 7.11 (1H, dd), 3.28 (2H, q), 1.44 (3H, t).
Present compound 3: ¹H-NMR (CDCl₃) δ: 8.45 (1H, dd), 7.34 (1H, dd), 7.31-7.24 (3H, m), 7.14 (1H, dd), 3.19 (2H, t), 2.33 (3H, d), 1.82-1.71 (2H, m), 1.06 (3H, t).
Present compound 67: ¹H-NMR (CDCl₃) δ: 8.96 (1H, d), 7.78-7.68 (2H, m), 7.52 (1H, d), 7.32 (1H, t), 3.06 (2H, t), 2.35 (3H, d), 1.88-1.76 (2H, m), 1.12 (3H, t).

### Preparation Example 2

A mixture of 2-bromo-4-(propylthio)pyridine 0.50 g, 3-fluoro-4-methylphenyl boronic acid 0.39 g, {1,1'-bis(diphenylphosphino)ferrocene}dichloropalladium (II) 0.15 g, tripotassium phosphate 0.91 g, DME 9 mL and water 1 mL was stirred at reflux for 5 hours. Water was added to the resulting mixture, and the mixture was extracted with ethyl acetate. The resulting organic layer was concentrated under reduced pressure, and the resulting residue was subjected to a silica gel column chromatography (hexane : ethyl acetate = 10 : 1) to obtain the present compound 4 represented by the following formula 0.53 g. 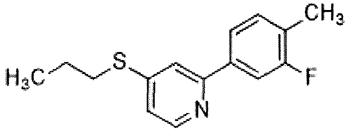 Present compound 4: ¹H-NMR (CDCl₃) δ: 8.45 (1H, dd), 7.66-7.60 (2H, m), 7.48 (1H, dd), 7.29-7.24 (1H, m), 7.05 (1H, dd), 3.01 (2H, t), 2.33 (3H, d), 1.84-1.72 (2H, m), 1.09 (3H, t).

### Preparation Example 2-1

The compounds which were prepared according to the Preparation Example 2 and their physical property values are shown.

The compounds (IA-1) wherein the combination of n, X¹, X², X³, X⁴, R¹, R², R³, R^{4A}, R^{4B}, and R^{4C} represents any combinations described in [Table A-2], [Table A-2A] and [Table A-2B].

**[Table A-2]**

| Present compound | n | X¹ | X² | X³ | X⁴ | R¹ | R² | R³ | R^{4A} | R^{4B} | R^{4C} |
|---|---|---|---|---|---|---|---|---|---|---|---|
| 5 | 0 | CH | N | CH | N | Me | H | H | H | Me | F |
| 6 | 0 | CH | CH | N | CH | Pr | H | H | F | Cl | F |
| 7 | 0 | CH | CH | N | CH | Pr | H | H | H | Me | F |
| 8 | 0 | N | CH | N | CH | Pr | H | H | H | Me | F |
| 9 | 2 | CH | CCl | N | CH | Et | H | H | H | Me | F |
| 68 | 0 | CH | CH | N | N | Pr | H | H | H | Me | F |
| 69 | 2 | CH | CH | N | CH | Et | H | H | H | Cl | F |
| 70 | 2 | CH | CH | N | CH | Et | H | H | H | Me | Cl |
| 71 | 2 | CH | CH | N | CH | Et | H | H | H | Cl | Me |
| 72 | 2 | CH | CH | N | CH | Et | H | H | H | Cl | Cl |
| 73 | 2 | CH | CH | N | CH | Et | H | H | H | F | F |
| 74 | 2 | CH | CH | N | CH | Et | H | H | H | Me | F |
| 75 | 2 | CH | CH | N | CH | Et | H | H | H | Me | Me |
| 76 | 2 | CH | CH | N | CH | Et | H | H | F | F | F |
| 77 | 2 | CH | CH | N | CH | Et | F | H | H | H | H |
| 78 | 2 | CH | CH | N | CH | Et | H | H | H | Cl | H |
| 79 | 2 | CH | CH | N | CH | Et | H | H | H | Me | H |
| 80 | 2 | CH | CH | N | CH | Et | H | H | H | F | Me |
| 81 | 2 | CH | CH | N | CH | Et | H | H | H | H | F |
| 82 | 2 | CH | CH | N | CH | Et | H | H | H | H | Cl |
| 83 | 2 | CH | CH | N | CH | Et | H | H | H | OMe | H |
| 84 | 2 | CH | CH | N | CH | Et | H | H | H | H | NO₂ |
| 85 | 2 | CH | CH | N | CH | Et | Me | H | H | H | H |
| 86 | 0 | N | CH | CH | CH | Et | H | H | H | H | C(O) NMe₂ |
| 87 | 2 | CH | CH | N | CH | Et | H | H | H | -CH₂CH₂CH₂- | |
| 88 | 2 | CH | CH | N | CH | Et | H | H | H | H | Me |
| 89 | 2 | CH | CH | N | CH | Et | H | H | H | Br | F |
| 90 | 2 | CH | CH | N | CH | Et | Cl | H | H | H | H |
| 91 | 2 | CH | CH | N | CH | Et | H | H | Me | F | Me |
| 92 | 2 | CH | CH | N | CH | Et | H | H | H | NMe₂ | H |
| 93 | 2 | CH | CH | N | CH | Et | H | H | Cl | H | Cl |
| 94 | 2 | CH | CH | N | CH | Et | H | H | H | Et | H |
| 95 | 2 | CH | CH | N | CH | Et | H | H | H | CN | H |
| 96 | 2 | CH | CH | N | CH | Et | H | H | F | Me | F |
| 97 | 2 | CH | CH | N | CH | Et | H | H | H | H | OMe |
| 98 | 2 | CH | CH | N | CH | Et | H | H | H | H | NMe₂ |
| 99 | 2 | CH | CH | N | CH | Et | H | H | H | H | SMe |

**[Table A-2A]**

| Present compound | n | X¹ | X² | X³ | X⁴ | R¹ | R² | R³ | R^{4A} | R^{4B} | R^{4C} |
|---|---|---|---|---|---|---|---|---|---|---|---|
| 100 | 2 | CH | CH | N | CH | Pr | H | H | H | Me | H |
| 101 | 2 | CH | CH | N | CH | Pr | H | H | F | Me | H |
| 102 | 2 | CH | CH | N | CH | Pr | H | H | F | F | F |
| 103 | 2 | CH | CH | N | CH | Pr | H | H | H | Cl | F |
| 104 | 2 | CH | CH | N | CH | NMe₂ | H | H | H | Me | F |
| 105 | 2 | CH | CH | N | CH | NMe₂ | H | H | F | F | F |
| 106 | 2 | CH | CH | N | CH | NMeEt | H | H | H | Me | F |
| 107 | 2 | CH | CH | N | CH | NMeEt | H | H | F | F | F |
| 108 | 2 | CH | CH | N | CH | Et | H | H | H | -CH=CH-O- | |
| 109 | 2 | N | CH | N | CH | Pr | H | H | H | F | CF₃ |
| 110 | 2 | N | CH | N | CH | Pr | H | H | H | -OCF₂O- | |
| 111 | 2 | N | CH | N | CH | Pr | H | H | F | F | F |
| 112 | 2 | N | CH | N | CH | Pr | H | H | H | CF₂H | H |
| 113 | 2 | N | CH | N | CH | Pr | H | H | F | C1 | F |
| 114 | 2 | N | CH | N | CH | Pr | H | H | H | c-Pr | H |
| 115 | 2 | N | CH | N | CH | Pr | H | H | H | OPh | H |
| 116 | 2 | N | CH | N | CH | Pr | H | H | H | -N=CH-S- | |
| 117 | 2 | N | CH | N | CH | Pr | H | H | H | Me | H |
| 118 | 2 | CH | CH | N | CH | Pr | H | H | F | Me | F |
| 119 | 2 | N | CH | N | CH | Pr | H | H | F | Me | F |
| 120 | 0 | CH | CH | N | CH | CH₂ (4-OMe-Ph) | H | H | H | Me | F |
| 160 | 2 | CH | CH | N | CH | Pr | H | H | H | F | F |
| 161 | 2 | CH | CH | N | CH | Pr | H | H | H | Me | Me |
| 162 | 2 | CH | CH | N | CH | Pr | H | H | H | Me | Cl |

**[Table A-2B]**

| Present compound | n | X¹ | X² | X³ | X⁴ | R¹ | R² | R³ | R^{4A} | R^{4B} | R^{4C} |
|---|---|---|---|---|---|---|---|---|---|---|---|
| 169 | 2 | CH | CH | N | CH | Pr | H | H | F | H | F |
| 170 | 2 | CH | CH | N | CH | Bu | H | H | H | Me | H |
| 171 | 2 | CH | CH | N | CH | Bu | H | H | H | F | F |
| 172 | 2 | CH | CH | N | CH | Bu | H | H | H | Me | F |
| 173 | 2 | CH | CH | N | CH | Bu | H | H | H | Me | Cl |
| 174 | 2 | CH | CH | N | CH | c-Pn | H | H | H | Me | H |
| 175 | 2 | CH | CH | N | CH | c-Pn | H | H | H | F | F |
| 176 | 2 | CH | CH | N | CH | c-Pn | H | H | H | Me | F |
| 177 | 2 | CH | CH | N | CH | c-Pn | H | H | H | Me | Cl |
| 178 | 2 | CH | CH | N | CH | 2-thienyl | H | H | H | Me | H |
| 179 | 2 | CH | CH | N | CH | 2-thienyl | H | H | H | F | Me |
| 180 | 2 | CH | CH | N | CH | 2-thienyl | H | H | H | F | F |
| 181 | 2 | CCH₃ | CH | N | CH | Pr | H | H | H | Cl | F |
| 182 | 2 | CCH₃ | CH | N | CH | Pr | H | H | H | Me | H |
| 183 | 2 | CH | CH | N | CH | Pr | H | H | H | Cl | H |
| 184 | 2 | CH | CH | N | CH | Pr | H | H | H | H | F |
| 185 | 2 | CH | CH | N | CH | 2-thienyl | H | H | H | Cl | H |
| 186 | 2 | CH | CH | N | CH | 2-thienyl | H | H | H | H | F |
| 187 | 2 | CH | CH | N | CH | Et | H | H | H | Br | H |
| 188 | 2 | CH | CH | N | CH | Ph | H | H | F | H | F |
| 189 | 2 | CH | CH | N | CH | Ph | H | H | H | F | F |
| 190 | 2 | CH | CH | N | CH | Ph | H | H | F | F | F |
| 191 | 2 | CH | CH | N | CH | Ph | H | H | H | Cl | F |
| 192 | 2 | CH | CH | N | CH | Ph | H | H | H | Cl | H |
| 193 | 2 | CH | CH | N | CH | Ph | H | H | H | F | H |
| 194 | 2 | CH | CH | N | CH | Ph | H | H | H | Me | H |
| 195 | 2 | CH | CH | N | CH | Ph | H | H | H | H | Cl |
| 196 | 2 | CH | CH | N | CH | Ph | H | H | H | H | F |
| 197 | 2 | CH | CH | N | CH | Ph | H | H | H | F | Cl |
| 198 | 2 | CH | CH | N | CH | Pr | H | H | H | F | Cl |

Present compound 5: ¹H-NMR (CDCl₃) δ: 8.98 (1H, d), 7.74-7.69 (2H, m), 7.51 (1H, d), 7.30 (1H, t), 2.62 (3H, s), 2.34 (3H, d).
Present compound 6: ¹H-NMR (CDCl₃) δ: 8.56 (2H, t), 7.75 (1H, t), 7.23-7.18 (1H, m), 6.53-6.47 (1H, m), 2.99 (2H, t), 1.77-1.68 (2H, m), 1.07 (3H, t).
Present compound 7: ¹H-NMR (CDCl₃) δ: 8.59 (1H, d), 8.52 (1H, d), 7.77 (1H, t), 7.33-7.20 (3H, m), 2.97 (2H, t), 2.33 (3H, d), 1.78-1.66 (2H, m), 1.05 (3H, t).
Present compound 8: ¹H-NMR (CDCl₃) δ: 8.60 (1H, s), 8.35 (1H, s), 7.74-7.67 (2H, m), 7.33-7.27 (1H, m), 3.25 (2H, t), 2.35 (3H, d), 1.86-1.76 (2H, m), 1.09 (3H, t).
Present compound 9: ¹H-NMR (CDCl₃) δ: 8.80 (1H, d), 8.58 (1H, d), 7.36-7.27 (3H, m), 3.55 (2H, q), 2.35 (3H, d), 1.35 (3H, t).
Present compound 68: ¹H-NMR (CDCl₃) δ: 8.96 (1H, d), 7.76-7.68 (2H, m), 7.52 (1H, d), 7.35-7.30 (1H, m), 3.06 (2H, t), 2.36 (3H, d), 1.87-1.78 (2H, m), 1.12 (3H, t).
Present compound 69: LCMS: 300 [M+H]⁺, RT = 1.753 min
Present compound 70: LCMS: 296 [M+H]⁺, RT = 1.841 min
Present compound 71: LCMS: 296 [M+H]⁺, RT = 1.857 min
Present compound 72: LCMS: 316 [M+H]⁺, RT = 1.855 min
Present compound 73: LCMS: 284 [M+H]⁺, RT = 1.642 min
Present compound 74: LCMS: 280 [M+H]⁺, RT = 1.729 min
Present compound 75: LCMS: 276 [M+H]⁺, RT = 1.798 min
Present compound 76: LCMS: 302 [M+H]⁺, RT = 1.712 min
Present compound 77: LCMS: 266 [M+H]⁺, RT = 1.584 min
Present compound 78: LCMS: 282 [M+H]⁺, RT = 1.724 min
Present compound 79: LCMS: 262 [M+H]⁺, RT = 1.692 min
Present compound 80: LCMS: 280 [M+H]⁺, RT = 1.724 min
Present compound 81: LCMS: 266 [M+H]⁺, RT = 1.599 min
Present compound 82: LCMS: 282 [M+H]⁺, RT = 1.716 min
Present compound 83: LCMS: 278 [M+H]⁺, RT = 1.569 min
Present compound 84: LCMS: 293 [M+H]⁺, RT = 1.547 min
Present compound 85: LCMS: 262 [M+H]⁺, RT = 1.600 min
Present compound 86: LCMS: 287 [M+H]⁺, RT = 1.846 min
Present compound 87: LCMS: 288 [M+H]⁺, RT = 1.860 min
Present compound 88: LCMS: 262 [M+H]⁺, RT = 1.695 min
Present compound 89: LCMS: 344 [M+H]⁺, RT = 1.778 min
Present compound 90: LCMS: 282 [M+H]⁺, RT = 1.666 min
Present compound 91: LCMS: 294 [M+H]⁺, RT = 1.859 min
Present compound 92: LCMS: 291 [M+H]⁺, RT = 1.568 min
Present compound 93: LCMS: 316 [M+H]⁺, RT = 1.891 min
Present compound 94: LCMS: 276 [M+H]⁺, RT = 1.815 min
Present compound 95: LCMS: 273 [M+H]⁺, RT = 1.466 min
Present compound 96: LCMS: 298 [M+H]⁺, RT = 1.791 min
Present compound 97: LCMS: 278 [M+H]⁺, RT = 1.593 min
Present compound 98: LCMS: 291 [M+H]⁺, RT = 1.451 min
Present compound 99: LCMS: 294 [M+H]⁺, RT = 1.711 min
Present compound 100: ¹H-NMR (CDCl₃) δ: 9.08 (1H, d), 9.04 (1H, d), 8.34 (1H, t), 7.55-7.52 (2H, m), 7.35-7.32 (2H, m), 3.18-3.13 (2H, m), 2.44 (3H, s), 1.86-1.77 (2H, m), 1.04 (3H, t) .
Present compound 101: ¹H-NMR (CDCl₃) δ: 9.07-9.04 (2H, m), 8.31 (1H, t), 7.47-7.39 (2H, m), 7.16 (1H, t), 3.18-3.14 (2H, m), 2.38 (3H, d), 1.87-1.77 (2H, m), 1.05 (3H, t).
Present compound 102: ¹H-NMR (CDCl₃) δ: 9.12 (1H, d), 9.03 (1H, d), 8.28 (1H, t), 7.29-7.25 (2H, m), 3.19-3.15 (2H, m), 1.88-1.77 (2H, m), 1.06 (3H, t).
Present compound 103: ¹H-NMR (CDCl₃) δ: 9.11 (1H, d), 9.06 (1H, d), 8.32 (1H, t), 7.60-7.55 (1H, m), 7.43 (1H, dd), 7.39-7.35 (1H, m), 3.19-3.15 (2H, m), 1.88-1.77 (2H, m), 1.06 (3H, t).
Present compound 104: ¹H-NMR (CDCl₃) δ: 9.01 (1H, d), 8.96 (1H, d), 8.19 (1H, t), 7.37-7.27 (3H, m), 2.81 (6H, s), 2.36 (3H, d).
Present compound 105: ¹H-NMR (CDCl₃) δ: 9.01 (1H, d), 8.97 (1H, d), 8.15 (1H, t), 7.28-7.23 (2H, m), 2.82 (6H, s).
Present compound 106: ¹H-NMR (CDCl₃) δ: 8.99 (1H, d), 8.96 (1H, d), 8.20 (1H, t), 7.36-7.25 (3H, m), 3.20 (2H, q), 2.83 (3H, s), 2.35 (3H, d), 1.19 (3H, t).
Present compound 107: ¹H-NMR (CDCl₃) δ: 9.01 (1H, d), 8.94 (1H, d), 8.16 (1H, t), 7.26-7.22 (2H, m), 3.21 (2H, q), 2.84 (3H, s), 1.20 (3H, t).
Present compound 108: ¹H-NMR (CDCl₃) δ: 9.16 (1H, d), 9.07 (1H, d), 8.41 (1H, t), 7.80-7.72 (3H, m), 7.52 (1H, dd), 6.85 (1H, dd), 3.23 (2H, q), 1.38 (3H, t).
Present compound 109: ¹H-NMR (CDCl₃) δ: 9.30 (1H, s), 9.26 (1H, s), 8.34-8.29 (2H, m), 7.43 (1H, t), 3.46-3.42 (2H, m), 1.94-1.84 (2H, m), 1.09 (3H, t).
Present compound 110: ¹H-NMR (CDCl₃) δ: 9.24 (1H, s), 9.21 (1H, s), 7.86-7.83 (2H, m), 7.26-7.23 (1H, m), 3.45-3.41 (2H, m), 1.93-1.83 (2H, m), 1.08 (3H, t).
Present compound 111: ¹H-NMR (CDCl₃) δ: 9.26 (1H, s), 9.21 (1H, s), 7.78-7.75 (2H, m), 3.45-3.41 (2H, m), 1.91-1.84 (2H, m), 1.09 (3H, t).
Present compound 112: ¹H-NMR (CDCl₃) δ: 9.32 (1H, s), 9.25 (1H, s), 8.19 (2H, d), 7.71 (2H, d), 6.74 (1H, t), 3.47-3.43 (2H, m), 1.93-1.84 (2H, m), 1.08 (3H, t).
Present compound 113: ¹H-NMR (CDCl₃) δ: 9.28 (1H, s), 9.25 (1H, s), 7.78-7.73 (2H, m), 3.46-3.42 (2H, m), 1.93-1.84 (2H, m), 1.09 (3H, t).
Present compound 114: LCMS: 303 [M+H]⁺, RT = 1.942 min
Present compound 115: LCMS: 355 [M+H]⁺, RT = 2.044 min
Present compound 116: LCMS: 320 [M+H]⁺, RT = 1.578 min
Present compound 117: LCMS: 277 [M+H]⁺, RT = 1.841 min
Present compound 118: ¹H-NMR (CDCl₃) δ: 9.09 (1H, d), 9.05 (1H, d), 8.31 (1H, t), 7.19-7.10 (2H, m), 3.19-3.15 (2H, m), 2.27 (3H, d), 1.89-1.77 (2H, m), 1.06 (3H, t).
Present compound 119: ¹H-NMR (CDCl₃) δ: 9.24-9.21 (2H, m), 7.64-7.57 (2H, m), 3.47-3.42 (2H, m), 2.29 (3H, t), 1.93-1.83 (2H, m), 1.09 (3H, t).
Present compound 120: ¹H-NMR (CDCl₃) δ: 8.61 (1H, d), 8.50 (1H, d), 7.62 (1H, t), 7.28-7.24 (1H, m), 7.22-7.08 (4H, m), 6.86-6.80 (2H, m), 4.10 (2H, s), 3.79 (3H, s), 2.32 (3H, d).
Present compound 160: ¹H-NMR (CDCl₃) δ: 9.10 (1H, d), 9.05 (1H, d), 8.30 (1H, t), 7.48-7.43 (1H, m), 7.38-7.31 (2H, m), 3.19-3.15 (2H, m), 1.89-1.76 (2H, m), 1.06 (3H, t).
Present compound 161: ¹H-NMR (CDCl₃) δ: 9.08 (1H, d), 9.03 (1H, d), 8.34 (1H, t), 7.43-7.35 (2H, m), 7.31-7.27 (1H, m), 3.21-3.12 (2H, m), 2.36 (3H, s), 2.34 (3H, s), 1.88-1.76 (2H, m), 1.04 (3H, t).
Present compound 162: ¹H-NMR (CDCl₃) δ: 9.09-9.06 (2H, m), 8.32 (1H, t), 7.62 (1H, d), 7.45-7.37 (2H, m), 3.20-3.12 (2H, m), 2.46 (3H, s), 1.88-1.76 (2H, m), 1.05 (3H, t).
Present compound 169: ¹H-NMR (CDCl₃) δ: 9.13 (1H, d), 9.07 (1H, d), 8.32 (1H, t), 7.20-7.13 (2H, m), 6.98-6.91 (1H, m), 3.20-3.14 (2H, m), 1.89-1.77 (2H, m), 1.06 (3H, t).
Present compound 170: ¹H-NMR (CDCl₃) δ: 9.09 (1H, d), 9.04 (1H, d), 8.34 (1H, t), 7.56-7.51 (2H, m), 7.36-7.31 (2H, m), 3.22-3.12 (2H, m), 2.44 (3H, s), 1.80-1.72 (2H, m), 1.49-1.38 (2H, m), 0.92 (3H, t).
Present compound 171: ¹H-NMR (CDCl₃) δ: 9.10 (1H, d), 9.05 (1H, d), 8.30 (1H, t), 7.50-7.42 (1H, m), 7.40-7.30 (2H, m), 3.22-3.13 (2H, m), 1.82-1.71 (2H, m), 1.51-1.38 (2H, m), 0.93 (3H, t).
Present compound 172: ¹H-NMR (CDCl₃) δ: 9.07 (2H, d), 8.32 (1H, t), 7.38-7.27 (3H, m), 3.21-3.12 (2H, m), 2.36 (3H, d), 1.82-1.70 (2H, m), 1.50-1.38 (2H, m), 0.93 (3H, t).
Present compound 173: ¹H-NMR (CDCl₃) δ: 9.07 (2H, t), 8.32 (1H, t), 7.62 (1H, d), 7.45-7.37 (2H, m), 3.22-3.15 (2H, m), 2.45 (3H, s), 1.82-1.71 (2H, m), 1.50-1.38 (2H, m), 0.93 (3H, t).
Present compound 174: ¹H-NMR (CDCl₃) δ: 9.07 (1H, d), 9.03 (1H, d), 8.34 (1H, t), 7.56-7.51 (2H, m), 7.36-7.31 (2H, m), 3.62-3.50 (1H, m), 2.44 (3H, s), 2.18-2.07 (2H, m), 2.00-1.88 (2H, m), 1.88-1.76 (2H, m), 1.70-1.61 (2H, m).
Present compound 175: ¹H-NMR (CDCl₃) δ: 9.09 (1H, d), 9.03 (1H, d), 8.30 (1H, t), 7.49-7.42 (1H, m), 7.39-7.30 (2H, m), 3.62-3.49 (1H, m), 2.21-2.06 (2H, m), 2.00-1.76 (4H, m), 1.74-1.61 (2H, m).
Present compound 176: ¹H-NMR (CDCl₃) δ: 9.06 (1H, d), 9.05 (1H, d), 8.32 (1H, t), 7.37-7.27 (3H, m), 3.61-3.50 (1H, m), 2.36 (3H, d), 2.18-2.07 (2H, m), 2.01-1.89 (2H, m), 1.88-1.77 (2H, m), 1.72-1.61 (2H, m).
Present compound 177: ¹H-NMR (CDCl₃) δ: 9.06 (1H, d), 9.05 (1H, d), 8.32 (1H, t), 7.62 (1H, d), 7.45-7.37 (2H, m), 3.63-3.49 (1H, m), 2.45 (3H, s), 2.19-2.05 (2H, m), 2.00-1.89 (2H, m), 1.87-1.78 (2H, m), 1.73-1.61 (2H, m).
Present compound 178: ¹H-NMR (CDCl₃) δ: 9.11 (1H, d), 8.99 (1H, d), 8.39 (1H, t), 7.79 (1H, dd), 7.72 (1H, dd), 7.52-7.47 (2H, m), 7.35-7.30 (2H, m), 7.14 (1H, dd), 2.43 (3H, s).
Present compound 179: ¹H-NMR (CDCl₃) δ: 9.12 (1H, d), 8.96 (1H, d), 8.36 (1H, t), 7.79 (1H, dd), 7.73 (1H, dd), 7.44-7.35 (2H, m), 7.18-7.11 (2H, m), 2.37 (3H, d).
Present compound 180: H-NMR (CDCl₃) δ: 9.16 (1H, d), 8.95 (1H, d), 8.35 (1H, t), 7.80 (1H, dd), 7.74 (1H, dd), 7.45-7.39 (1H, m), 7.36-7.30 (2H, m), 7.16 (1H, dd).
Present compound 181: ¹H-NMR (CDCl₃) δ: 9.15 (1H, s), 8.63 (1H, s), 7.55 (1H, t), 7.13 (1H, dd), 7.07-7.02 (1H, m), 3.23-3.15 (2H, m), 2.59 (3H, s), 1.92-1.79 (2H, m), 1.07 (3H, t) .
Present compound 182: ¹H-NMR (CDCl₃) δ: 9.11 (1H, s), 8.64 (1H, s), 7.33-7.28 (2H, m), 7.21-7.15 (2H, m), 3.23-3.14 (2H, m), 2.59 (3H, s), 2.44 (3H, s), 1.90-1.78 (2H, m), 1.07 (3H, t) .
Present compound 183: LCMS: 296[M+H]⁺, RT =1.844 min
Present compound 184: LCMS: 280[M+H]⁺, RT =1.723 min
Present compound 185: LCMS: 336[M+H]⁺, RT =1.966 min
Present compound 186: LCMS: 320[M+H]⁺, RT =1.850 min
Present compound 187: ¹H-NMR (CDCl₃) δ: 9.09 (1H, d), 9.08 (1H, d), 8.34 (1H, t), 7.70-7.64 (2H, m), 7.54-7.46 (2H, m), 3.22 (2H, q), 1.36 (3H, t).
Present compound 188: LCMS: 332[M+H]⁺, RT =1.915 min
Present compound 189: ¹H-NMR (CDCl₃) δ: 9.11 (1H, d), 8.94 (1H, d), 8.33 (1H, t), 8.04-7.99 (2H, m), 7.70-7.53 (3H, m), 7.46-7.38 (1H, m), 7.35-7.30 (2H, m).
Present compound 190: ¹H-NMR (CDCl₃) δ: 9.13 (1H, d), 8.92 (1H, d), 8.30 (1H, t), 8.04-7.98 (2H, m), 7.69-7.55 (3H, m), 7.25-7.19 (2H, m).
Present compound 191: ¹H-NMR (CDCl₃) δ: 9.12 (1H, d), 8.95 (1H, d), 8.34 (1H, t), 8.04-7.99 (2H, m), 7.67-7.62 (1H, m), 7.61-7.53 (3H, m), 7.41-7.30 (2H, m).
Present compound 192: LCMS: 330[M+H]⁺, RT =1.992 min
Present compound 193: LCMS: 314[M+H]⁺, RT =1.863 min
Present compound 194: LCMS: 310[M+H]⁺, RT =1.965 min
Present compound 195: LCMS: 330[M+H]⁺, RT =1.980 min
Present compound 196: LCMS: 314[M+H]⁺, RT =1.872 min
Present compound 197: LCMS: 348[M+H]⁺, RT =1.987 min
Present compound 198: LCMS: 314[M+H]⁺, RT =1.857 min.

### Preparation Example 3

To a mixture of the present compound 4 0.41 g, and chloroform 6 mL was added mCPBA (purity 75 %, containing 25 % water) 0.53 g at 0 °C, and the mixture was stirred at room temperature for 1 hour. To the resulting mixture was added saturated aqueous sodium thiosulfate solution 10 mL and a saturated aqueous sodium hydrocarbonate solution 10 mL, and the mixture was stirred for 1 hour, and extracted with chloroform. The resulting organic layer was washed with saturated brine, and dried over sodium sulfate, and concentrated under reduced pressure. The resulting residue was subjected to a silica gel column chromatography (hexane : ethyl acetate = 1 : 1) to obtain the present compound 10 represented by the following formula 0.18 g and the present compound 11 represented by the following formula 0.22 g. 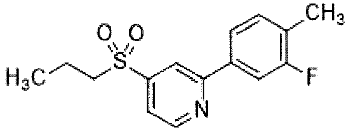 Present compound 10: ¹H-NMR (CDCl₃) δ: 8.93 (1H, dd), 8.13 (1H, dd), 7.80-7.72 (2H, m), 7.67 (1H, dd), 7.32 (1H, t), 3.16-3.11 (2H, m), 2.35 (3H, d), 1.86-1.75 (2H, m), 1.04 (3H, t) . 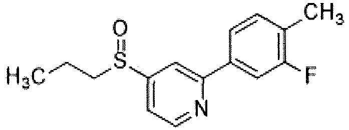 Present compound 11: ¹H-NMR (CDCl₃) δ: 8.79 (1H, d), 7.98-7.95 (1H, m), 7.79-7.72 (2H, m), 7.38 (1H, dd), 7.30 (1H, t), 2.89-2.83 (2H, m), 2.34 (3H, d), 1.98-1.86 (1H, m), 1.77-1.66 (1H, m), 1.09 (3H, t).

### Preparation Example 3-1

The compounds which were prepared according to the Preparation Example 3 and their physical property values are shown.

The compounds (IA-1) wherein the combination of n, X¹, X², X³, X⁴, R¹, R², R³, R^{4A}, R^{4B}, and R^{4C} 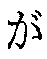 represents any combinations described in [Table A-3].

**[Table A-3]**

| Present compound | n | X¹ | X² | X³ | X⁴ | R¹ | R² | R³ | R^{4A} | R^{4B} | R^{4C} |
|---|---|---|---|---|---|---|---|---|---|---|---|
| 12 | 2 | N | CH | CH | CH | Et | H | H | H | H | Cl |
| 13 | 1 | N | CH | CH | CH | Et | H | H | H | H | Cl |
| 14 | 2 | CH | CH | N | CH | Pr | H | H | H | Me | F |
| 15 | 1 | CH | CH | N | CH | Pr | H | H | H | Me | F |
| 16 | 2 | CH | N | CH | CH | Pr | H | H | H | Me | F |
| 17 | 1 | CH | N | CH | CH | Pr | H | H | H | Me | F |
| 18 | 2 | CH | N | CH | N | Me | H | H | H | Me | F |
| 19 | 1 | CH | N | CH | N | Me | H | H | H | Me | F |
| 20 | 2 | CH | CH | N | CH | Pr | H | H | F | Cl | F |
| 21 | 1 | CH | CH | N | CH | Pr | H | H | F | Cl | F |
| 22 | 2 | N | CH | N | CH | Pr | H | H | H | Me | F |
| 23 | 1 | N | CH | N | CH | Pr | H | H | H | Me | F |
| 121 | 2 | CH | CH | N | CH | 4-pyridyl | H | H | H | Me | F |
| 122 | 1 | CH | CH | N | CH | 4-pyridyl | H | H | H | Me | F |
| 123 | 2 | CH | CH | N | CH | 3-F-Ph | H | H | H | Me | F |
| 124 | 1 | CH | CH | N | CH | 3-F-Ph | H | H | H | Me | F |
| 125 | 2 | CH | CH | N | CH | 2-furyl | H | H | H | Me | F |
| 126* | 2 | CH | CH | N | CH | 2-furyl | H | H | H | Me | F |
| 127 | 2 | CH | CH | N | CH | 3-furyl | H | H | H | Me | F |
| 128 | 1 | CH | CH | N | CH | 3-furyl | H | H | H | Me | F |
| 129 | 2 | CH | CH | N | CH | 2-thienyl | H | H | H | Me | F |
| 130 | 1 | CH | CH | N | CH | 2-thienyl | H | H | H | Me | F |
| 131 | 2 | CH | CH | N | CH | 3-thienyl | H | H | H | Me | F |
| 132 | 1 | CH | CH | N | CH | 3-thienyl | H | H | H | Me | F |
| 133 | 2 | CH | CH | N | CH | 2-pyridyl | H | H | H | Me | F |
| 134 | 2 | CH | CH | N | CH | i-Pr | H | H | H | Me | F |
| 135 | 2 | CH | CH | N | CH | 2-pyrazinyl | H | H | H | Me | F |
| 136 | 2 | N | CH | CH | CH | Et | H | H | H | Me | F |
| 137 | 1 | N | CH | CH | CH | Et | H | H | H | Me | F |
| 138 | 2 | N | CH | CH | CH | Et | H | H | H | H | C(O)NMe₂ |
| 139 | 1 | N | CH | CH | CH | Et | H | H | H | H | C(O)NMe₂ |
| 140 | 2 | CH | N | N | CH | Pr | H | H | H | Me | F |
| 141 | 1 | CH | N | N | CH | Pr | H | H | H | Me | F |
| 142 | 2 | CH | CH | N | N | Pr | H | H | H | Me | F |
| 143 | 1 | CH | CH | N | N | Pr | H | H | H | Me | F |
| 144 | 2 | N | N | CH | CH | Pr | H | H | H | Me | F |
| 145 | 1 | N | N | CH | CH | Pr | H | H | H | Me | F |
| 158 | 2 | CH | CH | N | CH | Ph | H | H | H | Me | F |
| 159* | 2 | CH | CH | N | CH | Ph | H | H | H | Me | F |
| 163 | 2 | CH | CH | N | CH | 7-quinolyl | H | H | H | Me | F |
| 164 | 2 | CH | CH | N | CH | 3-furyl | H | H | H | Me | H |
| 165 | 2 | CH | CH | N | CH | 3-thienyl | H | H | H | Me | H |
| 166 | 2 | CH | CH | N | CH | 3-furyl | H | H | H | Cl | H |
| 167 | 2 | CH | CH | N | CH | 3-thienyl | H | H | H | Cl | H |

Here the symbol of "*" described in the number of the present compound for the compounds which are indicated in the above [Table A-3] represents N-oxide. Specifically, the compounds represented by the following structures are included.

### Present compound 126

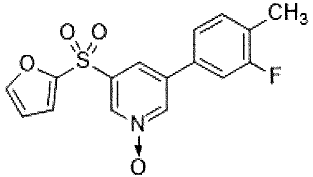

### Present compound 159

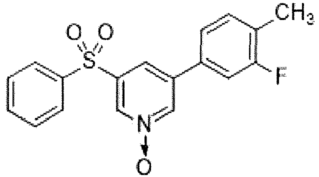

Present compound 12: ¹H-NMR (CDCl₃) δ: 8.06-7.91 (5H, m), 7.52-7.46 (2H, m), 3.52 (2H, q), 1.36 (3H, t).
Present compound 13: ¹H-NMR (CDCl₃) δ: 8.03-7.92 (4H, m), 7.78 (1H, d), 7.49-7.43 (2H, m), 3.31-3.18 (1H, m), 3.05-2.96 (1H, m), 1.29-1.23 (3H, m).
Present compound 14: ¹H-NMR (CDCl₃) δ: 9.07 (2H, d), 8.32 (1H, t), 7.37-7.27 (3H, m), 3.19-3.12 (2H, m), 2.36 (3H, d), 1.89-1.76 (2H, m), 1.05 (3H, t).
Present compound 15: ¹H-NMR (CDCl₃) δ: 8.93 (1H, s), 8.69 (1H, s), 8.22 (1H, t), 7.35-7.29 (3H, m), 2.96-2.84 (2H, m), 2.35 (3H, d), 1.95-1.82 (1H, m), 1.79-1.69 (1H, m), 1.10 (3H, t).
Present compound 16: ¹H-NMR (CDCl₃) δ: 8.77 (1H, dd), 8.28 (1H, dd), 7.71 (1H, dd), 7.42-7.32 (3H, m), 3.46-3.39 (2H, m), 2.36 (3H, d), 1.87-1.77 (2H, m), 1.05 (3H, t).
Present compound 17: ¹H-NMR (CDCl₃) δ: 8.65 (1H, d), 8.19 (1H, t), 7.55-7.52 (1H, m), 7.47-7.37 (2H, m), 7.35-7.29 (1H, m), 3.18-3.07 (1H, m), 2.98-2.87 (1H, m), 2.35 (3H, s), 2.03-1.88 (1H, m), 1.73-1.62 (1H, m), 1.08 (3H, t).
Present compound 18: ¹H-NMR (CDCl₃) δ: 9.36 (1H, d), 8.34 (1H, d), 7.93-7.82 (2H, m), 7.37 (1H, t), 3.31 (3H, s), 2.38 (3H, d).
Present compound 19: ¹H-NMR (CDCl₃) δ: 9.21 (1H, s), 8.40 (1H, s), 7.95-7.87 (2H, m), 7.35 (1H, t), 2.95 (3H, s), 2.37 (3H, d).
Present compound 20: ¹H-NMR (CDCl₃) δ: 9.13 (1H, d), 9.06 (1H, d), 8.34 (1H, t), 7.33-7.25 (2H, m), 3.22-3.15 (2H, m), 1.88-1.78 (2H, m), 1.06 (3H, t).
Present compound 21: ¹H-NMR (CDCl₃) δ: 8.91 (1H, d), 8.74 (1H, d), 8.25 (1H, t), 7.33-7.27 (2H, m), 2.97-2.81 (2H, m), 1.97-1.84 (1H, m), 1.80-1.67 (1H, m), 1.11 (3H, t).
Present compound 22: ¹H-NMR (CDCl₃) δ: 9.24 (1H, s), 9.19 (1H, s), 7.81-7.74 (2H, m), 7.41-7.34 (1H, m), 3.48-3.41 (2H, m), 2.38 (3H, d), 1.94-1.82 (2H, m), 1.08 (3H, t).
Present compound 23: ¹H-NMR (CDCl₃) δ: 9.10 (1H, s), 9.09 (1H, s), 7.75-7.69 (2H, m), 7.35 (1H, t), 3.24-3.12 (1H, m), 3.08-2.98 (1H, m), 2.37 (3H, d), 2.07-1.95 (1H, m), 1.79-1.66 (1H, m), 1.10 (3H, t).
Present compound 121: ¹H-NMR (CDCl₃) δ: 9.12 (1H, d), 9.03 (1H, d), 8.90 (2H, dd), 8.35 (1H, t), 7.84 (2H, dd), 7.38-7.22 (3H, m), 2.35 (3H, d).
Present compound 122: ¹H-NMR (CDCl₃) δ: 8.94-8.89 (1H, m), 8.84-8.81 (1H, m), 8.80-8.75 (2H, m), 8.17-8.13 (1H, m), 7.63-7.60 (2H, m), 7.37-7.20 (3H, m), 2.33 (3H, s).
Present compound 123: ¹H-NMR (CDCl₃) δ: 9.09 (1H, d), 8.99 (1H, d), 8.33 (1H, t), 7.83-7.79 (1H, m), 7.73-7.69 (1H, m), 7.59-7.53 (1H, m), 7.36-7.22 (4H, m), 2.35 (3H, d).
Present compound 124: ¹H-NMR (CDCl₃) δ: 8.88 (1H, d), 8.74 (1H, d), 8.16 (1H, t), 7.51-7.45 (3H, m), 7.33-7.17 (4H, m), 2.33 (3H, d).
Present compound 125: ¹H-NMR (CDCl₃) δ: 9.14 (1H, d), 9.02 (1H, d), 8.40 (1H, t), 7.61 (1H, dd), 7.38-7.24 (4H, m), 6.56 (1H, dd), 2.35 (3H, d).
Present compound 126: ¹H-NMR (CDCl₃) δ: 8.66 (1H, t), 8.50 (1H, t), 7.91 (1H, t), 7.67 (1H, dd), 7.38-7.32 (2H, m), 7.26-7.20 (2H, m), 6.61 (1H, dd), 2.35 (3H, d).
Present compound 127: ¹H-NMR (CDCl₃) δ: 9.11 (1H, d), 9.01 (1H, d), 8.35 (1H, t), 8.12 (1H, dd), 7.51 (1H, t), 7.36-7.23 (3H, m), 6.68 (1H, dd), 2.35 (3H, d).
Present compound 128: ¹H-NMR (CDCl₃) δ: 8.92 (1H, d), 8.68 (1H, d), 8.23 (1H, t), 7.96 (1H, t), 7.51 (1H, t), 7.36-7.27 (3H, m), 6.45 (1H, dd), 2.34 (3H, d).
Present compound 129: ¹H-NMR (CDCl₃) δ: 9.13 (1H, d), 8.98 (1H, d), 8.37 (1H, t), 7.80 (1H, dd), 7.73 (1H, dd), 7.35-7.24 (3H, m), 7.15 (1H, dd), 2.34 (3H, d).
Present compound 130: ¹H-NMR (CDCl₃) δ: 8.91 (1H, d), 8.70 (1H, d), 8.28 (1H, t), 7.69-7.67 (2H, m), 7.34-7.27 (3H, m), 7.15-7.12 (1H, m), 2.34 (3H, d).
Present compound 131: ¹H-NMR (CDCl₃) δ: 9.10 (1H, d), 8.98 (1H, d), 8.35 (1H, t), 8.21 (1H, dd), 7.46 (1H, dd), 7.40 (1H, dd), 7.35-7.23 (3H, m), 2.35 (3H, d).
Present compound 132: ¹H-NMR (CDCl₃) δ: 8.89 (1H, d), 8.68 (1H, d), 8.21 (1H, t), 7.92 (1H, dd), 7.47 (1H, dd), 7.34-7.25 (3H, m), 7.15 (1H, dd), 2.34 (3H, d).
Present compound 133: ¹ H-NMR (CDCl₃) δ: 9.19 (1H, d), 9.02 (1H, d), 8.71-8.69 (1H, m), 8.52 (1H, t), 8.28-8.25 (1H, m), 7.99 (1H, td), 7.54-7.50 (1H, m), 7.36-7.28 (3H, m), 2.34 (3H, d).
Present compound 134: ¹H-NMR (CDCl₃) δ: 9.07 (1H, d), 9.04 (1H, d), 8.30 (1H, t), 7.38-7.27 (3H, m), 3.33-3.23 (1H, m), 2.36 (3H, d), 1.37 (6H, d).
Present compound 135: ¹H-NMR (CDCl₃) δ: 9.45 (1H, d), 9.21 (1H, d), 9.05 (1H, d), 8.83 (1H, d), 8.67 (1H, dd), 8.50 (1H, t), 7.38-7.27 (3H, m), 2.35 (3H, d).
Present compound 136: ¹H-NMR (CDCl₃) δ: 8.05-7.98 (2H, m), 7.96-7.91 (1H, m), 7.78-7.68 (2H, m), 7.31 (1H, t), 3.53 (2H, q), 2.35 (3H, d), 1.37 (3H, t).
Present compound 137: ¹H-NMR (CDCl₃) δ: 8.00-7.91 (2H, m), 7.79-7.64 (3H, m), 7.29 (1H, t), 3.28-3.21 (1H, m), 3.05-2.98 (1H, m), 2.34 (3H, d), 1.25 (3H, t).
Present compound 138: ¹H-NMR (CDCl₃) δ: 8.15-7.97 (5H, m), 7.59-7.49 (2H, m), 3.52 (2H, q), 3.16 (3H, s), 3.02 (3H, s), 1.35 (3H, t).
Present compound 139: ¹H-NMR (CDCl₃) δ: 8.12-7.93 (4H, m), 7.85-7.79 (1H, m), 7.56-7.47 (2H, m), 3.31-3.20 (1H, m), 3.16 (3H, s), 3.07-2.96 (4H, m), 1.25 (3H, t).
Present compound 140: ¹H-NMR (CDCl₃) δ: 9.52 (1H, d), 8.22 (1H, d), 7.91 (1H, d), 7.83 (1H, d), 7.40 (1H, t), 3.22-3.18 (2H, m), 2.39 (3H, s), 1.89-1.79 (2H, m), 1.08 (3H, t).
Present compound 141: ¹H-NMR (CDCl₃) δ: 9.17 (1H, d), 8.21 (1H, d), 7.92 (1H, dd), 7.84 (1H, dd), 7.37 (1H, t), 3.02-2.85 (2H, m), 2.38 (3H, d), 2.04-1.89 (1H, m), 1.80-1.66 (1H, m), 1.12 (3H, t).
Present compound 142: LCMS: 295 [M+H]⁺, RT = 1.804 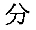
Present compound 143: ¹H-NMR (CDCl₃) δ: 9.19 (1H, d), 8.21-8.20 (1H, m), 7.93-7.82 (2H, m), 7.37 (1H, t), 3.03-2.87 (2H, m), 2.37 (3H, s), 2.03-1.88 (1H, m), 1.80-1.64 (1H, m), 1.15-1.08 (3H, m).
Present compound 144: ¹H-NMR (CDCl₃) δ: 8.93 (1H, d), 7.88-7.82 (3H, m), 7.36 (1H, t), 3.61-3.56 (2H, m), 2.38 (3H, d), 2.02-1.92 (2H, m), 1.12 (3H, t).
Present compound 145: ¹H-NMR (CDCl₃) δ: 8.90 (1H, d), 7.88-7.81 (2H, m), 7.71 (1H, d), 7.35 (1H, t), 3.22-3.08 (2H, m), 2.37 (3H, d), 2.10-1.96 (1H, m), 1.83-1.68 (1H, m), 1.10 (3H, t).
Present compound 158: ¹H-NMR (CDCl₃) δ: 9.08 (1H, d), 8.96 (1H, d), 8.34 (1H, t), 8.03-8.00 (2H, m), 7.66-7.61 (1H, m), 7.59-7.52 (2H, m), 7.34-7.28 (2H, m), 7.24-7.22 (1H, m), 2.34 (3H, d).
Present compound 159: ¹H-NMR (CDCl₃) δ: 8.59 (1H, t), 8.45 (1H, t), 8.01-8.00 (1H, m), 7.99-7.98 (1H, m), 7.85 (1H, t), 7.71-7.67 (1H, m), 7.62-7.58 (2H, m), 7.38-7.30 (1H, m), 7.24-7.18 (2H, m), 2.35 (3H, d).
Present compound 163: ¹H-NMR (CDCl₃) δ: 9.17 (1H, d), 9.06 (1H, dd), 8.97 (1H, d), 8.83 (1H, t), 8.41 (1H, t), 8.26-8.22 (1H, m), 8.07-7.98 (2H, m), 7.58 (1H, dd), 7.34-7.22 (3H, m), 2.34 (3H, d).
Present compound 164: ¹H-NMR (CDCl₃) δ: 9.08 (1H, d), 9.02 (1H, d), 8.36 (1H, t), 8.11 (1H, dd), 7.52-7.49 (3H, m), 7.33 (2H, d), 6.67 (1H, dd), 2.43 (3H, s).
Present compound 165: :¹H-NMR (CDCl₃) δ: 9.08 (1H, d), 9.00 (1H, d), 8.37 (1H, t), 8.20 (1H, dd), 7.53-7.48 (2H, m), 7.46-7.43 (1H, m), 7.41-7.38 (1H, m), 7.35-7.31 (2H, m), 2.43 (3H, s).
Present compound 166: ¹H-NMR (CDCl₃) δ: 9.12 (1H, d), 9.01 (1H, d), 8.36 (1H, t), 8.12 (1H, dd), 7.57-7.48 (5H, m), 6.68 (1H, dd).
Present compound 167: ¹H-NMR (CDCl₃) δ: 9.12 (1H, d), 8.99 (1H, d), 8.37 (1H, t), 8.21 (1H, dd), 7.56-7.45 (5H, m), 7.41 (1H, dd).

### Preparation Example 4

A mixture of 2-chloro-4-(ethylsulfonyl)pyrimidine 0.21 g, 4-chlorophenylboronic acid 0.17 g, {1,1'-bis(diphenylphosphino)ferrocene}dichloropalladium (II) 0.07 g, tripotassium phosphate 0.64 g, and DME 4 mL was stirred at reflux for 5 hours. Water was added to the resulting mixture, and the mixture was extracted with ethyl acetate. The resulting organic layer was concentrated under reduced pressure, and the resulting residue was subjected to a silica gel column chromatography (hexane : ethyl acetate = 4 : 1) to obtain the present compound 24 represented by the following formula 0.01 g. 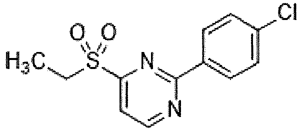 Present compound 24: ¹H-NMR (CDCl₃) δ: 9.14 (1H, d), 8.44 (2H, dt), 7.89 (1H, d), 7.50 (2H, dt), 3.55 (2H, q), 1.41 (3H, t).

### Preparation Example 5

A mixture of the intermediate compound 14 0.40 g, 3-bromofluorobenzene 0.24 mL, palladium acetate 0.02 g, Xantphos 0.11 g, and sodium tert-butoxide 0.35 g, and toluene 5 mL was stirred at reflux for 7 hours. Ethyl acetate was added to the resulting mixture, and the mixture was filtered through Celite. The resulting filtrate was concentrated under reduced pressure, and the resulting residue was subjected to a silica gel column chromatography (hexane : ethyl acetate = 2 : 1) to obtain the present compound 146 represented by the following formula 0.53 g. 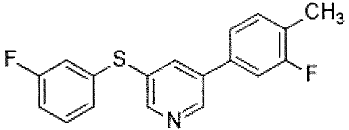 Present compound 146: ¹H-NMR (CDCl₃) δ: 8.72 (1H, d), 8.56 (1H, d), 7.83 (1H, t), 7.35-7.26 (2H, m), 7.24-7.17 (2H, m), 7.16-7.12 (1H, m), 7.07-7.02 (1H, m), 7.01-6.95 (1H, m), 2.32 (3H, d).

### Preparation Example 5-1

The compounds which were prepared according to the Preparation Example 5 and their physical property values are shown.

Compounds (IA-1) wherein a combination of n, X¹, X², X³, X⁴, R¹, R², R³, R^{4A}, R^{4B}, and R^{4C} represents any combination described in [Table A-9].

**[Table A-9]**

| Present compound | n | X¹ | X² | X³ | X⁴ | R¹ | R² | R³ | R^{4A} | R^{4B} | R^{4C} |
|---|---|---|---|---|---|---|---|---|---|---|---|
| 147 | 0 | CH | CH | N | CH | 7-quinolyl | H | H | H | Me | F |
| 148 | 0 | CH | CH | N | CH | 4-pyridyl | H | H | H | Me | F |
| 149 | 0 | CH | CH | N | CH | 2-furyl | H | H | H | Me | F |
| 150 | 0 | CH | CH | N | CH | 2-thienyl | H | H | H | Me | F |
| 151 | 0 | CH | CH | N | CH | 3-furyl | H | H | H | Me | F |
| 152 | 0 | CH | CH | N | CH | 3-thienyl | H | H | H | Me | F |
| 153 | 0 | CH | CH | N | CH | 3-pyridyl | H | H | H | Me | F |
| 168 | 0 | CH | CH | N | CH | 3-thienyl | H | H | H | Me | H |

Present compound 147: LCMS: 347 [M+H]⁺, RT = 1.954 min
Present compound 148: ¹H-NMR (CDCl₃) δ: 8.88 (1H, d), 8.71 (1H, d), 8.42 (2H, dd), 8.02 (1H, t), 7.34-7.22 (3H, m), 7.02 (2H, dd), 2.34 (3H, d).
Present compound 149: ¹H-NMR (CDCl₃) δ: 8.60 (1H, d), 8.39 (1H, d), 7.63-7.59 (2H, m), 7.29-7.23 (1H, m), 7.22-7.13 (2H, m), 6.82 (1H, dd), 6.50 (1H, dd), 2.31 (3H, d).
Present compound 150: ¹H-NMR (CDCl₃) δ: 8.57 (1H, d), 8.40 (1H, d), 7.59 (1H, t), 7.52 (1H, d), 7.37-7.34 (1H, m), 7.30-7.08 (4H, m), 2.31 (3H, d).
Present compound 151: ¹H-NMR (CDCl₃) δ: 8.57 (1H, d), 8.41 (1H, d), 7.69-7.68 (1H, m), 7.61 (1H, t), 7.56-7.54 (1H, m), 7.30-7.23 (1H, m), 7.21-7.13 (2H, m), 6.48 (1H, dd), 2.32 (3H, d).
Present compound 152: ¹H-NMR (CDCl₃) δ: 8.58 (1H, d), 8.40 (1H, d), 7.61 (1H, t), 7.50 (1H, dd), 7.43 (1H, dd), 7.29-7.23 (1H, m), 7.20-7.13 (2H, m), 7.09 (1H, dd), 2.31 (3H, d) .
Present compound 153: ¹H-NMR (CDCl₃) δ: 8.70 (1H, d), 8.66 (1H, d), 8.56-8.53 (2H, m), 7.79 (1H, t), 7.72-7.68 (1H, m), 7.30-7.26 (2H, m), 7.22-7.16 (2H, m), 2.32 (3H, d).
Present compound 168: ¹H-NMR (CDCl₃) δ: 8.61 (1H, d), 8.39 (1H, d), 7.64 (1H, t), 7.48 (1H, dd), 7.44-7.38 (3H, m), 7.29-7.24 (2H, m), 7.09 (1H, dd), 2.40 (3H, s).

### Preparation Example 6

To a mixture of the intermediate compound 14 0.20 g, 2-propanol 0.14 mL, and chloroform 3 mL was added N-chlorosuccinimide 0.49 g at 0 °C, and the mixture was stirred at room temperature for 4 hours. To the resulting mixture were added an aqueous sodium sulfite solution and a saturated aqueous sodium hydrogen carbonate solution, and the mixture was extracted with chloroform. The resulting organic layer was dried over sodium sulfate, and concentrated under reduced pressure.

Pyrrolidine 0.11 mL was added to a mixture of the resulting residue, chloroform 5 mL, and triethylamine 0.19 mL at 0 °C, and the mixture was stirred at room temperature for 2 hours. To the resulting mixture was added an aqueous sodium sulfite solution and the mixture was extracted with chloroform. The resulting organic layer was washed with saturated brine, dried over sodium sulfate, and concentrated under reduced pressure. The resulting residue was subjected to a silica gel column chromatography (hexane : ethyl acetate = 1 : 1) to obtain the present compound 154 represented by the following formula 0.23 g. 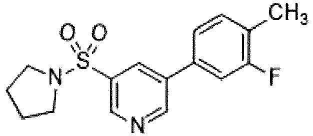 Present compound 154: ¹H-NMR (CDCl₃) δ: 9.02-8.99 (2H, m), 8.25 (1H, t), 7.37-7.26 (3H, m), 3.37-3.29 (4H, m), 2.35 (3H, d), 1.87-1.80 (4H, m).

### Preparation Example 6-1

The compounds which were prepared according to the Preparation Example 6 and their physical property values are shown.

Compounds (IA-1) wherein the combination of n, X¹, X², X³, X⁴, R¹, R², R^{4A}, R^{4B}, and R^{4C} represents the combination described in [Table A-10].

**[Table A-10]**

| Present compound | n | X¹ | X² | X³ | X⁴ | R¹ | R² | R³ | R^{4A} | R^{4B} | R^{4C} |
|---|---|---|---|---|---|---|---|---|---|---|---|
| 199 | 2 | CH | CH | N | CH | NHMe | H | H | H | Cl | H |

Present compound 199: 1H-NMR (CDCl₃) δ: 9.05 (1H, d), 9.01 (1H, d), 8.29 (1H, t), 7.60-7.48 (4H, m), 4.56 (1H, d), 2.78 (3H, d).

### Preparation Example 7

A mixture of the intermediate compound 14 0.10 g, 2-chloropyrazine 0.05 mL, cesium carbonate 0.22 g, and NMP 2 mL was stirred at 80 °C for 2 hours. Water was added to the resulting mixture, and the mixture was extracted with ethyl acetate. The resulting organic layer was dried over sodium sulfate, and concentrated under reduced pressure, and the resulting residue was subjected to a silica gel column chromatography (hexane : ethyl acetate = 1 : 1) to obtain the present compound 155 represented by the following formula 0.12 g. 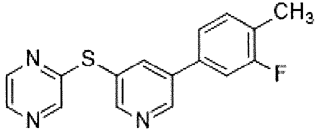 Present compound 155: ¹H-NMR (CDCl₃) δ: 8.85 (1H, d), 8.74 (1H, d), 8.44 (1H, d), 8.35 (1H, dd), 8.32 (1H, d), 8.08 (1H, t), 7.33-7.22 (3H, m), 2.34 (3H, d).

### Preparation Example 7-1

The compounds which were prepared according to the Preparation Example 7 and their physical property values are shown.

Compounds (IA-1) wherein the combination of n, X¹, X², X³, X⁴, R¹, R², R³, R^{4A}, R^{4B}, and R^{4C} represents any combination described in [Table A-8].

**[Table A-8]**

| Present compound | n | X¹ | X² | X³ | X⁴ | R¹ | R² | R³ | R^{4A} | R^{4B} | R^{4C} |
|---|---|---|---|---|---|---|---|---|---|---|---|
| 156 | 0 | CH | CH | N | CH | 2-pyridyl | H | H | H | Me | F |
| 157 | 0 | CH | CH | N | CH | i-Pr | H | H | H | Me | F |

Present compound 156: ¹H-NMR (CDCl₃) δ: 8.81 (1H, d), 8.72 (1H, d), 8.46-8.41 (1H, m), 8.06 (1H, t), 7.54 (1H, td), 7.33-7.21 (3H, m), 7.13-7.05 (2H, m), 2.33 (3H, d).
Present compound 157: ¹H-NMR (CDCl₃) δ: 8.66 (1H, d), 8.59 (1H, d), 7.86 (1H, t), 7.32-7.21 (3H, m), 3.49-3.39 (1H, m), 2.33 (3H, d), 1.34 (6H, d).

### Preparation Example 8

Under nitrogen atmosphere, to a mixture of 5-bromo-1,3-difluoro-2-methylbenzene 0.50 g and THF 5.5 mL was added isopropylmagnesium chloride lithium chloride complex solution (1.3M in THF) 2.4 mL at 0 °C, and resulting mixture was stirred at room temperature for 2 hours. Four(4) mL of the resulting mixture was added dropwise at room temperature to a mixture of intermediate compound 12 0.3 g, nickel(II) chloride 27 mg, zinc(II) chloride 96 mg and THF 3 mL, and stirred at 60 °C for 4.5 hours. To the resulting mixture was added saturated aqueous ammonium chloride solution, and the mixture was extracted with ethyl acetate. The resulting organic layer was concentrated under reduced pressure, and the resulting residue was subjected to a silica gel column chromatography (hexane : ethyl acetate = 1 : 1) to obtain the present compound 96 represented by the following formula.

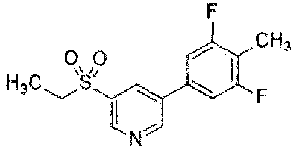

### Present compound 96: LCMS: 298 [M+H]⁺, RT = 1.791 min

The compounds which were prepared according to the above-mentioned Process and Preparation examples and their physical property values are shown.

Compound (IA-1) wherein the combination of n, X¹, X², X³, X⁴, R¹, R², R³, R^{4A}, R^{4B}, and R^{4C} represents any combination described in [Table A-4], [Table A-5], [Table A-6], and [Table A-7].

**[Table A-4]**

| Present compound | n | X¹ | X² | X³ | X⁴ | R¹ | R² | R³ | R^{4A} | R^{4B} | R^{4C} |
|---|---|---|---|---|---|---|---|---|---|---|---|
| 26 | 2 | CH | CH | N | CH | Et | H | H | H | F | H |
| 27 | 0 | N | C (CN) | CCF₃ | CH | Et | H | H | H | Cl | H |
| 28 | 0 | N | C (CN) | CCF₃ | CH | Et | H | H | H | OPr | H |
| 29 | 0 | N | C (CN) | CCF₃ | CH | Et | H | H | H | Me | H |
| 30 | 0 | N | C (CN) | CCF₃ | CH | Et | H | H | H | F | H |
| 31 | 0 | N | C (CN) | CCF₃ | CH | Et | H | H | H | OCH₂CH=CH₂ | H |
| 32 | 0 | N | C (CN) | CCF₃ | CH | Et | H | H | H | Br | H |
| 33 | 0 | N | C (CN) | CCF₃ | CH | Et | H | H | H | OCH₂(4-F-Ph) | H |
| 34 | 0 | N | C (CN) | CCF₃ | CH | Et | H | H | H | OMe | H |
| 35 | 0 | N | C (CN) | CCF₃ | CH | Et | H | H | H | OMe | OMe |

**[Table A-5]**

| Present compound | n | X¹ | X² | X³ | X⁴ | R¹ | R² | R³ | R^{4A} | R^{4B} | R^{4C} |
|---|---|---|---|---|---|---|---|---|---|---|---|
| 36 | 0 | C (CN) | N | CNH₂ | C (CN) | CH₂C≡CH | H | H | H | OH | H |
| 37 | 0 | C (CN) | N | CNH₂ | C (CN) | CH₂C≡CH | H | H | H | H | Cl |
| 39 | 0 | N | N | CH | CH | Me | H | H | H | OMe | H |
| 40 | 2 | N | N | CCF₃ | CH | Et | H | H | H | Me | H |
| 41 | 0 | N | N | CH | CH | (CH₂)₄OR | H | H | H | H | Cl |
| 42 | 0 | N | N | CH | CH | (CH₂)₄OH | H | H | H | Cl | H |
| 43 | 0 | N | N | CH | CH | (CH₂)₄OH | H | H | H | Cl | Cl |

**[Table A-6]**

| Present compound | n | X¹ | X² | X³ | X⁴ | R¹ | R² | R³ | R^{4A} | R^{4B} | R^{4C} |
|---|---|---|---|---|---|---|---|---|---|---|---|
| 44 | 0 | N | N | CH | C(CN) | Me | H | H | H | H | Cl |
| 45 | 0 | N | N | CH | C(CN) | Me | H | H | H | Cl | H |
| 46 | 0 | N | N | CH | CC(O)OMe | Me | H | H | H | H | Cl |
| 47 | 0 | N | N | CH | CC(O)OEt | Me | H | H | H | OMe | OMe |
| 48 | 0 | N | N | CH | CC(O)NH₂ | Me | H | H | H | OMe | OMe |
| 49 | 0 | N | N | CH | CNMe₂ | Me | Me | H | H | Me | H |
| 50 | 0 | N | N | CH | CS(O)₂Me | Me | H | H | H | H | CF₃ |
| 51 | 0 | N | N | CH | CC(O)NMe₂ | Me | H | H | H | H | CF₃ |
| 52 | 0 | N | N | CCl | CH | CH₂Ph | H | H | H | H | CF₃ |
| 53 | 0 | N | N | CH | COMe | CH₂Ph | H | H | H | H | CF₃ |
| 54 | 0 | N | N | COMe | CH | CH₂Ph | H | H | H | H | CF₃ |
| 55 | 0 | N | N | CH | COMe | CH₂CF₃ | H | H | H | H | CF₃ |
| 56 | 0 | N | N | CH | CCl | Me | H | F | H | Cl | OMe |
| 57 | 0 | N | N | CH | CSMe | Me | H | F | H | Cl | OMe |
| 58 | 0 | N | N | CH | CH | Me | H | H | H | OMe | H |

**[Table A-7]**

| Present compound | n | X¹ | X² | X³ | X⁴ | R¹ | R² | R³ | R^{4A} | R^{4B} | R^{4C} |
|---|---|---|---|---|---|---|---|---|---|---|---|
| 59 | 0 | N | CH | CMe | N | CH₂ (4-tBu-Ph) | H | H | H | Cl | H |
| 60 | 0 | CH | N | CH | N | (CH₂)₂OMe | H | H | H | H | Cl |
| 61 | 0 | CH | N | CH | N | CH₂(2-Cl-Ph) | H | H | H | H | Cl |
| 62 | 0 | CH | N | CH | N | CH₂(3-CF₃-Ph) | H | H | H | H | Cl |
| 63 | 0 | CH | N | CH | N | Me | F | H | H | H | SMe |
| 64 | 0 | CH | N | CMe | N | Me | H | H | H | H | CF₃ |
| 65 | 1 | CCl | N | CH | N | Me | F | H | NS(O)₂Et | CN | H |

Present compound 26: ¹H-NMR (CDCl₃) δ: 9.07 (2H, t), 8.32 (1H, t), 7.64-7.58 (2H, m), 7.26-7.20 (2H, m), 3.22 (2H, q), 1.36 (3H, t).
Present compound 28: LCMS: 367 [M+H]⁺, RT = 2.486 min
Present compound 29: LCMS: 323 [M+H]⁺, RT = 2.414 min
Present compound 30: LCMS: 327 [M+H]⁺, RT = 2.316 min
Present compound 31: LCMS: 365 [M+H]⁺, RT = 2.402 min
Present compound 32: LCMS: 387 [M+H]⁺, RT = 2.444 min
Present compound 33: LCMS: 433 [M+H]⁺, RT = 2.452 min
Present compound 34: LCMS: 339 [M+H]⁺, RT = 2.322 min
Present compound 36: LCMS: 305 [M-H]⁻, RT = 1.574 min
Present compound 37: LCMS: 323 [M-H]⁻, RT = 1.873 min
Present compound 39: LCMS: 233 [M+H]⁺, RT = 1.919 min
Present compound 40: LCMS: 331 [M+H]⁺, RT = 1.934 min
Present compound 41: LCMS: 295 [M+H]⁺, RT = 1.837 min
Present compound 42: LCMS: 295 [M+H]⁺, RT = 1.846 min
Present compound 43: LCMS: 329 [M+H]⁺, RT = 1.997 min
Present compound 44: LCMS: 262 [M+H]⁺, RT = 2.065 min
Present compound 45: LCMS: 262 [M+H]⁺, RT = 2.077 min
Present compound 46: LCMS: 295 [M+H]⁺, RT = 2.068 min
Present compound 47: LCMS: 335 [M+H]⁺, RT = 1.882 min
Present compound 48: LCMS: 306 [M+H]⁺, RT = 1.317 min
Present compound 50: LCMS: 349 [M+H]⁺, RT = 1.862 min
Present compound 52: LCMS: 381 [M+H]⁺, RT = 2.481 min
Present compound 53: LCMS: 377 [M+H]⁺, RT = 2.352 min
Present compound 54: LCMS: 377 [M+H]⁺, RT = 2.470 min
Present compound 55: LCMS: 369 [M+H]⁺, RT = 2.235 min
Present compound 56: LCMS: 319 [M+H]⁺, RT = 2.213 min
Present compound 57: LCMS: 331 [M+H]⁺, RT = 2.135 min
Present compound 58: LCMS: 233 [M+H]⁺, RT = 1.917 min
Present compound 59: LCMS: 383 [M+H]⁺, RT = 2.801 min
Present compound 60: LCMS: 281 [M+H]⁺, RT = 2.118 min
Present compound 61: LCMS: 347 [M+H]⁺, RT = 2.520 min
Present compound 62: LCMS: 381 [M+H]⁺, RT = 2.473 min
Present compound 63: LCMS: 267 [M+H]⁺, RT = 1.913 min
Present compound 64: LCMS: 285 [M+H]⁺, RT = 2.258 min
Present compound 65: LCMS: 403 [M+H]⁺, RT = 1.240 min

### Reference Preparation Example 1

A mixture of 2,4-dichloropyrimidine 1.0 g, 3-fluoro-4-methylphenylboronic acid 1.1 g, {1,1'-bis(diphenylphoshino)ferrocene}dichloropalladium (II) 0.24 g, tripotassium phoshoric acid 2.9 g, DME 20 mL, and water 2 mL was stirred at reflux for 7 hours. Water was added to the resulting mixture, and the mixture was extracted with ethyl acetate. The resulting organic layer was concentrated under reduced pressure, and the resulting residue was subjected to a silica gel column chromatography (hexane : ethyl acetate = 2 : 1) to obtain the intermediate compound 1 represented by the following formula 1.03 g. 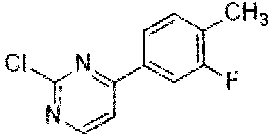 Intermediate compound 1: ¹H-NMR (CDCl₃) δ: 8.64 (1H, d), 7.82-7.74 (2H, m), 7.60 (1H, d), 7.33 (1H, t), 2.36 (3H, d).

### Reference Preparation Example 1-1

The intermediate compounds which was prepared according to the Reference Preparation Example 1 are shown below.

Compounds represented by the following formula (IB-1): 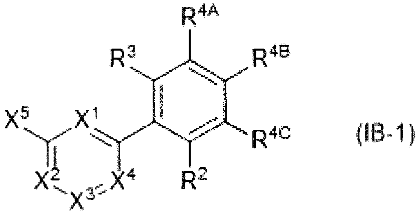 (hereinafter, referred to as "Compound (IB-1)") wherein the combination of X¹, X², X³, X⁴, X⁵, R², R³, R^{4A}, R^{4B}, and R^{4C} represents any combination described in [Table B-1].

**[Table B-1]**

| Intermediate compound | X¹ | X² | X³ | X⁴ | X⁵ | R² | R³ | R^{4A} | R^{4B} | R^{4C} |
|---|---|---|---|---|---|---|---|---|---|---|
| 2 | CH | CH | CH | N | F | H | H | H | Me | F |
| 3 | CH | N | N | CH | Cl | H | H | H | Me | F |

Intermediate compound 2: ¹H-NMR (CDCl₃) δ: 8.26 (1H, d), 7.38-7.35 (1H, m), 7.34-7.27 (3H, m), 7.11-7.09 (1H, m), 2.35 (3H, d).
Intermediate compound 3: ¹H-NMR (CDCl₃) δ: 9.15 (1H, d), 7.84 (1H, d), 7.79 (1H, dd), 7.73 (1H, dd), 7.35 (1H, t), 2.37 (3H, d).

### Reference Preparation Example 2

Under nitrogen atmosphere, to a mixture of sodium hydride (dispersion in Paraffin Liquid, 60 %) 0.31 g and DMF 10 mL, 1-propanethiol 0.50 mL was added at 0°C, and the mixture was stirred at room temperature for 30 minutes. To the resulting mixture was added 2-bromo-4-chloropyridine 1.0 g, and the mixture was stirred at room temperature for 2 hours. Water was added to the resulting mixture, and the mixture was extracted with ethyl acetate. The resulting organic layer was washed with saturated brine, dried over anhydrous sodium sulfate, and concentrated under reduced pressure. The resulting residue was subjected to a silica gel column chromatography (hexane : ethyl acetate = 3 : 1) to obtain the intermediate compound 4 represented by the following formula 1.1 g. 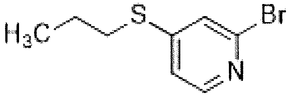 Intermediate compound 4: ¹H-NMR (CDCl₃) δ: 8.12 (1H, d), 7.26 (1H, d), 7.04 (1H, dd), 2.95 (2H, t), 1.81-1.71 (2H, m), 1.08 (3H, t).

### Reference Preparation Example 2-1

The intermediate compounds which were prepared according to the Reference Preparation Example 2 are shown below.

Compounds represented by formula (IB-2): 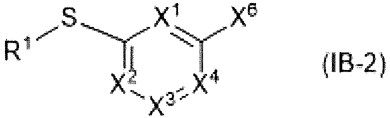 (hereinafter, referred to as "Compound (IB-2)") wherein the combination of X¹, X², X³, X⁴, X⁶, and R¹ represents any combination described in [Table B-2].

**[Table B-2]**

| Intermediate compound | X¹ | X² | X³ | X⁴ | X⁶ | R¹ |
|---|---|---|---|---|---|---|
| 5 | CH | CH | N | CH | Br | Pr |
| 6 | N | CH | N | CH | Br | Pr |
| 7 | CH | CH | N | N | Cl | Pr |
| 9 | N | CH | N | CH | Cl | Pr |
| 10 | CH | CH | N | CH | Br | CH₂(4-OMe-Ph) |
| 15 | CH | CH | N | CH | Br | Bu |
| 16 | CH | CH | N | CH | Br | c-Pn |
| 17 | CH | CH | N | CH | Br | Ph |
| 18 | CH | CH | N | CH | Br | 2-thienyl |
| 19 | CCH₃ | CH | N | CH | Br | Pr |

Intermediate compound 5: ¹H-NMR (CDCl₃) δ: 8.40 (1H, d), 8.35 (1H, d), 7.59 (1H, t), 2.93 (2H, t), 1.76-1.65 (2H, m), 1.05 (3H, t).
Intermediate compound 6: ¹H-NMR (CDCl₃) δ: 8.34 (1H, s), 8.27 (1H, s), 3.15 (2H, t), 1.80-1.70 (2H, m), 1.05 (3H, t).
Intermediate compound 7: ¹H-NMR (CDCl₃) δ: 8.88 (1H, d), 7.21 (1H, d), 3.00 (2H, t), 1.85-1.75 (2H, m), 1.11 (3H, t).
Intermediate compound 9: ¹H-NMR (CDCl₃) δ: 8.32 (1H, s), 8.18 (1H, s), 3.16 (2H, t), 1.81-1.70 (2H, m), 1.05 (3H, t).
Intermediate compound 10: ¹H-NMR (CDCl₃) δ: 8.46 (1H, d), 8.39 (1H, d), 7.68 (1H, t), 7.21-7.16 (2H, m), 6.86-6.81 (2H, m), 4.08 (2H, s), 3.79 (3H, s).
Intermediate compound 15: ¹H-NMR (CDCl₃) δ: 8.44 (1H, d), 8.43 (1H, d), 7.74 (1H, t), 2.94 (2H, t), 1.70-1.61 (2H, m), 1.51-1.41 (2H, m), 0.94 (3H, t).
Intermediate compound 16: ¹H-NMR (CDCl₃) δ: 8.47-8.44 (2H, m), 7.78 (1H, t), 3.66-3.56 (1H, m), 2.18-2.05 (2H, m), 1.86-1.73 (2H, m), 1.71-1.56 (4H, m).
Intermediate compound 17: ¹H-NMR (CDCl₃) δ: 8.47 (1H, d), 8.39 (1H, d), 7.63 (1H, t), 7.47-7.36 (5H, m).
Intermediate compound 18: ¹H-NMR (CDCl₃) δ: 8.43 (1H, d), 8.33 (1H, d), 7.56 (1H, dd), 7.53 (1H, t), 7.35 (1H, dd), 7.13 (1H, dd).
Intermediate compound 19: ¹H-NMR (CDCl₃) δ: 8.48 (1H, s), 8.36 (1H, s), 2.90 (2H, t), 2.50 (3H, s), 1.75-1.63 (2H, m), 1.05 (3H, t).

### Reference Preparation Example 3

To a mixture of 2,4-dichloropyrimidine 1.49 g and DMSO 20 mL was added sodium ethanesulfinate 1.16 g, and the mixture was stirred at room temperature for 8 hours. To the resulting mixture was added saturated brine 20 mL, and the mixture was extracted with MTBE. The resulting organic layer was dried over sodium sulfate, and concentrated under reduced pressure. The resulting residue was subjected to a silica gel column chromatography (hexane : ethyl acetate = 1 : 1) to obtain the intermediate compound 8 represented by the following formula 0.76 g. 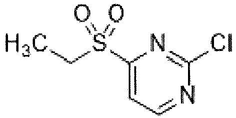 Intermediate compound 8: ¹H-NMR (CDCl₃) δ: 9.00 (1H, d), 7.97 (1H, d), 3.51 (2H, q), 1.39 (3H, t).

### Reference Preparation Example 4

To a mixture of 2-chloro-6-(propylthio)pyrazine 18.5 g, and chloroform 250 mL was added mCPBA (purity 75 %, containing 25 % water) 48.8 g at 0 °C, and the mixture was stirred at room temperature for 2 hours. To the resulting mixture was added mCPBA (purity 75 %, containing 25 %) 1.43 g, and the mixture was left to stand overnight. To the resulting mixture was added a saturated aqueous sodium hydrogen carbonate solution 400 mL, and the mixture was stirred for 1 hour, and then was extracted with chloroform. The resulting organic layer was washed with saturated brine, dried over sodium sulfate, and concentrated under reduced pressure to obtain the intermediate compound 11 24.7 g. 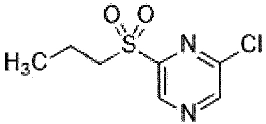 Intermediate compound 11: ¹H-NMR (CDCl₃) δ: 9.18 (1H, s), 8.86 (1H, s), 3.39 (2H, q), 1.85 (2H, m), 1.08 (3H, t).

### Reference Preparation Example 4-1

The intermediate compounds which were prepared according to the Reference Preparation Example 4 are shown below.

Compounds represented by formula (IB-3): 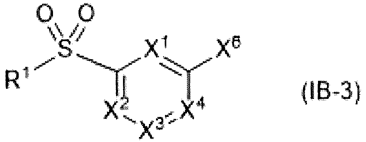 (hereinafter, referred to as "Compound (IB-3)") wherein the combination of X¹, X², X³, X⁴, X⁶, and R¹ represents any combination described in [Table B-3].

**[Table B-3]**

| Intermediate compound | X¹ | X² | X³ | X⁴ | X⁶ | R¹ |
|---|---|---|---|---|---|---|
| 12 | CH | CH | N | CH | Br | Et |
| 13 | CH | CH | N | CH | Br | Pr |
| 20 | CH | CH | N | CH | Br | Bu |
| 21 | CH | CH | N | CH | Br | c-Pn |
| 22 | CH | CH | N | CH | Br | Ph |
| 23 | CH | CH | N | CH | Br | 2-thienyl |
| 24 | CCH₃ | CH | N | CH | Br | Pr |

Intermediate compound 12: ¹H-NMR (CDCl₃) δ: 9.02 (1H, d), 8.95 (1H, d), 8.34 (1H, t), 3.22-3.17 (2H, q), 1.35 (3H, t).
Intermediate compound 13: ¹H-NMR (CDCl₃) δ: 9.01 (1H, d), 8.94 (1H, d), 8.33 (1H, t), 3.16-3.11 (2H, m), 1.85-1.75 (2H, m), 1.05 (3H, t).
Intermediate compound 20: ¹H-NMR (CDCl₃) δ: 9.02 (1H, d), 8.95 (1H, d), 8.34 (1H, t), 3.20-3.12 (2H, m), 1.81-1.67 (2H, m), 1.51-1.38 (2H, m), 0.93 (3H, t).
Intermediate compound 21: ¹H-NMR (CDCl₃) δ: 9.01 (1H, d), 8.93 (1H, d), 8.33 (1H, t), 3.57-3.49 (1H, m), 2.15-2.03 (2H, m), 1.99-1.77 (4H, m), 1.73-1.59 (2H, m).
Intermediate compound 22: ¹H-NMR (CDCl₃) δ: 9.03 (1H, d), 8.83 (1H, d), 8.34 (1H, t), 8.00-7.96 (2H, m), 7.69-7.63 (1H, m), 7.62-7.55 (2H, m).
Intermediate compound 23: ¹H-NMR (CDCl₃) δ: 9.08 (1H, d), 8.85 (1H, d), 8.37 (1H, t), 7.80-7.74 (2H, m), 7.19-7.14 (1H, m) .
Intermediate compound 24: ¹H-NMR (CDCl₃) δ: 9.05 (1H, s), 8.91 (1H, s), 3.18-3.12 (2H, m), 2.79 (3H, s), 1.85-1.75 (2H, m), 1.05 (3H, t).

### Reference Preparation Example 5

To a mixture of the present compound 120 0.7 g and toluene 10 mL was added aluminium chloride 0.41 g at room temperature, and the mixture was stirred at room temperature overnight. The resulting mixture was added dropwise to ice water, and 5% aqueous sodium hydroxide solution was added thereto. The resulting mixture was stirred at room temperature for 1 hours, and then the mixture was neutralized with 1M hydrochloric acid solution and extracted with ethyl acetate. The resulting organic layer was washed with saturated brine, dried over sodium sulfate, and concentrated under reduced pressure. The resulting residue was subjected to a silica gel column chromatography (hexane : ethyl acetate = 1 : 3) to obtain the intermediate compound 14 represented by the following formula 0.38 g. 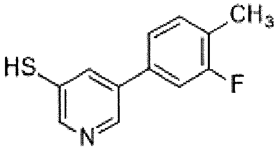 Intermediate compound 14: ¹H-NMR (CDCl₃) δ: 8.59 (1H, d), 8.48 (1H, d), 7.76 (1H, t), 7.33-7.18 (3H, m), 3.49 (1H, s), 2.33 (3H, d).

### Reference Preparation Example 5-1

The intermediate compounds which were prepared according to the Reference Preparation Example 5 are shown below.

Compounds represented by formula (IB-4): 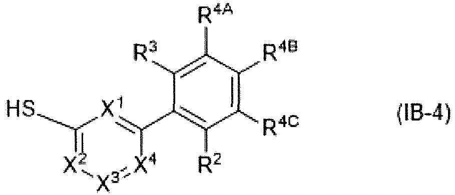 (hereinafter, referred to as "Compound (IB-4)") wherein the combination of X¹, X², X³, X⁴, R², R³, R^{4A}, R^{4B}, and R^{4C} represents any combination described in [Table B-4].

**[Table B-4]**

| Intermediate compound | X¹ | X² | X³ | X⁴ | R² | R³ | R^{4A} | R^{4B} | R^{4C} |
|---|---|---|---|---|---|---|---|---|---|
| 25 | CH | CH | N | CH | H | H | H | Cl | H |
| 26 | CH | CH | N | CH | H | H | H | Me | H |

Intermediate compound 25: ¹H-NMR (CDCl₃) δ: 8.59 (1H, d), 8.50 (1H, d), 7.76 (1H, t), 7.51-7.44 (4H, m), 3.50 (1H, s).
Intermediate compound 26: ¹H-NMR (CDCl₃) δ: 8.61 (1H, d), 8.46 (1H, d), 7.78 (1H, t), 7.47-7.43 (2H, m), 7.31-7.27 (2H, m), 3.48 (1H, s), 2.41 (3H, s).

The present compounds which are prepared according to the above-mentioned Preparation process and Examples of the present compounds are shown below.

Compounds represented by formula (A-1): 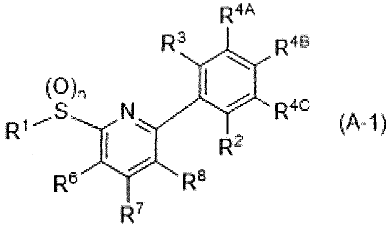 (hereinafter, referred to as "Compound (A-1)") wherein n is 0, each of R⁶, R⁷, and R⁸ represents a hydrogen atom, and the combination of R¹, R^{4A}, R^{4B}, R^{4C}, R² and R³ represents any combination described in the combination A (hereinafter, referred to as "Compound class SX1").

The combination A consists of Substituent Group Nos. A1 to A1863. The Substituents Group Nos. A1 to A1863 represents the combination of R¹, R^{4A}, R^{4B}, R^{4C}, R² and R³ in the compound represented by formula (A-1), the compound represented by formula (A-2), the compound represented by formula (A-3), the compound represented by formula (A-4), the compound represented by formula (A-5), the compound represented by formula (A-6), the compound represented by formula (A-7), the compound represented by formula (A-9), and the compound represented by formula (A-10), which is hereafter referred to as [Substituent Group No.; R¹, R^{4A}, R^{4B}, R^{4C}, R², R³].

For example, Substituent Group No. A5 represents the combination where R¹ represents an ethyl group, each of R^{4A}, R^{4B} and R^{4C} represents a methyl group, and R² and R³ represent a hydrogen atom.

### Combination A

[A1;Et,Me,H,H,H,H], [A2;Et,H,Me,H,H,H], [A3;Et,Me,Me,H,H,H], [A4;Et,Me,H,Me,H,H], [A5;Et,Me,Me,Me,H,H], [A6;Et,F,H,H,H,H], [A7;Et,H,F,H,H,H], [A8;Et,F,F,H,H,H], [A9;Et,F,H,F,H,H], [A10;Et,F,F,F,H,H], [A11;Et,Cl,H,H,H, H], [A12;Et,H,Cl,H,H,H], [A13;Et,Cl,Cl,H,H,H], [A14;Et,Cl,H,Cl,H,H], [A15;Et,Cl,Cl,Cl,H,H], [A16;Et,Br,H,H,H,H], [A17;Et,H,Br,H,H,H], [A18;Et,Br,Br,H,H,H], [A19;Et,Br,H,Br,H,H], [A20;Et,Br,Br,Br,H,H], [A21;Et,I,H,H,H,H], [A22;Et,H,I,H,H,H], [A23;Et,I,I,H,H,H], [A24;Et,I,H,I,H,H], [A25;Et,I,I,I,H,H], [A26;Et,Et,H,H,H,H], [A27;Et,H,Et,H,H,H], [A28;Et,Et,H,Et,H,H], [A29;Et,CF₃,H,H,H,H], [A30;Et,H,CF₃,H,H,H], [A31;Et,CF₃,H,CF₃,H,H], [A32;Et,c-Pr,H,H,H,H], [A33;Et,H,c-Pr,H,H,H], [A34;Et,OMe,H,H,H,H], [A35;Et,H,OMe,H,H,H], [A36;Et,OMe,H,OMe,H,H], [A37;Et,SMe,H,H,H,H], [A38;Et,H,SMe,H,H,H], [A39;Et,S(O)Me,H,H,H,H], [A40;Et,H,S(O)Me,H,H,H], [A41;Et,S(O)₂Me,H,H,H,H], [A42;Et,H,S(O)₂Me,H,H,H], [A43;Et,C(O)OMe,H,H,H,H], [A44;Et,H,C(O)OMe,H,H,H], [A45;Et,C(O)Me,H,H,H,H], [A46;Et,H,C(O)Me,H,H,H], [A47;Et,CN,H,H,H,H], [A48;Et,H,CN,H,H,H], [A49;Et,NO₂,H,H,H,H], [A50;Et,H,NO₂,H,H,H], [A51;Et,NH₂,H,H,H,H], [A52;Et,H,NH₂,H,H,H], [A53;Et,NMe₂,H,H,H,H], [A54;Et,H,NMe₂,H,H,H], [A55;Et,Ph,H,H,H,H], [A56;Et,H,Ph,H,H,H], [A57;Et,OPh,H,H,H,H], [A58;Et,H,OPh,H,H,H], [A59;Et,Me,F,H,H,H], [A60;Et,Me,H,F,H,H], [A61;Et,Me,F,F,H,H], [A62;Et,Me,Cl,H,H,H], [A63;Et,Me,H,Cl,H,H], [A64;Et,Me,Cl,Cl,H,H], [A65;Et,Me,Br,H,H,H], [A66;Et,Me,H,Br,H,H], [A67;Et,Me,Br,Br,H,H], [A68;Et,Me,I,H,H,H], [A69;Et,Me,H,I,H,H], [A70;Et,Me,I,I,H,H], [A71;Et,Me,F,Me,H,H], [A72;Et,Me,Cl,Me,H,H], [A73;Et,Me,Br,Me,H,H], [A74;Et,Me,I,Me,H,H], [A75;Et,Me,Me,F,H,H], [A76;Et,Me,Me,Cl,H,H], [A77;Et,Me,Me,Br,H,H], [A78;Et,Me,Me,I,H,H], [A79;Et,F,Me,H,H,H], [A80;Et,F,Me,F,H,H], [A81;Et,Cl,Me,H,H,H], [A82;Et,Cl,Me,Cl,H,H], [A83;Et,Br,Me,H,H,H], [A84;Et,Br,Me,Br,H,H], [A85;Et,I,Me,H,H,H], [A86;Et,I,Me,I,H,H], [A87;Et,F,Cl,H,H,H], [A88;Et,F,H,Cl,H,H], [A89;Et,F,Cl,Cl,H,H], [A90;Et,F,Br,H,H,H], [A91;Et,F,H,Br,H,H], [A92;Et,F,Br,Br,H,H], [A93;Et,F,I,H,H,H], [A94;Et,F,H,I,H,H], [A95;Et,F,I,I,H,H], [A96;Et,F,F,F,H,H], [A97;Et,F,Cl,F,H,H], [A98;Et,F,Br,F,H,H], [A99;Et,F,I,F,H,H], [A100;Et,F,F,Cl,H,H], [A101;Et,F,F,Br,H,H], [A102;Et,F,F,I,H,H], [A103;Et,Cl,F,H,H,H], [A104;Et,Cl,F,Cl,H,H], [A105;Et,Br,F,H,H,H], [A106;Et,Br,F,Br,H,H], [A107;Et,I,F,H,H,H], [A108;Et,I,F,I,H,H], [A109;Et,Cl,Br,H,H,H], [A110;Et,Cl,H,Br,H,H], [A111;Et,Cl,Br,Br,H,H], [A112;Et,Cl,I,H,H,H], [A113;Et,Cl,H,I,H,H], [A114;Et,Cl,I,I,H,H], [A115;Et,Br,Cl,H,H,H], [A116;Et,Br,Cl,Br,H,H], [A117;Et,I,Cl,H,H,H], [A118;Et,I,Cl,I,H,H], [A119;Et,Cl,Cl,Br,H,H], [A120;Et,Cl,Cl,I,H,H], [A121;Et,Cl,Br,Cl,H,H], [A122;Et,Cl,I,Cl,H,H], [A123;Et,Br,I,H,H,H], [A124;Et,Br,H,I,H,H], [A125;Et,Br,I,I,H,H], [A126;Et,Br,Br,I,H,H], [A127;Et,Br,I,Br,H,H], [A128;Et,Me,Me,H,F,H], [A129;Et,Me,H,Me,F,H], [A130;Et,Me,Me,Me,F,H], [A131;Et,F,F,H,F,H], [A132;Et,F,H,F,F,H], [A133;Et,Cl,Cl,H,F,H], [A134;Et,Cl,H,Cl,F,H], [A135;Et,Br,Br,H,F,H], [A136;Et,Br,H,Br,F,H], [A137;Et,I,I,H,F,H], [A138;Et,I,H,I,F,H], [A139;Et,F,Me,F,F,H], [A140;Et,F,F,F,F,H], [A141;Et,F,Cl,F,F,H], [A142;Et,F,Br,F,F,H], [A143;Et,F,I,F,F,H], [A144;Pr,Me,H,H,H,H], [A145;Pr,H,Me,H,H,H], [A146;Pr,Me,Me,H,H,H], [A147;Pr,Me,H,Me,H,H], [A148;Pr,Me,Me,Me,H,H], [A149;Pr,F,H,H,H,H], [A150;Pr,H,F,H,H,H], [A151;Pr,F,F,H,H,H], [A152;Pr,F,H,F,H,H], [A153;Pr,F,F,F,H,H], [A154;Pr,Cl,H,H,H,H], [A155;Pr,H,Cl,H,H,H], [A156;Pr,Cl,Cl,H,H,H], [A157;Pr,Cl,H,Cl,H,H], [A158;Pr,Cl,Cl,Cl,H,H], [A159;Pr,Br,H,H,H,H], [A160;Pr,H,Br,H,H,H], [A161;Pr,Br,Br,H,H,H], [A162;Pr,Br,H,Br,H,H], [A163;Pr,Br,Br,Br,H,H], [A164;Pr,I,H,H,H,H], [A165;Pr,H,I,H,H,H], [A166;Pr,I,I,H,H,H], [A167;Pr,I,H,I,H,H], [A168;Pr,I,I,I,H,H], [A169;Pr,Et,H,H,H,H], [A170;Pr,H,Et,H,H,H], [A171;Pr,Et,H,Et,H,H], [A172;Pr,CF₃,H,H,H,H], [A173;Pr,H,CF₃,H,H,H], [A174;Pr,CF₃,H,CF₃,H,H], [A175;Pr,c-Pr,H,H,H,H], [A176;Pr,H,c-Pr,H,H,H], [A177;Pr,OMe,H,H,H,H], [A178;Pr,H,OMe,H,H,H], [A179;Pr,OMe,H,OMe,H,H], [A180;Pr,SMe,H,H,H,H], [A181;Pr,H,SMe,H,H,H], [A182;Pr,S(O)Me,H,H,H,H], [A183;Pr,H,S(O)Me,H,H,H], [A184;Pr,S(O)₂Me,H,H,H,H], [A185;Pr,H,S(O)₂Me,H,H,H], [A186;Pr,C(O)OMe,H,H,H,H], [A187;Pr,H,C(O)OMe,H,H,H], [A188;Pr,C(O)Me,H,H,H,H], [A189;Pr,H,C(O)Me,H,H,H], [A190;Pr,CN,H,H,H,H], [A191;Pr,H,CN,H,H,H], [A192;Pr,NO₂,H,H,H,H], [A193;Pr,H,NO₂,H,H,H], [A194;Pr,NH₂,H,H,H,H], [A195;Pr,H,NH₂,H,H,H], [A196;Pr,NMe₂,H,H,H,H], [A197;Pr,H,NMe₂,H,H,H], [A198;Pr,Ph,H,H,H,H], [A199;Pr,H,Ph,H,H,H], [A200;Pr,OPh,H,H,H,H], [A201;Pr,H,OPh,H,H,H], [A202;Pr,Me,F,H,H,H], [A203;Pr,Me,H,F,H,H], [A204;Pr,Me,F,F,H,H], [A205;Pr,Me,Cl,H,H,H], [A206;Pr,Me,H,Cl,H,H], [A207;Pr,Me,Cl,Cl,H,H], [A208;Pr,Me,Br,H,H,H], [A209;Pr,Me,H,Br,H,H], [A210;Pr,Me,Br,Br,H,H], [A211;Pr,Me,I,H,H,H], [A212;Pr,Me,H,I,H,H], [A213;Pr,Me,I,I,H,H], [A214;Pr,Me,F,Me,H,H], [A215;Pr,Me,Cl,Me,H,H], [A216;Pr,Me,Br,Me,H,H], [A217;Pr,Me,I,Me,H,H], [A218;Pr,Me,Me,F,H,H], [A219;Pr,Me,Me,Cl,H,H], [A220;Pr,Me,Me,Br,H,H], [A221;Pr,Me,Me,I,H,H], [A222;Pr,F,Me,H,H,H], [A223;Pr,F,Me,F,H,H], [A224;Pr,Cl,Me,H,H,H], [A225;Pr,Cl,Me,Cl,H,H], [A226;Pr,Br,Me,H,H,H], [A227;Pr,Br,Me,Br,H,H], [A228;Pr,I,Me,H,H,H], [A229;Pr,I,Me,I,H,H], [A230;Pr,F,Cl,H,H,H], [A231;Pr,F,H,Cl,H,H], [A232;Pr,F,Cl,Cl,H,H], [A233;Pr,F,Br,H,H,H], [A234;Pr,F,H,Br,H,H], [A235;Pr,F,Br,Br,H,H], [A236;Pr,F,I,H,H,H], [A237;Pr,F,H,I,H,H], [A238;Pr,F,I,I,H,H], [A239;Pr,F,F,F,H,H], [A240;Pr,F,Cl,F,H,H], [A241;Pr,F,Br,F,H,H], [A242;Pr,F,I,F,H,H , [A243;Pr,F,F,Cl,H,H], [A244;Pr,F,F,Br,H,H], [A245;Pr,F,F,I,H,H], [A246;Pr,Cl,F,H,H,H], [A247;Pr,Cl,F,Cl,H,H], [A248;Pr,Br,F,H,H,H], [A249;Pr,Br,F,Br,H,H], [A250;Pr,I,F,H,H,H], [A251;Pr,I,F,I,H,H], [A252;Pr,Cl,Br,H,H,H], [A253;Pr,Cl,H,Br,H,H], [A254;Pr,Cl,Br,Br,H,H], [A255;Pr,Cl,I,H,H,H], [A256;Pr,Cl,H,I,H,H], [A257;Pr,Cl,I,I,H,H], [A258;Pr,Br,Cl,H,H,H], [A259;Pr,Br,Cl,Br,H,H], [A260;Pr,I,Cl,H,H,H], [A261;Pr,I,Cl,I,H,H], [A262;Pr,Cl,Cl,Br,H,H], [A263;Pr,Cl,Cl,I,H,H], [A264;Pr,Cl,Br,Cl,H,H], [A265;Pr,Cl,I,Cl,H,H], [A266;Pr,Br,I,H,H,H], [A267;Pr,Br,H,I,H,H], [A268;Pr,Br,I,I,H,H], [A269;Pr,Br,Br,I,H,H], [A270;Pr,Br,I,Br,H,H], [A271;Pr,Me,Me,H,F,H], [A272;Pr,Me,H,Me,F,H], [A273;Pr,Me,Me,Me,F,H], [A274;Pr,F,F,H,F,H], [A275;Pr,F,H,F,F,H], [A276;Pr,Cl,Cl,H,F,H], [A277;Pr,Cl,H,Cl,F,H], [A278;Pr,Br,Br,H,F,H], [A279;Pr,Br,H,Br,F,H], [A280;Pr,I,I,H,F,H], [A281;Pr,I,H,I,F,H], [A282;Pr,F,Me,F,F,H], [A283;Pr,F,F,F,F,H], [A284;Pr,F,Cl,F,F,H], [A285;Pr,F,Br,F,F,H], [A286;Pr,F,I,F,F,H], [A287;CH=CH₂,Me,Me,H,H,H], [A288;CH=CH₂,Me,H,Me,H,H], [A289;CH=CH₂, Me,Me,Me,H,H], [A290;CH=CH₂,F,F,H,H,H], [A291;CH=CH₂,F,H,F,H,H], [A292;CH=CH₂,Cl,Cl,H,H,H], [A293;CH=CH₂,Cl,H,Cl,H,H], [A294;CH=CH₂,Br,Br,H,H,H], [A295;CH=CH₂,Br,H,Br,H,H], [A296;CH=CH₂,I,I,H,H,H], [A297;CH=CH₂,I,H,I,H,H], [A298;CH=CH₂,F,Me,F,H,H], [A299;CH=CH₂,F,F,F,H,H], [A300;CH=CH₂,F,Cl,F,H,H], [A301;CH=CH₂,F,Br,F,H,H] [A302;CH=CH₂,F,I,F,H,H], [A303;CH=CH₂,F,Me,H,H,H], [A304;CH=CHCH₃,Me,Me,H,H,H], [A305;CH=CHCH₃,Me,H,Me,H,H], [A306;CH=CHCH₃,Me,Me,Me,H,H], [A307;CH=CHCH₃,F,F,H,H,H], [A308;CH=CHCH₃,F,H,F,H,H], [A309;CH=CHCH₃,Cl,Cl,H,H,H], [A310;CH=CHCH₃,Cl,H,Cl,H,H], [A311;CH=CHCH₃,Br,Br,H,H,H], [A312;CH=CHCH₃,Br,H,Br,H,H], [A313;CH=CHCH₃,I,I,H,H,H], [A314;CH=CHCH₃,I,H,I,H,H], [A315;CH=CHCH₃,F,Me,F,H,H], [A316;CH=CHCH₃,F,F,F,H,H], [A317;CH=CHCH₃,F,Cl,F,H,H], [A318;CH=CHCH₃,F,Br,F,H,H], [A319;CH=CHCH₃,F,I,F,H,H], [A320;CH=CHCH₃,F,Me,H,H,H], [A321;CH₂CH=CH₂,Me,Me,H,H,H], [A322;CH₂CH=CH₂,Me,H,Me,H,H], [A323;CH₂CH=CH₂,Me,Me,Me,H,H], [A324;CH₂CH=CH₂,F,F,H,H,H], [A325;CH_{2C}H=CH₂,F,H,F,H,H], [A326;CH₂CH=CH₂,Cl,Cl,H,H,H], [A327;CH₂CH=CH₂,Cl,H,Cl,H,H], [A328;CH₂CH=CH₂,Br,Br,H,H,H], [A329;CH₂CH=CH₂,Br,H,Br,H,H], [A330;CH₂CH=CH₂,I,I,H,H,H], [A331;CH₂CH=CH₂,I,H,I,H,H], [A332;CH₂CH=CH₂ ,F,Me,F,H,H], [A333;CH₂CH=CH₂,F,F,F,H,H], [A334;CH₂CH=CH₂,F,Cl,F,H,H], [A335;CH₂CH=CH₂,F,Br,F,H,H], [A336;CH₂CH=CH₂,F,I,F,H,H], [A337;CH₂CH=CH₂,F,Me,H,H,H], [A338;i-Pr,Me,Me,H,H,H], [A339;i-Pr,Me,H,Me,H,H], [A340;i-Pr,Me,Me,Me,H,H], [A341;i-Pr,F,F,H,H,H], [A342;i-Pr,F,H,F,H,H], [A343;i-Pr,Cl,Cl,H,H,H], [A344;i-Pr,Cl,H,Cl,H,H], [A345;i-Pr,Br,Br,H,H,H], [A346;i-Pr,Br,H,Br,H,H], [A347;i-Pr,I,I,H,H,H], [A348;i-Pr,I,H,I,H,H], [A349;i-Pr,F,Me,F,H,H], [A350;i-Pr,F,F,F,H,H], [A351;i-Pr,F,Cl,F,H,H], [A352;i-Pr,F,Br,F,H,H], [A353;i-Pr,F,I,F,H,H], [A354;i-Pr,F,Me,H,H,H], [A355;Bu,Me,Me,H,H,H], [A356;Bu,Me,H,Me,H,H], [A357;Bu,Me,Me,Me,H,H], [A358;Bu,F,F,H,H,H], [A359;Bu,F,H,F,H,H], [A360;Bu,Cl,Cl,H,H,H], [A361;Bu,Cl,H,Cl,H,H], [A362;Bu,Br,Br,H,H,H], [A363;Bu,Br,H,Br,H,H], [A364;Bu,I,I,H,H,H], [A365;Bu,I,H,I,H,H], [A366;Bu,F,Me,F,H,H], [A367;Bu,F,F,F,H,H], [A368;Bu,F,Cl,F,H,H], [A369;Bu,F,Br,F,H,H], [A370;Bu,F,I,F,H,H], [A371;Bu,F,Me,H,H,H], [A372;i-Bu,Me,Me,H,H,H], [A373;i-Bu,Me,H,Me,H,H], [A374;i-Bu,Me,Me,Me,H,H], [A375;i-Bu,F,F,H,H,H], [A376;i-Bu,F,H,F,H,H], [A377;i-Bu,Cl,Cl,H,H,H], [A378;i-Bu,Cl,H,Cl,H,H], [A379;i-Bu,Br,Br,H,H,H], [A380;i-Bu,Br,H,Br,H,H], [A381;i-Bu,I,I,H,H,H], [A382;i-Bu,I,H,I,H,H], [A383;i-Bu,F,Me,F,H,H], [A384;i-Bu,F,F,F,H,H], [A385;i-Bu,F,Cl,F,H,H], [A386;i-Bu,F,Br,F,H,H], [A387;i-Bu,F,I,F,H,H], [A388;i-Bu,F,Me,H,H,H], [A389;s-Bu,Me,Me,H,H,H], [A390;s-Bu,Me,H,Me,H,H], [A391;s-Bu,Me,Me,Me,H,H], [A392;s-Bu,F,F,H,H,H], [A393;s-Bu,F,H,F,H,H], [A394;s-Bu,Cl,Cl,H,H,H], [A395;s-Bu,Cl,H,Cl,H,H], [A396;s-Bu,Br,Br,H,H,H], [A397;s-Bu,Br,H,Br,H,H], [A398;s-Bu,I,I,H,H,H], [A399;s-Bu,I,H,I,H,H], [A400;s-Bu,F,Me,F,H,H], [A401;s-Bu,F,F,F,H,H], [A402;s-Bu,F,Cl,F,H,H], [A403;s-Bu,F,Br,F,H,H], [A404;s-Bu,F,I,F,H,H], [A405;s-Bu,F,Me,H,H,H], [A406;c-Pr,Me,Me,H,H,H], [A407;c-Pr,Me,H,Me,H,H], [A408;c-Pr,Me,Me,Me,H,H], [A409;c-Pr,F,F,H,H,H], [A410;c-Pr,F,H,F,H,H], [A411;c-Pr,Cl,Cl,H,H,H], [A412;c-Pr,Cl,H,Cl,H,H], [A413;c-Pr,Br,Br,H,H,H], [A414;c-Pr,Br,H,Br,H,H], [A415;c-Pr,I,I,H,H,H], [A416;c-Pr,I,H,I,H,H], [A417;c-Pr,F,Me,F,H,H], [A418;c-Pr,F,F,F,H,H], [A419;c-Pr,F,Cl,F,H,H], [A420;c-Pr,F,Br,F,H,H], [A421;c-Pr,F,I,F,H,H], [A422;c-Pr,F,Me,H,H,H], [A423;CH₂(c-Pr),Me,Me,H,H,H], [A424;CH₂(c-Pr),Me,H,Me,H,H], [A425;CH₂(c-Pr),Me,Me,Me,H,H], [A426;CH₂(c-Pr),F,F,H,H,H], [A427;CH₂(c-Pr),F,H,F,H,H], [A428;CH₂(c-Pr),Cl,Cl,H,H,H], [A429;CH₂(c-Pr),Cl,H,Cl,H,H], [A430;CH₂(c-Pr),Br,Br,H,H,H], [A431;CH₂(c-Pr),Br,H,Br,H,H], [A432;CH₂(c-Pr),I,I,H,H,H], [A433;CH₂(c-Pr),I,H,I,H,H], [A434;CH₂(c-Pr),F,Me,F,H,H], [A435;CH₂(c-Pr),F,F,F,H,H], [A436;CH₂(c-Pr),F,Cl,F,H,H], [A437;CH₂ (c-Pr),F,Br,F,H,H], [A438;CH₂(c-Pr),F,I,F,H,H], [A439;CH₂(c-Pr),F,Me,H,H,H], [A440;Bn,Me,Me,H,H,H], [A441;Bn,Me,H,Me,H,H], [A442;Bn,Me,Me,Me,H,H], [A443;Bn,F,F,H,H,H], [A444;Bn,F,H,F,H,H], [A445;Bn,Cl,Cl,H,H,H], [A446;Bn,Cl,H,Cl,H,H], [A447;Bn,Br,Br,H,H,H], [A448;Bn,Br,H,Br,H,H], [A449;Bn,I,I,H,H,H], [A450;Bn,I,H,I,H,H], [A451;Bn,F,Me,F,H,H], [A452;Bn,F,F,F,H,H], [A453;Bn,F,Cl,F,H,H], [A454;Bn,F,Br,F,H,H], [A455;Bn,F,I,F,H,H], [A456;Bn,F,Me,H,H,H], [A457;Me,Me,H,H,H,H], [A458;Me,H,Me,H,H,H], [A459;Me,Me,Me,H,H,H], [A460;Me,Me,H,Me,H,H], [A461;Me,Me,Me,Me,H,H], [A462;Me,F,H,H,H,H], [A463;Me,H,F,H,H,H], [A464;Me,F,F,H,H,H], [A465;Me,F,H,F,H,H], [A466;Me,F,F,F,H,H], [A467;Me,Cl,H,H,H,H], [A468;Me,H,Cl,H,H,H], [A469;Me,Cl,Cl,H,H,H], [A470;Me,Cl,H,Cl,H,H], [A471;Me,Cl,Cl,Cl,H,H], [A472;Me,Br,H,H,H,H], [A473;Me,H,Br,H,H,H], [A474;Me,Br,Br,H,H,H], [A475;Me,Br,H,Br,H,H], [A476;Me,Br,Br,Br,H,H], [A477;Me,I,H,H,H,H], [A478;Me,H,I,H,H,H], [A479;Me,I,I,H,H,H], [A480;Me,I,H,I,H,H], [A481;Me,I,I,I,H,H], [A482;Me,Et,H,H,H,H], [A483;Me,H,Et,H,H,H], [A484;Me,Et,H,Et,H,H], [A485;Me,CF₃,H,H,H,H], [A486;Me,H, CF₃,H,H,H], [A487;Me,CF₃,H,CF₃,H,H], [A488;Me,c-Pr,H,H,H,H], [A489;Me,H,c-Pr,H,H,H], [A490;Me,OMe,H,H,H,H], [A491;Me,H,OMe,H,H,H], [A492;Me,OMe,H,OMe,H,H], [A493;Me,SMe,H,H,H,H], [A494;Me,H,SMe,H,H,H], [A495;Me,S(O)Me,H,H,H,H], [A496;Me,H,S(O)Me,H,H,H], [A497;Me,S(O)₂Me,H,H,H,H], [A498;Me,H,S(O)₂Me,H,H,H], [A499;Me,C(O)OMe,H,H,H,H], [A500;M₂,H,C(O)OM₂,H,H,H], [A501;M₂,C(O)M₂,H,H,H,H], [A502;Me,H,C(O)Me,H,H,H], [A503;Me,CN,H,H,H,H], [A504;Me,H,CN,H,H,H], [A505;Me,NO₂,H,H,H,H], [A506;Me,H,NO₂,H,H,H], [A507;Me,NH₂,H,H,H,H], [A508;Me,H,NH₂,H,H,H], [A509;Me,NMe₂,H,H,H,H], [A510;Me,H,NMe₂,H,H,H], [A511;Me,Ph,H,H,H,H], [A512;Me,H,Ph,H,H,H], [A513;Me,OPh,H,H,H,H], [A514;Me,H,OPh,H,H,H], [A515;Me,Me,F,H,H,H], [A516;Me,Me,H,F,H,H], [A517;Me,Me,F,F,H,H], [A518;Me,Me,Cl,H,H,H], [A519;Me,Me,H,Cl,H,H], [A520;Me,Me,Cl,Cl,H,H], [A521;Me,Me,Br,H,H,H], [A522;Me,Me,H,Br,H,H], [A523;Me,Me,Br,Br,H,H], [A524;Me,Me,I,H,H,H], [A525;Me,Me,H,I,H,H], [A526;Me,Me,I,I,H,H], [A527;Me,Me,F,Me,H,H], [A528;Me,Me,Cl,Me,H,H], [A529;Me,Me,Br,Me,H,H], [A530;Me,Me,I,Me,H,H], [A531;Me,Me,Me,F,H,H], [A532;Me,Me,Me,Cl,H,H], [A533;Me,Me,Me,Br,H,H], [A534;Me,Me,Me,I,H,H], [A535;Me,F,Me,H,H,H], [A536;Me,F,Me,F,H,H], [A537;Me,Cl,Me,H,H,H], [A538;Me,Cl,Me,Cl,H,H], [A539;Me,Br,Me,H,H,H], [A540;Me,Br,Me,Br,H,H], [A541;Me,I,Me,H,H,H], [A542;Me,I,Me,I,H,H], [A543;Me,F,Cl,H,H,H], [A544;Me,F,H,Cl,H,H], [A545;Me,F,Cl,Cl,H,H], [A546;Me,F,Br,H,H,H], [A547;Me,F,H,Br,H,H], [A548;Me,F,Br,Br,H,H], [A549;Me,F,I,H,H,H], [A550;Me,F,H,I,H,H], [A551;Me,F,I,I,H,H], [A552;Me,F,F,F,H,H], [A553;Me,F,Cl,F,H,H], [A554;Me,F,Br,F,H,H], [A555;Me,F,I,F,H,H], [A556;Me,F,F,Cl,H,H], [A557;Me,F,F,Br,H,H], [A558;Me,F,F,I,H,H], [A559;Me,Cl,F,H,H,H], [A560;Me,Cl,F,Cl,H,H], [A561;Me,Br,F,H,H,H], [A562;Me,Br,F,Br,H,H], [A563;Me,I,F,H,H,H], [A564;Me,I,F,I,H,H], [A565;Me,Cl,Br,H,H,H], [A566;Me,Cl,H,Br,H,H], [A567;Me,Cl,Br,Br,H,H], [A568;Me,Cl,I,H,H,H], [A569;Me,Cl,H,I,H,H], [A570;Me,Cl,I,I,H,H], [A571;Me,Br,Cl,H,H,H], [A572;Me,Br,Cl,Br,H,H], [A573;Me,I,Cl,H,H,H], [A574;Me,I,Cl,I,H,H], [A575;Me,Cl,Cl,Br,H,H], [A576;Me,Cl,Cl,I,H,H], [A577;Me,Cl,Br,Cl,H,H], [A578;Me,Cl,I,Cl,H,H], [A579;Me,Br,I,H,H,H], [A580;Me,Br,H,I,H,H], [A581;Me,Br,I,I,H,H], [A582;Me,Br,Br,I,H,H], [A583;Me,Br,I,Br,H,H], [A584;Me,Me,Me,H,F,H], [A585;Me,Me,H,Me,F,H], [A586;Me,Me,Me,Me,F,H], [A587;Me,F,F,H,F,H], [A588;Me,F,H,F,F,H], [A589;Me,Cl,Cl,H,F,H], [A590;Me,Cl,H,Cl,F,H], [A591;Me,Br,Br,H,F,H], [A592;Me,Br,H,Br,F,H], [A593;Me,I,I,H,F,H], [A594;Me,I,H,I,F,H], [A595;Me,F,Me,F,F,H], [A596;Me,F,F,F,F,H], [A597;Me,F,Cl,F,F,H], [A598;Me,F,Br,F,F,H], [A599;Me,F,I,F,F,H], [A600;Me,H,OCH₂CH₂CH₃,H,H,H], [A601;Me,H,OCH₂CH=CH₂,H,H,H], [A602;Me,H,OCH₂(4-F-Ph),H,H,H], [A603;Me,OMe,OMe,H,H,H], [A604;Me,H,OH,H,H,H], [A605;Me,H,Me,H,Me,H], [A606;Me,OMe,Cl,H,F,H], [A607;Me,SMe,H,H,F,H], [A608;Me,NHS(O)₂Et,CN,H,F,H], [A609;Et,H,OCH₂CH₂CH₃,H,H,H], [A610;Et,H,OCH₂CH=CH₂,H,H,H], [A611;Et,H,OCH₂ (4-F-Ph),H,H,H], [A612;Et,OMe,OMe,H,H,H], [A613;Et,H,OH,H,H,H], [A614;Et,H,Me,H,Me,H], [A615;Et,OMe,Cl,H,F,H], [A616;Et,SMe,H,H,F,H], [A617;Et,NHS(O)₂Et,CN,H,F,H], [A618;Pr,H,OCH₂CH₂CH₃,H,H,H], [A619;Pr,H,OCH₂CH=CH₂,H,H,H], [A620;Pr,H,OCH₂(4-F-Ph),H,H,H], [A621;Pr,OMe,OMe,H,H,H], [A622;Pr,H,OH,H,H,H], [A623;Pr,H,Me,H,Me,H], [A624;Pr,OMe,Cl,H,F,H], [A625;Pr,SMe,H,H,F,H], [A626;Pr,NHS(O)₂Et,CN,H,F,H], [A627;Ph,F,H,H,H,H], [A628;Ph,H,F,H,H,H], [A629;Ph,Cl,H,H,H,H], [A630;Ph,H,Cl,H,H,H], [A631;Ph,Br,H,H,H,H], [A632;Ph,H,Br,H,H,H], [A633;Ph,I,H,H,H,H], [A634;Ph,H,I,H,H,H], [A635;Ph,Me,H,H,H,H], [A636;Ph,H,Me,H,H,H], [A637;Ph,CF₃,H,H,H,H], [A638;Ph,H,CF₃,H,H,H], [A639;Ph,CHF₂,H,H,H,H], [A640;Ph,H,CHF₂,H,H,H], [A641;Ph,c-Pr,H,H,H,H], [A642;Ph,H,c-Pr,H,H,H], [A643;Ph,F,F,H,H,H], [A644;Ph,F,Cl,H,H,H], [A645;Ph,F,Br,H,H,H], [A646;Ph,F,I,H,H,H], [A647;Ph,F,Me,H,H,H], [A648;Ph,Cl,F,H,H,H], [A649;Ph,Cl,Cl,H,H,H], [A650;Ph,Cl,Br,H,H,H], [A651;Ph,Cl,I,H,H,H], [A652;Ph,Cl,Me,H,H,H], [A653;Ph,Me,F,H,H,H], [A654;Ph,Me,Cl,H,H,H], [A655;Ph,Me,Br,H,H,H], [A656;Ph,Me,I,H,H,H], [A657;Ph,Me,Me,H,H,H], [A658;Ph,F,H,F,H,H], [A659;Ph,F,F,F,H,H], [A660;Ph,F,Cl,F,H,H], [A661;Ph,F,Br,F,H,H], [A662;Ph,F,I,F,H,H], [A663;Ph,F,Me,F,H,H], [A664;Ph,Cl,H,Cl,H,H], [A665;Ph,Cl,F,Cl,H,H], [A666;Ph,Cl,Cl,Cl,H,H], [A667;Ph,Cl,Me,Cl,H,H], [A668;Ph,Me,H,Me,H,H], [A669;Ph,Me,F,Me,H,H], [A670;Ph,Me,Cl,Me,H,H], [A671;2-F-Ph,F,H,H,H,H], [A672;2-F-Ph,H,F,H,H,H], [A673;2-F-Ph,Cl,H,H,H,H], [A674;2-F-Ph,H,Cl,H,H,H], [A675;2-F-Ph,Br,H,H,H,H], [A676;2-F-Ph,H,Br,H,H,H], [A677;2-F-Ph,I,H,H,H,H], [A678;2-F-Ph,H,I,H,H,H], [A679;2-F-Ph,Me,H,H,H,H], [A680;2-F-Ph,H,Me,H,H,H], [A681;2-F-Ph,F,F,H,H,H], [A682;2-F-Ph,F,C1,H,H,H], [A683;2-F-Ph,F,Br,H,H,H], [A684;2-F-Ph,F,I,H,H,H], [A685;2-F-Ph,F,Me,H,H,H], [A686;2-F-Ph,Cl,F,H,H,H], [A687;2-F-Ph,Cl,Cl,H,H,H], [A688;2-F-Ph,Cl,Me,H,H,H], [A689;2-F-Ph,Me,F,H,H,H], [A690;2-F-Ph,Me,Cl,H,H,H], [A691;2-F-Ph,Me,Me,H,H,H], [A692;2-F-Ph,F,H,F,H,H], [A693;2-F-Ph,F,F,F,H,H], [A694;2-F-Ph,F,Cl,F,H,H], [A695;2-F-Ph,F,Br,F,H,H], [A696;2-F-Ph,F,I,F,H,H], [A697;2-F-Ph,F,Me,F,H,H], [A698;3-F-Ph,F,H,H,H,H], [A699;3-F-Ph,H,F,H,H,H], [A700;3-F-Ph,Cl,H,H,H,H], [A701;3-F-Ph,H,Cl,H,H,H], [A702;3-F-Ph,Br,H,H,H,H], [A703;3-F-Ph,H,Br,H,H,H], [A704;3-F-Ph,I,H,H,H,H], [A705;3-F-Ph,H,I,H,H,H], [A706;3-F-Ph,Me,H,H,H,H], [A707;3-F-Ph,H,Me,H,H,H], [A708;3-F-Ph,F,F,H,H,H], [A709;3-F-Ph,F,Cl,H,H,H], [A710;3-F-Ph,F,Br,H,H,H], [A711;3-F-Ph,F,I,H,H,H], [A712;3-F-Ph,F,Me,H,H,H], [A713;3-F-Ph,Cl,F,H,H,H], [A714;3-F-Ph,Cl,Cl,H,H,H], [A715;3-F-Ph,Cl,Me,H,H,H], [A716;3-F-Ph,Me,F,H,H,H], [A717;3-F-Ph,Me,Cl,H,H,H], [A718;3-F-Ph,Me,Me,H,H,H], [A719;3-F-Ph,F,H,F,H,H], [A720;3-F-Ph,F,F,F,H,H], [A721;3-F-Ph,F,Cl,F,H,H], [A722;3-F-Ph,F,Br,F,H,H], [A723;3-F-Ph,F,I,F,H,H], [A724;3-F-Ph,F,Me,F,H,H], [A725;4-F-Ph,F,H,H,H,H], [A726;4-F-Ph,H,F,H,H,H], [A727;4-F-Ph,Cl,H,H,H,H], [A728;4-F-Ph,H,Cl,H,H,H], [A729;4-F-Ph,Br,H,H,H,H], [A730;4-F-Ph,H,Br,H,H,H], [A731;4-F-Ph,I,H,H,H,H], [A732;4-F-Ph,H,I,H,H,H], [A733;4-F-Ph,Me,H,H,H,H], [A734;4-F-Ph,H,Me,H,H,H], [A735;4-F-Ph,F,F,H,H,H], [A736;4-F-Ph,F,Cl,H,H,H], [A737;4-F-Ph,F,Br,H,H,H], [A738;4-F-Ph,F,I,H,H,H], [A739;4-F-Ph,F,Me,H,H,H], [A740;4-F-Ph,Cl,F,H,H,H], [A741;4-F-Ph,Cl,Cl,H,H,H], [A742;4-F-Ph,Cl,Me,H,H,H], [A743;4-F-Ph,Me,F,H,H,H], [A744;4-F-Ph,Me,Cl,H,H,H], [A745;4-F-Ph,Me,Me,H,H,H], [A746;4-F-Ph,F,H,F,H,H], [A747;4-F-Ph,F,F,F,H,H], [A748;4-F-Ph,F,Cl,F,H,H], [A749;4-F-Ph,F,Br,F,H,H], [A750;4-F-Ph,F,I,F,H,H], [A751;4-F-Ph,F,Me,F,H,H], [A752;2-Cl-Ph,F,H,H,H,H], [A753;2-Cl-Ph,H,F,H,H,H], [A754;2-Cl-Ph,Cl,H,H,H,H], [A755;2-Cl-Ph,H,Cl,H,H,H], [A756;2-Cl-Ph,Br,H,H,H,H], [A757;2-Cl-Ph,H,Br,H,H,H], [A758;2-Cl-Ph,I,H,H,H,H], [A759;2-Cl-Ph,H,I,H,H,H], [A760;2-Cl-Ph,Me,H,H,H,H], [A761;2-Cl-Ph,H,Me,H,H,H], [A762;2-Cl-Ph,F,F,H,H,H], [A763;2-Cl-Ph,F,Cl,H,H,H], [A764;2-Cl-Ph,F,Br,H,H,H], [A765;2-Cl-Ph,F,I,H,H,H], [A766;2-Cl-Ph,F,Me,H,H,H], [A767;2-Cl-Ph,Cl,F,H,H,H], [A768;2-Cl-Ph,Cl,Cl,H,H,H], [A769;2-Cl-Ph,Cl,Me,H,H,H], [A770;2-Cl-Ph,Me,F,H,H,H], [A771;2-Cl-Ph,Me,Cl,H,H,H], [A772;2-Cl-Ph,Me,Me,H,H,H], [A773;2-Cl-Ph,F,H,F,H,H], [A774;2-Cl-Ph,F,F,F,H,H], [A775;2-Cl-Ph,F,Cl,F,H,H], [A776;2-Cl-Ph,F,Br,F,H,H], [A777;2-Cl-Ph,F,I,F,H,H], [A778;2-Cl-Ph,F,Me,F,H,H], [A779;3-Cl-Ph,F,H,H,H,H], [A780;3-Cl-Ph,H,F,H,H,H], [A781;3-Cl-Ph,Cl,H,H,H,H], [A782;3-Cl-Ph,H,Cl,H,H,H], [A783;3-Cl-Ph,Br,H,H,H,H], [A784;3-Cl-Ph,H,Br,H,H,H], [A785;3-Cl-Ph,I,H,H,H,H], [A786;3-Cl-Ph,H,I,H,H,H], [A787;3-Cl-Ph,Me,H,H,H,H], [A788;3-Cl-Ph,H,Me,H,H,H], [A789;3-Cl-Ph,F,F,H,H,H], [A790;3-Cl-Ph,F,Cl,H,H,H], [A791;3-Cl-Ph,F,Br,H,H,H], [A792;3-Cl-Ph,F,I,H,H,H], [A793;3-Cl-Ph,F,Me,H,H,H], [A794;3-Cl-Ph,Cl,F,H,H,H], [A795;3-Cl-Ph,Cl,Cl,H,H,H], [A796;3-Cl-Ph,Cl,Me,H,H,H], [A797;3-Cl-Ph,Me,F,H,H,H], [A798;3-Cl-Ph,Me,Cl,H,H,H], [A799;3-Cl-Ph,Me,Me,H,H,H], [A800;3-Cl-Ph,F,H,F,H,H], [A801;3-Cl-Ph,F,F,F,H,H], [A802;3-Cl-Ph,F,Cl,F,H,H], [A803;3-Cl-Ph,F,Br,F,H,H], [A804;3-Cl-Ph,F,I,F,H,H], [A805;3-Cl-Ph,F,Me,F,H,H], [A806;4-Cl-Ph,F,H,H,H,H], [A807;4-Cl-Ph,H,F,H,H,H], [A808;4-Cl-Ph,Cl,H,H,H,H], [A809;4-Cl-Ph,H,Cl,H,H,H], [A810;4-Cl-Ph,Br,H,H,H,H], [A811;4-Cl-Ph,H,Br,H,H,H], [A812;4-Cl-Ph,I,H,H,H,H], [A813;4-Cl-Ph,H,I,H,H,H], [A814;4-Cl-Ph,Me,H,H,H,H], [A815;4-Cl-Ph,H,Me,H,H,H], [A816;4-Cl-Ph,F,F,H,H,H], [A817;4-Cl-Ph,F,Cl,H,H,H], [A818;4-Cl-Ph,F,Br,H,H,H], [A819;4-Cl-Ph,F,I,H,H,H], [A820;4-Cl-Ph,F,Me,H,H,H], [A821;4-Cl-Ph,Cl,F,H,H,H], [A822;4-Cl-Ph,Cl,Cl,H,H,H], [A823;4-Cl-Ph,Cl,Me,H,H,H], [A824;4-Cl-Ph,Me,F,H,H,H], [A825;4-Cl-Ph,Me,Cl,H,H,H], [A826;4-Cl-Ph,Me,Me,H,H,H], [A827;4-Cl-Ph,F,H,F,H,H], [A828;4-Cl-Ph,F,F,F,H,H], [A829;4-Cl-Ph,F,Cl,F,H,H], [A830;4-Cl-Ph,F,Br,F,H,H], [A831;4-Cl-Ph,F,I,F,H,H], [A832;4-Cl-Ph,F,Me,F,H,H], [A833;2-Me-Ph,F,H,H,H,H], [A834;2-Me-Ph,H,F,H,H,H], [A835;2-Me-Ph,Cl,H,H,H,H], [A836;2-Me-Ph,H,Cl,H,H,H], [A837;2-Me-Ph,Br,H,H,H,H], [A838;2-Me-Ph,H,Br,H,H,H], [A839;2-Me-Ph,I,H,H,H,H], [A840;2-Me-Ph,H,I,H,H,H], [A841;2-Me-Ph,Me,H,H,H,H], [A842;2-Me-Ph,H,Me,H,H,H], [A843;2-Me-Ph,F,F,H,H,H], [A844;2-Me-Ph,F,Cl,H,H,H], [A845;2-Me-Ph,F,Br,H,H,H], [A846;2-Me-Ph,F,I,H,H,H], [A847;2-Me-Ph,F,Me,H,H,H], [A848;2-Me-Ph,Cl,F,H,H,H], [A849;2-Me-Ph,Cl,Cl,H,H,H], [A850;2-Me-Ph,Cl,Me,H,H,H], [A851;2-Me-Ph,Me,F,H,H,H], [A852;2-Me-Ph,Me,Cl,H,H,H], [A853;2-Me-Ph,Me,Me,H,H,H], [A854;2-Me-Ph,F,H,F,H,H], [A855;2-Me-Ph,F,F,F,H,H], [A856;2-Me-Ph,F,Cl,F,H,H], [A857;2-Me-Ph,F,Br,F,H,H], [A858;2-Me-Ph,F,I,F,H,H], [A859;2-Me-Ph,F,Me,F,H,H], [A860;3-Me-Ph,F,H,H,H,H], [A861;3-Me-Ph,H,F,H,H,H], [A862;3-Me-Ph,Cl,H,H,H,H], [A863;3-Me-Ph,H,Cl,H,H,H], [A864;3-Me-Ph,Br,H,H,H,H], [A865;3-Me-Ph,H,Br,H,H,H], [A866;3-Me-Ph,I,H,H,H,H], [A867;3-Me-Ph,H,I,H,H,H], [A868;3-Me-Ph,Me,H,H,H,H], [A869;3-Me-Ph,H,Me,H,H,H], [A870;3-Me-Ph,F,F,H,H,H], [A871;3-Me-Ph,F,Cl,H,H,H], [A872;3-Me-Ph,F,Br,H,H,H], [A873;3-Me-Ph,F,I,H,H,H], [A874;3-Me-Ph,F,Me,H,H,H], [A875;3-Me-Ph,Cl,F,H,H,H], [A876;3-Me-Ph,Cl,Cl,H,H,H], [A877;3-Me-Ph,Cl,Me,H,H,H], [A878;3-Me-Ph,Me,F,H,H,H], [A879;3-Me-Ph,Me,Cl,H,H,H], [A880;3-Me-Ph,Me,Me,H,H,H], [A881;3-Me-Ph,F,H,F,H,H], [A882;3-Me-Ph,F,F,F,H,H], [A883;3-Me-Ph,F,Cl,F,H,H], [A884;3-Me-Ph,F,Br,F,H,H], [A885;3-Me-Ph,F,I,F,H,H], [A886;3-Me-Ph,F,Me,F,H,H], [A887;4-Me-Ph,F,H,H,H,H], [A888;4-Me-Ph,H,F,H,H,H], [A889;4-Me-Ph,Cl,H,H,H,H], [A890;4-Me-Ph,H,Cl,H,H,H], [A891;4-Me-Ph,Br,H,H,H,H], [A892;4-Me-Ph,H,Br,H,H,H], [A893;4-Me-Ph,I,H,H,H,H], [A894;4-Me-Ph,H,I,H,H,H], [A895;4-Me-Ph,Me,H,H,H,H], [A896;4-Me-Ph,H,Me,H,H,H], [A897;4-Me-Ph,F,F,H,H,H], [A898;4-Me-Ph,F,Cl,H,H,H], [A899;4-Me-Ph,F,Br,H,H,H], [A900;4-Me-Ph,F,I,H,H,H], [A901;4-Me-Ph,F,Me,H,H,H], [A902;4-Me-Ph,Cl,F,H,H,H], [A903;4-Me-Ph,Cl,Cl,H,H,H], [A904;4-Me-Ph,Cl,Me,H,H,H], [A905;4-Me-Ph,Me,F,H,H,H], [A906;4-Me-Ph,Me,Cl,H,H,H], [A907;4-Me-Ph,Me,Me,H,H,H], [A908;4-Me-Ph,F,H,F,H,H], [A909;4-Me-Ph,F,F,F,H,H], [A910;4-Me-Ph,F,Cl,F,H,H], [A911;4-Me-Ph,F,Br,F,H,H], [A912;4-Me-Ph,F,I,F,H,H], [A913;4-Me-Ph,F,Me,F,H,H], [A914;NMe₂,F,H,H,H,H], [A915;NMe₂,H,F,H,H,H], [A916;NMe₂,Cl,H,H,H,H], [A917;NMe₂,H,Cl,H,H,H], [A918;NMe₂,Br,H,H,H,H], [A919;NMe₂,H,Br,H,H,H], [A920;NMe₂,I,H,H,H,H], [A921;NMe₂,H,I,H,H,H], [A922;NMe₂,Me,H,H,H,H] , [A923;NMe₂,H,Me,H,H,H], [A924;NMe₂,F,F,H,H,H], [A925;NMe₂,F,Cl,H,H,H], [A926;NMe₂,F,Br,H,H,H], [A927;NMe₂,F,I,H,H,H], [A928;NMe₂,F,Me,H,H,H], [A929;NMe₂,Cl,F,H,H,H], [A930;NMe₂,Cl,Cl,H,H,H], [A931;NMe₂,Cl,Me,H,H,H], [A932;NMe₂,Me,F,H,H,H], [A933;NMe₂,Me,Cl,H,H,H], [A934;NMe₂,Me,Me,H,H,H], [A935;NMe₂,F,H,F,H,H], [A936;NMe₂,F,F,F,H,H], [A937;NMe₂,F,Cl,F,H,H], [A938;NMe₂,F,Br,F,H,H], [A939;NMe₂,F,I,F,H,H], [A940;NMe₂,F,Me,F,H,H], [A941;NMe₂,Cl,F,Cl,H,H], [A942;NMe₂,Cl,Cl,Cl,H,H], [A943;NMe₂,Cl,Me,Cl,H,H], [A944;NMe₂,Me,H,Me,H,H], [A945;NHMe,F,H,H,H,H], [A946;NHMe,H,F,H,H,H], [A947;NHMe,Cl,H,H,H,H], [A948;NHMe,H,Cl,H,H,H], [A949;NHMe,Br,H,H,H,H], [A950;NHMe,H,Br,H,H,H], [A951;NHMe,I,H,H,H,H], [A952;NHMe,H,I,H,H,H], [A953;NHMe,Me,H,H,H,H], [A954;NHMe,H,Me,H,H,H], [A955;NHMe,F,F,H,H,H], [A956;NHMe,F,Cl,H,H,H], [A957;NHMe,F,Br,H,H,H], [A958;NHMe,F,I,H,H,H], [A959;NHMe,F,Me,H,H,H], [A960;NHMe,Cl,F,H,H,H], [A961;NHMe,Cl,Cl,H,H,H], [A962;NHMe,Cl,Me,H,H,H], [A963;NHMe,Me,F,H,H,H], [A964;NHMe,Me,Cl,H,H,H], [A965;NHMe,Me,Me,H,H,H], [A966;NHMe,F,H,F,H,H], [A967;NHMe,F,F,F,H,H], [A968;NHMe,F,Cl,F,H,H], [A969;NHMe,F,Br,F,H,H], [A970;NHMe,F,I,F,H,H], [A971;NHMe,F,Me,F,H,H], [A972;NHMe,Cl,F,Cl,H,H], [A973;NHMe,Cl,Cl,Cl,H,H], [A974;NHMe,Cl,Me,Cl,H,H], [A975;NHMe,Me,H,Me,H,H], [A976;pyrrolizin-1-yl,F,H,H,H,H], [A977;pyrrolizin-1-yl,H,F,H,H,H], [A978;pyrrolizin-1-yl,Cl,H,H,H,H], [A979;pyrrolizin-1-yl,H,Cl,H,H,H], [A980;pyrrolizin-1-yl,Br,H,H,H,H], [A981;pyrrolizin-1-yl,H,Br,H,H,H], [A982;pyrrolizin-1-yl,I,H,H,H,H], [A983;pyrrolizin-1-yl,H,I,H,H,H], [A984;pyrrolizin-1-yl,Me,H,H,H,H], [A985;pyrrolizin-1-yl,H,Me,H,H,H], [A986;pyrrolizin-1-yl,F,F,H,H,H], [A987;pyrrolizin-1-yl,F,Cl,H,H,H], [A988;pyrrolizin-1-yl,F,Br,H,H,H], [A989;pyrrolizin-1-yl,F,I,H,H,H], [A990;pyrrolizin-1-yl,F,Me,H,H,H], [A991;pyrrolizin-1-yl,Cl,F,H,H,H], [A992;pyrrolizin-1-yl,Cl,Cl,H,H,H], [A993;pyrrolizin-1-yl,Cl,Me,H,H,H], [A994;pyrrolizin-1-yl,Me,F,H,H,H], [A995;pyrrolizin-1-yl,Me,Cl,H,H,H], [A996;pyrrolizin-1-yl,Me,Me,H,H,H], [A997;pyrrolizin-1-yl,F,H,F,H,H], [A998;pyrrolizin-1-yl,F,F,F,H,H], [A999;pyrrolizin-1-yl,F,Cl,F,H,H], [A1000;pyrrolizin-1-yl,F,Br,F,H,H], [A1001;pyrrolizin-1-yl,F,I,F,H,H], [A1002;pyrrolizin-1-yl,F,Me,F,H,H], [A1003;pyrrolizin-1-yl,Cl,F,Cl,H,H], [A1004;pyrrolizin-1-yl,Cl,Cl,Cl,H,H], [A1005;pyrrolizin-1-yl,Cl,Me,Cl,H,H], [A1006;pyrrolizin-1-yl,Me,H,Me,H,H], [A1007;azetidin-1-yl,F,H,H,H,H], [A1008;azetidin-1-yl,H,F,H,H,H], [A1009;azetidin-1-yl,Cl,H,H,H,H], [A1010;azetidin-1-yl,H,Cl,H,H,H], [A1011;azetidin-1-yl,Br,H,H,H,H], [A1012;azetidin-1-yl,H,Br,H,H,H], [A1013;azetidin-1-yl,I,H,H,H,H], [A1014;azetidin-1-yl,H,I,H,H,H], [A1015;azetidin-1-yl,Me,H,H,H,H], [A1016;azetidin-1-yl,H,Me,H,H,H], [A1017;azetidin-1-yl,F,F,H,H,H], [A1018;azetidin-1-yl,F,Cl,H,H,H], [A1019;azetidin-1-yl,F,Br,H,H,H], [A1020;azetidin-1-yl,F,I,H,H,H], [A1021;azetidin-1-yl,F,Me,H,H,H], [A1022;azetidin-1-yl,Cl,F,H,H,H], [A1023;azetidin-1-yl,Cl,Cl,H,H,H], [A1024;azetidin-1-yl,Cl,Me,H,H,H], [A1025;azetidin-1-yl,Me,F,H,H,H], [A1026;azetidin-1-yl,Me,Cl,H,H,H], [A1027;azetidin-1-yl,Me,Me,H,H,H], [A1028;azetidin-1-yl,F,H,F,H,H], [A1029;azetidin-1-yl,F,F,F,H,H], [A1030;azetidin-1-yl,F,Cl,F,H,H], [A1031;azetidin-1-yl,F,Br,F,H,H], [A1032;azetidin-1-yl,F,I,F,H,H], [A1033;azetidin-1-yl,F,Me,F,H,H], [A1034;azetidin-1-yl,Cl,F,Cl,H,H], [A1035;azetidin-1-yl,Cl,Cl,Cl,H,H], [A1036;azetidin-1-yl,Cl,Me,Cl,H,H], [A1037;azetidin-1-yl,Me,H,Me,H,H], [A1038;piperidin-1-yl,F,H,H,H,H], [A1039;piperidin-1-yl,H,F,H,H,H], [A1040;piperidin-1-yl,Cl,H,H,H,H], [A1041;piperidin-1-yl,H,Cl,H,H,H], [A1042;piperidin-1-yl,Br,H,H,H,H], [A1043;piperidin-1-yl,H,Br,H,H,H], [A1044;piperidin-1-yl,I,H,H,H,H], [A1045;piperidin-1-yl,H,I,H,H,H], [A1046;piperidin-1-yl,Me,H,H,H,H], [A1047;piperidin-1-yl,H,Me,H,H,H], [A1048;piperidin-1-yl,F,F,H,H,H], [A1049;piperidin-1-yl,F,Cl,H,H,H], [A1050;piperidin-1-yl,F,Br,H,H,H], [A1051;piperidin-1-yl,F,I,H,H,H], [A1052;piperidin-1-yl,F,Me,H,H,H], [A1053;piperidin-1-yl,Cl,F,H,H,H], [A1054;piperidin-1-yl,Cl,Cl,H,H,H], [A1055;piperidin-1-yl,Cl,Me,H,H,H], [A1056;piperidin-1-yl,Me,F,H,H,H], [A1057;piperidin-1-yl,Me,Cl,H,H,H], [A1058;piperidin-1-yl,Me,Me,H,H,H], [A1059;piperidin-1-yl,F,H,F,H,H], [A1060;piperidin-1-yl,F,F,F,H,H], [A1061;piperidin-1-yl,F,Cl,F,H,H], [A1062;piperidin-1-yl,F,Br,F,H,H], [A1063;piperidin-1-yl,F,I,F,H,H], [A1064;piperidin-1-yl,F,Me,F,H,H], [A1065;piperidin-1-yl,Cl,F,Cl,H,H], [A1066;piperidin-1-yl,Cl,Cl,Cl,H,H], [A1067;piperidin-1-yl,Cl,Me,Cl,H,H], [A1068;piperidin-1-yl,Me,H,Me,H,H], [A1069;i-Pr,F,H,H,H,H], [A1070;i-Pr,H,F,H,H,H], [A1071;i-Pr,Cl,H,H,H,H], [A1072;i-Pr,Br,H,H,H,H], [A1073;i-Pr,I,H,H,H,H], [A1074;i-Pr,Me,H,H,H,H], [A1075;i-Pr,H,Me,H,H,H], [A1076;i-Pr,F,F,H,H,H], [A1077;i-Pr,F,Cl,H,H,H], [A1078;i-Pr,F,Br,H,H,H], [A1079;i-Pr,F,I,H,H,H], [A1080;i-Pr,Cl,F,H,H,H], [A1081;i-Pr,Cl,Cl,H,H,H], [A1082;i-Pr,Cl,Me,H,H,H], [A1083;i-Pr,Me,F,H,H,H], [A1084;i-Pr,Me,Cl,H,H,H], [A1085;i-Pr,Me,Me,H,H,H], [A1086;i-Pr,Cl,F,Cl,H,H], [A1087;i-Pr,Cl,Cl,Cl,H,H], [A1088;i-Pr,Cl,Me,Cl,H,H], [A1089;i-Pr,Me,H,Me,H,H], [A1090;c-Pr,F,H,H,H,H], [A1091;c-Pr,H,F,H,H,H], [A1092;c-Pr,Cl,H,H,H,H], [A1093;c-Pr,Br,H,H,H,H], [A1094;c-Pr,I,H,H,H,H], [A1095;c-Pr,Me,H,H,H,H], [A1096;c-Pr,H,Me,H,H,H], [A1097;c-Pr,F,F,H,H,H], [A1098;c-Pr,F,Cl,H,H,H], [A1099;c-Pr,F,Br,H,H,H], [A1100;c-Pr,F,I,H,H,H], [A1101;c-Pr,Cl,F,H,H,H], [A1102;c-Pr,Cl,Cl,H,H,H], [A1103;c-Pr,Cl,Me,H,H,H], [A1104;c-Pr,Me,F,H,H,H], [A1105;c-Pr,Me,Cl,H,H,H], [A1106;c-Pr,Me,Me,H,H,H], [A1107;c-Pr,Cl,F,Cl,H,H], [A1108;c-Pr,Cl,Cl,Cl,H,H], [A1109;c-Pr,Cl,Me,Cl,H,H], [A1110;c-Pr,Me,H,Me,H,H], [A1111;Bu,F,H,H,H,H], [A1112;Bu,H,F,H,H,H], [A1113;Bu,Cl,H,H,H,H], [A1114;Bu,Br,H,H,H,H], [A1115;Bu,I,H,H,H,H], [A1116;Bu,Me,H,H,H,H], [A1117;Bu,H,Me,H,H,H], [A1118;Bu,F,F,H,H,H], [A1119;Bu,F,Cl,H,H,H], [A1120;Bu,F,Br,H,H,H], [A1121;Bu,F,I,H,H,H], [A1122;Bu,Cl,F,H,H,H], [A1123;Bu,Cl,Cl,H,H,H], [A1124;Bu,Cl,Me,H,H,H], [A1125;Bu,Me,F,H,H,H], [A1126;Bu,Me,Cl,H,H,H], [A1127;Bu,Me,Me,H,H,H], [A1128;Bu,Cl,F,Cl,H,H], [A1129;Bu,Cl,Cl,Cl,H,H], [A1130;Bu,Cl,Me,Cl,H,H], [A1131;Bu,Me,H,Me,H,H], [A1132;i-Bu,F,H,H,H,H], [A1133;i-Bu,H,F,H,H,H], [A1134;i-Bu,Cl,H,H,H,H], [A1135;i-Bu,Br,H,H,H,H], [A1136;i-Bu,I,H,H,H,H], [A1137;i-Bu,Me,H,H,H,H], [A1138;i-Bu,H,Me,H,H,H], [A1139;i-Bu,F,F,H,H,H], [A1140;i-Bu,F,Cl,H,H,H], [A1141;i-Bu,F,Br,H,H,H], [A1142;i-Bu,F,I,H,H,H], [A1143;i-Bu,Cl,F,H,H,H], [A1144;i-Bu,Cl,Cl,H,H,H], [A1145;i-Bu,Cl,Me,H,H,H], [A1146;i-Bu,Me,F,H,H,H], [A1147;i-Bu,Me,Cl,H,H,H], [A1148;i-Bu,Me,Me,H,H,H], [A1149;i-Bu,Cl,F,Cl,H,H], [A1150;i-Bu,Cl,Cl,Cl,H,H], [A1151;i-Bu,Cl,Me,Cl,H,H], [A1152;i-Bu,Me,H,Me,H,H], [A1153;s-Bu,F,H,H,H,H], [A1154;s-Bu,H,F,H,H,H], [A1155;s-Bu,Cl,H,H,H,H], [A1156;s-Bu,Br,H,H,H,H], [A1157;s-Bu,I,H,H,H,H], [A1158;s-Bu,Me,H,H,H,H], [A1159;s-Bu,H,Me,H,H,H], [A1160;s-Bu,F,F,H,H,H], [A1161;s-Bu,F,Cl,H,H,H], [A1162;s-Bu,F,Br,H,H,H], [A1163;s-Bu,F,I,H,H,H], [A1164;s-Bu,C1,F,H,H,H], [A1165;s-Bu,Cl,Cl,H,H,H], [A1166;s-Bu,Cl,Me,H,H,H], [A1167;s-Bu,Me,F,H,H,H], [A1168;s-Bu,Me,Cl,H,H,H], [A1169;s-Bu,Me,Me,H,H,H], [A1170;s-Bu,Cl,F,Cl,H,H], [A1171;s-Bu,Cl,Cl,Cl,H,H], [A1172;s-Bu,Cl,Me,Cl,H,H], [A1173;s-Bu,Me,H,Me,H,H], [A1174;pyridin-2-yl,F,H,H,H,H], [A1175;pyridin-2-yl,H,F,H,H,H], [A1176;pyridin-2-yl,Cl,H,H,H,H], [A1177;pyridin-2-yl,H,Cl,H,H,H], [A1178;pyridin-2-yl,Br,H,H,H,H], [A1179;pyridin-2-yl,H,Br,H,H,H], [A1180;pyridin-2-yl,I,H,H,H,H], [A1181;pyridin-2-yl,H,I,H,H,H], [A1182;pyridin-2-yl,Me,H,H,H,H], [A1183;pyridin-2-yl,H,Me,H,H,H], [A1184;pyridin-2-yl,CF₃,H,H,H,H], [A1185;pyridin-2-yl,H,CF₃,H,H,H], [A1186;pyridin-2-yl,CHF₂,H,H,H,H], [A1187;pyridin-2-yl,H,CHF₂,H,H,H], [A1188;pyridin-2-yl,c-Pr,H,H,H,H], [A1189;pyridin-2-yl,H,c-Pr,H,H,H], [A1190;pyridin-2-yl,F,F,H,H,H], [A1191;pyridin-2-yl,F,Cl,H,H,H], [A1192;pyridin-2-yl,F,Br,H,H,H], [A1193;pyridin-2-yl,F,I,H,H,H], [A1194;pyridin-2-yl,F,Me,H,H,H], [A1195;pyridin-2-yl,Cl,F,H,H,H], [A1196;pyridin-2-yl,Cl,Cl,H,H,H], [A1197;pyridin-2-yl,Cl,Br,H,H,H], [A1198;pyridin-2-yl,Cl,I,H,H,H], [A1199;pyridin-2-yl,Cl,Me,H,H,H], [A1200;pyridin-2-yl,Me,F,H,H,H], [A1201;pyridin-2-yl,Me,Cl,H,H,H], [A1202;pyridin-2-yl,Me,Br,H,H,H], [A1203;pyridin-2-yl,Me,I,H,H,H], [A1204;pyridin-2-yl,Me,Me,H,H,H], [A1205;pyridin-2-yl,F,H,F,H,H], [A1206;pyridin-2-yl,F,F,F,H,H], [A1207;pyridin-2-yl,F,Cl,F,H,H], [A1208;pyridin-2-yl,F,Br,F,H,H], [A1209;pyridin-2-yl,F,I,F,H,H], [A1210;pyridin-2-yl,F,Me,F,H,H], [A1211;pyridin-2-yl,Cl,H,Cl,H,H], [A1212;pyridin-2-yl,Cl,F,Cl,H,H], [A1213;pyridin-2-yl,Cl,Cl,Cl,H,H], [A1214;pyridin-2-yl,Cl,Me,Cl,H,H], [A1215;pyridin-2-yl,Me,H,Me,H,H], [A1216;pyridin-2-yl,Me,F,Me,H,H], [A1217;pyridin-2-yl,Me,Cl,Me,H,H], [A1218;pyridin-3-yl,F,H,H,H,H], [A1219;pyridin-3-yl,H,F,H,H,H], [A1220;pyridin-3-yl,Cl,H,H,H,H], [A1221;pyridin-3-yl,H,Cl,H,H,H], [A1222;pyridin-3-yl,Br,H,H,H,H], [A1223;pyridin-3-yl,H,Br,H,H,H], [A1224;pyridin-3-yl,I,H,H,H,H], [A1225;pyridin-3-yl,H,I,H,H,H], [A1226;pyridin-3-yl,Me,H,H,H,H], [A1227;pyridin-3-yl,H,Me,H,H,H], [A1228;pyridin-3-yl,CF₃,H,H,H,H], [A1229;pyridin-3-yl,H,CF₃,H,H,H], [A1230;pyridin-3-yl,CHF₂,H,H,H,H], [A1231;pyridin-3-yl,H,CHF₂,H,H,H], [A1232;pyridin-3-yl,c-Pr,H,H,H,H], [A1233;pyridin-3-yl,H,c-Pr,H,H,H], [A1234;pyridin-3-yl,F,F,H,H,H], [A1235;pyridin-3-yl,F,Cl,H,H,H], [A1236;pyridin-3-yl,F,Br,H,H,H], [A1237;pyridin-3-yl,F,I,H,H,H], [A1238;pyridin-3-yl,F,Me,H,H,H], [A1239;pyridin-3-yl,Cl,F,H,H,H], [A1240;pyridin-3-yl,Cl,Cl,H,H,H], [A1241;pyridin-3-yl,Cl,Br,H,H,H], [A1242;pyridin-3-yl,Cl,I,H,H,H], [A1243;pyridin-3-yl,Cl,Me,H,H,H], [A1244;pyridin-3-yl,Me,F,H,H,H], [A1245;pyridin-3-yl,Me,Cl,H,H,H], [A1246;pyridin-3-yl,Me,Br,H,H,H], [A1247;pyridin-3-yl,Me,I,H,H,H], [A1248;pyridin-3-yl,Me,Me,H,H,H], [A1249;pyridin-3-yl,F,H,F,H,H], [A1250;pyridin-3-yl,F,F,F,H,H], [A1251;pyridin-3-yl,F,Cl,F,H,H], [A1252;pyridin-3-yl,F,Br,F,H,H], [A1253;pyridin-3-yl,F,I,F,H,H], [A1254;pyridin-3-yl,F,Me,F,H,H], [A1255;pyridin-3-yl,Cl,H,Cl,H,H], [A1256;pyridin-3-yl,Cl,F,Cl,H,H], [A1257;pyridin-3-yl,Cl,Cl,Cl,H,H], [A1258;pyridin-3-yl,Cl,Me,Cl,H,H], [A1259;pyridin-3-yl,Me,H,Me,H,H], [A1260;pyridin-3-yl,Me,F,Me,H,H], [A1261;pyridin-3-yl,Me,Cl,Me,H,H], [A1262;pyridin-4-yl,F,H,H,H,H], [A1263;pyridin-4-yl,H,F,H,H,H], [A1264;pyridin-4-yl,Cl,H,H,H,H], [A1265;pyridin-4-yl,H,Cl,H,H,H], [A1266;pyridin-4-yl,Br,H,H,H,H], [A1267;pyridin-4-yl,H,Br,H,H,H], [A1268;pyridin-4-yl,I,H,H,H,H], [A1269;pyridin-4-yl,H,I,H,H,H], [A1270;pyridin-4-yl,Me,H,H,H,H], [A1271;pyridin-4-yl,H,Me,H,H,H], [A1272;pyridin-4-yl,CF₃,H,H,H,H], [A1273;pyridin-4-yl,H,CF₃,H,H,H], [A1274;pyridin-4-yl,CHF₂,H,H,H,H], [A1275;pyridin-4-yl,H,CHF₂,H,H,H], [A1276;pyridin-4-yl,c-Pr,H,H,H,H], [A1277;pyridin-4-yl,H,c-Pr,H,H,H], [A1278;pyridin-4-yl,F,F,H,H,H], [A1279;pyridin-4-yl,F,Cl,H,H,H], [A1280;pyridin-4-yl,F,Br,H,H,H], [A1281;pyridin-4-yl,F,I,H,H,H], [A1282;pyridin-4-yl,F,Me,H,H,H], [A1283;pyridin-4-yl,Cl,F,H,H,H], [A1284;pyridin-4-yl,Cl,Cl,H,H,H], [A1285;pyridin-4-yl,Cl,Br,H,H,H], [A1286;pyridin-4-yl,Cl,I,H,H,H], [A1287;pyridin-4-yl,Cl,Me,H,H,H], [A1288;pyridin-4-yl,Me,F,H,H,H], [A1289;pyridin-4-yl,Me,Cl,H,H,H], [A1290;pyridin-4-yl,Me,Br,H,H,H], [A1291;pyridin-4-yl,Me,I,H,H,H], [A1292;pyridin-4-yl,Me,Me,H,H,H], [A1293;pyridin-4-yl,F,H,F,H,H], [A1294;pyridin-4-yl,F,F,F,H,H], [A1295;pyridin-4-yl,F,Cl,F,H,H], [A1296;pyridin-4-yl,F,Br,F,H,H], [A1297;pyridin-4-yl,F,I,F,H,H], [A1298;pyridin-4-yl,F,Me,F,H,H], [A1299;pyridin-4-yl,Cl,H,Cl,H,H], [A1300;pyridin-4-yl,Cl,F,Cl,H,H], [A1301;pyridin-4-yl,Cl,Cl,Cl,H,H], [A1302;pyridin-4-yl,Cl,Me,Cl,H,H], [A1303;pyridin-4-yl,Me,H,Me,H,H], [A1304;pyridin-4-yl,Me,F,Me,H,H], [A1305;pyridin-4-yl,Me,Cl,Me,H,H], [A1306;pyrimidin-2-yl,F,H,H,H,H], [A1307;pyrimidin-2-yl,H,F,H,H,H], [A1308;pyrimidin-2-yl,Cl,H,H,H,H], [A1309;pyrimidin-2-yl,H,Cl,H,H,H], [A1310;pyrimidin-2-yl,Br,H,H,H,H], [A1311;pyrimidin-2-yl,H,Br,H,H,H], [A1312;pyrimidin-2-yl,I,H,H,H,H], [A1313;pyrimidin-2-yl,H,I,H,H,H], [A1314;pyrimidin-2-yl,Me,H,H,H,H], [A1315;pyrimidin-2-yl,H,Me,H,H,H], [A1316;pyrimidin-2-yl,F,F,H,H,H], [A1317;pyrimidin-2-yl,F,Cl,H,H,H], [A1318;pyrimidin-2-yl,F,Br,H,H,H], [A1319;pyrimidin-2-yl,F,I,H,H,H], [A1320;pyrimidin-2-yl,F,Me,H,H,H], [A1321;pyrimidin-2-yl,Cl,F,H,H,H], [A1322;pyrimidin-2-yl,Cl,Cl,H,H,H], [A1323;pyrimidin-2-yl,Cl,Me,H,H,H], [A1324;pyrimidin-2-yl,Me,F,H,H,H], [A1325;pyrimidin-2-yl,Me,Cl,H,H,H], [A1326;pyrimidin-2-yl,Me,Me,H,H,H], [A1327;pyrimidin-2-yl,F,H,F,H,H], [A1328;pyrimidin-2-yl,F,F,F,H,H], [A1329;pyrimidin-2-yl,F,Cl,F,H,H], [A1330;pyrimidin-2-yl,F,Br,F,H,H], [A1331;pyrimidin-2-yl,F,I,F,H,H], [A1332;pyrimidin-2-yl,F,Me,F,H,H], [A1333;pyrimidin-4-yl,F,H,H,H,H], [A1334;pyrimidin-4-yl,H,F,H,H,H], [A1335;pyrimidin-4-yl,Cl,H,H,H,H], [A1336;pyrimidin-4-yl,H,Cl,H,H,H], [A1337;pyrimidin-4-yl,Br,H,H,H,H], [A1338;pyrimidin-4-yl,H,Br,H,H,H], [A1339;pyrimidin-4-yl,I,H,H,H,H], [A1340;pyrimidin-4-yl,H,I,H,H,H], [A1341;pyrimidin-4-yl,Me,H,H,H,H], [A1342;pyrimidin-4-yl,H,Me,H,H,H], [A1343;pyrimidin-4-yl,F,F,H,H,H], [A1344;pyrimidin-4-yl,F,Cl,H,H,H], [A1345;pyrimidin-4-yl,F,Br,H,H,H], [A1346;pyrimidin-4-yl,F,I,H,H,H], [A1347;pyrimidin-4-yl,F,Me,H,H,H], [A1348;pyrimidin-4-yl,Cl,F,H,H,H], [A1349;pyrimidin-4-yl,Cl,Cl,H,H,H], [A1350;pyrimidin-4-yl,Cl,Me,H,H,H], [A1351;pyrimidin-4-yl,Me,F,H,H,H], [A1352;pyrimidin-4-yl,Me,Cl,H,H,H], [A1353;pyrimidin-4-yl,Me,Me,H,H,H], [A1354;pyrimidin-4-yl,F,H,F,H,H], [A1355;pyrimidin-4-yl,F,F,F,H,H], [A1356;pyrimidin-4-yl,F,Cl,F,H,H], [A1357;pyrimidin-4-yl,F,Br,F,H,H], [A1358;pyrimidin-4-yl,F,I,F,H,H], [A1359;pyrimidin-4-yl,F,Me,F,H,H], [A1360;pyrimidin-5-yl,F,H,H,H,H], [A1361;pyrimidin-5-yl,H,F,H,H,H], [A1362;pyrimidin-5-yl,Cl,H,H,H,H], [A1363;pyrimidin-5-yl,H,Cl,H,H,H], [A1364;pyrimidin-5-yl,Br,H,H,H,H], [A1365;pyrimidin-5-yl,H,Br,H,H,H], [A1366;pyrimidin-5-yl,I,H,H,H,H], [A1367;pyrimidin-5-yl,H,I,H,H,H], [A1368;pyrimidin-5-yl,Me,H,H,H,H], [A1369;pyrimidin-5-yl,H,Me,H,H,H], [A1370;pyrimidin-5-yl,F,F,H,H,H], [A1371;pyrimidin-5-yl,F,Cl,H,H,H], [A1372;pyrimidin-5-yl,F,Br,H,H,H], [A1373;pyrimidin-5-yl,F,I,H,H,H], [A1374;pyrimidin-5-yl,F,Me,H,H,H], [A1375;pyrimidin-5-yl,Cl,F,H,H,H], [A1376;pyrimidin-5-yl,Cl,Cl,H,H,H], [A1377;pyrimidin-5-yl,Cl,Me,H,H,H], [A1378;pyrimidin-5-yl,Me,F,H,H,H], [A1379;pyrimidin-5-yl,Me,Cl,H,H,H], [A1380;pyrimidin-5-yl,Me,Me,H,H,H], [A1381;pyrimidin-5-yl,F,H,F,H,H], [A1382;pyrimidin-5-yl,F,F,F,H,H], [A1383;pyrimidin-5-yl,F,Cl,F,H,H], [A1384;pyrimidin-5-yl,F,Br,F,H,H], [A1385;pyrimidin-5-yl,F,I,F,H,H], [A1386;pyrimidin-5-yl,F,Me,F,H,H], [A1387;pyridazin-3-yl,F,H,H,H,H], [A1388;pyridazin-3-yl,H,F,H,H,H], [A1389;pyridazin-3-yl,Cl,H,H,H,H], [A1390;pyridazin-3-yl,H,Cl,H,H,H], [A1391;pyridazin-3-yl,Br,H,H,H,H], [A1392;pyridazin-3-yl,H,Br,H,H,H], [A1393;pyridazin-3-yl,I,H,H,H,H], [A1394;pyridazin-3-yl,H,I,H,H,H], [A1395;pyridazin-3-yl,Me,H,H,H,H], [A1396;pyridazin-3-yl,H,Me,H,H,H], [A1397;pyridazin-3-yl,F,F,H,H,H], [A1398;pyridazin-3-yl,F,Cl,H,H,H], [A1399;pyridazin-3-yl,F,Br,H,H,H], [A1400;pyridazin-3-yl,F,I,H,H,H], [A1401;pyridazin-3-yl,F,Me,H,H,H], [A1402;pyridazin-3-yl,Cl,F,H,H,H], [A1403;pyridazin-3-yl,Cl,Cl,H,H,H], [A1404;pyridazin-3-yl,Cl,Me,H,H,H], [A1405;pyridazin-3-yl,Me,F,H,H,H], [A1406;pyridazin-3-yl,Me,Cl,H,H,H], [A1407;pyridazin-3-yl,Me,Me,H,H,H], [A1408;pyridazin-3-yl,F,H,F,H,H], [A1409;pyridazin-3-yl,F,F,F,H,H], [A1410;pyridazin-3-yl,F,Cl,F,H,H], [A1411;pyridazin-3-yl,F,Br,F,H,H], [A1412;pyridazin-3-yl,F,I,F,H,H], [A1413;pyridazin-3-yl,F,Me,F,H,H], [A1414;pyridazin-4-yl,F,H,H,H,H], [A1415;pyridazin-4-yl,H,F,H,H,H], [A1416;pyridazin-4-yl,Cl,H,H,H,H], [A1417;pyridazin-4-yl,H,Cl,H,H,H], [A1418;pyridazin-4-yl,Br,H,H,H,H], [A1419;pyridazin-4-yl,H,Br,H,H,H], [A1420;pyridazin-4-yl,I,H,H,H,H], [A1421;pyridazin-4-yl,H,I,H,H,H], [A1422;pyridazin-4-yl,Me,H,H,H,H], [A1423;pyridazin-4-yl,H,Me,H,H,H], [A1424;pyridazin-4-yl,F,F,H,H,H], [A1425;pyridazin-4-yl,F,Cl,H,H,H], [A1426;pyridazin-4-yl,F,Br,H,H,H], [A1427;pyridazin-4-yl,F,I,H,H,H], [A1428;pyridazin-4-yl,F,Me,H,H,H], [A1429;pyridazin-4-yl,Cl,F,H,H,H], [A1430;pyridazin-4-yl,Cl,Cl,H,H,H], [A1431;pyridazin-4-yl,Cl,Me,H,H,H], [A1432;pyridazin-4-yl,Me,F,H,H,H], [A1433;pyridazin-4-yl,Me,Cl,H,H,H], [A1434;pyridazin-4-yl,Me,Me,H,H,H], [A1435;pyridazin-4-yl,F,H,F,H,H], [A1436;pyridazin-4-yl,F,F,F,H,H], [A1437;pyridazin-4-yl,F,Cl,F,H,H], [A1438;pyridazin-4-yl,F,Br,F,H,H], [A1439;pyridazin-4-yl,F,I,F,H,H], [A1440;pyridazin-4-yl,F,Me,F,H,H], [A1441;pyrazin-2-yl,F,H,H,H,H], [A1442;pyrazin-2-yl,H,F,H,H,H], [A1443;pyrazin-2-yl,Cl,H,H,H,H], [A1444;pyrazin-2-yl,H,Cl,H,H,H], [A1445;pyrazin-2-yl,Br,H,H,H,H], [A1446;pyrazin-2-yl,H,Br,H,H,H], [A1447;pyrazin-2-yl,I,H,H,H,H], [A1448;pyrazin-2-yl,H,I,H,H,H], [A1449;pyrazin-2-yl,Me,H,H,H,H], [A1450;pyrazin-2-yl,H,Me,H,H,H], [A1451;pyrazin-2-yl,F,F,H,H,H], [A1452;pyrazin-2-yl,F,Cl,H,H,H], [A1453;pyrazin-2-yl,F,Br,H,H,H], [A1454;pyrazin-2-yl,F,I,H,H,H], [A1455;pyrazin-2-yl,F,Me,H,H,H], [A1456;pyrazin-2-yl,Cl,F,H,H,H], [A1457;pyrazin-2-yl,Cl,Cl,H,H,H], [A1458;pyrazin-2-yl,Cl,Me,H,H,H], [A1459;pyrazin-2-yl,Me,F,H,H,H], [A1460;pyrazin-2-yl,Me,Cl,H,H,H], [A1461;pyrazin-2-yl,Me,Me,H,H,H], [A1462;pyrazin-2-yl,F,H,F,H,H], [A1463;pyrazin-2-yl,F,F,F,H,H], [A1464;pyrazin-2-yl,F,Cl,F,H,H], [A1465;pyrazin-2-yl,F,Br,F,H,H], [A1466;pyrazin-2-yl,F,I,F,H,H], [A1467;pyrazin-2-yl,F,Me,F,H,H], [A1468;furan-2-yl,F,H,H,H,H], [A1469;furan-2-yl,H,F,H,H,H], [A1470;furan-2-yl,Cl,H,H,H,H], [A1471;furan-2-yl,H,Cl,H,H,H], [A1472;furan-2-yl,Br,H,H,H,H], [A1473;furan-2-yl,H,Br,H,H,H], [A1474;furan-2-yl,I,H,H,H,H], [A1475;furan-2-yl,H,I,H,H,H], [A1476;furan-2-yl,Me,H,H,H,H], [A1477;furan-2-yl,H,Me,H,H,H], [A1478;furan-2-yl,CF₃,H,H,H,H], [A1479;furan-2-yl,H,CF₃,H,H,H], [A1480;furan-2-yl,CHF₂,H,H,H,H], [A1481;furan-2-yl,H,CHF₂,H,H,H], [A1482;furan-2-yl,c-Pr,H,H,H,H], [A1483;furan-2-yl,H,c-Pr,H,H,H], [A1484;furan-2-yl,F,F,H,H,H], [A1485;furan-2-yl,F,Cl,H,H,H], [A1486;furan-2-yl,F,Br,H,H,H], [A1487;furan-2-yl,F,I,H,H,H], [A1488;furan-2-yl,F,Me,H,H,H], [A1489;furan-2-yl,Cl,F,H,H,H], [A1490;furan-2-yl,Cl,Cl,H,H,H], [A1491;furan-2-yl,Cl,Br,H,H,H], [A1492;furan-2-yl,Cl,I,H,H,H], [A1493;furan-2-yl,Cl,Me,H,H,H], [A1494;furan-2-yl,Me,F,H,H,H], [A1495;furan-2-yl,Me,Cl,H,H,H], [A1496;furan-2-yl,Me,Br,H,H,H], [A1497;furan-2-yl,Me,I,H,H,H], [A1498;furan-2-yl,Me,Me,H,H,H], [A1499;furan-2-yl,F,H,F,H,H], [A1500;furan-2-yl,F,F,F,H,H], [A1501;furan-2-yl,F,Cl,F,H,H], [A1502;furan-2-yl,F,Br,F,H,H], [A1503;furan-2-yl,F,I,F,H,H], [A1504;furan-2-yl,F,Me,F,H,H], [A1505;furan-2-yl,Cl,H,Cl,H,H], [A1506;furan-2-yl,Cl,F,Cl,H,H], [A1507;furan-2-yl,Cl,Cl,Cl,H,H], [A1508;furan-2-yl,Cl,Me,Cl,H,H], [A1509;furan-2-yl,Me,H,Me,H,H], [A1510;furan-2-yl,Me,F,Me,H,H], [A1511;furan-2-yl,Me,Cl,Me,H,H], [A1512;furan-3-yl,F,H,H,H,H], [A1513;furan-3-yl,H,F,H,H,H], [A1514;furan-3-yl,Cl,H,H,H,H], [A1515;furan-3-yl,H,Cl,H,H,H], [A1516;furan-3-yl,Br,H,H,H,H], [A1517;furan-3-yl,H,Br,H,H,H], [A1518;furan-3-yl,I,H,H,H,H], [A1519;furan-3-yl,H,I,H,H,H], [A1520;furan-3-yl,Me,H,H,H,H], [A1521;furan-3-yl,H,Me,H,H,H], [A1522;furan-3-yl,CF₃,H,H,H,H], [A1523;furan-3-yl,H,CF₃,H,H,H], [A1524;furan-3-yl,CHF₂,H,H,H,H], [A1525;furan-3-yl,H,CHF₂,H,H,H], [A1526;furan-3-yl,c-Pr,H,H,H,H], [A1527;furan-3-yl,H,c-Pr,H,H,H], [A1528;furan-3-yl,F,F,H,H,H], [A1529;furan-3-yl,F,Cl,H,H,H], [A1530;furan-3-yl,F,Br,H,H,H], [A1531;furan-3-yl,F,I,H,H,H], [A1532;furan-3-yl,F,Me,H,H,H], [A1533;furan-3-yl,Cl,F,H,H,H], [A1534;furan-3-yl,Cl,Cl,H,H,H], [A1535;furan-3-yl,Cl,Br,H,H,H], [A1536;furan-3-yl,Cl,I,H,H,H], [A1537;furan-3-yl,Cl,Me,H,H,H], [A1538;furan-3-yl,Me,F,H,H,H], [A1539;furan-3-yl,Me,Cl,H,H,H], [A1540;furan-3-yl,Me,Br,H,H,H], [A1541;furan-3-yl,Me,I,H,H,H], [A1542;furan-3-yl,Me,Me,H,H,H], [A1543;furan-3-yl,F,H,F,H,H], [A1544;furan-3-yl,F,F,F,H,H], [A1545;furan-3-yl,F,Cl,F,H,H], [A1546;furan-3-yl,F,Br,F,H,H], [A1547;furan-3-yl,F,I,F,H,H], [A1548;furan-3-yl,F,Me,F,H,H], [A1549;furan-3-yl,Cl,H,Cl,H,H], [A1550;furan-3-yl,Cl,F,Cl,H,H], [A1551;furan-3-yl,Cl,Cl,Cl,H,H], [A1552;furan-3-yl,Cl,Me,Cl,H,H], [A1553;furan-3-yl,Me,H,Me,H,H], [A1554;furan-3-yl,Me,F,Me,H,H], [A1555;furan-3-yl,Me,Cl,Me,H,H], [A1556;thien-2-yl,F,H,H,H,H], [A1557;thien-2-yl,H,F,H,H,H], [A1558;thien-2-yl,Cl,H,H,H,H], [A1559;thien-2-yl,H,Cl,H,H,H], [A1560;thien-2-yl,Br,H,H,H,H], [A1561;thien-2-yl,H,Br,H,H,H], [A1562;thien-2-yl,I,H,H,H,H], [A1563;thien-2-yl,H,I,H,H,H], [A1564;thien-2-yl,Me,H,H,H,H], [A1565;thien-2-yl,H,Me,H,H,H], [A1566;thien-2-yl,CF₃,H,H,H,H], [A1567;thien-2-yl,H,CF₃,H,H,H], [A1568;thien-2-yl,CHF₂,H,H,H,H], [A1569;thien-2-yl,H,CHF₂,H,H,H], [A1570;thien-2-yl,c-Pr,H,H,H,H], [A1571;thien-2-yl,H,c-Pr,H,H,H], [A1572;thien-2-yl,F,F,H,H,H], [A1573;thien-2-yl,F,Cl,H,H,H], [A1574;thien-2-yl,F,Br,H,H,H], [A1575;thien-2-yl,F,I,H,H,H], [A1576;thien-2-yl,F,Me,H,H,H], [A1577;thien-2-yl,Cl,F,H,H,H], [A1578;thien-2-yl,Cl,Cl,H,H,H], [A1579;thien-2-yl,Cl,Br,H,H,H], [A1580;thien-2-yl,Cl,I,H,H,H], [A1581;thien-2-yl,Cl,Me,H,H,H], [A1582;thien-2-yl,Me,F,H,H,H], [A1583;thien-2-yl,Me,Cl,H,H,H], [A1584;thien-2-yl,Me,Br,H,H,H], [A1585;thien-2-yl,Me,I,H,H,H], [A1586;thien-2-yl,Me,Me,H,H,H], [A1587;thien-2-yl,F,H,F,H,H], [A1588;thien-2-yl,F,F,F,H,H], [A1589;thien-2-yl,F,Cl,F,H,H], [A1590;thien-2-yl,F,Br,F,H,H], [A1591;thien-2-yl,F,I,F,H,H], [A1592;thien-2-yl,F,Me,F,H,H], [A1593;thien-2-yl,Cl,H,Cl,H,H], [A1594;thien-2-yl,Cl,F,Cl,H,H], [Al595;thien-2-yl,Cl,Cl,Cl,H,H], [A1596;thien-2-yl,Cl,Me,Cl,H,H], [A1597;thien-2-yl,Me,H,Me,H,H], [A1598;thien-2-yl,Me,F,Me,H,H], [A1599;thien-2-yl,Me,Cl,Me,H,H], [A1600;thien-3-yl,F,H,H,H,H], [A1601;thien-3-yl,H,F,H,H,H], [A1602;thien-3-yl,Cl,H,H,H,H], [A1603;thien-3-yl,H,Cl,H,H,H], [A1604;thien-3-yl,Br,H,H,H,H], [A1605;thien-3-yl,H,Br,H,H,H], [A1606;thien-3-yl,I,H,H,H,H], [A1607;thien-3-yl,H,I,H,H,H], [A1608;thien-3-yl,Me,H,H,H,H], [A1609;thien-3-yl,H,Me,H,H,H], [A1610;thien-3-yl,CF₃,H,H,H,H], [A1611;thien-3-yl,H,CF₃,H,H,H], [A1612;thien-3-yl,CHF₂,H,H,H,H], [A1613;thien-3-yl,H,CHF₂,H,H,H], [A1614;thien-3-yl,c-Pr,H,H,H,H], [A1615;thien-3-yl,H,c-Pr,H,H,H], [A1616;thien-3-yl,F,F,H,H,H], [A1617;thien-3-yl,F,Cl,H,H,H], [A1618;thien-3-yl,F,Br,H,H,H], [A1619;thien-3-yl,F,I,H,H,H], [A1620;thien-3-yl,F,Me,H,H,H], [A1621;thien-3-yl,Cl,F,H,H,H], [A1622;thien-3-yl,Cl,Cl,H,H,H], [A1623;thien-3-yl,Cl,Br,H,H,H], [A1624;thien-3-yl,Cl,I,H,H,H], [A1625;thien-3-yl,Cl,Me,H,H,H], [A1626;thien-3-yl,Me,F,H,H,H], [A1627;thien-3-yl,Me,Cl,H,H,H], [A1628;thien-3-yl,Me,Br,H,H,H], [A1629;thien-3-yl,Me,I,H,H,H], [A1630;thien-3-yl,Me,Me,H,H,H], [A1631;thien-3-yl,F,H,F,H,H], [A1632;thien-3-yl,F,F,F,H,H], [A1633;thien-3-yl,F,Cl,F,H,H], [A1634;thien-3-yl,F,Br,F,H,H], [A1635;thien-3-yl,F,I,F,H,H], [A1636;thien-3-yl,F,Me,F,H,H], [A1637;thien-3-yl,Cl,H,Cl,H,H], [A1638;thien-3-yl,Cl,F,Cl,H,H], [A1639;thien-3-yl,Cl,Cl,Cl,H,H], [A1640;thien-3-yl,Cl,Me,Cl,H,H], [A1641;thien-3-yl,Me,H,Me,H,H], [A1642;thien-3-yl,Me,F,Me,H,H], [A1643;thien-3-yl,Me,Cl,Me,H,H], [A1644;1,3-oxazol-2-yl,F,H,H,H,H], [A1645;1,3-oxazol-2-yl,H,F,H,H,H], [A1646;1,3-oxazol-2-yl,Cl,H,H,H,H], [A1647;1,3-oxazol-2-yl,H,Cl,H,H,H], [A1648;1,3-oxazol-2-yl,Br,H,H,H,H], [A1649;1,3-oxazol-2-yl,H,Br,H,H,H], [A1650;1,3-oxazol-2-yl,I,H,H,H,H], [A1651;1,3-oxazol-2-yl,H,I,H,H,H], [A1652;1,3-oxazol-2-yl,Me,H,H,H,H], [A1653;1,3-oxazol-2-yl,H,Me,H,H,H], [A1654;1,3-oxazol-2-yl,CF₃,H,H,H,H], [A1655;1,3-oxazol-2-yl,H,CF₃,H,H,H], [A1656;1,3-oxazol-2-yl,CHF₂,H,H,H,H], [A1657;1,3-oxazol-2-yl,H,CHF₂,H,H,H], [A1658;1,3-oxazol-2-yl,c-Pr,H,H,H,H], [A1659;1,3-oxazol-2-yl,H,c-Pr,H,H,H], [A1660;1,3-oxazol-2-yl,F,F,H,H,H], [A1661;1,3-oxazol-2-yl,F,Cl,H,H,H], [A1662;1,3-oxazol-2-yl,F,Br,H,H,H], [A1663;1,3-oxazol-2-yl,F,I,H,H,H], [A1664;1,3-oxazol-2-yl,F,Me,H,H,H], [A1665;1,3-oxazol-2-yl,Cl,F,H,H,H], [A1666;1,3-oxazol-2-yl,Cl,Cl,H,H,H], [A1667;1,3-oxazol-2-yl,Cl,Br,H,H,H], [A1668;1,3-oxazol-2-yl,Cl,I,H,H,H], [A1669;1,3-oxazol-2-yl,Cl,Me,H,H,H], [A1670;1,3-oXxazol-2-yl,Me,F,H,H,H], [A1671;1,3-oxazol-2-yl,Me,Cl,H,H,H], [A1672;1,3-oxazol-2-yl,Me,Br,H,H,H], [A1673;1,3-oxazol-2-yl,Me,I,H,H,H], [A1674;1,3-oxazol-2-yl,Me,Me,H,H,H], [A1675;1,3-oxazol-2-yl,F,H,F,H,H], [A1676;1,3-oxazol-2-yl,F,F,F,H,H], [A1677;1,3-oxazol-2-yl,F,Cl,F,H,H], [A1678;1,3-oxazol-2-yl,F,Br,F,H,H], [A1679;1,3-oxazol-2-yl,F,I,F,H,H], [A1680;1,3-oxazol-2-yl,F,Me,F,H,H], [A1681;1,3-oxazol-2-yl,Cl,H,Cl,H,H], [A1682;1,3-oxazol-2-yl,Cl,F,Cl,H,H], [A1683;1,3-oxazol-2-yl,Cl,Cl,Cl,H,H], [A1684;1,3-oxazol-2-yl,Cl,Me,Cl,H,H], [A1685;1,3-oxazol-2-yl,Me,H,Me,H,H], [A1686;1,3-oxazol-2-yl,Me,F,Me,H,H], [A1687;1,3-oxazol-2-yl,Me,Cl,Me,H,H], [A1688;1,3-thiazol-2-yl,F,H,H,H,H], [A1689;1,3-thiazol-2-yl,H,F,H,H,H], [A1690;1,3-thiazol-2-yl,Cl,H,H,H,H], [A1691;1,3-thiazol-2-yl,H,Cl,H,H,H], [A1692;1,3-thiazol-2-yl,Br,H,H,H,H], [A1693;1,3-thiazol-2-yl,H,Br,H,H,H], [A1694;1,3-thiazol-2-yl,I,H,H,H,H], [A1695;1,3-thiazol-2-yl,H,I,H,H,H], [A1696;1,3-thiazol-2-yl,Me,H,H,H,H], [A1697;1,3-thiazol-2-yl,H,Me,H,H,H], [A1698;1,3-thiazol-2-yl,CF₃,H,H,H,H], [A1699;1,3-thiazol-2-yl,H,CF₃,H,H,H], [A1700;1,3-thiazol-2-yl,CHF₂,H,H,H,H], [A1701;1,3-thiazol-2-yl,H,CHF₂,H,H,H], [A1702;1,3-thiazol-2-yl,c-Pr,H,H,H,H], [A1703;1,3-thiazol-2-yl,H,c-Pr,H,H,H], [A1704;1,3-thiazol-2-yl,F,F,H,H,H], [A1705;1,3-thiazol-2-yl,F,Cl,H,H,H], [A1706;1,3-thiazol-2-yl,F,Br,H,H,H], [A1707;1,3-thiazol-2-yl,F,I,H,H,H], [A1708;1,3-thiazol-2-yl,F,Me,H,H,H], [A1709;1,3-thiazol-2-yl,Cl,F,H,H,H], [A1710;1,3-thiazol-2-yl,Cl,Cl,H,H,H], [A1711;1,3-thiazol-2-yl,Cl,Br,H,H,H], [A1712;1,3-thiazol-2-yl,Cl,I,H,H,H], [A1713;1,3-thiazol-2-yl,Cl,Me,H,H,H], [A1714;1,3-thiazol-2-yl,Me,F,H,H,H], [A1715;1,3-thiazol-2-yl,Me,Cl,H,H,H], [A1716;1,3-thiazol-2-yl,Me,Br,H,H,H], [A1717;1,3-thiazol-2-yl,Me,I,H,H,H], [A1718;1,3-thiazol-2-yl,Me,Me,H,H,H], [A1719;1,3-thiazol-2-yl,F,H,F,H,H], [A1720;1,3-thiazol-2-yl,F,F,F,H,H], [A1721;1,3-thiazol-2-yl,F,Cl,F,H,H], [A1722;1,3-thiazol-2-yl,F,Br,F,H,H], [A1723;1,3-thiazol-2-yl,F,I,F,H,H], [A1724;1,3-thiazol-2-yl,F,Me,F,H,H], [A1725;1,3-thiazol-2-yl,Cl,H,Cl,H,H], [A1726;1,3-thiazol-2-yl,Cl,F,Cl,H,H], [A1727;1,3-thiazol-2-yl,Cl,Cl,Cl,H,H], [A1728;1,3-thiazol-2-yl,Cl,Me,Cl,H,H], [A1729;1,3-thiazol-2-yl,Me,H,Me,H,H], [A1730;1,3-thiazol-2-yl,Me,F,Me,H,H], [A1731;1,3-thiazol-2-yl,Me,Cl,Me,H,H], [A1732;1H-pyrazol-1-yl,F,H,H,H,H], [A1733;1H-pyrazol-1-yl,H,F,H,H,H], [A1734;1H-pyrazol-1-yl,Cl,H,H,H,H], [A1735;1H-pyrazol-1-yl,H,C1,H,H,H], [A1736;1H-pyrazol-1-yl,Br,H,H,H,H], [A1737;1H-pyrazol-1-yl,H,Br,H,H,H], [A1738;1H-pyrazol-1-yl,I,H,H,H,H], [A1739;1H-pyrazol-1-yl,H,I,H,H,H], [A1740;1H-pyrazol-1-yl,Me,H,H,H,H], [A1741;1H-pyrazol-1-yl,H,Me,H,H,H], [A1742;1H-pyrazol-1-yl,CF₃,H,H,H,H], [A1743;1H-pyrazol-1-yl,H,CF₃,H,H,H], [A1744;1H-pyrazol-1-yl,CHF₂,H,H,H,H], [A1745;1H-pyrazol-1-yl,H,CHF₂,H,H,H], [A1746;1H-pyrazol-1-yl,c-Pr,H,H,H,H], [A1747;1H-pyrazol-1-yl,H,c-Pr,H,H,H], [A1748;1H-pyrazol-1-yl,F,F,H,H,H], [A1749;1H-pyrazol-1-yl,F,Cl,H,H,H], [A1750;1H-pyrazol-1-yl,F,Br,H,H,H], [A1751;1H-pyrazol-1-yl,F,I,H,H,H], [A1752;1H-pyrazol-1-yl,F,Me,H,H,H], [A1753;1H-pyrazol-1-yl,C1,F,H,H,H], [A1754;1H-pyrazol-1-yl,C1,C1,H,H,H], [A1755;1H-pyrazol-1-yl,Cl,Br,H,H,H], [A1756;1H-pyrazol-1-yl,Cl,I,H,H,H], [A1757;1H-pyrazol-1-yl,Cl,Me,H,H,H], [A1758;1H-pyrazol-1-yl,Me,F,H,H,H], [A1759;1H-pyrazol-1-yl,Me,Cl,H,H,H], [A1760;1H-pyrazol-1-yl,Me,Br,H,H,H], [A1761;1H-pyrazol-1-yl,Me,I,H,H,H], [A1762;1H-pyrazol-1-yl,Me,Me,H,H,H], [A1763;1H-pyrazol-1-yl,F,H,F,H,H], [A1764;1H-pyrazol-1-yl,F,F,F,H,H], [A1765;1H-pyrazol-1-yl,F,Cl,F,H,H], [A1766;1H-pyrazol-1-yl,F,Br,F,H,H], [A1767;1H-pyrazol-1-yl,F,I,F,H,H], [A1768;1H-pyrazol-1-yl,F,Me,F,H,H], [A1769;1H-pyrazol-1-yl,Cl,H,Cl,H,H], [A1770;1H-pyrazol-1-yl,Cl,F,Cl,H,H], [A1771;1H-pyrazol-1-yl,Cl,Cl,CI,H,H], [A1772;1H-pyrazol-1-yl,Cl,Me,Cl,H,H], [A1773;1H-pyrazol-1-yl,Me,H,Me,H,H], [A1774;1H-pyrazol-1-yl,Me,F,Me,H,H], [A1775;1H-pyrazol-1-yl,Me,Cl,Me,H,H], [A1776;1-methyl-1H-pyazol-3-yl,F,H,H,H,H], [A1777;1-methyl-1H-pyazol-3-yl,H,F,H,H,H], [A1778;1-methyl-1H-pyazol-3-yl,Cl,H,H,H,H], [A1779;1-methyl-1H-pyazol-3-yl,H,Cl,H,H,H], [A1780;1-methyl-1H-pyazol-3-yl,Br,H,H,H,H], [A1781;1-methyl-1H-pyazol-3-yl,H,Br,H,H,H], [A1782;1-methyl-1H-pyazol-3-yl,I,H,H,H,H], [A1783;1-methyl-1H-pyazol-3-yl,H,I,H,H,H], [A1784;1-methyl-1H-pyazol-3-yl,Me,H,H,H,H], [A1785;1-methyl-1H-pyazol-3-yl,H,Me,H,H,H], [A1786;1-methyl-1H-pyazol-3-yl,CF₃,H,H,H,H], [A1787;1-methyl-1H-pyazol-3-yl,H,CF₃,H,H,H], [A1788;1-methyl-1H-pyazol-3-yl,CHF₂,H,H,H,H], [A1789;1-methyl-1H-pyazol-3-yl,H,CHF₂,H,H,H], [A1790;1-methyl-1H-pyazol-3-yl,c-Pr,H,H,H,H], [A1791;1-methyl-1H-pyazol-3-yl,H,c-Pr,H,H,H], [A1792;1-methyl-1H-pyazol-3-yl,F,F,H,H,H], [A1793;1-methyl-1H-pyazol-3-yl,F,Cl,H,H,H], [A1794;1-methyl-1H-pyazol-3-yl,F,Br,H,H,H], [A1795;1-methyl-1H-pyazol-3-yl,F,I,H,H,H], [A1796;1-methyl-1H-pyazol-3-yl,F,Me,H,H,H], [A1797;1-methyl-1H-pyazol-3-yl,Cl,F,H,H,H], [A1798;1-methyl-1H-pyazol-3-yl,Cl,Cl,H,H,H], [A1799;1-methyl-1H-pyazol-3-yl,Cl,Br,H,H,H], [A1800;1-methyl-1H-pyazol-3-yl,Cl,I,H,H,H], [A1801;1-methyl-1H-pyazol-3-yl,Cl,Me,H,H,H], [A1802;1-methyl-1H-pyazol-3-yl,Me,F,H,H,H], [A1803;1-methyl-1H-pyazol-3-yl,Me,Cl,H,H,H], [A1804;1-methyl-1H-pyazol-3-yl,Me,Br,H,H,H], [A1805;1-methyl-1H-pyazol-3-yl,Me,I,H,H,H], [A1806;1-methyl-1H-pyazol-3-yl,Me,Me,H,H,H], [A1807;1-methyl-1H-pyazol-3-yl,F,H,F,H,H], [A1808;1-methyl-1H-pyazol-3-yl,F,F,F,H,H], [A1809;1-methyl-1H-pyazol-3-yl,F,Cl,F,H,H], [A1810;1-methyl-1H-pyazol-3-yl,F,Br,F,H,H], [A1811;1-methyl-1H-pyazol-3-yl,F,I,F,H,H], [A1812;1-methyl-1H-pyazol-3-yl,F,Me,F,H,H], [A1813;1-methyl-1H-pyazol-3-yl,Cl,H,Cl,H,H], [A1814;1-methyl-1H-pyazol-3-yl,Cl,F,Cl,H,H], [A1815;1-methyl-1H-pyazol-3-yl,Cl,Cl,Cl,H,H], [A1816;1-methyl-1H-pyazol-3-yl,Cl,Me,Cl,H,H], [A1817;1-methyl-1H-pyazol-3-yl,Me,H,Me,H,H], [A1818;1-methyl-1H-pyazol-3-yl,Me,F,Me,H,H], [A1819;1-methyl-1H-pyazol-3-yl,Me,Cl,Me,H,H], [A1820;1H-pyrrol-1-yl,F,H,H,H,H], [A1821;1H-pyrrol-1-yl,H,F,H,H,H], [A1822;1H-pyrrol-1-yl,Cl,H,H,H,H], [A1823;1H-pyrrol-1-yl,H,Cl,H,H,H], [A1824;1H-pyrrol-1-yl,Br,H,H,H,H], [A1825;1H-pyrrol-1-yl,H,Br,H,H,H], [A1826;1H-pyrrol-1-yl,I,H,H,H,H], [A1827;1H-pyrrol-1-yl,H,I,H,H,H], [A1828;1H-pyrrol-1-yl,Me,H,H,H,H], [A1829;1H-pyrrol-1-yl,H,Me,H,H,H], [A1830;1H-pyrrol-1-yl,CF₃,H,H,H,H], [A1831;1H-pyrrol-1-yl,H,CF₃,H,H,H], [A1832;1H-pyrrol-1-yl,CHF₂,H,H,H,H], [A1833;1H-pyrrol-1-yl,H,CHF₂,H,H,H], [A1834;1H-pyrrol-1-yl,c-Pr,H,H,H,H], [A1835;1H-pyrrol-1-yl,H,c-Pr,H,H,H], [A1836;1H-pyrrol-1-yl,F,F,H,H,H], [A1837;1H-pyrrol-1-yl,F,Cl,H,H,H], [A1838;1H-pyrrol-1-yl,F,Br,H,H,H], [A1839;1H-pyrrol-1-yl,F,I,H,H,H], [A1840;1H-pyrrol-1-yl,F,Me,H,H,H], [A1841;1H-pyrrol-1-yl,Cl,F,H,H,H], [A1842;1H-pyrrol-1-yl,Cl,Cl,H,H,H], [A1843;1H-pyrrol-1-yl,Cl,Br,H,H,H], [A1844;1H-pyrrol-1-yl,Cl,I,H,H,H], [A1845;1H-pyrrol-1-yl,Cl,Me,H,H,H], [A1846;1H-pyrrol-1-yl,Me,F,H,H,H], [A1847;1H-pyrrol-1-yl,Me,Cl,H,H,H], [A1848;1H-pyrrol-1-yl,Me,Br,H,H,H], [A1849;1H-pyrrol-1-yl,Me,I,H,H,H], [A1850;1H-pyrrol-1-yl,Me,Me,H,H,H], [A1851;1H-pyrrol-1-yl,F,H,F,H,H], [A1852;1H-pyrrol-1-yl,F,F,F,H,H], [A1853;1H-pyrrol-1-yl,F,Cl,F,H,H], [A1854;1H-pyrrol-1-yl,F,Br,F,H,H], [A1855;1H-pyrrol-1-yl,F,I,F,H,H], [A1856;1H-pyrrol-1-yl,F,Me,F,H,H], [A1857;1H-pyrrol-1-yl,Cl,H,Cl,H,H], [A1858;1H-pyrrol-1-yl,Cl,F,Cl,H,H], [A1859;1H-pyrrol-1-yl,Cl,Cl,Cl,H,H], [A1860;1H-pyrrol-1-yl,Cl,Me,Cl,H,H], [A1861;1H-pyrrol-1-yl,Me,H,Me,H,H], [A1862;1H-pyrrol-1-yl,Me,F,Me,H,H], [A1863;1H-pyrrol-1-yl,Me,Cl,Me,H,H].

The compound (A-1) wherein n is 1, each of R⁶, R⁷ and R⁸ represents a hydrogen atom, and the combination of R¹, R^{4A}, R^{4B}, R^{4C}, R² and R³ represents any combination described in the combination A (hereinafter, referred to as "compound class SX2").

The compound (A-1) wherein n is 2, each of R⁶, R⁷ and R⁸ represents a hydrogen atom, and the combination of R¹, R^{4A}, R^{4B}, R^{4C}, R² and R³ represents any combination described in the combination A (hereinafter, referred to as "compound class SX3").

The compound (A-1) wherein n is 0, R⁶ represents a methyl group, each of R⁷ and R⁸ represents a hydrogen atom, and the combination of R¹, R^{4A}, R^{4B}, R^{4C}, R² and R³ represents any combination described in the combination A (hereinafter, referred to as "compound class SX4").

The compound (A-1) wherein n is 1, R⁶ represents a methyl group, each of R⁷ and R⁸ represents a hydrogen atom, and the combination of R¹, R^{4A}, R^{4B}, R^{4C}, R² and R³ represents any combination described in the combination A (hereinafter, referred to as "compound class SX5").

The compound (A-1) wherein n is 2, R⁶ represents a methyl group, each of R⁷ and R⁸ represents a hydrogen atom, and the combination of R¹, R^{4A}, R^{4B}, R^{4C}, R² and R³ represents any combination described in the combination A (hereinafter, referred to as "compound class SX6").

The compound (A-1) wherein n is 0, R⁷ represents a methyl group, each of R⁶ and R⁸ represents a hydrogen atom, and the combination of R¹, R^{4A}, R^{4B}, R^{4C}, R² and R³ represents any combination described in the combination A (hereinafter, referred to as "compound class SX7").

The compound (A-1) wherein n is 1, R⁷ represents a methyl group, each of R⁶ and R⁸ represents a hydrogen atom, and the combination of R¹, R^{4A}, R^{4B}, R^{4C}, R² and R³ represents any combination described in the combination A (hereinafter, referred to as "compound class SX8").

The compound (A-1) wherein n is 2, R⁷ represents a methyl group, each of R⁶ and R⁸ represents a hydrogen atom, and the combination of R¹, R^{4A}, R^{4B}, R^{4C}, R² and R³ represents any combination described in the combination A (hereinafter, referred to as "compound class SX9").

The compound (A-1) wherein n is 0, R⁸ represents a methyl group, each of R⁶ and R⁷ represents a hydrogen atom, and the combination of R¹, R^{4A}, R^{4B}, R^{4C}, R² and R³ represents any combination described in the combination A (hereinafter, referred to as "compound class SX10").

The compound (A-1) wherein n is 1, R⁸ represents a methyl group, each of R⁶ and R⁷ represents a hydrogen atom, and the combination of R¹, R^{4A}, R^{4B}, R^{4C}, R² and R³ represents any combination described in the combination A (hereinafter, referred to as "compound class SX11").

The compound (A-1) wherein n is 2, R⁸ represents a methyl group, each of R⁶ and R⁷ represents a hydrogen atom, and the combination of R¹, R^{4A}, R^{4B}, R^{4C}, R² and R³ represents any combination described in the combination A (hereinafter, referred to as "compound class SX12").

The compound (A-1) wherein n is 0, R⁶ represents a chlorine atom, each of R⁷ and R⁸ represents a hydrogen atom, and the combination of R¹, R^{4A}, R^{4B}, R^{4C}, R² and R³ represents any combination described in the combination A (hereinafter, referred to as "compound class SX13").

The compound (A-1) wherein n is 1, R⁶ represents a chlorine atom, each of R⁷ and R⁸ represents a hydrogen atom, and the combination of R¹, R^{4A}, R^{4B}, R^{4C}, R² and R³ represents any combination described in the combination A (hereinafter, referred to as "compound class SX14").

The compound (A-1) wherein n is 2, R⁶ represents a chlorine atom, each of R⁷ and R⁸ represents a hydrogen atom, and the combination of R¹, R^{4A}, R^{4B}, R^{4C}, R² and R³ represents any combination described in the combination A (hereinafter, referred to as "compound class SX15").

The compound (A-1) wherein n is 0, R⁷ represents a chlorine atom, each of R⁶ and R⁸ represents a hydrogen atom, and the combination of R¹, R^{4A}, R^{4B}, R^{4C}, R² and R³ represents any combination described in the combination A (hereinafter, referred to as "compound class SX16").

The compound (A-1) wherein n is 1, R⁷ represents a chlorine atom, each of R⁶ and R⁸ represents a hydrogen atom, and the combination of R¹, R^{4A}, R^{4B}, R^{4C}, R² and R³ represents any combination described in the combination A (hereinafter, referred to as "compound class SX17").

The compound (A-1) wherein n is 2, R⁷ represents a chlorine atom, each of R⁶ and R⁸ represents a hydrogen atom, and the combination of R¹, R^{4A}, R^{4B}, R^{4C}, R² and R³ represents any combination described in the combination A (hereinafter, referred to as "compound class SX18").

The compound (A-1) wherein n is 0, R⁸ represents a chlorine atom, each of R⁶ and R⁷ represents a hydrogen atom, and the combination of R¹, R^{4A}, R^{4B}, R^{4C}, R² and R³ represents any combination described in the combination A (hereinafter, referred to as "compound class SX19").

The compound (A-1) wherein n is 1, R⁸ represents a chlorine atom, each of R⁶ and R⁷ represents a hydrogen atom, and the combination of R¹, R^{4A}, R^{4B}, R^{4C}, R² and R³ represents any combination described in the combination A (hereinafter, referred to as "compound class SX20").

The compound (A-1) wherein n is 2, R⁸ represents a chlorine atom, each of R⁶ and R⁷ represents a hydrogen atom, and the combination of R¹, R^{4A}, R^{4B}, R^{4C}, R² and R³ represents any combination described in the combination A (hereinafter, referred to as "compound class SX21").

The compound represented by formula (A-2): 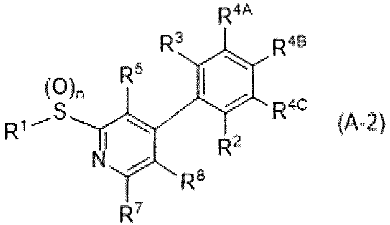 (hereinafter, referred to as "Compound (A-2)") wherein n is 0, each of R⁵, R⁷ and R⁸ represents a hydrogen atom, and the combination of R¹, R^{4A}, R^{4B}, R^{4C}, R² and R³ represents any combination described in the Combination A (hereinafter, referred to as "compound class SX22").

The compound (A-2) wherein n is 1, each of R⁵, R⁷ and R⁸ represents a hydrogen atom, and the combination of R¹, R^{4A}, R^{4B}, R^{4C}, R² and R³ represents any combination described in the combination A (hereinafter, referred to as "compound class SX23").

The compound (A-2) wherein n is 2, each of R⁵, R⁷ and R⁸ represents a hydrogen atom, and the combination of R¹, R^{4A}, R^{4B}, R^{4C}, R² and R³ represents any combination described in the combination A (hereinafter, referred to as "compound class SX24").

The compound (A-2) wherein n is 0, R⁵ represents a methyl group, each of R⁷ and R⁸ represents a hydrogen atom, and the combination of R¹, R^{4A}, R^{4B}, R^{4C}, R² and R³ represents any combination described in the combination A (hereinafter, referred to as "compound class SX25").

The compound (A-2) wherein n is 1, R⁵ represents a methyl group, each of R⁷ and R⁸ represents a hydrogen atom, and the combination of R¹, R^{4A}, R^{4B}, R^{4C}, R² and R³ represents any combination described in the combination A (hereinafter, referred to as "compound class SX26").

The compound (A-2) wherein n is 2, R³ represents a methyl group, each of R⁷ and R⁸ represents a hydrogen atom, and the combination of R¹, R^{4A}, R^{4B}, R^{4C}, R² and R³ represents any combination described in the combination A (hereinafter, referred to as "compound class SX27").

The compound (A-2) wherein n is 0, R⁷ represents a methyl group, each of R⁵ and R⁸ represents a hydrogen atom, and the combination of R¹, R^{4A}, R^{4B}, R^{4C}, R² and R³ represents any combination described in the combination A (hereinafter, referred to as "compound class SX28").

The compound (A-2) wherein n is 1, R⁷ represents a methyl group, each of R⁵ and R⁸ represents a hydrogen atom, and the combination of R¹, R^{4A}, R^{4B}, R^{4C}, R² and R³ represents any combination described in the combination A (hereinafter, referred to as "compound class SX29").

The compound (A-2) wherein n is 2, R⁷ represents a methyl group, each of R⁵ and R⁸ represents a hydrogen atom, and the combination of R¹, R^{4A}, R^{4B}, R^{4C}, R² and R³ represents any combination described in the combination A (hereinafter, referred to as "compound class SX30").

The compound (A-2) wherein n is 0, R⁸ represents a methyl group, each of R⁵ and R⁷ represents a hydrogen atom, and the combination of R¹, R^{4A}, R^{4B}, R^{4C}, R² and R³ represents any combination described in the combination A (hereinafter, referred to as "compound class SX31").

The compound (A-2) wherein n is 1, R⁸ represents a methyl group, each of R⁵ and R⁷ represents a hydrogen atom, and the combination of R¹, R^{4A}, R^{4B}, R^{4C}, R² and R³ represents any combination described in the combination A (hereinafter, referred to as "compound class SX32").

The compound (A-2) wherein n is 2, R⁸ represents a methyl group, each of R⁵ and R⁷ represents a hydrogen atom, and the combination of R¹, R^{4A}, R^{4B}, R^{4C}, R² and R³ represents any combination described in the combination A (hereinafter, referred to as "compound class SX33").

The compound (A-2) wherein n is 0, R⁵ represents a chlorine atom, each of R⁷ and R⁸ represents a hydrogen atom, and the combination of R¹, R^{4A}, R^{4B}, R^{4C}, R² and R³ represents any combination described in the combination A (hereinafter, referred to as "compound class SX34").

The compound (A-2) wherein n is 1, R⁵ represents a chlorine atom, each of R⁷ and R⁸ represents a hydrogen atom, and the combination of R¹, R^{4A}, R^{4B}, R^{4C}, R² and R³ represents any combination described in the combination A (hereinafter, referred to as "compound class SX35").

The compound (A-2) wherein n is 2, R⁵ represents a chlorine atom, each of R⁷ and R⁸ represents a hydrogen atom, and the combination of R¹, R^{4A}, R^{4B}, R^{4C}, R² and R³ represents any combination described in the combination A (hereinafter, referred to as "compound class SX36").

The compound (A-2) wherein n is 0, R⁷ represents a chlorine atom, each of R⁵ and R⁸ represents a hydrogen atom, and the combination of R¹, R^{4A}, R^{4B}, R^{4C}, R² and R³ represents any combination described in the combination A (hereinafter, referred to as "compound class SX37").

The compound (A-2) wherein n is 1, R⁷ represents a chlorine atom, each of R⁵ and R⁸ represents a hydrogen atom, and the combination of R¹, R^{4A}, R^{4B}, R^{4C}, R² and R³ represents any combination described in the combination A (hereinafter, referred to as "compound class SX38").

The compound (A-2) wherein n is 2, R⁷ represents a chlorine atom, each of R⁵ and R⁸ represents a hydrogen atom, and the combination of R¹, R^{4A}, R^{4B}, R^{4C}, R² and R³ represents any combination described in the combination A (hereinafter, referred to as "compound class SX39").

The compound (A-2) wherein n is 0, R⁸ represents a chlorine atom, each of R⁵ and R⁷ represents a hydrogen atom, and the combination of R¹, R^{4A}, R^{4B}, R^{4C}, R² and R³ represents any combination described in the combination A (hereinafter, referred to as "compound class SX40").

The compound (A-2) wherein n is 1, R⁸ represents a chlorine atom, each of R⁵ and R⁷ represents a hydrogen atom, and the combination of R¹, R^{4A}, R^{4B}, R^{4C}, R² and R³ represents any combination described in the combination A (hereinafter, referred to as "compound class SX41").

The compound (A-2) wherein n is 2, R⁸ represents a chlorine atom, each of R⁵ and R⁷ represents a hydrogen atom, and the combination of R¹, R^{4A}, R^{4B}, R^{4C}, R² and R³ represents any combination described in the combination A (hereinafter, referred to as "compound class SX42").

The compound represented by formula (A-3): 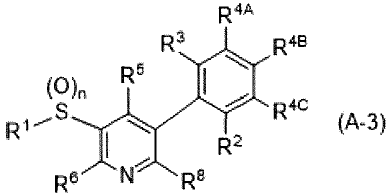 (hereinafter, referred to as "Compound (A-3)") wherein n is 0, each of R⁵, R⁶ and R⁸ represents a hydrogen atom, and the combination of R¹, R^{4A}, R^{4B}, R^{4C}, R² and R³ represents any combination described in the Combination A (hereinafter, referred to as "compound class SX43").

The compound (A-3) wherein n is 1, each of R⁵, R⁶ and R⁸ represents a hydrogen atom, and the combination of R¹, R^{4A}, R^{4B}, R^{4C}, R² and R³ represents any combination described in the combination A (hereinafter, referred to as "compound class SX44").

The compound (A-3) wherein n is 2, each of R⁵, R⁶ and R⁸ represents a hydrogen atom, and the combination of R¹, R^{4A}, R^{4B}, R^{4C}, R² and R³ represents any combination described in the combination A (hereinafter, referred to as "compound class SX45").

The compound (A-3) wherein n is 0, R⁵ represents a methyl group, each of R⁶ and R⁸ represents a hydrogen atom, and the combination of R¹, R^{4A}, R^{4B}, R^{4C}, R² and R³ represents any combination described in the combination A (hereinafter, referred to as "compound class SX46").

The compound (A-3) wherein n is 1, R⁵ represents a methyl group, each of R⁶ and R⁸ represents a hydrogen atom, and the combination of R¹, R^{4A}, R^{4B}, R^{4C}, R² and R³ represents any combination described in the combination A (hereinafter, referred to as "compound class SX47").

The compound (A-3) wherein n is 2, R⁵ represents a methyl group, each of R⁶ and R⁸ represents a hydrogen atom, and the combination of R¹, R^{4A}, R^{4B}, R^{4C}, R² and R³ represents any combination described in the combination A (hereinafter, referred to as "compound class SX48").

The compound (A-3) wherein n is 0, R⁶ represents a methyl group, each of R⁵ and R⁸ represents a hydrogen atom, and the combination of R¹, R^{4A}, R^{4B}, R^{4C}, R² and R³ represents any combination described in the combination A (hereinafter, referred to as "compound class SX49").

The compound (A-3) wherein n is 1, R⁶ represents a methyl group, each of R⁵ and R⁸ represents a hydrogen atom, and the combination of R¹, R^{4A}, R^{4B}, R^{4C}, R² and R³ represents any combination described in the combination A (hereinafter, referred to as "compound class SX50").

The compound (A-3) wherein n is 2, R⁶ represents a methyl group, each of R⁵ and R⁸ represents a hydrogen atom, and the combination of R¹, R^{4A}, R^{4B}, R^{4C}, R² and R³ represents any combination described in the combination A (hereinafter, referred to as "compound class SX51").

The compound (A-3) wherein n is 2, R⁸ represents a methyl group, each of R⁵ and R⁶ represents a hydrogen atom, and the combination of R¹, R^{4A}, R^{4B}, R^{4C}, R² and R³ represents any combination described in the combination A (hereinafter, referred to as "compound class SX52").

The compound (A-3) wherein n is 1, R⁸ represents a methyl group, each of R⁵ and R⁶ represents a hydrogen atom, and the combination of R¹, R^{4A}, R^{4B}, R^{4C}, R² and R³ represents any combination described in the combination A (hereinafter, referred to as "compound class SX53").

The compound (A-3) wherein n is 2, R⁸ represents a methyl group, each of R⁵ and R⁶ represents a hydrogen atom, and the combination of R¹, R^{4A}, R^{4B}, R^{4C}, R² and R³ represents any combination described in the combination A (hereinafter, referred to as "compound class SX54").

The compound (A-3) wherein n is 0, R⁵ represents a chlorine atom, each of R⁶ and R⁸ represents a hydrogen atom, and the combination of R¹, R^{4A}, R^{4B}, R^{4C}, R² and R³ represents any combination described in the combination A (hereinafter, referred to as "compound class SX55").

The compound (A-3) wherein n is 1, R⁵ represents a chlorine atom, each of R⁶ and R⁸ represents a hydrogen atom, and the combination of R¹, R^{4A}, R^{4B}, R^{4C}, R² and R³ represents any combination described in the combination A (hereinafter, referred to as "compound class SX56").

The compound (A-3) wherein n is 2, R⁵ represents a chlorine atom, each of R⁶ and R⁸ represents a hydrogen atom, and the combination of R¹, R^{4A}, R^{4B}, R^{4C}, R² and R³ represents any combination described in the combination A (hereinafter, referred to as "compound class SX57").

The compound (A-3) wherein n is 0, R⁶ represents a chlorine atom, each of R⁵ and R⁸ represents a hydrogen atom, and the combination of R¹, R^{4A}, R^{4B}, R^{4C}, R² and R³ represents any combination described in the combination A (hereinafter, referred to as "compound class SX58").

The compound (A-3) wherein n is 1, R⁶ represents a chlorine atom, each of R⁵ and R⁸ represents a hydrogen atom, and the combination of R¹, R^{4A}, R^{4B}, R^{4C}, R² and R³ represents any combination described in the combination A (hereinafter, referred to as "compound class SX59").

The compound (A-3) wherein n is 2, R⁶ represents a chlorine atom, each of R⁵ and R⁸ represents a hydrogen atom, and the combination of R¹, R^{4A}, R^{4B}, R^{4C}, R² and R³ represents any combination described in the combination A (hereinafter, referred to as "compound class SX60").

The compound (A-3) wherein n is 0, R⁸ represents a chlorine atom, each of R⁵ and R⁶ represents a hydrogen atom, and the combination of R¹, R^{4A}, R^{4B}, R^{4C}, R² and R³ represents any combination described in the combination A (hereinafter, referred to as "compound class SX61").

The compound (A-3) wherein n is 1, R⁸ represents a chlorine atom, each of R⁵ and R⁶ represents a hydrogen atom, and the combination of R¹, R^{4A}, R^{4B}, R^{4C}, R² and R³ represents any combination described in the combination A (hereinafter, referred to as "compound class SX62").

The compound (A-3) wherein n is 2, R⁸ represents a chlorine atom, each of R⁵ and R⁶ represents a hydrogen atom, and the combination of R¹, R^{4A}, R^{4B}, R^{4C}, R² and R³ represents any combination described in the combination A (hereinafter, referred to as "compound class SX63").

The compound represented by formula (A-4): 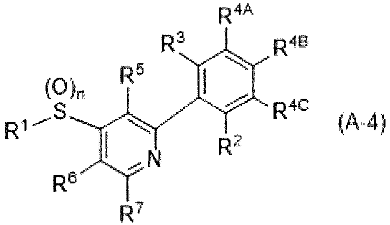 (hereinafter, referred to as "compound (A-4)") wherein n is 0, each of R⁵, R⁶ and R⁷ represents a hydrogen atom, and the combination of R¹, R^{4A}, R^{4B}, R^{4C}, R² and R³ represents any combination described in the combination A (hereinafter, referred to as "compound class SX64").

The compound (A-4) wherein n is 1, each of R⁵, R⁶ and R⁷ represents a hydrogen atom, and the combination of R¹, R^{4A}, R^{4B}, R^{4C}, R² and R³ represents any combination described in the combination A (hereinafter, referred to as "compound class SX65").

The compound (A-4) wherein n is 2, each of R⁵, R⁶ and R⁷ represents a hydrogen atom, and the combination of R¹, R^{4A}, R^{4B}, R^{4C}, R² and R³ represents any combination described in the combination A (hereinafter, referred to as "compound class SX66").

The compound (A-4) wherein n is 0, R⁵ represents a methyl group, each of R⁶ and R⁷ represents a hydrogen atom, and the combination of R¹, R^{4A}, R^{4B}, R^{4C}, R² and R³ represents any combination described in the combination A (hereinafter, referred to as "compound class SX67").

The compound (A-4) wherein n is 1, R⁵ represents a methyl group, each of R⁶ and R⁷ represents a hydrogen atom, and the combination of R¹, R^{4A}, R^{4B}, R^{4C}, R² and R³ represents any combination described in the combination A (hereinafter, referred to as "compound class SX68").

The compound (A-4) wherein n is 2, R⁵ represents a methyl group, each of R⁶ and R⁷ represents a hydrogen atom, and the combination of R¹, R^{4A}, R^{4B}, R^{4C}, R² and R³ represents any combination described in the combination A (hereinafter, referred to as "compound class SX69").

The compound (A-4) wherein n is 0, R⁶ represents a methyl group, each of R⁵ and R⁷ represents a hydrogen atom, and the combination of R¹, R^{4A}, R^{4B}, R^{4C}, R² and R³ represents any combination described in the combination A (hereinafter, referred to as "compound class SX70").

The compound (A-4) wherein n is 1, R⁶ represents a methyl group, each of R⁵ and R⁷ represents a hydrogen atom, and the combination of R¹, R^{4A}, R^{4B}, R^{4C}, R² and R³ represents any combination described in the combination A (hereinafter, referred to as "compound class SX71").

The compound (A-4) wherein n is 2, R⁶ represents a methyl group, each of R⁵ and R⁷ represents a hydrogen atom, and the combination of R¹, R^{4A}, R^{4B}, R^{4C}, R² and R³ represents any combination described in the combination A (hereinafter, referred to as "compound class SX72").

The compound (A-4) wherein n is 0, R⁶ represents a methyl group, each of R⁵ and R⁷ represents a hydrogen atom, and the combination of R¹, R^{4A}, R^{4B}, R^{4C}, R² and R³ represents any combination described in the combination A (hereinafter, referred to as "compound class SX73").

The compound (A-4) wherein n is 1, R⁷ represents a methyl group, each of R⁵ and R⁶ represents a hydrogen atom, and the combination of R¹, R^{4A}, R^{4B}, R^{4C}, R² and R³ represents any combination described in the combination A (hereinafter, referred to as "compound class SX74").

The compound (A-4) wherein n is 2, R⁷ represents a methyl group, each of R⁵ and R⁶ represents a hydrogen atom, and the combination of R¹, R^{4A}, R^{4B}, R^{4C}, R² and R³ represents any combination described in the combination A (hereinafter, referred to as "compound class SX75").

The compound (A-4) wherein n is 0, R⁵ represents a chlorine atom, each of R⁶ and R⁷ represents a hydrogen atom, and the combination of R¹, R^{4A}, R^{4B}, R^{4C}, R² and R³ represents any combination described in the combination A (hereinafter, referred to as "compound class SX76").

The compound (A-4) wherein n is 1, R⁵ represents a chlorine atom, each of R⁶ and R⁷ represents a hydrogen atom, and the combination of R¹, R^{4A}, R^{4B}, R^{4C}, R² and R³ represents any combination described in the combination A (hereinafter, referred to as "compound class SX77").

The compound (A-4) wherein n is 2, R⁵ represents a chlorine atom, each of R⁶ and R⁷ represents a hydrogen atom, and the combination of R¹, R^{4A}, R^{4B}, R^{4C}, R² and R³ represents any combination described in the combination A (hereinafter, referred to as "compound class SX78").

The compound (A-4) wherein n is 0, R⁶ represents a chlorine atom, each of R⁵ and R⁷ represents a hydrogen atom, and the combination of R¹, R^{4A}, R^{4B}, R^{4C}, R² and R³ represents any combination described in the combination A (hereinafter, referred to as "compound class SX79").

The compound (A-4) wherein n is 1, R⁶ represents a chlorine atom, each of R⁵ and R⁷ represents a hydrogen atom, and the combination of R¹, R^{4A}, R^{4B}, R^{4C}, R² and R³ represents any combination described in the combination A (hereinafter, referred to as "compound class SX80").

The compound (A-4) wherein n is 2, R⁶ represents a chlorine atom, each of R⁵ and R⁷ represents a hydrogen atom, and the combination of R¹, R^{4A}, R^{4B}, R^{4C}, R² and R³ represents any combination described in the combination A (hereinafter, referred to as "compound class SX81").

The compound (A-4) wherein n is 0, R⁷ represents a chlorine atom, each of R⁵ and R⁶ represents a hydrogen atom, and the combination of R¹, R^{4A}, R^{4B}, R^{4C}, R² and R³ represents any combination described in the combination A (hereinafter, referred to as "compound class SX82").

The compound (A-4) wherein n is 1, R⁷ represents a chlorine atom, each of R⁵ and R⁶ represents a hydrogen atom, and the combination of R¹, R^{4A}, R^{4B}, R^{4C}, R² and R³ represents any combination described in the combination A (hereinafter, referred to as "compound class SX83").

The compound (A-4) wherein n is 2, R⁷ represents a chlorine atom, each of R⁵ and R⁶ represents a hydrogen atom, and the combination of R¹, R^{4A}, R^{4B}, R^{4C}, R² and R³ represents any combination described in the combination A (hereinafter, referred to as "compound class SX84").

The compound represented by formula (A-5): 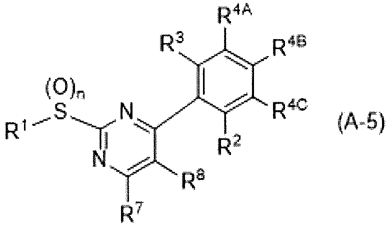 (hereinafter, referred to as "compound (A-5)") wherein n is 0, each of R⁷ and R⁸ represents a hydrogen atom, and the combination of R¹, R^{4A}, R^{4B}, R^{4C}, R² and R³ represents any combination described in the combination A (hereinafter, referred to as "Compound class SX85").

The compound (A-5) wherein n is 1, each of R⁷ and R⁸ represents a hydrogen atom, and the combination of R¹, R^{4A}, R^{4B}, R^{4C}, R² and R³ represents any combination described in the combination A (hereinafter, referred to as "compound class SX86").

The compound (A-5) wherein n is 2, each of R⁷ and R⁸ represents a hydrogen atom, and the combination of R¹, R^{4A}, R^{4B}, R^{4C}, R² and R³ represents any combination described in the combination A (hereinafter, referred to as "compound class SX87").

The compound (A-5) wherein n is 0, R⁷ represents a methyl group, R⁸ represents a hydrogen atom, and the combination of R¹, R^{4A}, R^{4B}, R^{4C}, R² and R³ represents any combination described in the combination A (hereinafter, referred to as "compound class SX88").

The compound (A-5) wherein n is 1, R⁷ represents a methyl group, R⁸ represents a hydrogen atom, and the combination of R¹, R^{4A}, R^{4B}, R^{4C}, R² and R³ represents any combination described in the combination A (hereinafter, referred to as "compound class SX89").

The compound (A-5) wherein n is 2, R⁷ represents a methyl group, R⁸ represents a hydrogen atom, and the combination of R¹, R^{4A}, R^{4B}, R^{4C}, R² and R³ represents any combination described in the combination A (hereinafter, referred to as "compound class SX90").

The compound (A-5) wherein n is 0, R⁸ represents a methyl group, R⁷ represents a hydrogen atom, and the combination of R¹, R^{4A}, R^{4B}, R^{4C}, R² and R³ represents any combination described in the combination A (hereinafter, referred to as "compound class SX91").

The compound (A-5) wherein n is 1, R⁸ represents a methyl group, R⁷ represents a hydrogen atom, and the combination of R¹, R^{4A}, R^{4B}, R^{4C}, R² and R³ represents any combination described in the combination A (hereinafter, referred to as "compound class SX92").

The compound (A-5) wherein n is 2, R⁸ represents a methyl group, R⁷ represents a hydrogen atom, and the combination of R¹, R^{4A}, R^{4B}, R^{4C}, R² and R³ represents any combination described in the combination A (hereinafter, referred to as "compound class SX93").

The compound (A-5) wherein n is 0, R⁷ represents a chlorine atom, R⁸ represents a hydrogen atom, and the combination of R¹, R^{4A}, R^{4B}, R^{4C}, R² and R³ represents any combination described in the combination A (hereinafter, referred to as "compound class SX94").

The compound (A-5) wherein n is 1, R⁷ represents a chlorine atom, R⁸ represents a hydrogen atom, and the combination of R¹, R^{4A}, R^{4B}, R^{4C}, R² and R³ represents any combination described in the combination A (hereinafter, referred to as "compound class SX95").

The compound (A-5) wherein n is 2, R⁷ represents a chlorine atom, R⁸ represents a hydrogen atom, and the combination of R¹, R^{4A}, R^{4B}, R^{4C}, R² and R³ represents any combination described in the combination A (hereinafter, referred to as "compound class SX96").

The compound (A-5) wherein n is 0, R⁸ represents a chlorine atom, R⁷ represents a hydrogen atom, and the combination of R¹, R^{4A}, R^{4B}, R^{4C}, R² and R³ represents any combination described in the combination A (hereinafter, referred to as "compound class SX97").

The compound (A-5) wherein n is 1, R⁸ represents a chlorine atom, R⁷ represents a hydrogen atom, and the combination of R¹, R^{4A}, R^{4B}, R^{4C}, R² and R³ represents any combination described in the combination A (hereinafter, referred to as "compound class SX98").

The compound (A-5) wherein n is 2, R⁸ represents a chlorine atom, R⁷ represents a hydrogen atom, and the combination of R¹, R^{4A}, R^{4B}, R^{4C}, R² and R³ represents any combination described in the combination A (hereinafter, referred to as "compound class SX99").

The compound represented by formula (A-6): 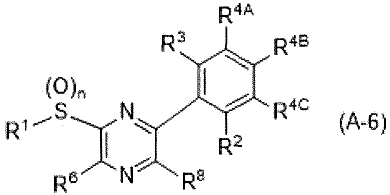 (hereinafter, referred to as "compound (A-6)") wherein n is 0, each of R⁶ and R⁸ represents a hydrogen atom, and the combination of R¹, R^{4A}, R^{4B}, R^{4C}, R² and R³ represents any combination described in the combination A) (hereinafter, referred to as "compound class SX100").

The compound (A-6) wherein n is 1, each of R⁶ and R⁸ represents a hydrogen atom, and the combination of R¹, R^{4A}, R^{4B}, R^{4C}, R² and R³ represents any combination described in the combination A (hereinafter, referred to as "compound class SX101").

The compound (A-6) wherein n is 2, each of R⁶ and R⁸ represents a hydrogen atom, and the combination of R¹, R^{4A}, R^{4B}, R^{4C}, R² and R³ represents any combination described in the combination A (hereinafter, referred to as "compound class SX102").

The compound (A-6) wherein n is 0, R⁶ represents a methyl group, R⁸ represents a hydrogen atom, and the combination of R¹, R^{4A}, R^{4B}, R^{4C}, R² and R³ represents any combination described in the combination A (hereinafter, referred to as "compound class SX103").

The compound (A-6) wherein n is 1, R⁶ represents a methyl group, R⁸ represents a hydrogen atom, and the combination of R¹, R^{4A}, R^{4B}, R^{4C}, R² and R³ represents any combination described in the combination A (hereinafter, referred to as "compound class SX104").

The compound (A-6) wherein n is 2, R⁶ represents a methyl group, R⁸ represents a hydrogen atom, and the combination of R¹, R^{4A}, R^{4B}, R^{4C}, R² and R³ represents any combination described in the combination A (hereinafter, referred to as "compound class SX105").

The compound (A-6) wherein n is 0, R⁸ represents a methyl group, R⁶ represents a hydrogen atom, and the combination of R¹, R^{4A}, R^{4B}, R^{4C}, R² and R³ represents any combination described in the combination A (hereinafter, referred to as "compound class SX106").

The compound (A-6) wherein n is 1, R⁸ represents a methyl group, R⁶ represents a hydrogen atom, and the combination of R¹, R^{4A}, R^{4B}, R^{4C}, R² and R³ represents any combination described in the combination A (hereinafter, referred to as "compound class SX107").

The compound (A-6) wherein n is 2, R⁸ represents a methyl group, R⁶ represents a hydrogen atom, and the combination of R¹, R^{4A}, R^{4B}, R^{4C}, R² and R³ represents any combination described in the combination A (hereinafter, referred to as "compound class SX108").

The compound (A-6) wherein n is 0, R⁶ represents a chlorine atom, R⁸ represents a hydrogen atom, and the combination of R¹, R^{4A}, R^{4B}, R^{4C}, R² and R³ represents any combination described in the combination A (hereinafter, referred to as "compound class SX109").

The compound (A-6) wherein n is 1, R⁶ represents a chlorine atom, R⁸ represents a hydrogen atom, and the combination of R¹, R^{4A}, R^{4B}, R^{4c}, R² and R³ represents any combination described in the combination A (hereinafter, referred to as "compound class SX110").

The compound (A-6) wherein n is 2, R⁶ represents a chlorine atom, R⁸ represents a hydrogen atom, and the combination of R¹, R^{4A}, R^{4B}, R^{4C}, R² and R³ represents any combination described in the combination A (hereinafter, referred to as "compound class SX111").

The compound (A-6) wherein n is 0, R⁸ represents a chlorine atom, R⁶ represents a hydrogen atom, and the combination of R¹, R^{4A}, R^{4B}, R^{4C}, R² and R³ represents any combination described in the combination A (hereinafter, referred to as "compound class SX112").

The compound (A-6) wherein n is 1, R⁸ represents a chlorine atom, R⁶ represents a hydrogen atom, and the combination of R¹, R^{4A}, R^{4B}, R^{4C}, R² and R³ represents any combination described in the combination A (hereinafter, referred to as "compound class SX113").

The compound (A-6) wherein n is 2, R⁸ represents a chlorine atom, R⁶ represents a hydrogen atom, and the combination of R¹, R^{4A}, R^{4B}, R^{4C}, R² and R³ represents any combination described in the combination A (hereinafter, referred to as "compound class SX114").

The compound represented by formula (A-7): 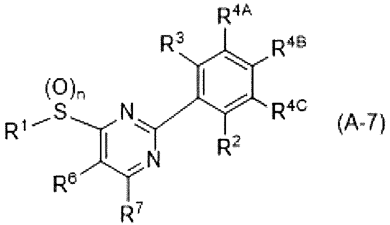 (hereinafter, referred to as "compound (A-7)") wherein n is 0, each of R⁶ and R⁷ represents a hydrogen atom, and the combination of R¹, R^{4A}, R^{4B}, R^{4C}, R² and R³ represents any combination described in the combination A) (hereinafter, referred to as "Compound class SX115").

The compound (A-7) wherein n is 1, each of R⁶ and R⁷ represents a hydrogen atom, and the combination of R¹, R^{4A}, R^{4B}, R^{4C}, R² and R³ represents any combination described in the combination A (hereinafter, referred to as "compound class SX116").

The compound (A-7) wherein n is 2, each of R⁶ and R⁷ represents a hydrogen atom, and the combination of R¹, R^{4A}, R^{4B}, R^{4C}, R² and R³ represents any combination described in the combination A (hereinafter, referred to as "compound class SX117").

The compound (A-7) wherein n is 0, R⁶ represents a methyl group, R⁷ represents a hydrogen atom, and the combination of R¹, R^{4A}, R^{4B}, R^{4C}, R² and R³ represents any combination described in the combination A (hereinafter, referred to as "compound class SX118").

The compound (A-7) wherein n is 1, R⁶ represents a methyl group, R⁷ represents a hydrogen atom, and the combination of R¹, R^{4A}, R^{4B}, R^{4C}, R² and R³ represents any combination described in the combination A (hereinafter, referred to as "compound class SX119").

The compound (A-7) wherein n is 2, R⁶ represents a methyl group, R⁷ represents a hydrogen atom, and the combination of R¹, R^{4A}, R^{4B}, R^{4C}, R² and R³ represents any combination described in the combination A (hereinafter, referred to as "compound class SX120").

The compound (A-7) wherein n is 0, R⁷ represents a methyl group, R⁶ represents a hydrogen atom, and the combination of R¹, R^{4A}, R^{4B}, R^{4C}, R² and R³ represents any combination described in the combination A (hereinafter, referred to as "compound class SX121").

The compound (A-7) wherein n is 1, R⁷ represents a methyl group, R⁶ represents a hydrogen atom, and the combination of R¹, R^{4A}, R^{4B}, R^{4C}, R² and R³ represents any combination described in the combination A (hereinafter, referred to as "compound class SX122").

The compound (A-7) wherein n is 2, R⁷ represents a methyl group, R⁶ represents a hydrogen atom, and the combination of R¹, R^{4A}, R^{4B}, R^{4C}, R² and R³ represents any combination described in the combination A (hereinafter, referred to as "compound class SX123").

The compound (A-7) wherein n is 0, R⁶ represents a chlorine atom, R⁷ represents a hydrogen atom, and the combination of R¹, R^{4A}, R^{4B}, R^{4C}, R² and R³ represents any combination described in the combination A (hereinafter, referred to as "compound class SX124").

The compound (A-7) wherein n is 1, R⁶ represents a chlorine atom, R⁷ represents a hydrogen atom, and the combination of R¹, R^{4A}, R^{4B}, R^{4C}, R² and R³ represents any combination described in the combination A (hereinafter, referred to as "compound class SX125").

The compound (A-7) wherein n is 2, R⁶ represents a chlorine atom, R⁷ represents a hydrogen atom, and the combination of R¹, R^{4A}, R^{4B}, R^{4C}, R² and R³ represents any combination described in the combination A (hereinafter, referred to as "compound class SX126").

The compound (A-7) wherein n is 0, R⁷ represents a chlorine atom, R⁶ represents a hydrogen atom, and the combination of R¹, R^{4A}, R^{4B}, R^{4C}, R² and R³ represents any combination described in the combination A (hereinafter, referred to as "compound class SX127").

The compound (A-7) wherein n is 1, R⁷ represents a chlorine atom, R⁶ represents a hydrogen atom, and the combination of R¹, R^{4A}, R^{4B}, R^{4C}, R² and R³ represents any combination described in the combination A (hereinafter, referred to as "compound class SX128").

The compound (A-7) wherein n is 2, R⁷ represents a chlorine atom, R⁶ represents a hydrogen atom, and the combination of R¹, R^{4A}, R^{4B}, R^{4C}, R² and R³ represents any combination described in the combination A (hereinafter, referred to as "compound class SX129").

The compound represented by formula (A-8): 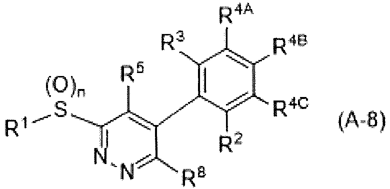 (hereinafter, referred to as "compound (A-8)") wherein n is 0, each of R⁵ and R⁸ represents a hydrogen atom, and the combination of R¹, R^{4A}, R^{4B}, R^{4C}, R² and R³ represents any combination described in the combination A (hereinafter, referred to as "compound class SX130").

The compound (A-8) wherein n is 1, each of R⁵ and R⁸ represents a hydrogen atom, and the combination of R¹, R^{4A}, R^{4B}, R^{4C}, R² and R³ represents any combination described in the combination A (hereinafter, referred to as "compound class SX131").

The compound (A-8) wherein n is 2, each of R⁵ and R⁸ represents a hydrogen atom, and the combination of R¹, R^{4A}, R^{4B}, R^{4C}, R² and R³ represents any combination described in the combination A (hereinafter, referred to as "compound class SX132").

The compound (A-8) wherein n is 0, R⁵ represents a methyl group, R⁸ represents a hydrogen atom, and the combination of R¹, R^{4A}, R^{4B}, R^{4C}, R² and R³ represents any combination described in the combination A (hereinafter, referred to as "compound class SX133").

The compound (A-8) wherein n is 1, R³ represents a methyl group, R⁸ represents a hydrogen atom, and the combination of R¹, R^{4A}, R^{4B}, R^{4C}, R² and R³ represents any combination described in the combination A (hereinafter, referred to as "compound class SX134").

The compound (A-8) wherein n is 2, R⁵ represents a methyl group, R⁸ represents a hydrogen atom, and the combination of R¹, R^{4A}, R^{4B}, R^{4C}, R² and R³ represents any combination described in the combination A (hereinafter, referred to as "compound class SX135").

The compound (A-8) wherein n is 0, R⁸ represents a methyl group, R⁵ represents a hydrogen atom, and the combination of R¹, R^{4A}, R^{4B}, R^{4C}, R² and R³ represents any combination described in the combination A (hereinafter, referred to as "compound class SX136").

The compound (A-8) wherein n is 1, R⁸ represents a methyl group, R⁵ represents a hydrogen atom, and the combination of R¹, R^{4A}, R^{4B}, R^{4C}, R² and R³ represents any combination described in the combination A (hereinafter, referred to as "compound class SX137").

The compound (A-8) wherein n is 2, R⁸ represents a methyl group, R³ represents a hydrogen atom, and the combination of R¹, R^{4A}, R^{4B}, R^{4C}, R² and R³ represents any combination described in the combination A (hereinafter, referred to as "compound class SX138").

The compound (A-8) wherein n is 0, R⁵ represents a chlorine atom, R⁸ represents a hydrogen atom, and the combination of R¹, R^{4A}, R^{4B}, R^{4C}, R² and R³ represents any combination described in the combination A (hereinafter, referred to as "compound class SX139").

The compound (A-8) wherein n is 1, R⁵ represents a chlorine atom, R⁸ represents a hydrogen atom, and the combination of R¹, R^{4A}, R^{4B}, R^{4C}, R² and R³ represents any combination described in the combination A (hereinafter, referred to as "compound class SX140").

The compound (A-8) wherein n is 2, R⁵ represents a chlorine atom, R⁸ represents a hydrogen atom, and the combination of R¹, R^{4A}, R^{4B}, R^{4C}, R² and R³ represents any combination described in the combination A (hereinafter, referred to as "compound class SX141").

The compound (A-8) wherein n is 0, R⁸ represents a chlorine atom, R⁵ represents a hydrogen atom, and the combination of R¹, R^{4A}, R^{4B}, R^{4C}, R² and R³ represents any combination described in the combination A (hereinafter, referred to as "compound class SX142").

The compound (A-8) wherein n is 1, R⁸ represents a chlorine atom, R⁵ represents a hydrogen atom, and the combination of R¹, R^{4A}, R^{4B}, R^{4C}, R² and R³ represents any combination described in the combination A (hereinafter, referred to as "compound class SX143").

The compound (A-8) wherein n is 2, R⁸ represents a chlorine atom, R⁵ represents a hydrogen atom, and the combination of R¹, R^{4A}, R^{4B}, R^{4C}, R² and R³ represents any combination described in the combination A (hereinafter, referred to as "compound class SX144").

The compound represented by formula (A-9): 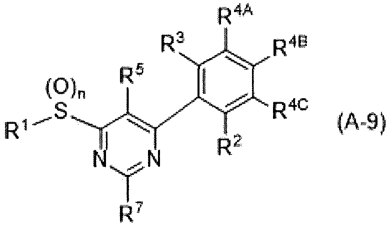 (hereinafter, referred to as "compound (A-9)") wherein n is 0, each of R⁵ and R⁷ represents a hydrogen atom, and the combination of R¹, R^{4A}, R^{4B}, R^{4C}, R² and R³ represents any combination described in the combination A) (hereinafter, referred to as "compound class SX145").

The compound (A-9) wherein n is 1, each of R⁵ and R⁷ represents a hydrogen atom, and the combination of R¹, R^{4A}, R^{4B}, R^{4C}, R² and R³ represents any combination described in the combination A (hereinafter, referred to as "compound class SX146").

The compound (A-9) wherein n is 2, each of R⁵ and R⁷ represents a hydrogen atom, and the combination of R¹, R^{4A}, R^{4B}, R^{4C}, R² and R³ represents any combination described in the combination A (hereinafter, referred to as "compound class SX147").

The compound (A-9) wherein n is 0, R⁵ represents a methyl group, R⁷ represents a hydrogen atom, and the combination of R¹, R^{4A}, R^{4B}, R^{4C}, R² and R³ represents any combination described in the combination A (hereinafter, referred to as "compound class SX148").

The compound (A-9) wherein n is 1, R⁵ represents a methyl group, R⁷ represents a hydrogen atom, and the combination of R¹, R^{4A}, R^{4B}, R^{4C}, R² and R³ represents any combination described in the combination A (hereinafter, referred to as "compound class SX149").

The compound (A-9) wherein n is 2, R⁵ represents a methyl group, R⁷ represents a hydrogen atom, and the combination of R¹, R^{4A}, R^{4B}, R^{4C}, R² and R³ represents any combination described in the combination A (hereinafter, referred to as "compound class SX150").

The compound (A-9) wherein n is 0, R⁷ represents a methyl group, R⁵ represents a hydrogen atom, and the combination of R¹, R^{4A}, R^{4B}, R^{4C}, R² and R³ represents any combination described in the combination A (hereinafter, referred to as "compound class SX151").

The compound (A-9) wherein n is 1, R⁷ represents a methyl group, R⁵ represents a hydrogen atom, and the combination of R¹, R^{4A}, R^{4B}, R^{4C}, R² and R³ represents any combination described in the combination A (hereinafter, referred to as "compound class SX152").

The compound (A-9) wherein n is 2, R⁷ represents a methyl group, R⁵ represents a hydrogen atom, and the combination of R¹, R^{4A}, R^{4B}, R^{4C}, R² and R³ represents any combination described in the combination A (hereinafter, referred to as "compound class SX153").

The compound (A-9) wherein n is 0, R⁵ represents a chlorine atom, R⁷ represents a hydrogen atom, and the combination of R¹, R^{4A}, R^{4B}, R^{4C}, R² and R³ represents any combination described in the combination A (hereinafter, referred to as "compound class SX154").

The compound (A-9) wherein n is 1, R⁵ represents a chlorine atom, R⁷ represents a hydrogen atom, and the combination of R¹, R^{4A}, R^{4B}, R^{4C}, R² and R³ represents any combination described in the combination A (hereinafter, referred to as "compound class SX155").

The compound (A-9) wherein n is 2, R⁵ represents a chlorine atom, R⁷ represents a hydrogen atom, and the combination of R¹, R^{4A}, R^{4B}, R^{4C}, R² and R³ represents any combination described in the combination A (hereinafter, referred to as "compound class SX156").

The compound (A-9) wherein n is 0, R⁷ represents a chlorine atom, R⁵ represents a hydrogen atom, and the combination of R¹, R^{4A}, R^{4B}, R^{4C}, R² and R³ represents any combination described in the combination A (hereinafter, referred to as "compound class SX157").

The compound (A-9) wherein n is 1, R⁷ represents a chlorine atom, R⁵ represents a hydrogen atom, and the combination of R¹, R^{4A}, R^{4B}, R^{4C}, R² and R³ represents any combination described in the combination A (hereinafter, referred to as "compound class SX158").

The compound (A-9) wherein n is 2, R⁷ represents a chlorine atom, R⁵ represents a hydrogen atom, and the combination of R¹, R^{4A}, R^{4B}, R^{4C}, R² and R³ represents any combination described in the combination A (hereinafter, referred to as "compound class SX159").

The compound represented by formula (A-10): 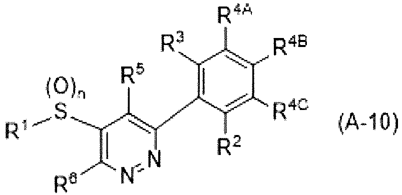 (hereinafter, referred to as "compound (A-10)") wherein n is 0, each of R⁵ and R⁶ represents a hydrogen atom, and the combination of R¹, R^{4A}, R^{4B}, R^{4C}, R² and R³ represents any combination described in the combination A (hereinafter, referred to as "compound class SX160").

The compound (A-10) wherein n is 1, each of R⁵ and R⁶ represents a hydrogen atom, and the combination of R¹, R^{4A}, R^{4B}, R^{4C}, R² and R³ represents any combination described in the combination A (hereinafter, referred to as "compound class SX161").

The compound (A-10) wherein n is 2, each of R⁵ and R⁶ represents a hydrogen atom, and the combination of R¹, R^{4A}, R^{4B}, R^{4C}, R² and R³ represents any combination described in the combination A (hereinafter, referred to as "compound class SX162").

The compound (A-10) wherein n is 0, R³ represents a methyl group, R⁶ represents a hydrogen atom, and the combination of R¹, R^{4A}, R^{4B}, R^{4C}, R² and R³ represents any combination described in the combination A (hereinafter, referred to as "compound class SX163").

The compound (A-10) wherein n is 1, R⁵ represents a methyl group, R⁶ represents a hydrogen atom, and the combination of R¹, R^{4A}, R^{4B}, R^{4C}, R² and R³ represents any combination described in the combination A (hereinafter, referred to as "compound class SX164").

The compound (A-10) wherein n is 2, R⁵ represents a methyl group, R⁶ represents a hydrogen atom, and the combination of R¹, R^{4A}, R^{4B}, R^{4C}, R² and R³ represents any combination described in the combination A (hereinafter, referred to as "compound class SX165").

The compound (A-10) wherein n is 0, R⁶ represents a methyl group, R⁵ represents a hydrogen atom, and the combination of R¹, R^{4A}, R^{4B}, R^{4C}, R² and R³ represents any combination described in the combination A (hereinafter, referred to as "compound class SX166").

The compound (A-10) wherein n is 1, R⁶ represents a methyl group, R⁵ represents a hydrogen atom, and the combination of R¹, R^{4A}, R^{4B}, R^{4C}, R² and R³ represents any combination described in the combination A (hereinafter, referred to as "compound class SX167").

The compound (A-10) wherein n is 2, R⁶ represents a methyl group, R⁵ represents a hydrogen atom, and the combination of R¹, R^{4A}, R^{4B}, R^{4C}, R² and R³ represents any combination described in the combination A (hereinafter, referred to as "compound class SX168").

The compound (A-10) wherein n is 0, R⁵ represents a chlorine atom, R⁶ represents a hydrogen atom, and the combination of R¹, R^{4A}, R^{4B}, R^{4C}, R² and R³ represents any combination described in the combination A (hereinafter, referred to as "compound class SX169").

The compound (A-10) wherein n is 1, R⁵ represents a chlorine atom, R⁶ represents a hydrogen atom, and the combination of R¹, R^{4A}, R^{4B}, R^{4C}, R² and R³ represents any combination described in the combination A (hereinafter, referred to as "compound class SX170").

The compound (A-10) wherein n is 2, R⁵ represents a chlorine atom, R⁶ represents a hydrogen atom, and the combination of R¹, R^{4A}, R^{4B}, R^{4C}, R² and R³ represents any combination described in the combination A (hereinafter, referred to as "compound class SX171").

The compound (A-10) wherein n is 0, R⁶ represents a chlorine atom, R⁵ represents a hydrogen atom, and the combination of R¹, R^{4A}, R^{4B}, R^{4C}, R² and R³ represents any combination described in the combination A (hereinafter, referred to as "compound class SX172").

The compound (A-10) wherein n is 1, R⁶ represents a chlorine atom, R⁵ represents a hydrogen atom, and the combination of R¹, R^{4A}, R^{4B}, R^{4C}, R² and R³ represents any combination described in the combination A (hereinafter, referred to as "compound class SX173").

The compound (A-10) wherein n is 2, R⁶ represents a chlorine atom, R⁵ represents a hydrogen atom, and the combination of R¹, R^{4A}, R^{4B}, R^{4C}, R² and R³ represents any combination described in the combination A (hereinafter, referred to as "compound class SX174").

The compound represented by formula (B-1): 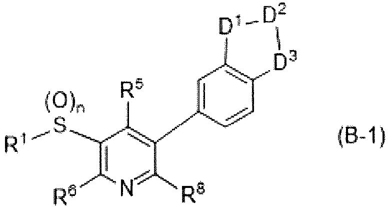 (hereinafter, referred to as "compound (B-1)") wherein n is 0, each of R⁵, R⁶ and R⁸ represents a hydrogen atom, and the combination of R¹, D¹, D², and D³ represents any combination described in the combination B (hereinafter, referred to as "compound class SX175").

The combination B consists of the substituent group Nos. B1 to B352. The substituent group Nos B1 to B352 represents the combination of R¹, D¹, D² and D³ in the compound represented by formula (B-1) and the compound represented by formula (B-2) [hereinafter, referres as [Substitution Group No.: R¹, D¹, D², D³].

For example, the Substitution group No. B5 represents the combination wherein R¹ represents an ethyl group, D¹ represents C(CH₃)₂, and D² and D³ represents CH₂.

### Combination B:

[B1;Et,CH₂,CH₂,CH₂], [B2;Et,O,CH₂,O], [B3;Et,O,CF₂,O], [B4;Et,CH₂,O,CH₂], [B5;Et,C(CH₃)₂,CH₂,CH₂], [B6;Et,CH₂,CH₂,C(CH₃)₂], [B7;Et,C(CH₃)₂,CH₂,CH(CH₃)], [B8;Et,CH(CH₃),CH₂,C(CH₃)₂], [B9;CH=CH₂,CH₂,CH₂,CH₂], [B10;CH=CH₂,O,CH₂,O], [B11;CH=CH₂,O,CF₂,O], [B12;CH=CH₂,CH₂,O,CH₂], [B13;CH=CH₂,C(CH₃)₂,CH₂,CH₂], [B14;CH=CH₂,CH₂,CH₂,C(CH₃)₂], [B15;CH=CH₂,C(CH₃)₂,CH₂,CH(CH₃)], [B16;CH=CH₂,CH(CH₃),CH₂,C(CH₃)₂], [B17;Pr,CH₂,CH₂,CH₂], [B18;Pr,O,CH₂,O], [B19;Pr,O,CF₂,O], [B20;Pr,CH₂,O,CH₂], [B21;Pr,C(CH₃)₂,CH₂,CH₂], [B22;Pr,CH₂,CH₂,C(CH₃)₂], [B23;Pr,C(CH₃)₂,CH₂,CH(CH₃)], [B24;Pr,CH(CH₃),CH₂,C(CH₃)₂], [B25;CH=CHCH₃,CH₂,CH₂,CH₂], [B26;CH=CHCH₃,O,CH₂,O], [B27;CH=CHCH₃,O,CF₂,O], [B28;CH=CHCH₃,CH₂,O,CH₂], [B29;CH=CHCH₃,C(CH₃)₂,CH₂,CH₂], [B30;CH=CHCH₃,CH₂,CH₂,C(CH₃)₂], [B31;CH=CHCH₃,C(CH₃)₂,CH₂,CH(CH₃)], [B32;CH=CHCH₃,CH(CH₃),CH₂,C(CH₃)₂], [B33;CH₂CH=CH₂,CH₂,CH₂,CH₂], [B34;CH₂CH=CH₂,O,CH₂,O], [B35;CH₂CH=CH₂,O,CF₂,O], [B36;CH₂CH=CH₂,CH₂,O,CH₂], [B37;CH₂CH=CH₂,C(CH₃)₂,CH₂,CH₂], [B38;CH₂CH=CH₂,CH₂,CH₂,C(CH₃)₂], [B39;CH₂CH=CH₂,C(CH₃)₂,CH₂,CH(CH₃)], [B40;CH₂CH=CH₂,CH(CH₃),CH₂,C(CH₃)₂], [B41;i-Pr,CH₂,CH₂,CH₂], [B42;i-Pr,O,CH₂,O], [B43;i-Pr,O,CF₂,O], [B44;i-Pr,CH₂,O,CH₂], [B45;i-Pr,C(CH₃)₂,CH₂,CH₂], [B46;i-Pr,CH₂,CH₂,C(CH₃)₂], [B47;i-Pr,C(CH₃)₂,CH₂,CH(CH₃)], [B48;i-Pr,CH(CH₃),CH₂,C(CH₃)₂], [B49;Bu,CH₂,CH₂,CH₂], [B50;Bu,O,CH₂,O], [B51;Bu,O,CF₂,O], [B52;Bu,CH₂,O,CH₂], [B53;Bu,C(CH₃)₂,CH₂,CH₂], [B54;Bu,CH₂,CH₂,C(CH₃)₂], [B55;Bu,C(CH₃)₂,CH₂,CH(CH₃)], [B56;Bu,CH(CH₃),CH₂,C(CH₃)2], [B57;i-Bu,CH₂,CH₂,CH₂], [B58;i-Bu,O,CH₂,O], [B59;i-Bu,O,CF₂,O], [B60;i-Bu,CH₂,O,CH₂], [B61;i-Bu,C(CH₃)₂,CH₂,CH₂], [B62;i-Bu,CH₂,CH₂,C(CH₃)₂], [B63;i-Bu,C(CH₃)₂,CH₂,CH(CH₃)], [B64;i-Bu,CH(CH₃),CH₂,C(CH₃)₂], [B65;s-Bu,CH₂,CH₂,CH₂], [B66;s-Bu,O,CH₂,O], [B67;s-Bu,O,CF₂,O], [B68;s-Bu,CH₂,O,CH₂], [B69;s-Bu,C(CH₃)₂,CH₂,CH₂], [B70;s-Bu,CH₂,CH₂,C(CH₃)₂], [B71;s-Bu,C(CH₃)₂,CH₂,CH(CH₃)], [B72;s-Bu,CH(CH₃),CH₂,C(CH₃)₂], [B73;c-Pr,CH₂,CH₂,CH₂], [B74;c-Pr,O,CH₂,O], [B75;c-Pr,O,CF₂,O], [B76;c-Pr,CH₂,O,CH₂], [B77;c-Pr,C(CH₃)₂,CH₂,CH₂], [B78;c-Pr,CH₂,CH₂,C(CH₃)₂], [B79;c-Pr,C(CH₃)₂,CH₂,CH(CH₃)], [B80;c-Pr,CH(CH₃),CH₂,C(CH₃)₂], [B81;CH₂(c-Pr),CH₂,CH₂,CH₂], [B82;CH₂(c-Pr),O,CH₂,O], [B83;CH₂(c-Pr),O,CF₂,O], [B84;CH₂(c-Pr),CH₂,O,CH₂], [B85;CH₂(c-Pr),C(CH₃)₂,CH₂,CH₂], [B86;CH₂(c-Pr),CH₂,CH₂,C(CH₃)₂], [B87;CH₂(c-Pr),C(CH₃)₂,CH₂,CH(CH₃)], [B88;CH₂(c-Pr),CH(CH₃),CH₂,C(CH₃)₂], [B89;Bn,CH₂,CH₂,CH₂], [B90;Bn,O,CH₂,O], [B91;Bn,O,CF₂,O], [B92;Bn,CH₂,O,CH₂], [B93;Bn,C(CH₃)₂,CH₂,CH₂], [B94;Bn,CH₂,CH₂,C(CH₃)₂], [B95;Bn,C(CH₃)₂,CH₂,CH(CH₃)], [B96;Bn,CH(CH₃),CH₂,C(CH₃)₂], [B97;Me,CH₂,CH₂,CH₂], [B98;e,O,CH₂,O], [B99;Me,O,CF₂,O], [B100;Me,CH₂,O,CH₂], [B101;Me,C(CH₃)₂,CH₂,CH₂], [B102;Me,CH₂,CH₂,C(CH₃)₂], [B103;Me,C(CH₃)₂,CH₂,CH(CH₃)], [B104;Me,CH(CH₃),CH₂,C(CH₃)₂], [B105;Ph,CH₂,CH₂,CH₂], [B106;Ph,O,CH₂,O], [B107;Ph,O,CF₂,O], [B108;Ph,CH₂,O,CH₂], [B109;Ph,C(CH₃)₂,CH₂,CH₂], [B110;Ph,CH₂,CH₂,C(CH₃)₂], [B111;Ph,C(CH₃)₂,CH₂,CH(CH₃)], [B112;Ph,CH(CH₃),CH₂,C(CH3)₂], [B113;2-F-Ph,CH₂,CH₂,CH₂], [B114;2-F-Ph,O,CH₂,O], [B115;2-F-Ph,O,CF₂,O], [B116;2-F-Ph,CH₂,O,CH₂], [B117;2-F-Ph,C(CH₃)₂,CH₂,CH₃], [B118;2-F-Ph,CH₂,CH₂,C(CH₃)₂], [B119;2-F-Ph,C(CH₃)2,CH₂,CH(CH₃)], [B120;2-F-Ph,CH(CH₃),CH₂,C(CH₃)₂], [B121;3-F-Ph,CH₂,CH₂,CH₂], [B122;3-F-Ph,O,CH₂,O], [B123;3-F-Ph,O,CF₂,O], [B124;3-F-Ph,CH₂,O,CH₂], [B125;3-F-Ph,C(CH₃)₂,CH₂,CH₂] , [B126;3-F-Ph,CH₂,CH₂,C(CH₃)₂], [B127;3-F-Ph,C(CH₃)₂,CH₂,CH(CH₃)], [B128;3-F-Ph,CH(CH₃),CH₂,C(CH₃)₂], [B129;4-F-Ph,CH₂,CH₂,CH₂], [B130;4-F-Ph,O,CH₂,O], [B131;4-F-Ph,O,CF₂,O], [B132;4-F-Ph,CH₂,O,CH₂], [B133;4-F-Ph,C(CH₃)₂,CH₂,CH₂], [B134;4-F-Ph,CH₂,CH₂,C(CH₃)₂], [B135;4-F-Ph,C(CH₃)₂,CH₂,CH(CH₃)], [B136;4-F-Ph,CH(CH₃),CH₂,C(CH₃)₂], [B137;2-Cl-Ph,CH₂,CH₂,CH₂], [B138;2-Cl-Ph,O,CH₂,O], [B139;2-Cl-Ph,O,CF₂,O], [B140;2-Cl-Ph,CH₂,O,CH₂], [B141;2-Cl-Ph,C(CH₃)₂,CH₂,CH₂], [B142;2-C1-Ph,CH₂,CH₂,C(CH₃)₂], [B143;2-Cl-Ph,C(CH₃)₂,CH₂,CH(CH₃)], [B144;2-Cl-Ph,CH(CH₃),CH₂,C(CH₃)₂], [B145;3-Cl-Ph,CH₂,CH₂,CH₂], [B146;3-Cl-Ph,O,CH₂,O], [B147;3-Cl-Ph,O,CF₂,O], [B148;3-Cl-Ph,CH₂,O,CH₂], [B149;3-Cl-Ph,C(CH₃)₂,CH₂,CH₂], [B150;3-Cl-Ph,C₂,CH₂,C(CH₃)₂], [B151;3-Cl-Ph,C(CH₃)₂,CH₂,CH(CH₃)], [B152;3-Cl-Ph,CH(CH₃),CH₂,C(CH₃)₂], [B153;4-Cl-Ph,CH₂,CH₂,CH₂], [B154;4-Cl-Ph,O,CH₂,O], [B155;4-Cl-Ph,O,CF₂,O], [B156;4-Cl-Ph,CH₂,O,CH₂], [B157;4-Cl-Ph,C(CH₃)₂,CH₂,CH₂], [B158;4-Cl-Ph,CH₂,CH₂,C(CH₃)₂], [B159;4-Cl-Ph,C(CH₃)₂,CH₂,CH(CH₃)], [B160;4-Cl-Ph,CH(CH₃),CH₂,C(CH₃)₂], [B161;2-Me-Ph,CH₂,CH₂,CH₂], [B162;2-Me-Ph,O,CH₂,O], [B163;2-Me-Ph,O,CF₂,O], [B164;2-Me-Ph,CH₂,O,CH_{2]}, [B165;2-Me-Ph,C(CH₃)₂,CH₂,CH₂], [B166;2-Me-Ph,CH₂,CH₂,C(CH₃)₂], [B167;2-Me-Ph,C(CH₃)₂,CH₂,CH(CH₃)], [B168;2-Me-Ph,CH(CH₃),CH₂,C(CH₃)₂], [B169;3-Me-Ph,CH₂,CH₂,CH₂], [B170;3-Me-Ph,O,CH₂,O], [B171;3-Me-Ph,O,CF₂,O], [B172;3-Me-Ph,CH₂,O,CH₂], [B173;3-Me-Ph,C(CH₃)₂,CH₂,CH₂], [B174;3-Me-Ph,CH₂,CH₂,C(CH₃)₂], [B175;3-Me-Ph,C(CH₃)₂,CH₂,CH(CH₃)], [B176;3-Me-Ph,CH(CH₃),CH₂,C(CH₃)₂], [B177;4-Me-Ph,CH₂,CH₂,CH₂], [B178;4-Me-Ph,O,CH₂,O], [B179;4-Me-Ph,O,CF₂,O], [B180;4-Me-Ph,CH₂,O,CH₂], [B181;4-Me-Ph,C(CH₃)₂,CH₂,CH₂], [B182;4-Me-Ph,CH₂,CH₂,C(CH₃)₂], [B183;4-Me-Ph,C(CH₃)₂,CH2,CH(CH₃)], [B184;4-Me-Ph,CH(CH₃),CH₂,C(CH₃)₂], [B185;NMe₂,CH₂,CH₂,CH₂], [B186;NMe₂,O,CH₂,O], [B187;NMe₂,O,CF₂,O], [B188;NMe₂,CH₂,O,CH₂], [B189;NMe₂,C(CH₃)₂,CH₂,CH₂], [B190;NMe₂,CH₂,CH₂,C(CH₃)₂], [B191;NMe₂,C(CH₃)₂,CH₂,CH(CH₃) [B192;NMe₂,CH(CH₃),CH₂,C(CH₃)₂], [B193;NHMe,CH₂,CH₂,CH₂], [B194;NHMe,O,CH₂,O], [B195;NHMe,O,CF₂,O], [B196;NHMe,CH₂,O,CH₂], [B197;NHMe,C(CH₃)₂,CH₂,CH₂], [B198;NHMe,CH₂,CH₂,C(CH₃)₂], [B199;NHMe,C(CH₃)₂,CH₂,CH(CH₃)], [B200;NHMe,CH(CH₃),CH₂,C(CH₃)₂], [B201;pyrrolizin-1-yl,CH₂,CH₂,CH₂], [B202;pyrrolizin-1-yl,O,CH₂,O], [B203;pyrrolizin-1-yl,O,CF₂,O], [B204;pyrrolizin-1-yl,CH₂,O,CH₂], [B205;pyrrolizin-1-yl,C(CH₃)₂,CH₂,CH₂], [B206;pyrrolizin-1-yl,CH₂,CH₂,C(CH₃)₂], [B207;pyrrolizin-1-yl,C(CH₃)₂,CH₂,CH(CH₃)], [B208;pyrrolizin-1-yl,CH(CH₃),CH₂,C(CH₃)₂], [B209;azetidin-1-yl,CH₂,CH₂,CH₂], [B210;azetidin-1-yl,O,CH₂,O], [B211;azetidin-1-yl,O,CF₂,O], [B212;azetidin-1-yl,CH₂,O,CH₂], [B213;azetidin-1-yl,C(CH₃)₂,CH₂,CH₂], [B214;azetidin-1-yl,CH₂,CH₂,C(CH₃)₂], [B215;azetidin-1-yl,C(CH₃)₂,CH₂,CH(CH₃)], [B216;azetidin-1-yl,CH(CH₃),CH₂,C(CH₃)₂], [B217;piperidin-1-yl,CH₂,CH₂,CH₂], [B218;piperidin-1-yl,O,CH₂,O], [B219;piperidin-1-yl,O,CF₂,O], [B220;piperidin-1-yl,CH₂,O,CH₂], [B221;piperidin-1-yl,C(CH₃)₂,CH₂,CH₂], [B222;piperidin-1-yl,CH₂,CH₂,C(CH₃)₂], [B223;piperidin-1-yl,C(CH₃)₂,CH₂,CH(CH₃)], [B224;piperidin-1-yl,CH(CH₃),CH₂,C(CH₃)₂], [B225;pyridin-2-yl,CH₂,CH₂,CH₂], [B226;pyridin-2-yl,O,CH₂,O], [B227;pyridin-2-yl,O,CF₂,O], [B228;pyridin-2-yl,CH₂,O,CH₂], [B229;pyridin-2-yl,C(CH₃)₂,CH₂,CH₂], [B230;pyridin-2-yl,CH₂,CH₂,C(CH₃)₂], [B231;pyridin-2-yl,C(CH₃)₂,CH₂,CH(CH₃)], [B232;pyridin-2-yl,CH(CH₃),CH_{2,}C(CH₃)₂], [B233;pyridin-3-yl,CH₂,CH₂,CH₂], [B234;pyridin-3-yl,O,CH₂,O], [B235;pyridin-3-yl,O,CF₂,O], [B236;pyridin-3-yl,CH₂,O,CH₂], [B237;pyridin-3-yl,C(CH₃)₂,CH₂,CH₂], [B238;pyridin-3-yl,CH₂,CH₂,C(CH₃)₂], [B239;pyridin-3-yl,C(CH₃)₂,CH₂,CH(CH₃)], [B240;pyridin-3-yl,CH(CH₃),CH₂,C(CH₃)₂], [B241;pyrimidin-4-yl,CH₂,CH₂,CH₂], [B242;pyrimidin-4-yl,O,CH₂,O], [B243;pyrimidin-4-yl,O,CF₂,O], [B244;pyrimidin-4-yl,CH₂,O,CH₂], [B245;pyrimidin-4-yl,C(CH₃)₂,CH₂,CH₂], [B246;pyrimidin-4-yl,CH₂,CH₂,C(CH₃)₂], [B247;pyrimidin-4-yl,C(CH₃)₂,CH₂,CH(CH₃)], [B248;pyrimidin-4-yl,CH(CH₃),CH₂,C(CH₃)₂], [B249;pyrimidin-5-yl,CH₂,CH₂,CH₂], [B250;pyrimidin-5-yl,O,CH₂,O], [B251;pyrimidin-5-yl,O,CF₂,O], [B252;pyrimidin-5-yl,CH₂,O,CH₂], [B253;pyrimidin-5-yl,C(CH₃)₂,CH₂,CH₂], [B254;pyrimidin-5-yl,CH₂,CH₂,C(CH₃)₂], [B255;pyrimidin-5-yl,C(CH₃)₂,CH₂,CH(CH₂)], [B256;pyrimidin-5-yl,CH(CH₃),CH₂,C(CH₃)₂], [B257;pyridazin-3-yl,CH₂,CH₂,CH₂], [B258;pyridazin-3-yl,O,CH₂,O], [B259;pyridazin-3-yl,O,CF₂,O], [B260;pyridazin-3-yl,CH₂,O,CH₂], [B261;pyridazin-3-yl,C(CH₃)₂,CH₂,CH₂], [B262;pyridazin-3-yl,CH₂,CH₂,C(CH₃)₂], [B263;pyridazin-3-yl,C(CH₃)₂,CH₂,CH(CH₃)], [B264;pyridazin-3-yl,CH(CH₃),CH₂,C(CH₃)₂], [B265;pyridazin-4-yl,CH₂,CH₂,CH₂], [B266;pyridazin-4-yl,O,CH₂,O], [B267;pyridazin-4-yl,O,CF₂,O], [B268;pyridazin-4-yl,CH₂,O,CH₂], [B269;pyridazin-4-yl,C(CH₃)₂,CH₂,CH₂], [B270;pyridazin-4-yl,CH₂,CH₂,C(CH₃)₂], [B271;pyridazin-4-yl,C(CH₃)₂,CH₂,CH(CH₃)], [B272;pyridazin-4-yl,CH(CH₃),CH₂,C(CH₃)₂], [B273;pyrazin-2-yl,CH₂,CH₂,CH₂], [B274;pyrazin-2-yl,O,CH₂,O], [B275;pyrazin-2-yl,O,CF₂,O], [B276;pyrazin-2-yl,CH₂,O,CH₂], [B277;pyrazin-2-yl,C(CH₃)₂,CH₂,CH₂], [B278;pyrazin-2-y1,CH₂,CH₂,C(CH₃)₂], [B279;pyrazin-2-yl,C(CH₃)₂,CH₂,CH(CH₃)], [B280;pyrazin-2-yl,CH(CH₃),CH₂,C(CH₃)₂], [B281;furan-2-yl,CH₂,CH₂,CH₂], [B282;furan-2-yl,O,CH₂,O], [B283;furan-2-yl,O,CF₂,O], [B284;furan-2-yl,CH₂,O,CH₂], [B285;furan-2-yl,C(CH₃)2₂,CH₂,CH₂], [B286;furan-2-yl,CH₂,CH₂,C(CH₃)₂], [B287;furan-2-yl,C(CH₃)₂,CH₂,CH(CH₃)], [B288;furan-2-yl,CH(CH₃),CH₂,C(CH₃)₂], [B289;furan-3-yl,CH₂,CH₂,CH₂], [B290;furan-3-yl,O,CH₂,O], [B291;furan-3-yl,O,CF₂,O], [B292;furan-3-yl,CH₂,O,CH_{2]}, [B293;furan-3-yl,C(CH₃)₂,CH₂,CH₂], [B294;furan-3-yl,CH₂,CH₂,C(CH₃)₂ [B295;furan-3-yl,C(CH₃)₂,CH₂,CH(CH₃)], [B296;furan-3-yl,CH(CH₃),CH₂,C(CH₃)₂], [B297;thien-2-yl,CH₂,CH₂,CH₂], [B298;thien-2-yl,O,CH₂,O], [B299;thien-2-yl,O,CF₂,O], [B300;thien-2-yl,CH₂,O,CH₂], [B301;thien-2-yl,C(CH₃)₂,CH₂,CH₂], [B302;thien-2-yl,CH₂,CH₂,C(CH₃)₂], [B303;thien-2-yl,C(CH₃)₂,CH₂,CH(CH₃)], [B304;thien-2-yl,CH(CH₃),CH₂,C(CH₃)₂], [B305;thien-3-yl,CH₂,CH₂,CH₂], [B306;thien-3-yl,O,CH₂,O], [B307;thien-3-yl,O,CF₂,O], [B308;thien-3-yl,CH₂,O,CH₂], [B309;thien-3-yl,C(CH₃)₂,CH₂,CH₂], [B310;thien-3-yl,CH₂,CH₂,C(CH₃)₂], [B311;thien-3-yl,C(CH₃)₂,CH₂,CH(CH₃)], [B312;thien-3-yl,CH(CH₃),CH₂,C(CH₃)₂], [B313;1,3-oxazol-2-yl,CH₂,CH₂,CH₂], [B314;1,3-oxazol-2-yl,O,CH₂,O], [B315;1,3-oxazol-2-yl,O,CF₂,O], [B316;1,3-oxazol-2-yl,CH₂,O,CH₂], [B317;1,3-oxazol-2-yl,C(CH₃)₂,CH₂,CH₂], [B318;1,3-oxazol-2-yl,CH₂,CH₂,C(CH₃)₂], [B319;1,3-oxazol-2-yl,C(CH₃)₂,CH₂,CH(CH₃)], [B320;1,3-oxazol-2-yl,CH(CH₃),CH₂,C(CH₃)₂], [B321;1,3-thiazol-2-yl,CH₂,CH₂,CH₂], [B322;1,3-thiazol-2-yl,O,CH₂,O], [B323;1,3-thiazol-2-yl,O,CF₂,O], [B324;1,3-thiazol-2-yl,CH₂,O,CH₂], [B325;1,3-thiazol-2-yl,C(CH₃)₂,CH₂,CH₂], [B326;1,3-thiazol-2-yl,CH₂,CH₂,C(CH₃)₂], [B327;1,3-thiazol-2-yl,C(CH₃)₂,CH₂,CH(CH₃)], [B328;1,3-thiazol-2-yl,CH(CH₃),CH₂,C(CH₃)₂], [B329;1H-pyrazol-1-yl,CH₂,CH₂,CH₂], [B330;1H-pyrazol-1-yl,O,CH₂,O], [B331;1H-pyrazol-1-yl,O,CF₂,O], [B332;1H-pyrazol-1-yl,CH₂,O,CH₂], [B333;1H-pyrazol-1-yl,C(CH₃)₂,CH₂,CH₂], [B334;1H-pyrazol-1-yl,CH₂,CH₂,C(CH₃)₂], [B335;1H-pyrazol-1-yl,C(CH₃)₂,CH₂,CH(CH₃)], [B336;1H-pyrazol-1-yl,CH(CH₃),CH₂,C(CH₃)₂], [B337;1-methyl-1H-pyazol-3-yl,CH₂,CH₂,CH₂], [B338;1-methyl-1H-pyazol-3-yl,O,CH₂,O], [B339;1-methyl-1H-pyazol-3-yl,O,CF₂,O], [B340;1-methyl-1H-pyazol-3-yl,CH₂,O,CH₂], [B341;1-methyl-1H-pyazol-3-yl,C(CH₃)₂,CH₂,CH₂], [B342;1-methyl-1H-pyazol-3-yl,CH₂,CH₂,C(CH₃)₂, [B343;1-methyl-1H-pyazol-3-yl,C(CH₃)₂,CH₂,CH(CH₃)], [B344;1-methyl-1H-pyazol-3-yl,CH(CH₃),CH₂,C(CH₃)₂], [B345;1H-pyrrol-1-yl,CH₂,CH₂,CH₂], [B346;1H-pyrrol-1-yl,O,CH₂,O], [B347;1H-pyrrol-1-yl,O,CF₂,O], [B348;1H-pyrrol-1-yl,CH₂,O,CH₂], [B349;1H-pyrrol-1-yl,C(CH₃)₂,CH₂,CH₂], [B350;1H-pyrrol-1-yl,CH₂,CH₂,C(CH₃)₂], [B351;1H-pyrrol-1-yl,C(CH₃)₂,CH₂,CH(CH₃)], [B352;1H-pyrrol-1-yl,CH(CH₃),CH₂,C(CH₃)₂].

The compound (B-1) wherein n is 1, each of R⁵, R⁶ and R⁸ represents a hydrogen atom, and the combination of R¹, D¹, D² and D³ represents any combination described in the combination B (hereinafter, referred to as "compound class SX176").

The compound (B-1) wherein n is 2, each of R⁵, R⁶ and R⁸ represents a hydrogen atom, and the combination of R¹, D¹, D² and D³ represents any combination described in the combination B (hereinafter, referred to as "compound class SX177"). The compound (B-1) wherein n is 0, R⁵ represents a methyl group, each of R⁶ and R⁸ represents a hydrogen atom, and the combination of R¹, D¹, D² and D³ represents any combination described in the combination B (hereinafter, referred to as "compound class SX178").

The compound (B-1) wherein n is 1, R⁵ represents a methyl group, each of R⁶ and R⁸ represents a hydrogen atom, and the combination of R¹, D¹, D² and D³ represents any combination described in the combination B (hereinafter, referred to as "compound class SX179").

The compound (B-1) wherein n is 2, R⁵ represents a methyl group, each of R⁶ and R⁸ represents a hydrogen atom, and the combination of R¹, D¹, D² and D³ represents any combination described in the combination B (hereinafter, referred to as "compound class SX180").

The compound (B-1) wherein n is 0, R⁵ represents a methyl group, each of R⁶ and R⁸ represents a hydrogen atom, and the combination of R¹, D¹, D² and D³ represents any combination described in the combination B (hereinafter, referred to as "compound class SX181").

The compound (B-1) wherein n is 1, R⁶ represents a methyl group, each of R⁵ and R⁸ represents a hydrogen atom, and the combination of R¹, D¹, D² and D³ represents any combination described in the combination B (hereinafter, referred to as "compound class SX182").

The compound (B-1) wherein n is 2, R⁶ represents a methyl group, each of R⁵ and R⁸ represents a hydrogen atom, and the combination of R¹, D¹, D² and D³ represents any combination described in the combination B (hereinafter, referred to as "compound class SX183").

The compound (B-1) wherein n is 0, R⁸ represents a methyl group, each of R⁵ and R⁶ represents a hydrogen atom, and the combination of R¹, D¹, D² and D³ represents any combination described in the combination B (hereinafter, referred to as "compound class SX184").

The compound (B-1) wherein n is 1, R⁸ represents a methyl group, each of R⁵ and R⁶ represents a hydrogen atom, and the combination of R¹, D¹, D² and D³ represents any combination described in the combination B (hereinafter, referred to as "compound class SX185").

The compound (B-1) wherein n is 2, R⁸ represents a methyl group, each of R⁵ and R⁶ represents a hydrogen atom, and the combination of R¹, D¹, D² and D³ represents any combination described in the combination B (hereinafter, referred to as "compound class SX186").

The compound (B-1) wherein n is 0, R⁵ represents a chlorine atom, each of R⁶ and R⁸ represents a hydrogen atom, and the combination of R¹, D¹, D² and D³ represents any combination described in the combination B (hereinafter, referred to as "compound class SX187").

The compound (B-1) wherein n is 1, R⁵ represents a chlorine atom, each of R⁶ and R⁸ represents a hydrogen atom, and the combination of R¹, D¹, D² and D³ represents any combination described in the combination B (hereinafter, referred to as "compound class SX188").

The compound (B-1) wherein n is 2, R⁵ represents a chlorine atom, each of R⁶ and R⁸ represents a hydrogen atom, and the combination of R¹, D¹, D² and D³ represents any combination described in the combination B (hereinafter, referred to as "compound class SX189").

The compound (B-1) wherein n is 0, R⁶ represents a chlorine atom, each of R⁵ and R⁸ represents a hydrogen atom, and the combination of R¹, D¹, D² and D³ represents any combination described in the combination B (hereinafter, referred to as "compound class SX190").

The compound (B-1) wherein n is 1, R⁶ represents a chlorine atom, each of R⁵ and R⁸ represents a hydrogen atom, and the combination of R¹, D¹, D² and D³ represents any combination described in the combination B (hereinafter, referred to as "compound class SX191").

The compound (B-1) wherein n is 2, R⁶ represents a chlorine atom, each of R⁵ and R⁸ represents a hydrogen atom, and the combination of R¹, D¹, D² and D³ represents any combination described in the combination B (hereinafter, referred to as "compound class SX192").

The compound (B-1) wherein n is 0, R⁸ represents a chlorine atom, each of R⁵ and R⁶ represents a hydrogen atom, and the combination of R¹, D¹, D² and D³ represents any combination described in the combination B (hereinafter, referred to as "compound class SX193").

The compound (B-1) wherein n is 1, R⁸ represents a chlorine atom, each of R⁵ and R⁶ represents a hydrogen atom, and the combination of R¹, D¹, D² and D³ represents any combination described in the combination B (hereinafter, referred to as "compound class SX194").

The compound (B-1) wherein n is 2, R⁸ represents a chlorine atom, each of R⁵ and R⁶ represents a hydrogen atom, and the combination of R¹, D¹, D² and D³ represents any combination described in the combination B (hereinafter, referred to as "compound class SX195").

The compound represented by formula (B-2): 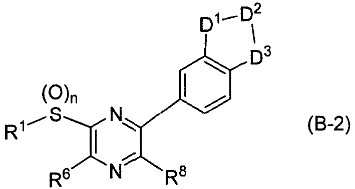 (hereinafter, referred to as "compound (B-2)") wherein n is 0, each of R⁶ and R⁸ represents a hydrogen atom, and the combination of R¹, D¹, D², and D³ represents any combination described in the combination B (hereinafter, referred to as "compound class SX196").

The compound (B-2) wherein n is 1, each of R⁶ and R⁸ represents a hydrogen atom, and the combination of R¹, D¹, D² and D³ represents any combination described in the combination B (hereinafter, referred to as "compound class SX197").

The compound (B-2) wherein n is 2, each of R⁶ and R⁸ represents a hydrogen atom, and the combination of R¹, D¹, D² and D³ represents any combination described in the combination B (hereinafter, referred to as "compound class SX198").

The compound (B-2) wherein n is 0, R⁶ represents a methyl group, R⁸ represents a hydrogen atom, and the combination of R¹, D¹, D² and D³ represents any combination described in the combination B (hereinafter, referred to as "compound class SX199").

The compound (B-2) wherein n is 1, R⁶ represents a methyl group, R⁸ represents a hydrogen atom, and the combination of R¹, D¹, D² and D³ represents any combination described in the combination B (hereinafter, referred to as "compound class SX200") .

The compound (B-2) wherein n is 2, R⁶ represents a methyl group, R⁸ represents a hydrogen atom, and the combination of R¹, D¹, D² and D³ represents any combination described in the combination B (hereinafter, referred to as "compound class SX201").

The compound (B-2) wherein n is 0, R⁸ represents a methyl group, R⁶ represents a hydrogen atom, and the combination of R¹, D¹, D² and D³ represents any combination described in the combination B (hereinafter, referred to as "compound class SX202").

The compound (B-2) wherein n is 1, R⁸ represents a methyl group, R⁶ represents a hydrogen atom, and the combination of R¹, D¹, D² and D³ represents any combination described in the combination B (hereinafter, referred to as "compound class SX203").

The compound (B-2) wherein n is 2, R⁸ represents a methyl group, R⁶ represents a hydrogen atom, and the combination of R¹, D¹, D² and D³ represents any combination described in the combination B (hereinafter, referred to as "compound class SX204").

The compound (B-2) wherein n is 0, R⁶ represents a chlorine atom, R⁸ represents a hydrogen atom, and the combination of R¹, D¹, D² and D³ represents any combination described in the combination B (hereinafter, referred to as "compound class SX205").

The compound (B-2) wherein n is 1, R⁶ represents a chlorine atom, R⁸ represents a hydrogen atom, and the combination of R¹, D¹, D² and D³ represents any combination described in the combination B (hereinafter, referred to as "compound class SX206").

The compound (B-2) wherein n is 2, R⁶ represents a chlorine atom, R⁸ represents a hydrogen atom, and the combination of R¹, D¹, D² and D³ represents any combination described in the combination B (hereinafter, referred to as "compound class SX207").

The compound (B-2) wherein n is 0, R⁸ represents a chlorine atom, R⁶ represents a hydrogen atom, and the combination of R¹, D¹, D² and D³ represents any combination described in the combination B (hereinafter, referred to as "compound class SX208").

The compound (B-2) wherein n is 1, R⁸ represents a chlorine atom, R⁶ represents a hydrogen atom, and the combination of R¹, D¹, D² and D³ represents any combination described in the combination B (hereinafter, referred to as "compound class SX209").

The compound (B-2) wherein n is 1, R⁸ represents a chlorine atom, R⁶ represents a hydrogen atom, and the combination of R¹, D¹, D² and D³ represents any combination described in the combination B (hereinafter, referred to as "compound class SX210").

Next, the Formulation Examples are shown below. The "parts" represents "part by weight" unless otherwise specified. Also "Present compound S" represents any compound described in the compound class SX1 to SX210.

### Formulation Example 1

Thirty five (35) parts of a mixture of ammonium polyoxyethylene alkyl ether sulfate and wet silica (weight ratio: 1:1), 10 parts of any one of the present compound S, and 55 parts of water are mixed, and the mixture is then finely-ground by a wet grinding method to obtain a formulation.

### Formulation Example 2

Fifty (50) parts of any one of the present compound S, 3 parts of calcium lignin sulfonate, 2 parts of sodium lauryl sulfate, and 45 parts of silica are well mixed-grinding to obtain a formulation.

### Formulation Example 3

Five (5) parts of any one of the present compound S, 9 parts of polyoxyethylene styryl phenyl ether, 5 parts of polyoxyethylene decyl ether (Number of ethylene oxide additions: 5), 6 parts of calcium dodecylbenzene sulfonate and 75 parts of xylene are well mixed to obtain a formulation.

### Formulation Example 4

Two (2) parts of any one of the present compound S, 1 part of silica, 2 parts of calcium lignin sulfonate, 30 parts of bentonite and 65 parts of kaolin clay are mixed-grinding, and thereto is added an appropriate amount of water, and the mixture is well kneaded and is then granulated with a granulator and dried to obtain a formulation.

### Formulation Example 5

Ten (10) parts of any one of the present compound S is mixed with a mixture of 18 parts of benzyl alcohol and 9 parts of DMSO, and thereto are added 6.3 parts of GERONOL (registered trademark) TE250, 2.7 parts of Ethylan (registered trademark) NS-500LQ, and 54 parts of Solvent naphtha, and the mixture is mixed to obtain a formulation.

Next, the test examples are shown below.

The term of "untreated well" in the Test Examples 1, 1-2, 2, 3, 5 and 6 respectively represents a test area in which the procedures are carried out under the same conditions as those described in each of Test Examples except that DMSO is dispensed in place of a diluted DMSO solution containing the Present compound. The term of "untreated leaf disk" in the Test Examples 7 and 7-2 represents a leaf disk in which the procedures were carried out under the same conditions as those described in the Test Example except that DMSO was dispensed in place of a diluted DMSO solution containing the present compound. Also, the term of "untreated" in the Test Examples 10, 11, 11-2, and 15 respectively means that a diluted solution of the formulation comprising the present compound with water was not sprayed to foliage. The term of "untreated" in the Test Example 16 represents a test in which the procedures were carried out under the same conditions as those described in the Test Example except that DMSO was dispensed in place of a diluted DMSO solution containing the present compound.

### Test Example 1: Control test against wheat leaf blotch fungus (Septoria tritici)

The present compound 18 was diluted with DMSO so as to contain 150 ppm, and 1 µL of the dilution solutions were dispensed into titer plate (96 well), and thereafter, thereto was then dispensed 150 µL of YBG medium to which spores of Septoria tritici were inoculated in advance. This plate was cultured at 18°C for 3 days, thereby allowing Septoria tritici to undergo proliferation, and the absorbance at 550 nm of each well of the titer plate was then measured to determine a degree of growth of Septoria tritici. As a result, the growth in the well in treated groups treated with the above present compound showed 50% or less compared to the growth in an untreated well.

### Test Example 1-2: Control test against wheat leaf blotch fungus (Septoria tritici)

The present compound 12 or 13 was diluted with DMSO so as to contain 1500 ppm, and 1 µL of the dilution solutions were dispensed into titer plate (96 well), and thereafter, thereto was then dispensed 150 µL of YBG medium to which spores of Septoria tritici were inoculated in advance. This plate was cultured at 18°C for 3 days, thereby allowing Septoria tritici to undergo proliferation, and the absorbance at 550 nm of each well of the titer plate was then measured to determine a degree of growth of Septoria tritici. As a result, the growth in the well in treated groups treated with each of the above present compound showed 50% or less compared to the growth in an untreated well.

### Test Example 2: Control test against Corn smut fungus (Ustilago maydis)

Each of the present compounds 18, 131, 153, 164, 165, or 167 was diluted with DMSO so as to contain 150 ppm, and 1 µL of the dilution solutions were dispensed into titer plate (96 well), and thereafter, thereto was then dispensed 150 µL of a potato dextrose broth (PDB broth) to which spores of Ustilago maydis were inoculated in advance. This plate was cultured at 18°C for 4 days, thereby allowing Ustilago maydis to undergo proliferation, and the absorbance at 550 nm of each well of the titer plate was then measured to determine a degree of growth of the Ustilago maydis. As a result, the growth in the well in treated groups treated with each of the above present compound showed 50% or less compared to the growth in an untreated well.

### Test Example 3: Control test against Barley scald fungus (Rhynchosporium secalis)

Each of the present compounds 18, 19 or 153 was diluted with DMSO so as to contain 150 ppm, and 1 µL of the dilution solutions were dispensed into titer plate (96 well), and thereafter, thereto was then dispensed 150 µL of a potato dextrose broth (PDB broth) to which spores of Rhynchosporium secalis were inoculated in advance. This plate was cultured at 18°C for 7 days, thereby allowing Rhynchosporium secalis to undergo proliferation, and the absorbance at 550 nm of each well of the titer plate was then measured to determine a degree of growth of the Rhynchosporium secalis. As a result, the growth in the well in treated groups treated with each of the above present compound showed 50% or less compared to the growth in an untreated well.

### Test Example 4: Control test against Cucumber botrytis rot fungus (Botrytis cinerea)

The present compound is diluted with DMSO so as to contain 150 ppm, and 1 µL of the dilution solutions are dispensed into titer plate (96 well), and thereafter, thereto is then dispensed 150 µL of complete medium to which spores of Botrytis cinerea are inoculated in advance. This plate is cultured at 18°C for 4 days, thereby allowing Botrytis cinerea to undergo proliferation, and the absorbance at 550 nm of each well of the titer plate is then measured to determine a degree of growth of the Botrytis cinerea. As a result, each of the present compounds shows an effect on controlling plant diseases.

### Test Example 5: Control test against Peach scab fungus (Cladosporium carpophilum)

The present compound 18 was diluted with DMSO so as to contain 150 ppm, and 1 µL of the dilution solutions were dispensed into titer plate (96 well), and thereafter, thereto was then dispensed 150 µL of a potato dextrose broth (PDB broth) to which spores of Cladosporium carpophilum were inoculated in advance. This plate was cultured at 18°C for 5 days, thereby allowing Cladosporium carpophilum to undergo proliferation, and the absorbance at 550 nm of each well of the titer plate was then measured to determine a degree of growth of the Cladosporium carpophilum. As a result, the growth in the well in treated groups treated with the above present compound showed 50% or less compared to the growth in an untreated well.

### Test Example 6: Control test against rice brown spot fungus (Cochliobolus miyabeanus)

Each of the present compound 18 or 192 was diluted with DMSO so as to contain 150 ppm, and 1 µL of the dilution solutions were dispensed into titer plate (96 well), and thereafter, thereto was then dispensed 150 µL of YB liquid medium to which spores of Cochliobolus miyabeanus were inoculated in advance. This plate was cultured at 23°C for 3 days, thereby allowing Cochliobolus miyabeanus to undergo proliferation, and the absorbance at 550 nm of each well of the titer plate was then measured to determine a degree of growth of the Cochliobolus miyabeanus. As a result, the growth in the well in treated groups treated with the above present compound showed 50% or less compared to the growth in an untreated well.

### Test Example 7: Control test against soybean rust (Phakopsora pachyrhizi)

Soybean leaf (cv; Kurosengoku) was punched out to 1 cm diameter to prepare a leaf disk. Each 1 mL of an agar medium (agar concentration 1.2 %) was dispensed in each well of 24 well microplate. A piece of the leaf disk was placed on agar medium on each well. To a mixture of 0.5 µL of Sorpol (registered trademark) 1200KX, 4.5 µL of DMSO, and 5 µL of xylene was added 20 µL of a solution containing 10000 ppm of the test compounds in DMSO. The resulting mixture was diluted with ion exchange water to prepare a mixture containing a predetermined concentration of the test compounds. The resulting mixture was sprayed in 10 µL per one leaf disk. After 1 day, an aqueous suspension of spores of *Phakopsora pachyrhizi* having an amino acid substitution of F129L on mitochondrial cytochrome b protein (1.0 × 10⁵/mL) was inoculated onto the leaf disks. After the inoculation, the microplate was placed in a growth chamber (light on for 6 hours, light off for 18 hours, 23°C temperature, 60% humidity). After 1 day, the leaf disks were air-dried to disappear water droplets on the surface of the leaf disk, and the microplate was placed again in the growth chamber for 12 days. Thereafter, a lesion area of soybean rust disease was assessed. As a result, lesion areas in the leaf disk treated with any one of the present compounds 3, 4, 6, 7, 8, 9, 10, 11, 12, 14, 15, 16, 17, 19, 20, 21, 22, 23, or 26 as a tested compound at a prescribed concentration of 50 ppm showed 30% or less compared to the lesion areas in an untreated leaf disk.

### Test Example 7-2: Control test against soybean rust (Phakopsora pachyrhizi)

The lesion areas in the leaf disk treated with any one of the present compounds 67, 69, 70, 71, 72, 73, 74, 75, 76, 77, 78, 79, 80, 81, 82, 83, 85, 87, 88, 89, 90, 91, 94, 96, 97, 98, 100, 101, 102, 103, 104, 105, 106, 107, 108, 109, 110, 111, 112, 113, 114, 116, 117, 118, 119, 121, 122, 123, 124, 125, 126, 127, 128, 129, 130, 131, 132, 133, 134, 136, 137, 140, 141, 142, 143, 144, 145, 146, 147, 148, 150, 151, 152, 153, 154, 155, 158, 160, 161, 162, 163, 164, 165, 166, 167, 168, 169, 170, 171, 172, 173, 174, 175, 176, 177, 178, 179, 180, 183, 184, 185, 186, 187, 188, 189, 190, 191, 192, 193, 194, 195, 196, 197, 198, or 199 as a tested compound at a prescribed concentration of 200 ppm according to the Test Example 7 showed 30% or less compared to the lesion areas in an untreated leaf disk.

### Test Example 8: Control test against barley net blotch (Pyrenophora teres)

Each of plastic pots is filled with soil and thereto barley (cv; NISHINOHOSHI) seeds are sown and the barleys are grown in a greenhouse for 7 days. Thereafter, the present compound each of which is made to a formulation according to the similar method to that of Formulation Example 1, is mixed with water so as to be a prescribed concentration (200 ppm). The resulting mixtures are sprayed to foliar parts so as to adhere adequately on the leaves of the above-mentioned barley. After spraying the mixtures, the barleys are air-dried and after 1 day, an aqueous suspension of the spores of Pyrenophora teres is spraying-inoculated. After the inoculation, the barleys are placed at 23°C during daytime and 20 °C during nighttime under a high humidity for 3 days and then cultivated in a greenhouse for 7 days, and a lesion area is observed. As a result, each of the present compounds shows an effect on controlling plant diseases.

### Test Example 9: Control test against wheat brown rust (Puccinia recondita)

Each of plastic pots is filled with soil and thereto wheat (cv; SHIROGANE) seeds are sown and the wheats are grown in a greenhouse for 9 days. The present compound each of which is made to a formulation according to the similar method to that of Formulation Example 1, is mixed with water so as to be 200 ppm, and the mixtures are sprayed to foliar parts so as to adhere adequately on the leaves of the above-mentioned wheat. After spraying the mixtures, the wheats are air-dried and are then cultivated at 20 °C under lighting for 5 to 7 days. The spores of Puccinia recondita are sprinkling-inoculated. After the inoculation, the wheats are placed under a dark and humid condition at 23 °C for 1 day and are then cultivated at 20 °C under lighting for 8 days, and a lesion area is observed. As a result, each of the present compounds shows an effect on controlling plant diseases.

### Test Example 10: Control test against septoria leaf blotch (Septoria tritici)

Each of plastic pots was filled with soil and thereto wheat (cv; Apogee) seeds were sown and the wheats were grown in a greenhouse for 10 days. Thereafter, the present compound 10, 13, 14, 15, 69, 89, 91, 93, 97, 111, 113, or 139 each of which was made to a formulation according to the similar method to that of Formulation Example 1, was mixed with water so as to be 200 ppm. The mixtures were sprayed to foliar parts so as to adhere adequately on the leaves of the above-mentioned wheat. After spraying the mixtures, the wheats were air-dried and after 4 days, an aqueous suspension of the spores of Septoria tritici was spraying-inoculated. After the inoculation, the wheats were placed at 18 °C under a high humidity for 3 days and then under lighting for 14 to 18 days, and a lesion area was observed. As a result, every of the lesion areas in wheats treated with each of the above present compounds showed 30% or less compared to the lesion area in an untreated wheat.

### Test Example 11: Control test against soybean rust (Phakopsora pachyrhizi)

Each of plastic pots was filled with soil and thereto soybean (cv: Kurosengoku) seeds were sown and the soybeans were grown in a greenhouse for 10 to 14 days. Thereafter, the present compound 2, 3, 4, 6, 7, 8,9, 10, 11, 12, 13, 14, 15, 16, 17, 18, 19, 20, 21, 22, 23, 26, 67, 69, 70, 71, 72, 73, 74, 75, 76, 77, 78, 79, 80, 81, 82, 83, 84, 85, 87, 88, 89, 90, 91, 93, 94, 96, 97, 98, 108, 109, 110, 111, 112, 113, 114, 116, 117, 118, 119, 121, 122, 123, 124, 125, 126, 127, 128, 129, 130, 131, 132, 133, 136, 137, 140, 141, 142, 143, 144, 145, 146, 149, 150, 151, 152, 153, 154, 158, 160, 161, 162, 164, 165, 166, 167, 168, 169, 170, 171, 172, 173, 174, 175, 176, 177, 178, 179, 180, 183, 184, 185, 186, 187, 188, 189, 190, 191, 192, 193, 194, 195, 196, 197, or 198 each of which was made to a formulation according to the similar method to that of Formulation Example 1, was mixed with water so as to be 200 ppm. The resulting mixtures were sprayed to foliar parts so as to adhere adequately on the leaves of the above-mentioned soybean. After spraying the mixtures, the soybeans were air-dried and after 2 to 5 days, an aqueous suspension of the spores of Phakopsora pachyrhizi was spraying-inoculated. After the inoculation, the soybeans were placed in a greenhouse of 23 °C during daytime and 20 °C during nighttime under a high humidity for 1 to 2 days, and were then cultivated in the greenhouse for 12 days, and a lesion area was observed. As a result, every of the lesion areas in soybean treated with each of the above present compounds showed 30% or less compared to the lesion area in an untreated soybean.

### Test Example 11-2: Control test against soybean rust (Phakopsora pachyrhizi)

Each of plastic pots was filled with soil and thereto soybean (cv: Kurosengoku) seeds were sown and the soybeans were grown in a greenhouse for 10 to 14 days. Thereafter, the present compound 100, 101, 102, 103, 104, 105, 106, 107, 134, 135, 155, 156, or 157 each of which was made to a formulation according to the similar method to that of Formulation Example 1, was mixed with water so as to be 50 ppm. The resulting mixtures were sprayed to foliar parts so as to adhere adequately on the leaves of the above-mentioned soybean. After spraying the mixtures, the soybeans were air-dried and after 2 to 5 days, an aqueous suspension of the spores of Phakopsora pachyrhizi was spraying-inoculated. After the inoculation, the soybeans were placed in a greenhouse of 23 °C during daytime and 20 °C during nighttime under a high humidity for 1 to 2 days, and were then cultivated in the greenhouse for 12 days, and a lesion area was observed. As a result, every of the lesion areas in soybean treated with each of the above present compounds showed 30% or less compared to the lesion area in an untreated soybean.

### Test Example 12: Control test against soybean rust (Phakopsora pachyrhizi)

Each of plastic pots is filled with soil and thereto soybean (cv: Kurosengoku) seeds are sown and the soybeans were grown in a greenhouse for 10 days, and an aqueous suspension containing the spores of Phakopsora pachyrhizi is spraying-inoculated. After the inoculation, the soybeans are placed in a greenhouse of 23 °C during daytime and 20 °C during nighttime under a high humidity for 1 day, and are then cultivated in the greenhouse for 2 days, and thereafter, the present compound each of which was made to a formulation according to the similar method to that of Formulation Example 1, is mixed with water so as to be 200 ppm, and the resulting mixtures are sprayed to foliar parts so as to adhere adequately on the leaves of the above-mentioned soybean. After spraying the mixtures, the soybeans are air-dried and cultivated in a greenhouse for 8 days, and a lesion area is then observed. As a result, each of the present compounds shows an effect on controlling plant diseases.

### Test Example 13: Control test against soybean leaf spot (Cercospora sojina)

Each of plastic pots is filled with soil and thereto soybean (cv: Tachinagaha) seeds are sown and the soybeans were grown in a greenhouse for 13 days. Thereafter, the present compound each of which is made to a formulation according to the similar method to that of Formulation Example 1, is mixed with water so as to be 200 ppm. The resulting mixtures are sprayed to foliar parts so as to adhere adequately on the leaves of the above-mentioned soybean. After spraying the mixtures, the soybeans are air-dried and after 1 day, an aqueous suspension of the spores of Cercospora sojina is spraying-inoculated. After the inoculation, the soybeans are placed in a greenhouse of 23 °C during daytime and 20 °C during nighttime under a high humidity for 3 days, and are then cultivated in the greenhouse for 16 days, and a lesion area is observed. As a result, each of the present compounds shows an effect on controlling plant diseases.

### Test Example 14: Control test against tomato early blight (Alternaria solani)

Each of plastic pots is filled with soils and thereto tomato (cv; PATIO) seeds are sown and the tomatoes were grown in a greenhouse for 20 days. Thereafter, the present compound each of which is made to a formulation according to the similar method to that of Formulation Example 1, is mixed with water so as to be 200 ppm. The resulting mixtures are sprayed to foliar parts so as to adhere adequately on the leaves of the above-mentioned tomato. After spraying the mixtures, the tomatoes are air-dried and after 1 day, an aqueous suspension of the spores of Alternaria solani are spraying-inoculated. After the inoculation, the tomatoes are placed at 18°C under a high humidity for 6 days, and a lesion area is observed. As a result, each of the present compounds shows an effect on controlling plant diseases.

### Test Example 15: Control test against soybean powdery mildew (Microsphaera diffusa)

Each of plastic pots was filled with soil and thereto soybean (cv: Kurosengoku) seeds were sown, the soybeans were grown in a greenhouse for 7 to 13 days, and spores of soybean powdery mildew was spraying-inoculated. After the inoculating, the soybeans were placed in a greenhouse of 24 °C during daytime and 20 °C during nighttime for 2 days. Thereafter, the present compounds 6, 7 or 81, each of which was made to a formulation according to the similar method to that of Formulation Example 1, was mixed with water so as to be 200 ppm. The mixtures were sprayed to foliar parts so as to adhere adequately on the leaves of the above-mentioned soybean. After the spraying, the soybeans were air-dried, and were then cultivated in the greenhouse for 7 to 11 days, and a lesion area was observed. As a result, every of the lesion areas in soybean treated with each of the above present compounds showed 30% or less compared to the lesion area in an untreated soybean.

### Test Example 16: Control test against soybean rust (Phakopsora pachyrhizi)

The test compound was diluted with DMSO so as to contain 150 ppm, 1 µL thereof was dispensed to a titer plate (96 well), and thereafter, an aqueous suspension of soybean rust fungus spores (1.0 × 10⁴/mL) 149 µL was further dispensed to the plate. This plate was cultivated at 23 °C for a few hours, and thereafter, the number of germinated spores of soybean rust fungus was counted. As a result, in the case of 1 ppm as the treated concentration, the number of germinated spores in a well containing any one of the present compound 160, 161 or 162 as a test compound showed 50% or less compared to the number of germinated spores in an untreated group.

### INDUSTRIAL APPLICABILITY

The present compound Y (including the compound Z of the present invention) has a control efficacy against plant diseases, and can be thus used to control plant diseases.

## Claims

1. A method for controlling a plant disease which comprises applying a compound represented by formula (I): 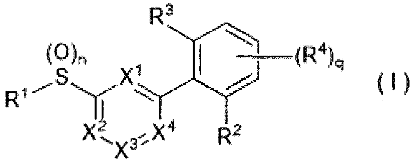 [wherein
n is 0, 1, or 2,
R¹ represents a C1-C6 alkyl group, a C2-C6 alkynyl group {the C1-C6 alkyl group, and the C2-C6 alkynyl group may optionally have one or more substituents selected from Group A}, a C2-C6 alkenyl group which may optionally have one or more substituents selected from Group C, a C3-C8 alicyclic hydrocarbon group, a three (3)- to eight (8)- membered non-aromatic heterocyclic group {the C3-C8 alicyclic hydrocarbon group, and the three (3)- to eight (8)- membered non-aromatic heterocyclic group may optionally have one or more substituents selected from Group B}, a C6-C10 aryl group which may optionally have one or more substituents selected from Group F, a five (5)- to ten (10)- membered aromatic heterocyclic group which may optionally have one or more substituents selected from Group D, or NR³³R³⁴,
R² and R³ are identical to or different from each other, and each represents a C1-C6 chain hydrocarbon group which may be optionally substituted with one or more halogen atoms, a C3-C8 alicyclic hydrocarbon group which may optionally have one or more substituents selected from Group B, OR⁹, SR¹⁰, S(O)R¹¹, S(O)₂R¹², NR¹³R¹⁴, C(O)R¹⁵, a nitro group, a cyano group, a halogen atom, or a hydrogen atom,
R⁴ represents a C1-C6 chain hydrocarbon group which may optionally have one or more substituents selected from Group A, a C3-C8 alicyclic hydrocarbon group, a three (3)- to eight (8)- membered non-aromatic heterocyclic group {the C3-C8 alicyclic hydrocarbon group, and the three (3)- to eight (8)- membered non-aromatic heterocyclic group may optionally have one or more substituents selected from Group B}, a phenyl group, a five (5)- to six (6)- membered aromatic heterocyclic group {the phenyl group, and the five (5)- to six (6)- membered aromatic heterocyclic group may optionally have one or more substituents selected from Group D}, OR¹⁶, SR¹⁷, S(O)R¹⁸, S(O)₂R19, NR²⁰R²¹, C(O)R²², C(R²³)=NOR²⁴, a cyano group, a nitro group, an amino group, a hydroxy group, or a halogen atom,
q is 1, 2, or 3,
when q is 2 or 3, 2 or 3 of R⁴ may be identical to or different from each other,
a combination of X¹, X², X³, and X⁴ represents,
a combination wherein X¹ represents CR⁵, X² represents CR⁶, X³ represents CR⁷, and X⁴ represents a nitrogen atom;
a combination wherein X¹ represents CR⁵, X² represents CR⁶, X³ represents a nitrogen atom, and X⁴ represents CR⁸;
a combination wherein X¹ represents CR⁵, X² represents a nitrogen atom, X³ represents CR^{7A}, and X⁴ represents CR⁸;
a combination wherein X¹ represents a nitrogen atom, X² represents CR⁶, X³ represents CR^{7B}, and X⁴ represents CR⁸;
a combination wherein X¹ represents a nitrogen atom, X² represents a nitrogen atom, X³ represents CR^{7C}, and X⁴ represents CH;
a combination wherein X¹ represents a nitrogen atom, X² represents a nitrogen atom, X³ represents CH, and X⁴ represents CR⁸;
a combination wherein X¹ represents a nitrogen atom, X² represents CR⁶, X³ represents a nitrogen atom, and X⁴ represents CR⁸;
a combination wherein X¹ represents a nitrogen atom, X² represents CR⁶, X³ represents CR⁷, and X⁴ represents a nitrogen atom;
a combination wherein X¹ represents CR⁵, X² represents a nitrogen atom, X³ represents a nitrogen atom, and X⁴ represents CR⁸;
a combination wherein X¹ represents CR⁵, X² represents a nitrogen atom, X³ represents CH, and X⁴ represents a nitrogen atom;
a combination wherein X¹ represents CH, X² represents a nitrogen atom, X³ represents CR^{7D}, and X⁴ represents a nitrogen atom; or
a combination wherein X¹ represents CR⁵, X² represents CR⁶, X³ represents a nitrogen atom, and X⁴ represents a nitrogen atom;
R⁵, R⁶, R⁷ and R⁸ are identical to or different from each other, and each represents a C1-C6 chain hydrocarbon group which may be optionally substituted with one or more halogen atoms, a C3-C8 alicyclic hydrocarbon group which may optionally have one or more substituents selected from Group B, OR²⁵, SR²⁶, S(O)R²⁷, S(O)₂R²⁸, NR²⁹R³⁰, C(O)R³¹, a nitro group, a cyano group, a halogen atom, or a hydrogen atom,
R⁹, R¹⁰, R¹³, and R²⁹ are identical to or different from each other, and each represents a C1-C6 chain hydrocarbon group which may be optionally substituted with one or more halogen atoms, or a hydrogen atom,
R^{7A} represents a C1-C6 chain hydrocarbon group which may be optionally substituted with one or more halogen atoms, a C3-C8 alicyclic hydrocarbon group which may optionally have one or more substituents selected from Group B, OR²⁵, SR²⁶, S(O)R²⁷, S(O)₂R²⁸, NR²⁹R³², C(O)R³¹, a nitro group, a cyano group, a halogen atom, or a hydrogen atom,
R^{7B} represents a C1-C6 chain hydrocarbon group which may be optionally substituted with one or more halogen atoms, a C3-C8 alicyclic hydrocarbon group which may optionally have one or more substituents selected from Group B, OR²⁵, NR²⁹R³⁰, C(O)R³¹, a nitro group, a cyano group, a halogen atom, or a hydrogen atom,
R^{7C} represents a C1-C6 chain hydrocarbon group which may be optionally substituted with one or more halogen atoms, a C3-C8 alicyclic hydrocarbon group which may optionally have one or more substituents selected from Group B, OR²⁵, NR²⁹R³⁰, C(O)R³¹, a nitro group, a cyano group, or a halogen atom,
R^{7D} represents a C1-C6 chain hydrocarbon group which may be optionally substituted with one or more halogen atoms, a C3-C8 alicyclic hydrocarbon group which may optionally have one or more substituents selected from Group B, OR²⁵, S(O)R²⁷, S(O)2R²⁸, NR²⁹R³⁰, C(O)R³¹, a nitro group, a cyano group, a fluorine atom, a chlorine atom, or an iodine atom,
R¹¹, R¹², R²⁵, R²⁶, R²⁷, and R²⁸ are identical to or different from each other, and each represents a C1-C6 chain hydrocarbon group which may be optionally substituted with one or more halogen atoms,
R¹⁴ and R³⁰ are identical to or different from each other, and each represents a C1-C6 chain hydrocarbon group which may be optionally substituted with one or more halogen atoms, a (C1-C6 alkyl)carbonyl group, a (C1-C6 alkoxy)carbonyl group {the (C1-C6 alkyl)carbonyl group, and the (C1-C6 alkoxy) carbonyl group may be optionally substituted with one or more halogen atoms}, or a hydrogen atom,
R¹⁵, R²² and R³¹ are identical to or different from each other, and each represents a C1-C6 chain hydrocarbon group, a C1-C6 alkoxy group, a C1-C6 alkylamino group, or a C2-C8 dialkylamino group {the C1-C6 chain hydrcarbon group, the C1-C6 alkoxy group, the C1-C6 alkylamino group, and the C2-C8 dialkylamino group may be optionally substituted with one or more halogen atoms},
R¹⁶ represents a C1-C6 chain hydrocarbon group which may optionally have one or more substituents selected from Group A, a C1-C6 alkylsulfonyl group, a (C1-C6 alkyl)carbonyl group, a (C1-C6 alkoxy)carbonyl group, a (C1-C6 alkylamino)carbonyl group, a (C2-C8 dialkylamino)carbonyl group {the C1-C6 alkylsulfonyl group, the (C1-C6 alkyl)carbonyl group, the (C1-C6 alkoxy)carbonyl group, the (C1-C6 alkylamino)carbonyl group, and the (C2-C8 dialkylamino)carbonyl group may be optionally substituted with one or more halogen atoms}, a C3-C8 alicyclic hydrocarbon group, a three (3)- to eight (8)- membered non-aromatic heterocyclic group {the C3-C8 alicyclic hydrocarbon group, and the three (3)- to eight (8)- membered non-aromatic heterocyclic group may optionally have one or more substituents selected from Group B}, a phenyl group, a five (5)- to six (6)- membered aromatic heterocyclic group {the phenyl group, and the five (5)- to six (6)- membered aromatic heterocyclic group may optionally have one or more substituents selected from Group D}, or a hydrogen atom,
R¹⁷, R¹⁸, R¹⁹, and R²⁴ are identical to or different from each other, and each represents a C1-C6 chain hydrocarbon group which may optionally have one or more substituents selected from Group A, a C3-C8 alicyclic hydrocarbon group, a three (3)- to eight (8)- membered non-aromatic heterocyclic group {the C3-C8 alicyclic hydrocarbon group, and the three (3)- to eight (8)- membered non-aromatic heterocyclic group may optionally have one or more substituents selected from Group B}, a phenyl group, or a five (5)- to six (6)- membered aromatic heterocyclic group {the phenyl group, and the five (5)- to six (6)- membered aromatic heterocyclic group may optionally have one or more substituents selected from Group D},
R²⁰ represents a C1-C6 chain hydrocarbon group which may optionally have one or more substituents selected from Group A, a C3-C8 alicyclic hydrocarbon group, a three (3)- to eight (8)- membered non-aromatic heterocyclic group {the C3-C8 alicyclic hydrocarbon group, and the three (3)- to eight (8)- membered non-aromatic heterocyclic group may optionally have one or more substituents selected from Group B}, a phenyl group, a five (5)- to six (6)- membered aromatic heterocyclic group {the phenyl group, and the five (5)- to six (6)- membered aromatic heterocyclic group may optionally have one or more substituents selected from Group D}, a (C1-C6 alkyl)carbonyl group, a (C1-C6 alkoxy)carbonyl group, or a C1-C6 alkylsulfonyl group {the (C1-C6 alkyl)carbonyl group, the (C1-C6 alkoxy)carbonyl group, and the C1-C6 alkylsulfonyl group may be optionally substituted with one or more halogen atoms},
R²¹ and R²³ are identical to or different from each other, and each represents a C1-C6 chain hydrocarbon group which may optionally have one or more substituents selected from Group A, or a hydrogen atom,
R³² represents a C1-C6 chain hydrocarbon group which may be optionally substituted with one or more halogen atoms, a (C1-C6 alkyl)carbonyl group, or a (C1-C6 alkoxy)carbonyl group {the (C1-C6 alkyl)carbonyl group, and the (C1-C6 alkoxy)carbonyl group may be optionally substituted with one or more halogen atoms},
R³³ and R³⁴ are identical to or different from each other, and each represents a C1-C6 chain hydrocarbon group which may optionally have one or more substituents selected from Group G, a C3-C6 alicyclic hydrocarbon group which may be optionally substituted with one or more halogen atoms, or a hydrogen atom,
the neighboring R² and R⁴ may combine together with a carbon atom to which they are attached to form a C5-C6 alicyclic hydrocarbon, a five (5)- to six (6)- membered non-aromatic heterocyclic ring {the C5-C6 alicyclic hydrocarbon, and the five (5)- to six (6)- membered non-aromatic heterocyclic ring may optionally have one or more substituents selected from Group B}, a benzene ring, or a five (5)- to six (6)- membered aromatic heterocyclic ring {the benzene ring, and the five (5)- to six (6)- membered aromatic heterocyclic ring may optionally have one or more substituents selected from Group E},
two of neighboring R⁴ and R⁴ may combine together with a carbon atom to which they are attached to form a C5-C6 alicyclic hydrocarbon, a five (5)- to six (6)- membered non-aromatic heterocyclic ring {the C5-C6 alicyclic hydrocarbon, and the five (5)- to six (6)- membered non-aromatic heterocyclic ring may optionally have one or more substituents selected from Group B}, a benzene ring, or a five (5)- to six (6)- membered aromatic heterocyclic ring {the benzene ring, and the five (5)- to six (6)- membered aromatic heterocyclic ring may optionally have one or more substituents selected from Group E}.
Group A is a group consisting of a C1-C6 alkoxy group, a C1-C6 alkylsulfanyl group, a C1-C6 alkylsulfinyl group, a C1-C6 alkylsulfonyl group {the C1-C6 alkoxy group, the C1-C6 alkylsulfanyl group, the C1-C6 alkylsulfinyl group, and the C1-C6 alkylsulfonyl group may be optionally substituted with one or more halogen atoms}, a C3-C8 alicyclic hydrocarbon group, a three (3)- to eight (8)- membered non-aromatic heterocyclic group {the C3-C8 alicyclic hydrocarbon group, and the three (3)- to eight (8)- membered non-aromatic heterocyclic group may optionally have one or more substituents selected from Group B}, a phenyl group, a five (5)- to six (6)- membered aromatic heterocyclic group {the phenyl group and the five (5)- to six (6)- membered aromatic heterocyclic group may optionally have one or more substituents selected from Group D}, a halogen atom, a cyano group, a nitro group, a hydroxy group, and a sulfanyl group.
Group B is a group consisting of an oxo group, a thioxo group, a C1-C6 chain hydrocarbon group, a C1-C6 alkoxy group, a C1-C6 alkylsulfanyl group, a C1-C6 alkylsulfinyl group, a C1-C6 alkylsulfonyl group {the C1-C6 chain hydrocarbon group, the C1-C6 alkoxy group, the C1-C6 alkylsulfanyl group, the C1-C6 alkylsulfinyl group, and the C1-C6 alkylsulfonyl group may be optionally substituted with one or more halogen atoms}, a hydroxy group, a sulfanyl group, a halogen atom, and a cyano group.
Group C is a group consisting of a C1-C6 alkoxy group, a C1-C6 alkylsulfanyl group, a C1-C6 alkylsulfinyl group, a C1-C6 alkylsulfonyl group {the C1-C6 alkoxy group, the C1-C6 alkylsulfanyl group, the C1-C6 alkylsulfinyl group, and the C1-C6 alkylsulfonyl group may be optionally substituted with one or more halogen atoms}, a C3-C8 alicyclic hydrocarbon group, a three (3)- to eight (8)- membered non-aromatic heterocyclic group {the C3-C8 alicyclic hydrocarbon group, and the three (3)- to eight (8)- membered non-aromatic heterocyclic group may optionally have one or more substituents selected from Group B}, a phenyl group, a five (5)- to six (6)- membered aromatic heterocyclic group {the phenyl group and the five (5)- to six (6)- membered aromatic heterocyclic group may optionally have one or more substituents selected from Group D}, a chlorine atom, a bromine atom, an iodine atom, a cyano group, a nitro group, a hydroxy group, and a sulfanyl group.
Group D is a group consisting of a C1-C6 chain hydrocarbon group, a C1-C6 alkoxy group, a C1-C6 alkylsulfanyl group, a C1-C6 alkylsulfinyl group, a C1-C6 alkylsulfonyl group, a C1-C6 alkylamino group, a C2-C8 dialkylamino group, a (C1-C6 alkyl)carbonyl group, a (C1-C6 alkoxy)carbonyl group, a (C1-C6 alkylamino)carbonyl group, a (C2-C8 dialkylamino)carbonyl group, a (C1-C6 alkyl)carbonylamino group, a (C1-C6 alkoxy)carbonylamino group {the C1-C6 chain hydrocarbon group, the C1-C6 alkoxy group, the C1-C6 alkylsulfanyl group, the C1-C6 alkylsulfinyl group, the C1-C6 alkylsulfonyl group, the C1-C6 alkylamino group, the C2-C8 dialkylamino group, the (C1-C6 alkyl)carbonyl group, the (C1-C6 alkoxy)carbonyl group, the (C1-C6 alkylamino)carbonyl group, the (C2-C8 dialkylamino)carbonyl group, the (C1-C6 alkyl)carbonylamino group, and the (C1-C6 alkoxy)carbonylamino group may be optionally substituted with one or more halogen atoms}, a C3-C8 alicyclic hydrocarbon group which may optionally have one or more substituents selected from Group B, an amino group, a nitro group, a cyano group, a hydroxy group, a sulfanyl group, and a halogen atom.
Group E is a group consisting of a C1-C6 chain hydrocarbon group, a C1-C6 alkoxy group, a C1-C6 alkylsulfanyl group, a C1-C6 alkylsulfinyl group, a C1-C6 alkylsulfonyl group {the C1-C6 chain hydrocarbon group, the C1-C6 alkoxy group, the C1-C6 alkylsulfanyl group, the C1-C6 alkylsulfinyl group, and the C1-C6 alkylsulfonyl group may be optionally substituted with one or more halogen atoms}, a hydroxy group, a sulfanyl group, a halogen atom, and a cyano group.
Group F is a group consisting of a C1-C6 chain hydrocarbon group, a C1-C6 alkoxy group, a C1-C6 alkylsulfanyl group, a C1-C6 alkylsulfinyl group, a C1-C6 alkylsulfonyl group, a C1-C6 alkylamino group, a C2-C8 dialkylamino group, a (C1-C6 alkyl)carbonyl group, a (C1-C6 alkoxy)carbonyl group, a (C1-C6 alkylamino)carbonyl group, a (C2-C8 dialkylamino)carbonyl group, a (C1-C6 alkyl)carbonylamino group, a (C1-C6 alkoxy)carbonylamino group {the C1-C6 chain hydrocarbon group, the C1-C6 alkoxy group, the C1-C6 alkylsulfanyl group, the C1-C6 alkylsulfinyl group, the C1-C6 alkylsulfonyl group, the C1-C6 alkylamino group, the C2-C8 dialkylamino group, the (C1-C6 alkyl)carbonyl group, the (C1-C6 alkoxy)carbonyl group, the (C1-C6 alkylamino)carbonyl group, the (C2-C8 dialkylamino)carbonyl group, the (C1-C6 alkyl)carbonylamino group, and the (C1-C6 alkoxy)carbonylamino group may be optionally substituted with one or more halogen atoms}, a C3-C8 alicyclic hydrocarbon group which may optionally have one or more substituents selected from Group B, an amino group, a cyano group, a hydroxy group, a sulfanyl group, and a halogen atom.
Group G is a group consisting of a C3-C6 cycloalkyl group, a C1-C3 alkoxy group, a C1-C3 alkylthio group {the C3-C6 cycloalkyl group, the C1-C3 alkoxy group, and the C1-C3 alkylthio group may be optionally substituted with one or more halogen atoms}, a halogen atom, and a cyano group] , or a N-oxide thereof, or a salt thereof
to a plant or soil for cultivating the plant.

2. The method according to claim 1 wherein the compound represented by formula (I), or the N-oxide thereof, or the salt thereof represents the compound wherein R¹ represents a C1-C6 alkyl group, a C2-C6 alkynyl group {the C1-C6 alkyl group, and the C2-C6 alkynyl group may optionally have one or more substituents selected from Group A}, a C2-C6 alkenyl group which may optionally have one or more substituents selected from Group C, or a C3-C8 alicyclic hydrocarbon group {the C3-C8 alicyclic hydrocarbon group may optionally have one or more substituents selected from Group B} , or the N-oxide thereof, or the salt thereof.

3. The method according to claim 2 wherein the compound represented by formula (I), or the N-oxide thereof, or the salt thereof represents the compound wherein the combination of X¹, X², X³, and X⁴ represents a combination wherein X¹ represents CR⁵, X² represents CR⁶, X³ represents CR⁷, and X⁴ represents a nitrogen atom; a combination wherein X¹ represents CR⁵, X² represents CR⁶, X³ represents a nitrogen atom, and X⁴ represents CR⁸; a combination wherein X¹ represents CR⁵, X² represents a nitrogen atom, X³ represents CR^{7A}, and X⁴ represents CR⁸; a combination wherein X¹ represents a nitrogen atom, X² represents CR⁶, X³ represents CR^{7B}, and X⁴ represents CR⁸; a combination wherein X¹ represents a nitrogen atom, X² represents CR⁶, X³ represents a nitrogen atom, and X⁴ represents CR⁸; a combination wherein X¹ represents CR⁵, X² represents a nitrogen atom, X³ represents a nitrogen atom, and X⁴ represents CR⁸; or a combination wherein X¹ represents CR⁵, X² represents CR⁶, X³ represents a nitrogen atom, and X⁴ represents a nitrogen atom, or the N-oxide thereof, or the salt thereof.

4. The method according to claim 2 or 3 wherein the compound represented by formula (I), or the N-oxide thereof, or the salt thereof represents a compound wherein R¹ represents a C2-C6 alkyl group, a C2-C6 alkynyl group {the C2-C6 alkyl group, and the C2-C6 alkynyl group may optionally have one or more substituents selected from Group A}, a methyl group which has one or more substituents selected from Group C, or a C3-C8 alicyclic hydrocarbon group {the C3-C8 alicyclic hydrocarbon group may optionally have one or more substituents selected from Group B}
or the N-oxide thereof, or the salt thereof.

5. A compound represented by formula (II): 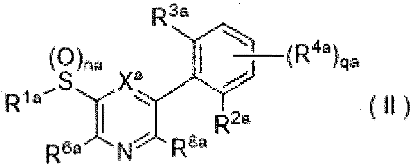 [wherein
na is 0, 1, or 2,
qa is 1, 2, or 3,
when qa is 2 or 3, 2 or 3 of R^{4a} may be identical to or different from each other,
R^{1a} represents a C2-C6 alkyl group, a C2-C6 alkynyl group {the C2-C6 alkyl group, and the C2-C6 alkynyl group may optionally have one or more substituents selected from Group A^{a}}, a C2-C6 alkenyl group which may optionally have one or more substituents selected from Group F^{a}, a C3-C8 alicyclic hydrocarbon group which may optionally have one or more substituents selected from Group B^{a}, a methyl group which has one or more substituents selected from Group C^{a}, or a five (5)- to six (6)- membered aromatic heterocyclic group which may optionally have one or more substituents selected from Group G^{a},
R^{8a} represents a C1-C6 chain hydrocarbon group which may be optionally substituted with one or more halogen atoms, a C3-C8 alicyclic hydrocarbon group which may optionally have one or more substituents selected from Group B^{a}, OR^{7a}, SR^{9a}, S(O)R^{10a}, S(O)₂R^{11a}, NR^{12a}R^{13a}, C(O)R^{14a}, a nitro group, a cyano group, a halogen atom, or a hydrogen atom,
R^{2a} and R^{3a} are identical to or different from each other, and each represents a C1-C6 chain hydrocarbon group which may be optionally substituted with one or more halogen atoms, a C3-C8 alicyclic hydrocarbon group which may optionally have one or more substituents selected from Group B^{a}, OR^{15a}, SR^{16a}, S(O)R^{17a}, S(O)₂R^{18a}, NR^{19a}R^{20a}, C(O)R^{21a}, a nitro group, a cyano group, a halogen atom, or a hydrogen atom,
a combination of X^{a}, R^{4a}, and R^{6a} represents
a combination wherein
X^{a} represents nitrogen atom,
R^{4a} represents a C1-C6 chain hydrocarbon group which may optionally have one or more substituents selected from Group A^{a}, a C3-C8 alicyclic hydrocarbon group, a three (3)- to eight (8)- membered non-aromatic heterocyclic group {the C3-C8 alicyclic hydrocarbon group, and the three (3)-to eight (8)- membered non-aromatic heterocyclic group may optionally have one or more substituents selected from Group B^{a}}, a phenyl group, a five (5)- to six (6)- membered aromatic heterocyclic group {the phenyl group, and the five (5)- to six (6)- membered aromatic heterocyclic group may optionally have one or more substituents selected from Group D^{a}}, OR^{23a}, SR^{24a}, S(O)R^{25a}, S(O)₂R^{26a}, NR^{27a}R^{28a}, C(O)R^{29a}, C(R^{30a})=NOR^{31a}, a cyano group, a nitro group, an amino group, a hydroxy group, or a halogen atom,
R^{6a} represents a C1-C6 chain hydrocarbon group which may be optionally substituted with one or more halogen atoms, a C3-C8 alicyclic hydrocarbon group which may optionally have one or more substituents selected from Group B^{a}, OR^{7a}, SR^{9a}, S(O)R^{10a}, S(O)₂R^{11a}, NR^{12a}R^{13a}, C(O)R^{14a}, a nitro group, a cyano group, a halogen atom, or a hydrogen atom; or
a combination wherein
X^{a} represents CR^{5a},
R^{4a} represents a methyl group which may optionally have one or more substituents selected from Group F^{a}, a CH₂F group, a CHF₂ group, a C2-C6 chain hydrocarbon group which may optionally have one or more substituents selected from Group A^{a}, a C3-C8 alicyclic hydrocarbon group, a three (3)-to eight (8)- membered non-aromatic heterocyclic group {the C3-C8 alicyclic hydrocarbon group, and the three (3)- to eight (8)- membered non-aromatic heterocyclic group may optionally have one or more substituents selected from Group B^{a}}, a phenyl group, a five (5)- to six (6)- membered aromatic heterocyclic group {the phenyl group, and the five (5)- to six (6)- membered aromatic heterocyclic group may optionally have one or more substituents selected from Group D^{a}}, OR^{23a}, SR^{24a}, S(O)R^{25a}, S(O)₂R^{22a}, NR^{27a}R^{28a}, C(O)R^{29a}, C(R^{30a})=NOR^{31a}, a cyano group, a nitro group, an amino group, a hydroxy group, or a halogen atom,
R^{6a} represents a C1-C6 chain hydrocarbon group which may be optionally substituted with one or more halogen atoms, a C3-C8 alicyclic hydrocarbon group which may optionally have one or more substituents selected from Group B^{a}, OR^{7a}, a nitro group, a fluorine atom, a bromine atom, an iodine atom, or a hydrogen atom,
R^{5a} represents a C1-C6 chain hydrocarbon group which may be optionally substituted with one or more halogen atoms, a C3-C8 alicyclic hydrocarbon group which may optionally have one or more substituents selected from Group B^{a}, OR^{7a}, SR^{9a}, S(O)R^{10a}, S(O)₂R^{11a}, NR^{12a}R^{13a}, C(O)R^{14a}, a nitro group, a cyano group, a halogen atom, or a hydrogen atom,
R^{7a}, R^{9a}, R^{10a}, R^{11a}, R^{15a}, R^{16a}, R^{17a}, and R^{18a} are identical to or different from each other, and each represents a C1-C6 chain hydrocarbon group which may be optionally substituted with one or more halogen atoms,
R^{12a} and R^{19a} are identical to or different from each other, and each represents a C1-C6 chain hydrocarbon group which may be optionally substituted with one or more halogen atoms, or a hydrogen atom,
R^{13a} and R^{20a} are identical to or different from each other, and each represents a C1-C6 chain hydrocarbon group which may be optionally substituted with one or more halogen atoms, a (C1-C6 alkyl)carbonyl group, a (C1-C6 alkoxy)carbonyl group {the (C1-C6 alkyl)carbonyl group, and the (C1-C6 alkoxy)carbonyl group may be optionally substituted with one or more halogen atoms}, or a hydrogen atom,
R^{14a}, R^{21a}, and R^{29a} are identical to or different from each other, and each represents a C1-C6 chain hydrocarbon group, a C1-C6 alkoxy group, a C1-C6 alkylamino group, or a C2-C8 dialkylamino group {the C1-C6 chain hydrocarbon group, the C1-C6 alkoxy group, the C1-C6 alkylamino group, and the C2-C8 dialkylamino group may be optionally substituted with one or more halogen atoms},
R^{23a} represents a C1-C6 chain hydrocarbon group which may optionally have one or more substituents selected from Group A^{a}, a C1-C6 alkylsulfonyl group, a (C1-C6 alkyl)carbonyl group, a (C1-C6 alkoxy)carbonyl group, a (C1-C6 alkylamino) carbonyl group, a (C2-C8 dialkylamino) carbonyl group {the C1-C6 alkylsulfonyl group, the (C1-C6 alkyl)carbonyl group, the (C1-C6 alkoxy)carbonyl group, the (C1-C6 alkylamino)carbonyl group, and the (C2-C8 dialkylamino) carbonyl group may be optionally substituted with one or more halogen atoms}, a C3-C8 alicyclic hydrocarbon group, a three (3)- to eight (8)- membered non-aromatic heterocyclic group {the C3-C8 alicyclic hydrocarbon group, and the three (3)- to eight (8)- membered non-aromatic heterocyclic group may optionally have one or more substituents selected from Group B^{a}}, a phenyl group, a five (5)- to six (6)- membered aromatic heterocyclic group {the phenyl group, and the five (5)- to six (6)- membered aromatic heterocyclic group may optionally have one or more substituents selected from Group D^{a}}, or a hydrogen atom,
R^{22a}, R^{24a}, R^{25a}, and R^{31a} are identical to or different from each other, and each represents a C1-C6 chain hydrocarbon group which may optionally have one or more substituents selected from Group A^{a}, a C3-C8 alicyclic hydrocarbon group, a three (3)- to eight (8)- membered non-aromatic heterocyclic group {the C3-C8 alicyclic hydrocarbon group, and the three (3)- to eight (8)- membered non-aromatic heterocyclic group may optionally have one or more substituents selected from Group B^{a}}, a phenyl group, or a five (5)- to six (6)- membered aromatic heterocyclic group {the phenyl group, and the five (5)- to six (6)- membered aromatic heterocyclic group may optionally have one or more substituents selected from Group D^{a}},
R^{26a} represents a methyl group which has one or more substituents selected from Group A^{a}, a C2-C6 chain hydrocarbon group which may optionally have one or more substituents selected from Group A^{a}, a C3-C8 alicyclic hydrocarbon group, a three (3)- to eight (8)- membered non-aromatic heterocyclic group {the C3-C8 alicyclic hydrocarbon group, and the three (3)- to eight (8)- membered non-aromatic heterocyclic group may optionally have one or more substituents selected from Group B^{a}}, a phenyl group, or a five (5)- to six (6)- membered aromatic heterocyclic group {the phenyl group, and the five (5)- to six (6)- membered aromatic heterocyclic group may optionally have one or more substituents selected from Group D^{a}},
R^{27a} represents a C1-C6 chain hydrocarbon group which may optionally have one or more substituents selected from Group A^{a}, a C3-C8 alicyclic hydrocarbon group, a three (3)-to eight (8)- membered non-aromatic heterocyclic group {the C3-C8 alicyclic hydrocarbon group, and the three (3)- to eight (8)- membered non-aromatic heterocyclic group may optionally have one or more substituents selected from Group B^{a}}, a phenyl group, a five (5)- to six (6)- membered aromatic heterocyclic group {the phenyl group, and the five (5)- to six (6)- membered aromatic heterocyclic group may optionally have one or more substituents selected from Group D^{a}}, a (C1-C6 alkyl)carbonyl group, a (C1-C6 alkoxy)carbonyl group, or a C1-C6 alkylsulfonyl group {the (C1-C6 alkyl)carbonyl group, the (C1-C6 alkoxy)carbonyl group, and the C1-C6 alkylsulfonyl group may be optionally substituted with one or more halogen atoms},
R^{28a} and R^{30a} are identical to or different from each other, and each represents a C1-C6 chain hydrocarbon group which may optionally have one or more substituents selected from Group A^{a}, or a hydrogen atom,
the neighboring R^{2a} and R^{4a} may combine together with a carbon atom to which they are attached to form a C5-C6 alicyclic hydrocarbon, a five (5)- to six (6)- membered non-aromatic heterocyclic ring {the C5-C6 alicyclic hydrocarbon, and the five (5)- to six (6)- membered non-aromatic heterocyclic ring may optionally have one or more substituents selected from Group B^{a}}, a benzene ring, or a five (5)- to six (6)- membered aromatic heterocyclic ring {the benzene ring, and the five (5)- to six (6)- membered aromatic heterocyclic ring may optionally have one or more substituents selected from Group E^{a}},
two of neighboring R^{4a} and R^{4a} may combine together with a carbon atom to which they are attached to form a C5-C6 alicyclic hydrocarbon, a five (5)- to six (6)- membered non-aromatic heterocyclic ring {the C5-C6 alicyclic hydrocarbon, and the five (5)- to six (6)- membered non-aromatic heterocyclic ring may optionally have one or more substituents selected from Group B^{a}}, a benzene ring, or a five (5)- to six (6)- membered aromatic heterocyclic ring {the benzene ring, and the five (5)- to six (6)- membered aromatic heterocyclic ring may optionally have one or more substituents selected from Group E^{a}}.
Group A^{a} is a group consisting of a C1-C6 alkoxy group, a C1-C6 alkylsulfanyl group, a C1-C6 alkylsulfinyl group, a C1-C6 alkylsulfonyl group {the C1-C6 alkoxy group, the C1-C6 alkylsulfanyl group, the C1-C6 alkylsulfinyl group, and the C1-C6 alkylsulfonyl group may be optionally substituted with one or more halogen atoms}, a C3-C8 alicyclic hydrocarbon group, a three (3)- to eight (8)- membered non-aromatic heterocyclic group {the C3-C8 alicyclic hydrocarbon group, and the three (3)- to eight (8)- membered non-aromatic heterocyclic group may optionally have one or more substituents selected from Group Ba}, a phenyl group, a five (5)- to six (6)- membered aromatic heterocyclic group {the phenyl group and the five (5)- to six (6)- membered aromatic heterocyclic group may optionally have one or more substituents selected from Group Da}, a halogen atom, a cyano group, a nitro group, a hydroxy group, and a sulfanyl group.
Group B^{a} is a group consisting of an oxo group, a thioxo group, a C1-C6 chain hydrocarbon group, a C1-C6 alkoxy group, a C1-C6 alkylsulfanyl group, a C1-C6 alkylsulfinyl group, a C1-C6 alkylsulfonyl group {the C1-C6 chain hydrocarbon group, the C1-C6 alkoxy group, the C1-C6 alkylsulfanyl group, the C1-C6 alkylsulfinyl group, and the C1-C6 alkylsulfonyl group may be optionally substituted with one or more halogen atoms}, a hydroxy group, a sulfanyl group, a halogen atom, and a cyano group.
Group C^{a} is a group consisting of a C1-C6 alkoxy group, a C1-C6 alkylsulfanyl group, a C1-C6 alkylsulfinyl group, a C1-C6 alkylsulfonyl group {the C1-C6 alkoxy group, the C1-C6 alkylsulfanyl group, the C1-C6 alkylsulfinyl group, and the C1-C6 alkylsulfonyl group may be optionally substituted with one or more halogen atoms}, a C3-C8 alicyclic hydrocarbon group, a three (3)- to eight (8)- membered non-aromatic heterocyclic group {the C3-C8 alicyclic hydrocarbon group, and the three (3)- to eight (8)- membered non-aromatic heterocyclic group may optionally have one or more substituents selected from Group B^{a}}, and a five (5)- to six (6)- membered aromatic heterocyclic group {the five (5)- to six (6)- membered aromatic heterocyclic group may optionally have one or more substituents selected from Group D^{a}}.
Group D^{a} is a group consisting of a C1-C6 chain hydrocarbon group, a C1-C6 alkoxy group, a C1-C6 alkylsulfanyl group, a C1-C6 alkylsulfinyl group, a C1-C6 alkylsulfonyl group, a C1-C6 alkylamino group, a C2-C8 dialkylamino group, a (C1-C6 alkyl)carbonyl group, a (C1-C6 alkoxy)carbonyl group, a (C1-C6 alkylamino)carbonyl group, a (C2-C8 dialkylamino)carbonyl group, a (C1-C6 alkyl)carbonylamino group, a (C1-C6 alkoxy)carbonylamino group {the C1-C6 chain hydrocarbon group, the C1-C6 alkoxy group, the C1-C6 alkylsulfanyl group, the C1-C6 alkylsulfinyl group, the C1-C6 alkylsulfonyl group, the C1-C6 alkylamino group, the C2-C8 dialkylamino group, the (C1-C6 alkyl)carbonyl group, the (C1-C6 alkoxy)carbonyl group, the (C1-C6 alkylamino)carbonyl group, the (C2-C8 dialkylamino)carbonyl group, the (C1-C6 alkyl)carbonylamino group, and the (C1-C6 alkoxy) carbonylamino group may be optionally substituted with one or more halogen atoms}, a C3-C8 alicyclic hydrocarbon group which may optionally have one or more substituents selected from Group B^{a}, an amino group, a nitro group, a cyano group, a hydroxy group, a sulfanyl group, and a halogen atom.
Group E^{a} is a group consisting of a C1-C6 chain hydrocarbon group, a C1-C6 alkoxy group, a C1-C6 alkylsulfanyl group, a C1-C6 alkylsulfinyl group, a C1-C6 alkylsulfonyl group {the C1-C6 chain hydrocarbon group, the C1-C6 alkoxy group, the C1-C6 alkylsulfanyl group, the C1-C6 alkylsulfinyl group, and the C1-C6 alkylsulfonyl group may be optionally substituted with one or more halogen atoms}, a hydroxy group, a sulfanyl group, a halogen atom, and a cyano group.
Group F^{a} is a group consisting of a C1-C6 alkoxy group, a C1-C6 alkylsulfanyl group, a C1-C6 alkylsulfinyl group, a C1-C6 alkylsulfonyl group {the C1-C6 alkoxy group, the C1-C6 alkylsulfanyl group, the C1-C6 alkylsulfinyl group, and the C1-C6 alkylsulfonyl group may be optionally substituted with one or more halogen atoms}, a C3-C8 alicyclic hydrocarbon group, a three (3)- to eight (8)- membered non-aromatic heterocyclic group {the C3-C8 alicyclic hydrocarbon group, and the three (3)- to eight (8)- membered non-aromatic heterocyclic group may optionally have one or more substituents selected from Group B^{a}}, a phenyl group, a five (5)- to six (6)- membered aromatic heterocyclic group {the phenyl group and the five (5)- to six (6)- membered aromatic heterocyclic group may optionally have one or more substituents selected from Group D^{a}}, a chlorine atom, a bromine atom, an iodine atom, a cyano group, a nitro group, a hydroxy group, and a sulfanyl group.
Group G^{a} is a group consisting of a C1-C6 chain hydrocarbon group, a C1-C6 alkoxy group, a C1-C6 alkylsulfanyl group, a C1-C6 alkylsulfinyl group, a C1-C6 alkylsulfonyl group, a C1-C6 alkylamino group, a C2-C8 dialkylamino group {the C1-C6 chain hydrocarbon group, the C1-C6 alkoxy group, the C1-C6 alkylsulfanyl group, the C1-C6 alkylsulfinyl group, the C1-C6 alkylsulfonyl group, the C1-C6 alkylamino group, and the C2-C8 dialkylamino group may be optionally substituted with one or more halogen atoms}, a C3-C8 alicyclic hydrocarbon group which may optionally have one or more substituents selected from Group B, an amino group, a nitro group, a cyano group, a hydroxy group, a sulfanyl group, and a halogen atom]
or a N-oxide thereof, or a salt thereof.

6. The compound according to claim 5 wherein R^{1a} represents a C2-C6 alkyl group, a C2-C6 alkynyl group {the C2-C6 alkyl group, and the C2-C6 alkynyl group may optionally have one or more substituents selected from Group A^{a}}, a C2-C6 alkenyl group which may optionally have one or more substituents selected from Group F^{a}, a C3-C8 alicyclic hydrocarbon group which may optionally have one or more substituents selected from Group B^{a}, or a methyl group which has one or more substituents selected from Group C^{a}
, or the N-oxide thereof, or the salt thereof.

7. The compound according to claim 6 wherein X^{a} represents a nitrogen atom, R^{4a} represents a C1-C6 chain hydrocarbon group which may optionally have one or more substituents selected from Group A^{a}, OR^{23a}, NR^{27a}R^{28a}, a cyano group, a nitro group, a hydroxy group, or a halogen atom,
or the N-oxide thereof, or the salt thereof.

8. The compound according to claim 6 wherein X^{a} represents CR^{5a}, R^{4a} represents a methyl group, a C2-C6 chain hydrocarbon group which may optionally have one or more substituents selected from Group A^{a}, OR^{23a}, SR^{24a}, S(O)R^{25a}, S(O)₂R^{22a}, NR^{27a}R^{28a}, a cyano group, a nitro group, a hydroxy group, or a halogen atom,
the N-oxide thereof, or the salt thereof.

9. The compound according to any one of claims 5 to 8 wherein R^{6a} represents a hydrogen atom, or the N-oxide thereof, or the salt thereof.

10. A compound represented by formula (III): 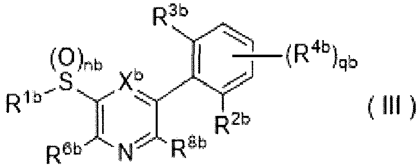 [wherein,
nb is 0, 1, or 2,
qb is 1, 2, or 3,
when qb is 2 or 3, 2 or 3 of R^{4b} may be identical to or different from each other,
R^{1b} represents a phenyl group which may optionally have one or more substituents selected from Group G^{b},
R^{8b} represents a C1-C6 chain hydrocarbon group which may be optionally substituted with one or more halogen atoms, a C3-C8 alicyclic hydrocarbon group which may optionally have one or more substituents selected from Group B^{b}, OR^{7b}, SR^{9b}, S(O)R^{10b}, S(O)₂R^{11b}, NR^{12b}R^{13b}, C(O)R^{14b}, a nitro group, a cyano group, a halogen atom, or a hydrogen atom,
R^{2b} and R^{3b} are identical to or different from each other, and each represents a C1-C6 chain hydrocarbon group which may be optionally substituted with one or more halogen atoms, a C3-C8 alicyclic hydrocarbon group which may optionally have one or more substituents selected from Group B^{b}, OR^{15b}, SR^{16b}, S(O)R^{17b}, S(O)2R^{18b}, NR^{19b}R^{20b}, C(O)R^{21b}, a nitro group, a cyano group, a halogen atom, or a hydrogen atom,
a combination of X^{b}, R^{4b} and R^{6b} represents
a combination wherein
X^{b} represents a nitrogen atom,
R^{4b} represents a methyl group which may optionally have one or more substituents selected from Group F^{b}, a CH₂F group, a CHF₂ group, a C2-C3 chain hydrocarbon group which may optionally have one or more substituents selected from Group A^{b}, a C3-C8 alicyclic hydrocarbon group, a three (3)-to eight (8)- membered non-aromatic heterocyclic group {the C3-C8 alicyclic hydrocarbon group, and the three (3)- to eight (8)- membered non-aromatic heterocyclic group may optionally have one or more substituents selected from Group B^{b}}, a phenyl group, a five (5)- to six (6)- membered aromatic heterocyclic group {the phenyl group, and the five (5)- to six (6)- membered aromatic heterocyclic group may optionally have one or more substituents selected from Group D^{b}}, OR^{23b}, SR^{24b}, S(O)R^{25b}, S(O)₂R^{22b}, NR^{27b}R^{28b}, C(O)R^{29b}, C(R^{30b})=NOR^{31b}, a cyano group, a nitro group, a hydroxy group, an amino group, or a halogen atom, and
R^{6b} represents a C1-C6 chain hydrocarbon group which may be optionally substituted with one or more halogen atoms, a C3-C8 alicyclic hydrocarbon group which may optionally have one or more substituents selected from Group B^{b}, OR^{7b}, SR^{9b}, S(O)R^{10b}, S(O)₂R^{11b}, NR^{12b}R^{13b}, C(O)R^{14b}, a nitro group, a halogen atom, or a hydrogen atom; or
a combination wherein
X^{b} represents CR^{5b},
R^{4b} represents a methyl group which may optionally have one or more substituents selected from Group F^{b}, a CH₂F group, a CHF₂ group, a C2-C3 chain hydrocarbon group which may optionally have one or more substituents selected from Group A^{b}, a C3-C8 alicyclic hydrocarbon group, a three (3)-to eight (8)- membered non-aromatic heterocyclic group {the C3-C8 alicyclic hydrocarbon group, and the three (3)- to eight (8)- membered non-aromatic heterocyclic group may optionally have one or more substituents selected from Group B^{b}}, a phenyl group, a five (5)- to six (6)- membered aromatic heterocyclic group {the phenyl group, and the five (5)- to six (6)- membered aromatic heterocyclic group may optionally have one or more substituents selected from Group D^{b}}, OR^{32b}, SR^{24b}, S(O)R^{25b}, S(O)₂R^{22b}, NR^{27b}R^{28b}, C(O)R^{29b}, C(R^{30b})=NOR^{31b}, a cyano group, a nitro group, a hydroxy group, or a halogen atom,
R^{6b} represents a C1-C6 chain hydrocarbon group which may be optionally substituted with one or more halogen atoms, a C3-C8 alicyclic hydrocarbon group which may optionally have one or more substituents selected from Group B^{b}, OR^{7b}, a nitro group, a fluorine atom, a bromine atom, an iodine atom, or a hydrogen atom,
R^{5b} represents a C1-C6 chain hydrocarbon group which may be optionally substituted with one or more halogen atoms, a C3-C8 alicyclic hydrocarbon group which may optionally have one or more substituents selected from Group B^{b}, OR^{7b}, SR^{9b}, S(O)R^{10b}, S(O)₂R^{11b}, NR^{12b}R^{13b}, C(O)R^{14b}, a nitro group, a cyano group, a halogen atom, or a hydrogen atom,
R^{7b}, R^{9b}, R^{10b}, R^{11b}, R^{15b}, R^{16b}, R^{17b}, and R^{18b} are identical to or different from each other, and each represents a C1-C6 chain hydrocarbon group which may be optionally substituted with one or more halogen atoms,
R^{12b} and R^{19b} are identical to or different from each other, and each represents a C1-C6 chain hydrocarbon group which may be optionally substituted with one or more halogen atoms, or a hydrogen atom,
R^{13b} and R^{20b} are identical to or different from each other, and each represents a C1-C6 chain hydrocarbon group which may be optionally substituted with one or more halogen atoms, a (C1-C6 alkyl)carbonyl group, a (C1-C6 alkoxy)carbonyl group {the (C1-C6 alkyl)carbonyl group, and the (C1-C6 alkoxy)carbonyl group may be optionally substituted with one or more halogen atoms}, or a hydrogen atom,
R^{14b}, R^{21b}, and R^{29b} are identical to or different from each other, and each represents a C1-C6 chain hydrocarbon group, a C1-C6 alkoxy group, a C1-C6 alkylamino group, or a C2-C8 dialkylamino group {the C1-C6 chain hydrocarbon group, the C1-C6 alkoxy group, the C1-C6 alkylamino group, and the C2-C8 dialkylamino group may be optionally substituted with one or more halogen atoms},
R^{23b} represents a C1-C6 chain hydrocarbon group which may optionally have one or more substituents selected from Group A^{b}, a C1-C6 alkylsulfonyl group, a (C1-C6 alkyl)carbonyl group, a (C1-C6 alkoxy)carbonyl group, a (C1-C6 alkylamino) carbonyl group, a (C2-C8 dialkylamino) carbonyl group {the C1-C6 alkylsulfonyl group, the (C1-C6 alkyl)carbonyl group, the (C1-C6 alkoxy)carbonyl group, the (C1-C6 alkylamino)carbonyl group, and the (C2-C8 dialkylamino) carbonyl group may be optionally substituted with one or more halogen atoms}, a C3-C8 alicyclic hydrocarbon group, a three (3)- to eight (8)- membered non-aromatic heterocyclic group {the C3-C8 alicyclic hydrocarbon group, and the three (3)- to eight (8)- membered non-aromatic heterocyclic group may optionally have one or more substituents selected from Group B^{b}}, a phenyl group, a five (5)- to six (6)- membered aromatic heterocyclic group {the phenyl group, and the five (5)- to six (6)- membered aromatic heterocyclic group may optionally have one or more substituents selected from Group D^{b}}, or a hydrogen atom,
R^{22b}, R^{24b}, R^{25b}, and R^{31b} are identical to or different from each other, and each represents a C1-C6 chain hydrocarbon group which may optionally have one or more substituents selected from Group A^{b}, a C3-C8 alicyclic hydrocarbon group, a three (3)- to eight (8)- membered non-aromatic heterocyclic group {the C3-C8 alicyclic hydrocarbon group, and the three (3)- to eight (8)- membered non-aromatic heterocyclic group may optionally have one or more substituents selected from Group B^{b}}, a phenyl group, or a five (5)- to six (6)- membered aromatic heterocyclic group {the phenyl group, and the five (5)- to six (6)- membered aromatic heterocyclic group may optionally have one or more substituents selected from Group D^{b}},
R^{27b} represents a C1-C6 chain hydrocarbon group which may optionally have one or more substituents selected from Group A^{b}, a C3-C8 alicyclic hydrocarbon group, a three (3)-to eight (8)- membered non-aromatic heterocyclic group {the C3-C8 alicyclic hydrocarbon group, and the three (3)- to eight (8)- membered non-aromatic heterocyclic group may optionally have one or more substituents selected from Group B^{b}}, a phenyl group, a five (5)- to six (6)- membered aromatic heterocyclic group {the phenyl group, and the five (5)- to six (6)- membered aromatic heterocyclic group may optionally have one or more substituents selected from Group D^{b}}, a (C1-C6 alkyl)carbonyl group, a (C1-C6 alkoxy)carbonyl group, or a C1-C6 alkylsulfonyl group {the (C1-C6 alkyl)carbonyl group, the (C1-C6 alkoxy)carbonyl group, and the C1-C6 alkylsulfonyl group may be optionally substituted with one or more halogen atoms},
R^{28b} and R^{30b} are identical to or different from each other, and each represents a C1-C6 chain hydrocarbon group which may optionally have one or more substituents selected from Group A^{b}, or a hydrogen atom,
R^{32b} represents a methyl group which has one or more substituents selected from Group A^{b}, a C2-C6 chain hydrocarbon group which may optionally have one or more substituents selected from Group A^{b}, a C1-C6 alkylsulfonyl group, a (C1-C6 alkyl)carbonyl group, a (C1-C6 alkoxy)carbonyl group, a (C1-C6 alkylamino)carbonyl group, a (C2-C8 dialkylamino)carbonyl group {the C1-C6 alkylsulfonyl group, the (C1-C6 alkyl)carbonyl group, the (C1-C6 alkoxy)carbonyl group, the (C1-C6 alkylamino) carbonyl group, and the (C2-C8 dialkylamino) carbonyl group may be optionally substituted with one or more halogen atoms}, a C3-C8 alicyclic hydrocarbon group, a three (3)- to eight (8)- membered non-aromatic heterocyclic group {the C3-C8 alicyclic hydrocarbon group, and the three (3)- to eight (8)- membered non-aromatic heterocyclic group may optionally have one or more substituents selected from Group B^{b}}, a phenyl group, a five (5)- to six (6)- membered aromatic heterocyclic group {the phenyl group, and the five (5)- to six (6)- membered aromatic heterocyclic group may optionally have one or more substituents selected from Group D^{b}}, or a hydrogen atom,
the neighboring R^{2b} and R^{4b} may combine together with a carbon atom to which they are attached to form a C5-C6 alicyclic hydrocarbon, a five (5)- to six (6)- membered non-aromatic heterocyclic ring {the C5-C6 alicyclic hydrocarbon, and the five (5)- to six (6)- membered non-aromatic heterocyclic ring may optionally have one or more substituents selected from Group B^{b}}, a benzene ring, or a five (5)- to six (6)- membered aromatic heterocyclic ring {the benzene ring, and the five (5)- to six (6)- membered aromatic heterocyclic ring may optionally have one or more substituents selected from Group E^{b}},
two of neighboring R^{4b} and R^{4b} may combine together with a carbon atom to which they are attached to form a C5-C6 alicyclic hydrocarbon, a five (5)- to six (6)- membered non-aromatic heterocyclic ring {the C5-C6 alicyclic hydrocarbon, and the five (5)- to six (6)- membered non-aromatic heterocyclic ring may optionally have one or more substituents selected from Group B^{b}}, a benzene ring, or a five (5)- to six (6)- membered aromatic heterocyclic ring {the benzene ring, and the five (5)- to six (6)- membered aromatic heterocyclic ring may optionally have one or more substituents selected from Group E^{b}}.
Group A^{b} is a group consisting of a C1-C6 alkoxy group, a C1-C6 alkylsulfanyl group, a C1-C6 alkylsulfinyl group, a C1-C6 alkylsulfonyl group {the C1-C6 alkoxy group, the C1-C6 alkylsulfanyl group, the C1-C6 alkylsulfinyl group, and the C1-C6 alkylsulfonyl group may be optionally substituted with one or more halogen atoms}, a C3-C8 alicyclic hydrocarbon group, a three (3)- to eight (8)- membered non-aromatic heterocyclic group {the C3-C8 alicyclic hydrocarbon group, and the three (3)- to eight (8)- membered non-aromatic heterocyclic group may optionally have one or more substituents selected from Group B^{b}}, a phenyl group, a five (5)- to six (6)- membered aromatic heterocyclic group {the phenyl group and the five (5)- to six (6)- membered aromatic heterocyclic group may optionally have one or more substituents selected from Group D^{b}}, a halogen atom, a cyano group, a nitro group, a hydroxy group, and a sulfanyl group.
Group B^{b} is a group consisting of an oxo group, a thioxo group, a C1-C6 chain hydrocarbon group, a C1-C6 alkoxy group, a C1-C6 alkylsulfanyl group, a C1-C6 alkylsulfinyl group, a C1-C6 alkylsulfonyl group {the C1-C6 chain hydrocarbon group, the C1-C6 alkoxy group, the C1-C6 alkylsulfanyl group, the C1-C6 alkylsulfinyl group, and the C1-C6 alkylsulfonyl group may be optionally substituted with one or more halogen atoms}, a hydroxy group, a sulfanyl group, a halogen atom, and a cyano group.
Group D^{b} is a group consisting of a C1-C6 chain hydrocarbon group, a C1-C6 alkoxy group, a C1-C6 alkylsulfanyl group, a C1-C6 alkylsulfinyl group, a C1-C6 alkylsulfonyl group, a C1-C6 alkylamino group, a C2-C8 dialkylamino group, a (C1-C6 alkyl)carbonyl group, a (C1-C6 alkoxy)carbonyl group, a (C1-C6 alkylamino)carbonyl group, a (C2-C8 dialkylamino)carbonyl group, a (C1-C6 alkyl)carbonylamino group, a (C1-C6 alkoxy)carbonylamino group {the C1-C6 chain hydrocarbon group, the C1-C6 alkoxy group, the C1-C6 alkylsulfanyl group, the C1-C6 alkylsulfinyl group, the C1-C6 alkylsulfonyl group, the C1-C6 alkylamino group, the C2-C8 dialkylamino group, the (C1-C6 alkyl)carbonyl group, the (C1-C6 alkoxy)carbonyl group, the (C1-C6 alkylamino)carbonyl group, the (C2-C8 dialkylamino)carbonyl group, the (C1-C6 alkyl)carbonylamino group, and the (C1-C6 alkoxy)carbonylamino group may be optionally substituted with one or more halogen atoms}, a C3-C8 alicyclic hydrocarbon group which may optionally have one or more substituents selected from Group B^{b}, an amino group, a nitro group, a cyano group, a hydroxy group, a sulfanyl group, and a halogen atom.
Group E^{b} is a group consisting of a C1-C6 chain hydrocarbon group, a C1-C6 alkoxy group, a C1-C6 alkylsulfanyl group, a C1-C6 alkylsulfinyl group, a C1-C6 alkylsulfonyl group {the C1-C6 chain hydrocarbon group, the C1-C6 alkoxy group, the C1-C6 alkylsulfanyl group, the C1-C6 alkylsulfinyl group, and the C1-C6 alkylsulfonyl group may be optionally substituted with one or more halogen atoms}, a hydroxy group, a sulfanyl group, a halogen atom, and a cyano group.
Group F^{b} is a group consisting of a C1-C6 alkoxy group, a C1-C6 alkylsulfanyl group, a C1-C6 alkylsulfinyl group, a C1-C6 alkylsulfonyl group {the C1-C6 alkoxy group, the C1-C6 alkylsulfanyl group, the C1-C6 alkylsulfinyl group, and the C1-C6 alkylsulfonyl group may be optionally substituted with one or more halogen atoms}, a C3-C8 alicyclic hydrocarbon group, a three (3)- to eight (8)- membered non-aromatic heterocyclic group {the C3-C8 alicyclic hydrocarbon group, and the three (3)- to eight (8)- membered non-aromatic heterocyclic group may optionally have one or more substituents selected from Group B^{b}}, a phenyl group, a five (5)- to six (6)- membered aromatic heterocyclic group {the phenyl group and the five (5)- to six (6)- membered aromatic heterocyclic group may optionally have one or more substituents selected from Group D^{b}}, a chlorine atom, a bromine atom, an iodine atom, a cyano group, a nitro group, a hydroxy group, and a sulfanyl group.
Group G^{b} is a group consisting of a C1-C6 chain hydrocarbon group, a C1-C6 alkoxy group, a C1-C6 alkylsulfanyl group, a C1-C6 alkylsulfinyl group, a C1-C6 alkylsulfonyl group, a C1-C6 alkylamino group, a C2-C8 dialkylamino group, a (C1-C6 alkyl)carbonyl group, a (C1-C6 alkoxy)carbonyl group, a (C1-C6 alkylamino)carbonyl group, a (C2-C8 dialkylamino)carbonyl group, a (C1-C6 alkyl)carbonylamino group, a (C1-C6 alkoxy)carbonylamino group {the C1-C6 chain hydrocarbon group, the C1-C6 alkoxy group, the C1-C6 alkylsulfanyl group, the C1-C6 alkylsulfinyl group, the C1-C6 alkylsulfonyl group, the C1-C6 alkylamino group, the C2-C8 dialkylamino group, the (C1-C6 alkyl)carbonyl group, the (C1-C6 alkoxy)carbonyl group, the (C1-C6 alkylamino)carbonyl group, the (C2-C8 dialkylamino)carbonyl group, the (C1-C6 alkyl)carbonylamino group, and the (C1-C6 alkoxy)carbonylamino group may be optionally substituted with one or more halogen atoms}, a C3-C8 alicyclic hydrocarbon group which may optionally have one or more substituents selected from Group B^{b}, an amino group, a cyano group, a hydroxy group, a sulfanyl group, and a halogen atom]
, or a N-oxide thereof, or a salt thereof.

11. A compound represented by formula (IV): 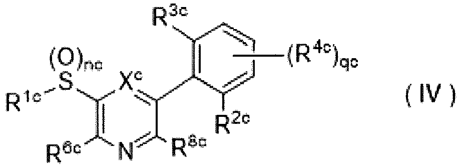 [wherein
nc is 0, 1, or 2,
qc is 1, 2, or 3,
when qc is 2 or 3, 2 or 3 of R^{4c} may be identical to or different from each other,
X^{c} is a nitrogen atom, or CR5c,
R^{1c} represents a three (3)- to eight (8)- membered non-aromatic heterocyclic group which may optionally have one or more substituents selected from Group B^{c}, or NR^{33c}R^{34c},
R^{8c} represents a C1-C6 chain hydrocarbon group which may be optionally substituted with one or more halogen atoms, a C3-C8 alicyclic hydrocarbon group which may optionally have one or more substituents selected from Group B^{c}, OR^{7c}, SR^{9c}, S(O)R^{10c}, S(O)₂R^{11c}, NR^{12c}R^{13c}, C(O)R^{14c}, a nitro group, a cyano group, a halogen atom, or a hydrogen atom,
R^{2c} and R^{3c} are identical to or different from each other, and each represents a C1-C6 chain hydrocarbon group which may be optionally substituted with one or more halogen atoms, a C3-C8 alicyclic hydrocarbon group which may optionally have one or more substituents selected from Group B^{c}, OR^{15c}, SR^{16c}, S(O)R^{17c}, S(O)₂R^{18c}, NR^{19c}R^{20c}, C(O)R^{21c}, a nitro group, a cyano group, a halogen atom, or a hydrogen atom,
R^{4c} represents a C1-C6 chain hydrocarbon group which may optionally have one or more substituents selected from Group A^{c}, a C3-C8 alicyclic hydrocarbon group, a three (3)- to eight (8)- membered non-aromatic heterocyclic group {the C3-C8 alicyclic hydrocarbon group, and the three (3)- to eight (8)- membered non-aromatic heterocyclic group may optionally have one or more substituents selected from the Group B^{c}}, a five (5)- to six (6)- membered aromatic heterocyclic group {the five (5)- to six (6)- membered aromatic heterocyclic group may optionally have one or more substituents selected from the Group D^{c}}, OR^{23c}, SR^{24c}, S(O)R^{25c}, S(O)₂R^{22c}, NR^{27c}R^{28c}, C(O)R^{29c}, C(R^{30c})=NOR^{31c}, a cyano group, a nitro group, an amino group, a hydroxy group, or a halogen atom,
R^{6c} represents a C1-C6 chain hydrocarbon group which may be optionally substituted with one or more halogen atoms, a C3-C8 alicyclic hydrocarbon group which may optionally have one or more substituents selected from Group B^{c}, OR^{7c}, SR^{9c}, S(O)R^{10c}, S(O)₂R^{11c}, NR^{12c}R^{13c}, C(O)R^{14c}, a nitro group, a cyano group, a halogen atom, or a hydrogen atom,
R^{5c} represents a C1-C6 chain hydrocarbon group which may be optionally substituted with one or more halogen atoms, a C3-C8 alicyclic hydrocarbon group which may optionally have one or more substituents selected from Group B^{c}, OR^{7c} , SR^{9c}, S(O)R^{10c}, S(O)₂R^{11c}, NR^{12c}R^{13c}, C(O)R^{14c}, a nitro group, a cyano group, a halogen atom, or a hydrogen atom,
R^{7c}, R^{9c}, R^{10c}, R^{11c}, R^{15c}, R^{16c}, R^{17c}, and R^{18c} are identical to or different from each other, and each represents a C1-C6 chain hydrocarbon group which may be optionally substituted with one or more halogen atoms,
R^{12c} and R^{19c} are identical to or different from each other, and each represents a C1-C6 chain hydrocarbon group which may be optionally substituted with one or more halogen atoms, or a hydrogen atom,
R^{13c} and R^{20c} are identical to or different from each other, and each represents a C1-C6 chain hydrocarbon group which may be optionally substituted with one or more halogen atoms, a (C1-C6 alkyl)carbonyl group, a (C1-C6 alkoxy)carbonyl group {the (C1-C6 alkyl)carbonyl group, and the (C1-C6 alkoxy)carbonyl group may be optionally substituted with one or more halogen atoms}, or a hydrogen atom,
R^{14e}, R^{21c}, and R^{29c} are identical to or different from each other, a C1-C6 chain hydrocarbon group, a C1-C6 alkoxy group, a C1-C6 alkylamino group, or a C2-C8 dialkylamino group {the C1-C6 chain hydrocarbon group, the C1-C6 alkoxy group, the C1-C6 alkylamino group, and the C2-C8 dialkylamino group may be optionally substituted with one or more halogen atoms},
R^{23c} represents a C1-C6 chain hydrocarbon group which may optionally have one or more substituents selected from Group A^{c}, a C1-C6 alkylsulfonyl group, a (C1-C6 alkyl)carbonyl group, a (C1-C6 alkoxy)carbonyl group, a (C1-C6 alkylamino)carbonyl group, a (C2-C8 dialkylamino)carbonyl group {the C1-C6 alkylsulfonyl group, the (C1-C6 alkyl)carbonyl group, the (C1-C6 alkoxy)carbonyl group, the (C1-C6 alkylamino)carbonyl group, and the (C2-C8 dialkylamino) carbonyl group may be optionally substituted with one or more halogen atoms}, a C3-C8 alicyclic hydrocarbon group, a three (3)- to eight (8)- membered non-aromatic heterocyclic group {the C3-C8 alicyclic hydrocarbon group, and the three (3)- to eight (8)- membered non-aromatic heterocyclic group may optionally have one or more substituents selected from Group B^{c}}, a phenyl group, a five (5)- to six (6)- membered aromatic heterocyclic group {the phenyl group, and the five (5)- to six (6)- membered aromatic heterocyclic group may optionally have one or more substituents selected from Group D^{c}}, or a hydrogen atom,
R^{22c}, R^{24c}, R^{25c}, and R^{31c} are identical to or different from each other, and each represents a C1-C6 chain hydrocarbon group which may optionally have one or more substituents selected from Group A^{c}, a C3-C8 alicyclic hydrocarbon group, a three (3)- to eight (8)- membered non-aromatic heterocyclic group {the C3-C8 alicyclic hydrocarbon group, and the three (3)- to eight (8)- membered non-aromatic heterocyclic group may optionally have one or more substituents selected from Group B^{c}}, a phenyl group, or a five (5)- to six (6)- membered aromatic heterocyclic group {the phenyl group, and the five (5)- to six (6)- membered aromatic heterocyclic group may optionally have one or more substituents selected from Group D^{c}},
R^{27c} represents a C1-C6 chain hydrocarbon group which may optionally have one or more substituents selected from Group A^{c}, a C3-C8 alicyclic hydrocarbon group, a three (3)-to eight (8)- membered non-aromatic heterocyclic group {the C3-C8 alicyclic hydrocarbon group, and the three (3)- to eight (8)- membered non-aromatic heterocyclic group may optionally have one or more substituents selected from Group B^{c}}, a phenyl group, a five (5)- to six (6)- membered aromatic heterocyclic group {the phenyl group, and the five (5)- to six (6)- membered aromatic heterocyclic group may optionally have one or more substituents selected from Group D^{c}}, a (C1-C6 alkyl)carbonyl group, a (C1-C6 alkoxy)carbonyl group, or a C1-C6 alkylsulfonyl group {the (C1-C6 alkyl)carbonyl group, the (C1-C6 alkoxy)carbonyl group, and the C1-C6 alkylsulfonyl group may be optionally substituted with one or more halogen atoms},
R^{28c} and R^{30c} are identical to or different from each other, and each represents a C1-C6 chain hydrocarbon group which may optionally have one or more substituents selected from Group A^{c}, or a hydrogen atom,
R^{33c} represents a C1-C6 chain hydrocarbon group which may optionally have one or more substituents selected from Group G^{c}, a C3-C6 cycloalkyl group which may be optionally substituted with one or more halogen atoms, or a hydrogen atom,
R^{34c} represents a C1-C6 chain hydrocarbon group which may optionally have one or more substituents selected from Group G^{c}, or a C3-C6 cycloalkyl group which may be optionally substituted with one or more halogen atoms,
the neighboring R^{2c} and R^{4c} may combine together with a carbon atom to which they are attached to form a C5-C6 alicyclic hydrocarbon, a five (5)- to six (6)- membered non-aromatic heterocyclic ring {the C5-C6 alicyclic hydrocarbon, and the five (5)- to six (6)- membered non-aromatic heterocyclic ring may optionally have one or more substituents selected from Group B^{c}}, a benzene ring, or a five (5)- to six (6)- membered aromatic heterocyclic ring {the benzene ring, and the five (5)- to six (6)- membered aromatic heterocyclic ring may optionally have one or more substituents selected from Group E^{c}}.
two of neighboring R^{4c} and R^{4c} may combine together with a carbon atom to which they are attached to form a C5-C6 alicyclic hydrocarbon which may optionally have one or more substituents selected from Group B^{c}, a five (5)- to six (6)-membered non-aromatic heterocyclic ring which may optionally have one or more substituents selected from Group C^{c}, a benzene ring, or a five (5)- to six (6)- membered aromatic heterocyclic ring {the benzene ring, and the five (5)- to six (6)- membered aromatic heterocyclic ring may optionally have one or more substituents selected from Group E^{c}}.
Group A^{c} is a group consisting of a C1-C6 alkoxy group, a C1-C6 alkylsulfanyl group, a C1-C6 alkylsulfinyl group, C1-C6 alkylsulfonyl group {the C1-C6 alkoxy group, the C1-C6 alkylsulfanyl group, the C1-C6 alkylsulfinyl group, and the C1-C6 alkylsulfonyl group may be optionally substituted with one or more halogen atoms}, a C3-C8 alicyclic hydrocarbon group, a three (3)- to eight (8)- membered non-aromatic heterocyclic group {the C3-C8 alicyclic hydrocarbon group, and the three (3)- to eight (8)- membered non-aromatic heterocyclic group may optionally have one or more substituents selected from Group B^{c}}, a phenyl group, a five (5)- to six (6)- membered aromatic heterocyclic group {the phenyl group and the five (5)- to six (6)- membered aromatic heterocyclic group may optionally have one or more substituents selected from Group D^{c}}, a halogen atom, a cyano group, a nitro group, a hydroxy group, and a sulfanyl group.
Group B^{c} is a group consisting of an oxo group, a thioxo group, a C1-C6 chain hydrocarbon group, a C1-C6 alkoxy group, a C1-C6 alkylsulfanyl group, a C1-C6 alkylsulfinyl group, a C1-C6 alkylsulfonyl group {the C1-C6 chain hydrocarbon group, the C1-C6 alkoxy group, the C1-C6 alkylsulfanyl group, the C1-C6 alkylsulfinyl group, and the C1-C6 alkylsulfonyl group may be optionally substituted with one or more halogen atoms}, a hydroxy group, a sulfanyl group, a halogen atom, and a cyano group.
Group C^{c} is a group consisting of a thioxo group, a C1-C6 chain hydrocarbon group, a C1-C6 alkoxy group, a C1-C6 alkylsulfanyl group, a C1-C6 alkylsulfinyl group, a C1-C6 alkylsulfonyl group {the C1-C6 chain hydrocarbon group, the C1-C6 alkoxy group, the C1-C6 alkylsulfanyl group, the C1-C6 alkylsulfinyl group, and the C1-C6 alkylsulfonyl group may be optionally substituted with one or more halogen atoms}, a hydroxy group, a sulfanyl group, a halogen atom, and a cyano group.
Group D^{c} is a group consisting of a C1-C6 chain hydrocarbon group, a C1-C6 alkoxy group, a C1-C6 alkylsulfanyl group, a C1-C6 alkylsulfinyl group, a C1-C6 alkylsulfonyl group, a C1-C6 alkylamino group, a C2-C8 dialkylamino group, a (C1-C6 alkyl)carbonyl group, a (C1-C6 alkoxy)carbonyl group, a (C1-C6 alkylamino)carbonyl group, (C2-C8 dialkylamino)carbonyl group, a (C1-C6 alkyl)carbonylamino group, a (C1-C6 alkoxy)carbonylamino group {the C1-C6 chain hydrocarbon group, the C1-C6 alkoxy group, the C1-C6 alkylsulfanyl group, the C1-C6 alkylsulfinyl group, the C1-C6 alkylsulfonyl group, the C1-C6 alkylamino group, the C2-C8 dialkylamino group, the (C1-C6 alkyl)carbonyl group, the (C1-C6 alkoxy)carbonyl group, the (C1-C6 alkylamino)carbonyl group, the (C2-C8 dialkylamino)carbonyl group, the (C1-C6 alkyl)carbonylamino group, and the (C1-C6 alkoxy)carbonylamino group may be optionally substituted with one or more halogen atoms}, a C3-C8 alicyclic hydrocarbon group which may optionally have one or more substituents selected from Group B^{c}, an amino group, a nitro group, a cyano group, a hydroxy group, a sulfanyl group, and halogen atom.
Group E^{c} is a group consisting of a C1-C6 chain hydrocarbon group, a C1-C6 alkoxy group, a C1-C6 alkylsulfanyl group, a C1-C6 alkylsulfinyl group, a C1-C6 alkylsulfonyl group {the C1-C6 chain hydrocarbon group, the C1-C6 alkoxy group, the C1-C6 alkylsulfanyl group, the C1-C6 alkylsulfinyl group, and the C1-C6 alkylsulfonyl group may be optionally substituted with one or more halogen atoms}, a hydroxy group, a sulfanyl group, a halogen atom, and a cyano group.
Group F^{c} is a group consisting of a C1-C6 alkoxy group, a C1-C6 alkylsulfanyl group, a C1-C6 alkylsulfinyl group, a C1-C6 alkylsulfonyl group {the C1-C6 alkoxy group, the C1-C6 alkylsulfanyl group, the C1-C6 alkylsulfinyl group, and the C1-C6 alkylsulfonyl group may be optionally substituted with one or more halogen atoms}, a C3-C8 alicyclic hydrocarbon group, a three (3)- to eight (8)- membered non-aromatic heterocyclic group {the C3-C8 alicyclic hydrocarbon group, and the three (3)- to eight (8)- membered non-aromatic heterocyclic group may optionally have one or more substituents selected from Group B^{c}}, a phenyl group, a five (5)- to six (6)- membered aromatic heterocyclic group {the phenyl group and the five (5)- to six (6)- membered aromatic heterocyclic group may optionally have one or more substituents selected from Group D^{c}}, a chlorine atom, a bromine atom, an iodine atom, a cyano group, a nitro group, a hydroxy group, and sulfanyl group.
Group G^{c} is a group consisting of a C3-C6 cycloalkyl group, a C1-C3 alkoxy group, a C1-C3 alkylthio group {the C3-C6 cycloalkyl group, the C1-C3 alkoxy group, and the C1-C3 alkylthio group may be optionally substituted with one or more halogen atoms}, a halogen atom, and a cyano group]
, or a N-oxide thereof, or a salt thereof.

12. An agrochemical composition comprising the compound according to any one of claims 5 to 11, the N-oxide thereof, or the salt thereof, and an inert carrier.

13. A composition comprising one or more ingredients selected from the group consisting of the following Group (a), Group (b), Group (c), and Group (d), as well as the compound according to any one of claims 5 to 11, or the N-oxide thereof, or the salt thereof:
Group (a): a group consisting of insecticidal active ingredients, miticidal active ingredients, and nematicidal active ingredients;
Group (b): fungicidal active ingredients;
Group (c): plant growth regulatory ingredients;
Group (d): repellent ingredients.

14. A method for controlling a plant disease which comprises applying an effective amount of the compound according to any one of claims 5 to 11, or the N-oxide thereof, or the salt thereof, or an effective amount of the composition according to claim 13 to a plant or soil for cultivating the plant.

15. A use of the compound according to any one of claims 5 to 11, or the N-oxide thereof, or the salt thereof, or the composition according to claim 13 for controlling a plant disease.

16. A seed or vegetative reproductive organ carrying an effective amount of the compound according to any one of claims 5 to 11, or the N-oxide, or the salt thereof, or an effective amount of the composition according to claim 13.

17. A compound represented by formula (V): 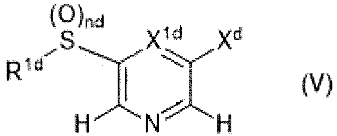 [wherein
R^{1d} represents a C2-C4 chain hydrocarbon group, a C3-C6 alicyclic hydrocarbon group, a five (5)- to six (6)- membered aromatic heterocyclic group {the five (5)- to six (6)-membered aromatic heterocyclic group may optionally have one or more substituents selected from Group V¹},
X^{d} represents a chlorine atom, or a bromine atom,
the combination of X^{d} and nd represents
a combination wherein X^{1d} represents CH, and nd is 0, 1, or 2; or
a combination wherein X^{1d} represents a nitrogen atom, and nd is 1, or 2.
Group V¹ is a group consisting of a C1-4 alkyl group, a C1-C4 alkoxy group {the C1-C4 alkyl group, and the C1-C4 alkoxy group may be optionally substituted with one or more halogen atoms}, and a halogen atom] with the proviso that the following compounds are excluded: 3-chloro-5-(ethylthio)pyridine, 3-bromo-5-(ethylthio)pyridine, 3-chloro-5-(ethylsulfonyl)pyridine, 3-bromo-5-(ethylsulfonyl)pyridine, 3-chloro-5-(propylthio)pyridine, 3-chloro-5-[(1-methylethyl)thio]pyridine, 3-bromo-5-[(1-methylethyl)thio]pyridine, , 3-chloro-5-[(1-methylethyl)sulfonyl]pyridine, 3-bromo-5-[(1-methylethyl)sulfonyl]pyridine, 3-chloro-5-[(1,1-dimethylethyl)thio]pyridine, 3-bromo-5-[(1,1-dimethylethyl)thio]pyridine, 3-chloro-5-[(1,1-dimethylethyl)sulfonyl]pyridine, 3-bromo-5-[(1,1-dimethylethyl)sulfonyl]pyridine, 3-chloro-5-(cyclohexylsulfonyl)pyridine, 3-bromo-5-(cyclopropylthio)pyridine, 3-bromo-5-(cyclopropylsulfonyl)pyridine, 5-[(5-bromo-3-pyridinyl)thio]-2-methylpyridine, 5-[(5-bromo-3-pyridinyl)sulfonyl]-2-methylpyridine, and 3,3'-sulfonylbis(5-bromopyridine).
